# EUROPÄISCHE PATENTSCHRIFT

(11) **EP 1 773 840 B1**
(45) Veröffentlichungstag und Bekanntmachung des Hinweises auf die Patenterteilung: **20.01.2010**
(21) Anmeldenummer: 05770450.4
(22) Anmeldetag: 22.07.2005
(51) Int. Cl.: C07D 495/00

(54) **SUBSTITUIERTE PYRIDO[3',2':4,5]THIENO[3,2-D]PYRIMIDINE UND PYRIDO[3',2':4,5]FURO[3,2-D]-PYRIMIDINE ZUR VERWENDUNG ALS INHIBITOREN DER PDA-4 UND/ODER TNF-ALPHA FREISETZUNG**
SUBSTITUTED PYRIDO[3',2':4,5]THIENO[3,2-D]PYRIMIDINES AND PYRIDO[3',2':4,5]FURO[3,2-D]-PYRIMIDINES FOR USE AS INHIBITORS OF PDA-4 AND/OR TNF-ALPHA RELEASE
PYRIDO[3',2':4,5]THIENO[3,2-D]PYRIMIDINES ET PYRIDO[3',2':4,5]FURO[3,2-D]-PYRIMIDINES SUBSTITUÉS COMME INHIBITEURS DE PDA-4 ET/OU LIBÉRATION DE TNF-ALPHA

(30) Priorität: 23.07.2004 EP 04017542; 02.08.2004 EP 04018272
(43) Veröffentlichungstag der Anmeldung: 18.04.2007
(73) Patentinhaber: The Medicines Company (Leipzig) GmbH, 04103 Leipzig (DE)
(72) Erfinder: REICHELT, Claudia, 04299 Leipzig (DE); LUDWIG, Alexander, 04275 Leipzig (DE); SCHULZE, Alexander, 04317 Leipzig (DE); DAGHISH, Mohammed, 04107 Leipzig (DE); LEISTNER, Siegfried, 04340 Leipzig (DE); KRÖDEL, Andreas, 04109 Leipzig (DE); HEINICKE, Jochen, 04229 Leipzig (DE)
(74) Vertreter: Schüssler, Andrea
(86) Internationale Anmeldenummer: PCT/EP2005/008030
(87) Internationale Veröffentlichungsnummer: WO 2006/010567

(56) Entgegenhaltungen:
- EP-A- 1 277 738
- EP-A- 1 323 719
- JOSÉ M. QUINTELA ET AL.: "Synthesis and Antiallergic Activity of Pyridothienopyrimidines" BIOORG. MED. CHEM, Bd. 6, 1998, Seiten 1911-1925, XP002310819
- PEINADOR C ET AL: "A Convenient Synthesis for Some Pyrido[3',2':4,5]thieno[3,2-d]pyrimi dine Derivatives with Potential Biological Activity" JOURNAL OF HETEROCYCLIC CHEMISTRY, HETEROCORPORATION. PROVO, US, Bd. 29, Dezember 1992 (1992-12), Seiten 1693-1702, XP002244954 ISSN: 0022-152X
- G. WAGNER ET AL.: "Synthese von acylierten 4-Hydrazino-pyrido[3',2':4,5]thieno[3,2-d] pyrimidinen und tetracyclischen Verbindungen gleicher tricyclischer Grundstruktur und einem am Pyrimidinring annelierten Heterocyclus" PHARMAZIE, Bd. 48, 1993, Seiten 20-23, XP002310820
- VIEWEG H. ET AL.: "Synthese von 2,3-Dihydro-imidazo[1,2-c]- und 3,4-Dihydro-2H-pyrimido[1,2-c]-annelierten Pyrido[3',2':4,5]thieno[2,3-e]pyrimidinen aus 4-(omega-Hydroxyalkylamino)-Verbindungen von Pyridothienopyrimidinen" PHARMAZIE, Bd. 47, 1992, Seiten 751-754, XP002310821
- DATABASE CHEMCATS Chemical Abstracts Service, Columbus, Ohio, US; XP002352717 & "Synthetic and Natrual Compounds Product List" 17. März 2004 (2004-03-17), PHARMEKS LTD., CHUKSIN TUPIK 4, OF. 15, MOSCOW, 125206, RUSSIA
- DATABASE CHEMCATS Chemical Abstracts Service, Columbus, Ohio, US; XP002352718 & "Interchim Intermediates" 18. Januar 2005 (2005-01-18), INTERCHIM, 211 BIS AV JF KENNEDY, BP 1140, MONTLUCON, 03103, FRANCE
- DATABASE CHEMCATS Chemical Abstracts Service, Columbus, Ohio, US; XP002352719 & "Ambinter Stock Screening Collection" 3. Juli 2005 (2005-07-03), AMBINTER, 50 AVENUE DE VERSAILLES, PARIS, F-75016, FRANCE
- PARK ET AL: 'Wortmannin, a specific inhibitor of phosphatidylinositol-3-kinase, enhances LPS-induced NO production form murine peritoneal macrophages' BIOCHEM. BIOPHYS. Bd. 240, 1997, Seiten 692 - 696
- SCHABBAUER ET AL: 'PI3K-Akt pathway suppresses coagulation and inflammation in endotoxemic mice' ARTERIOSCLER. THROMB. VASC. BIOL. Bd. 10, 2004, Seiten 1963 - 1969

## Beschreibung

### Technisches Gebiet

Die Erfindung betrifft folgende Verbindungen der allgemeinen Formeln 1a, 1b, 1c und 1 d.

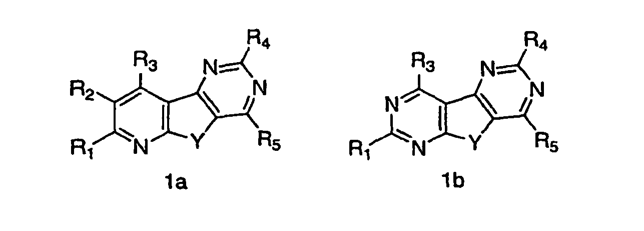

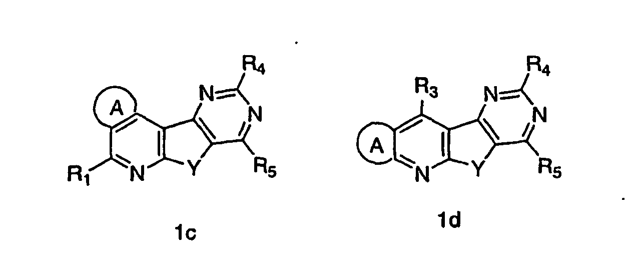

Verfahren zu deren Herstellung, pharmazeutische Zubereitungen, die diese Verbindungen und/oder daraus herstellbare physiologisch verträgliche Salze und/oder deren Solvate enthalten sowie die pharmazeutische Verwendung dieser Verbindungen, deren Salze oder Solvate als Inhibitoren der Phosphodiesterase 4. Die Verbindungen stellen Wirkstoffe zur Behandlung jener Erkrankungen dar, die durch Hemmung der Aktivität von Phosphodiesterase 4 und/oder TNFα-Freisetzung, z.B. in Lymphozyten, eosinophilen und basophilen Granulozyten, Makrophagen und Mastzellen, positiv zu beeinflussen sind.

### Stand der Technik

Die zyklischen Nukleotide cAMP und cGMP werden als intrazelluläre sekundäre Botenstoffe (second messengers) zusammengefasst und spielen bei vielfältigsten physiologischen aber auch bei pathophysiologischen Prozessen in lebenden Organismen eine zentrale Rolle. Bei hohen intrazellulären Konzentrationen dieser Nukleotide binden sich diese an spezifische Proteinkinasen, insbesondere an die Proteinkinase A und die Proteinkinase B, und aktivieren diese. Die so aktivierten Proteinkinasen sind nun in der Lage, eine Vielzahl von intrazellulären Proteinen zu phosphorylieren, die dann ihrerseits den zellulären Stoffwechsel und die Antwort dieser Zellen auf innere und äußere Signale maßgeblich beeinflussen.

In der lebenden Zelle wird die Konzentration an cAMP und cGMP hauptsächlich durch die Synthese und den enzymatischen Abbau dieser Nukleotide bestimmt. Die Synthese von cAMP und cGMP erfolgt aus ATP und GTP über die Enzyme Adenylatzyklase und Guanylatzyklase, die durch G-Protein-gekoppelte Rezeptoren in der Zellmembran aktiviert werden. Der Abbau dieser zyklischen Nukleotide erfolgt durch spezifische Phosphodiesterasen in der Zelle.

Die Phosphodiesterasen (PDEs) umfassen eine große Superenzymfamilie mit derzeit 11 bekannten Typen, die ihrerseits in zahlreiche Subtypen (Isoformen) und noch wesentlich mehr Splicevarianten unterteilt werden. Diese Isoformen unterscheiden sich in ihrer Substratspezifität, Enzymkinetik, Gewebespezifität, Sensitivität gegenüber Inhibitoren und Aktivatoren sowie in ihrer intrazellulären Kompartmentierung *(*Giernbycz MA. Phosphodiesterase 4 inhibitors and the treatment of asthma: where are we now and where do we go from here? Drugs [2000]; 59, 193-212*).*

Seit 1990 ist ein wachsendes Interesse der pharmazeutischen Industrie zu registrieren, selektive PDE Inhibitoren zu identifizieren und als Wirkstoffkandidaten zur Behandlung von z.B. chronischen Atemwegserkrankungen zu entwickeln.

Die cAMP spezifische PDE-4 stellt nach heutigem Wissensstand das wichtigste Isoenzym von immunkompetenten Zellen und von glatten Muskelzellen des Lungengewebes dar. Die PDE-4-Familie umfasst derzeit vier Gene mit den Genprodukten PDE-4A, PDE-4B, PDE-4C und PDE-4D. Erstmals wurden PDE-4 spezifische Isotypen von Nemoz et al. beschrieben (Nemoz G, Prigent AF, Moueqqit M, Fougier S, Macovschi O, Pacheco H. Selective inhibition of one of the cyclic AMP phosphodiesterases from rat brain by the neurotropic compound rolipram. Biochem Pharmacol [1985]; 34, 2997-3000). Alle Subtypen setzen sich aus einer konservierten katalytischen Domäne mit einer Länge von ca. 270 Aminosäuren und zwei in der Länge variierenden zusätzlichen Regionen (UCR1 und UCR2; upstream conserved region) innerhalb des N-Terminus des Moleküls zusammen (Engels P, Fichtel K, Lubbert H. Expression and regulation of human and rat phosphodiesterase IV. FEBS Letters [1994]; 350, 291-295). Eine weitere Diversivität in der Expression der PDE-4 Isoformen resultiert durch alternative Splicevarianten und unterschiedliches posttranslationales Prozessing (Beavo JA, Conti M, Heaslip RJ. Multiple cyclic nucleotide phosphodiesterases. Mol Pharmakol [1994]; 46, 399-405*).* Diese vier Subtypen werden in unterschiedlichen Geweben und Zellen differentiell exprimiert. Die PDE-4A bis -4D sind in den meisten immunkompetenten Zellen und in Entzündungszellen vorhanden. Mit Ausnahme der PDE-4B sind die anderen PDE-4 Typen auch in den Epithelzellen der Luftwege nachweisbar. Im menschlichen Gehirn ist gleichfalls eine differentielle Expression der vier Subtypen in Abhängigkeit von bestimmten anatomischen Regionen beschrieben.

In den vergangenen Jahren haben zahlreiche präklinische und klinische Studien die Modulation von Entzündungs- und Immunkompetenten Zellen durch selektive PDE-4 Inhibitoren belegen können und führten damit zu der wissenschaftlich abgesicherten Auffassung über das hohe therapeutische Potential von spezifischen PDE-4 Inhibitoren bei ganz unterschiedlichen entzündlichen Erkrankungen, wie z.B. chronisch obstruktiven Lungenerkrankungen (COPD), Asthma bronchiale, atopischer Dermatitis, Rheumatoider Arthritis (RA), Multiple Sklerose (MS) und zahlreichen neurologischen Erkrankungen (Doherty AM. Phosphodiesterase 4 inhibitors as novel anti-inflammatory agents. Curr Opin Chem Biol [1999]; 3, 466-473*;* Essayan DM. Cyclic nucleotide phosphodiesterase (PDE) inhibitors and immunomodulation. Biochem Pharmacol [1999]; 57, 565-573*;* Dal Piaz V, Giovannoni MP. Phosphodiesterase 4 inhibitors, structurally unrelated to Rolipram, as promising agents for treatment of asthma and other pathologies. Eur J Med Chem [2000]; 35, 463-480).

Eine zentrale Eigenschaft von selektiven PDE-4 Inhibitoren ist die Hemmung der Freisetzung des wichtigen pro-inflammatorischen Zytokins TNFα aus Entzündungszellen (Makrophagen, Mastzellen, T-Lymphozyten, basophilen und eosinophilen Granulozyten) aber auch aus Fibroblasten, Endothelzellen und Astrozyten. TNFα wiederum ist selbst in der Lage, weitere pro-inflammatorische Zytokine wie den granulocyte-macrophage colony-stimulating factor (GM-CSF) und Interleukine, besonders IL-1 und IL-8, zu stimulieren. Desweiteren aktiviert TNFα neutrophile und eosinophile Granulozyten, Fibroblasten und Endothelzellen, die ihrerseits pro-inflammatorische Zytokine und Proteasen, hauptsächlich MMP's, sezernieren. So stellen auch alle Krankheiten, bei denen TNFα eine zentrale Rolle im Erkrankungsprozess spielt, ein Target für PDE-4 Inhibitoren dar.

In eosinophilen Granulozyten konnten sowohl PDE-3 als auch PDE-4A, B, D, jedoch keine PDE-4C Subtypen nachgewiesen werden. Diese Zellen spielen eine bedeutende Rolle in der Pathophysiologie des Asthmas und sind deshalb auch hinsichtlich der PDE-4 Hemmung intensiv untersucht worden. Die selektive PDE-4 Hemmung bewirkt eine Verminderung der Freisetzung von reaktiven Sauerstoffradikalen und von Leukotrien C₄ (Sturton RG, Butt NM, Palfai SP, Tudhope SR, Abram TS, Fisher R, Braunlich G, Es-Sayed M. Am J Respir Crit Care Med [2000]; 161, A200*).* Die Synthese des eosinophil-derived neurotoxin (EDN), der Komplementkomponente C5ₐ und des platelet activating factor (PAF) wird gleichfalls durch selektive Inhibition der PDE-4 vermindert (Hatzelmann A, Tenor H, Schudt C. Differential effects of non-selective and selective phosphodiesterase inhibitors on human eosinophil functions. Br J Pharmacol. [1995];114, 821-831*).* PDE-4 Inhibitoren blockieren, untersucht in verschiedensten Tiermodellen für Asthma, signifikant das Einwandern von eosinophilen Granulozyten, hemmen die Akkumulation der Zytokine IL-4 und IL-5 in der broncho-alveolären Lavage (BAL) und verbessern die Funktion der Luftwege (Kanehiro A, Ikemura T, Makela MJ, Lahn M, Joetham A, Dakhama A, Gelfand EW. Inhibition of phosphodiesterase 4 attenuates airway hyperresponsiveness and airway inflammation in a model of secondary allergen challenge. Am J Respir Crit Care Med. [2001];163, 173-184).

Neutrophile Granulozyten spielen eine Schlüsselrolle bei entzündlichen Erkrankungen. Sie akkumulieren sehr schnell im entzündlichen Gewebe und sezernieren eine Vielzahl biologisch aktiver Mediatoren und Enzyme.

In diesen Zellen wird als cAMP-abbauendes Enzym hauptsächlich die PDE-4 nachgewiesen (Wang P, Wu P, Ohleth KM, Egan RW, Billah MM. Phosphodiesterase 482 is the predominant phosphodiesterase species and undergoes differential regulation of gene expression in human monocytes and neutrophils. Mol Pharmacol. [1999]; 56,170-174). PDE-4 Inhibitoren suprimieren eine Vielzahl von Abwehrfunktionen dieser Zellen, wie Superoxid-Bildung, Degranulierung, IL-8-Freisetzung, Adhesionsmolekül-Expression, eine stark verminderte Expression der humanen Lungen-Elastase, der MMP-9 und der Leukotrien B₄ Synthese (Barnette MS, Christensen SB, Essayan DM, Grous M, Prabhakar U, Rush JA, Kagey-Sobotka A, Torphy TJ. SB 207499 (Ariflo), a potent and selective second-generation phosphodiesterase 4 inhibitor: in vitro anti-inflammatory actions. J Pharmacol Exp Ther. [1998]; 284, 420-426*;* Berends C, Dijkhuizen B, de Monchy JG, Dubois AE, Gerritsen J, Kauffman HF. Inhibition of PAF-induced expression of CD11b and shedding of L-selectin on human neutrophils and eosinophils by the type IV selective PDE inhibitor, rolipram. Eur Respir J. [1997]; 10, 1000-1007). Eine pathophysiologische Hauptkonsequenz dieser Zellfunktionsänderung durch PDE-4 Inhibitoren ist in deren stark verminderter Adhäsion und Chemotaxis in Richtung des Entzündungsherdes zu sehen, es kommt somit nicht zum Einwandern der Entzündungszellen.

PDE-4 Inhibitoren blockieren in vivo in zahlreichen COPD Tiermodellen die Neutrophilie in der BAL und damit einen wesentlichen pathologischen Entzündungsweg (Spond J, Chapman R, Fine J, Jones H, Kreutner W, Kung TT, Minnicozzi M. Comparison of PDE 4 inhibitors, rolipram and SB 207499 (ariflo), in a rat model of pulmonary neutrophilia. Pulm Pharmacol Ther [2001]; 14, 157-164).

Das gleichfalls verstärktes Einwandern und die Akkumulation von Monozyten in das entzündliche Lungengewebe ist ein typischer pathophysiologischer Regulationsmechanismus bei diesen Erkrankungen. Als Leitphänomen kann hierbei die Freisetzung von pro-inflammatorischen Zytokinen, besonders von TNFα und GM-CSF und zahlreicher proteolytischer Enzyme angesehen werden. Selektive PDE-4 Inhibitoren blockieren die Synthese und Freisetzung von TNFα, GM-CSF und Proteasen und stimulieren die PGE₂ Produktion. Überraschenderweise wird die Synthese des anti-inflammatorische Zytokin IL-10 durch selektive PDE-4 Inhibitoren stark stimuliert (Suda Y, Tamura G, Ohno 1, Maeda K, Liu Y, Yamauchi K, Kurimoto F, Shirato K. Effects of phosphodiesterase inhibitors on secretions of human monokines. Allergol Int [1998]; 47, 219-224).

Die im entzündlichen Lungengewebe akkumulierten T-Lymphozyten sezernieren hauptsächlich pro-inflammatorische Zytokine wie IL-2, IL-4, IL-5, IL-13, TNFα, IFNγ und GM-CSF, deren Synthese durch PDE-4 Inhibitoren sehr effektiv gehemmt wird.

Die besonders bei Asthma eingewanderten B-Lymphozyten werden durch spezifische PDE-4 Inhibitoren in ihrer IgE Synthese gehemmt und damit direkt Einfluss auf den IgE-vermittelten allergischen Pathogeneseweg (Coqueret O, Boichot E, Lagente V. Selective type IV phosphodiesterase inhibitors prevent IL-4-induced IgE production by human peripheral blood mononuclear cells. Clin Exp Allergy [1997]; 27, 816-823*).*

Die Epithelzellen der Luftwege sind gleichfalls stark in die entzündlichen Reaktionen bei COPD und Asthma eingebunden. Bei ihrer Aktivierung setzen sie eine Vielzahl von biologisch hochaktiven Substanzen, wie z.B. Metabolite der Arachidonsäure und proinflammatorische Zytokine, wie das TNFα und GM-CSF, frei (Chipappara G, Merendino AM, Chimenti L, Rilcobono L, Mirabella F, La Rocca AM, Weck PK, Bonsignore G, Vignola AM. In vitro and ex vivo effects of the phosphodiesterase 4 inhibitors. Am J Resp Crit Cara Med [2001]; 163, A278*).* In vivo zeigen PDE-4 Inhibitoren durch eine signifikante Hemmung der Synthese und Freisetzung des pro-inflammatorischen Enzyms MMP-9, einen starken protektiven Effekt auf Epithelzellen der Luftwege, sowohl in Tiermodellen als auch in klinischen Studien (Rabinovici R, Feuerstein G, Abdullah F, Whiteford M, Borboroglu P, Sheikh E, Phillip DR, Ovadia P, Bobroski L, Bagasra O, Neville LF. Locally produced tumor necrosis factor-alpha mediates interleukin-2-induced lung injury. Circ Res (1996); 78, 329-236*;* Ortiz JL, Cortijo J, Valles JM, Bou J, Morcillo EJ. Rolipram inhibits airway microvascular leakage induced by platelet-activating factor, histamine and bradykinin in guineapigs. J Pharm Pharmacol (1993); 45, 1090-1092).
Ein erhöhter intrazellulärer cAMP Spiegel senkt die Aktivität von Immun- und Entzündungszellen sowohl in vitro als auch in vivo, wirkt protektiv auf Epithel- und Endothelzellen und vermittelt eine Relaxation der glatten Muskelzellen im Lungengewebe. Diese pharmakologischen Eigenschaften belegen nachhaltig die Bedeutung von PDE Hemmung als relevantes und abgesichertes Target für die therapeutische Intervention bei chronischen entzündlichen Lungenerkrankungen wie COPD und Asthma (Barnes PJ, Shapiro SD, Pauwels RA. Chronic obstructive pulmonary disease: molecular and cellular mechanisms. Eur Respir J (2003); 22, 672-88*).*

Unter COPD wird eine Gruppe von chronisch progressiven, entzündlichen Lungenerkrankungen zusammengefasst, die hauptsächlich durch ein Einwandern von neutrophilen Granulozyten und weiterer immunkompetenter Zellen in das Lungengewebe charakterisiert ist. Hauptsymptome sind hierbei chronischer Husten und Auswurf und die damit verbundene progrediente und irreversible Verschlechterung der Lungenfunktion bis hin zur Maximalvariante, dem Lungenemphysem. Die Erkrankung verläuft schubförmig und ist häufig mit bakteriellen Sekundärinfektionen der Lunge verbunden. Wichtigste Risikofaktoren sind das Rauchen und die Umweltverschmutzung. Durch die teilweise extreme Kurzatmigkeit und die geringe Belastbarkeit der Patienten auf Grund eines pulmonalen Rückstaus und der daraus resultierenden kardialen Probleme ist die Lebensqualität und Lebenserwartung der Patienten stark eingeschränkt. Weltweit leiden ca. 600 Mill. an dieser Erkrankung und ist laut WHO sechsthäufigste Erkrankung weltweit, die auch die vierthäufigste Todesursache ist. Mit einer jährlichen Wachstumsrate von 12% (Visiongain, 2004), wird sie global in den nächsten 20 Jahren die dritthäufigste Todesursache sein. Analysten schätzen deshalb ein, dass es sich bei dieser Erkrankungsgruppe um den mit am schnellsten wachsenden globalen therapeutischen Markt handelt. Die gegenwärtige Standardtherapie setzt sich aus der Gabe von β₂-Agonisten, muscarinergen Antagonisten, Kortikosteroiden und Antihistaminika zusammen. Diese Behandlungsstrategien sind jedoch nur symptomatischer Natur, ohne kausal in den progressiven Verlauf der Erkrankung einzugreifen. Es besteht deshalb seit Jahren die berechtigte Forderung mittels neuer Therapieansätze kausal in den Krankheitsprozess einzugreifen und damit insbesondere den fortschreitenden Charakter von COPD zu bekämpfen.

Beim Asthma bronchiale handelt es sich um eine anfallsweise, nichtinfektiöse und meist allergisch ausgelöste Lungenerkrankung, die hauptsächlich durch ein Einwandern von eosinophilen Granulozyten und weiteren immunkompetenten Zellen in das Lungengewebe und durch eine temporäre Bronchokonstriktion charakterisiert ist. Nach längerer Krankheitsdauer tritt vielfach eine chronische Bronchitis mit einem Lungenemphysem und Bronchiektasien bis hin zum Cor pulmonale auf. Die Lebensqualität der Patienten ist durch die Atemnot bis hin zu Dyspnoe Attacken, die erhebliche Schleimsekretion und starke Angstgefühle merklich eingeschränkt. In den Industrieländem leiden ca. 5% der erwachsenen Bevölkerung und nahezu 10% der Kinder an dieser Erkrankung. Weltweit geht man von einer Prävalenz von 155 Mill. Erkrankten bei einer jährlichen Steigerungsrate von 10 - 15 % (aus Lead Discovery, 2003). Die seit 25 Jahren übliche Standardtherapie mit β₂-Adeno-Rezeptor-Agonisten (Bronchodilatation) und Kortikosteroide (antientzündlich) zeigt neben einem guten therapeutischen Profil auch teilweise erhebliche unerwünschte Nebenwirkungen, besonders im Kindesalter (bekannte Glukokortikoid-Nebenwirkungen und Tachykardie, Herzklopfen, Kopfschmerz bei β₂- Adenorezeptor Agonisten). Es besteht auch bei diesem Krankheitsbild die Forderung nach neuen wirksameren Therapiestrategien, die nicht nur symptomatisch, sondern kausal in den Krankheitsprozess eingreifen.

Selektive PDE-4 Inhibitoren sollten daher zusammengefasst zumindest folgende invivo Wirkungen haben:
1. effektive Hemmung der TNFα-Synthese und -Freisetzung in Blutzellen;
2. signifikante Verminderung der TNFα Konzentration in der Broncho-alveolären Lavageflüssigkeit (BAL);
3. signifikante Verminderung von IL-4 und IL-5 in der BAL Flüssigkeit;
4. signifikante Hemmung des Einwandems von eosinophilen Granulozyten in die Lunge;
5. signifikante Hemmung des Einwanderns von neutrophilen Granulozyten in die Lunge;
6. Verhinderung des "oxidativen Burst";
7. Verhinderung der Bronchokonstriktion;
8. Verhinderung der Ausbildung von Lungenödemen (Emphysem);
9. Verhinderung der Proliferation und Hyperplasie von Zellen der Luftwege;
10. signifikante Hemmung der MMP-9 Aktivität in der BAL Flüssigkeit;
11. signifikante Hemmung der TGF-β Aktivität in der BAL Flüssigkeit.

In neuerer Zeit belegen experimentelle Daten auch den erfolgversprechenden Einsatz von PDE-4 Inhibitoren als immunmodulatorische Wirkstoffe. Das therapeutische Potential von PDE-4 Inhibitoren wurde erfolgreich in Tiermodellen für die atopische Dermatitis (Hanifin JM, Chan SC, Cheng JB, Tofte SJ, Henderson WR Jr, Kirby DS, Weiner ES. Type 4 phosphodiesterase inhibitors have clinical and in vitro anti-inflammatory effects in atopic dermatitis. J Invest Dermatol (1996);107, 51-56), die Rheumatoide Arthritis (Laemont KD, Schaefer CJ, Juneau PL, Schrier DJ. Effects of the phosphodiesterase inhibitor rolipram on streptococcal cell wall-induced arthritis in rats. Int J Immunopharmacol (1999); 21, 711-725), Colitis ulcerosa *(*Hartmann G, Bidlingmaier C, Siegmund B, Albrich S, Schulze J, Tschoep K, Eigler A, Lehr HA, Endres S. Specific type IV phosphodiesterase inhibitor rolipram mitigates experimental colitis in mice. J Pharmacol Exp Ther (2000); 292, 22-30*),* Multiple Sklerose (Dinter H, Onuffer J, Faulds D, Perez HD. Phosphodiesterase type IV inhibitors in the treatment of multiple sclerosis. J Mol Med (1997); 75, 95-102), Crohn's Disease (Prehn JL, Landers C, Muller GW, Man HW, Stirling DI, Targan SR. Potent inhibition of cytokine production from intestinal lamina propria T cells by phosphodiesterase-4 inhibitory thalidomide analogues. J Clin Immunol (2001); 21, 357-364), Entzündungsschmerz (Cunha FQ, Teixeira MM, Ferreira SH. Pharmacological modulation of secondary mediator systems--cyclic AMP and cyclic GMP--on inflammatory hyperalgesia. Br J Pharmacol (1999);127, 671-678), Septischer Schock (Cardelus I, Gras J, Jauregui J, Lienas J, Palacios JM. Inhibition of lipopolysaccharide-induced bowel erythrocyte extravasation in rats, and of mesenteric hypoperfusion in dogs, by phosphodiesterase inhibitors. Eur J Pharmacol (1996); 299, 153-159), Leishmaniose (Rascon A. Cyclic nucleotide phosphodiesterases: diversity, classification, structure and function. Acta Cient Venez (1997); 48, 145-153) und HIV Infektionen (Sun Y, Li L, Lau F, Beavo JA, Clark EA. Infection of CD4+ memory T cells by HIV-1 requires expression of phosphodiesterase 4. J Immunol (2000); 165, 1755-1761) nachgewiesen.
Klinisch zeigten die meisten der bisher getesteten PDE-4 Inhibitoren, mit Rolipram als Leitstruktur, unerwünschte Arzneimittelwirkungen (UAW) und fehlende in vivo Wirksamkeit, die ihren klinischen Einsatz bisher limitierten. Dazu zählen emetische Wirkungen wie Übelkeit und Erbrechen, aber auch gastro-intestinale Beschwerden, kardiovaskuläre und zentrale Nebenwirkungen (Zeller E, Stief HJ, Pflug B, Sastre-y-Hemandez M. Results of a phase II study of the antidepressant effect of rolipram. Pharmacopsychiatry (1984); 17, 188-190*;* Puurunen J, Lucke C, Schwabe U. Effect of the phosphodiesterase inhibitor 4-(3-cyclopentyloxy-4-methoxyphenyl)-2-pyrrolidone (ZK 62711) on gastric secretion and gastric mucosal cyclic AMP. Naunyn Schmiedebergs Arch Pharmacol (1978); 304, 69-75*;* Nicholson CD. Cyclic nucleotide phosphodiesterase isoenzymes and asthma--outstanding issues. Agents Actions Suppl (1993); 43, 3-12).

Ausgehend von Rolipram wurden in der Folgezeit zahlreiche strukturbasierte Abwandlungen entwickelt, die aber alle ein ähnliches Nebenwirkungsprofil aufweisen *(*Schneider HH, Schmiechen R, Brezinski M, Seidler J. Stereospecific binding of the antidepressant rolipram to brain protein structures. Eur J Pharmacol (1986); 127, 105-115*).* Die heute am weitesten entwickelten PDE-4 Inhibitoren sind Cilomilast (Fa. Glaxo-SmithKline) (Compton CH, Gubb J, Nieman R, Edelson J, Amit O, Bakst A, Ayres JG, Creemers JP, Schultze-Werninghaus G, Brambilla C, Barnes NC; International Study Group. Cilomilast, a selective phosphodiesterase-4 inhibitor for treatment of patients with chronic obstructive pulmonary disease: a randomised, dose-ranging study. Lancet (2001); 358, 265-270*;* Compton C, Edelson JD, Cedar E et al. Cilomilast (Ariflo) 15mg bid safey in a six month clinical trial program. Am J Resp Crit Care Med (2001); 163, A909*;* Zussman BD, Benincosa LJ, Webber DM, Clark DJ, Cowley H, Kelly J, Murdoch RD, Upward J, Wyld P, Port A, Fuder H. An overview of the pharmacokinetics of cilomilast (Ariflo), a new, orally active phosphodiesterase 4 inhibitor, in healthy young and elderly volunteers. J Clin Pharmacol (2001); 41, 950-958*;* Giembycz MA. Cilomilast: a second generation phosphodiesterase 4 inhibitor for asthma and chronic obstructive pulmonary disease. Expert Opin Investig Drugs (2001); 10, 1361-1379*)* und Roflumilast (Fa. Altana) *(*Schmidt BM, Kusma M, Feuring M, Timmer WE, Neuhauser M, Bethke T, Stuck BA, Hormann K, Wehling M. The phosphodiesterase 4 inhibitor roflumilast is effective in the treatment of allergic rhinitis. J Allergy Clin Immunol (2001); 108, 530-536*; SCRIP 2644, p.25*), die strukturell große Ähnlichkeiten zu Rolipram aufweisen, aber offensichtlich ein besseres Wirkprofil besitzen. Roflumilast ist im Vergleich zu Cilomilast mit einem IC₅₀ Wert von 0.8 nM ein wesentlich potenterer PDE-4 Inhibitor und zeigt auch in zahlreichen in vitro und in vivo Untersuchungen eine stärkere anti-inflammatorische Aktivität (Hatzelmann A, Schudt C. Anti-inflammatory and immunomodulatory potential of the novel PDE4 inhibitor roflumilast in vitro. J Pharmacol Exp Ther (2001); 297, 267-79). Beide Wirkstoffe haben trotz eines Nebenwirkungsprofils erfolgreich die klinische Studie Phase III für COPD und Asthma abgeschlossen, sind jedoch noch nicht von den zuständigen Behörden zugelassen worden.

Die bisher entwickelten PDE-4 Inhibitoren, die zum Teil ein umfangreiches Nebenwirkungsprofil aufweisen, lassen sich hauptsächlich in folgende chemische Hauptklassen untergliedern:
1. Catecholetherverbindungen mit struktureller Ähnlichkeit zu Rolipram, Cilomilast und Roflumilast,
2. Chinazolindione mit struktureller Ähnlichkeit zu Nitraquazone,
3. Xanthin-Derivate mit struktureller Ähnlichkeit zu Theophyllin und
4. Benzofuran-Derivate.

Es besteht deshalb der dringende Bedarf, neuartige nebenwirkungsarme PDE-4 Inhibitoren mit besserer therapeutischer Breite zu entwickeln und in die Klinik einzuführen.

Die Aufgabe der vorliegenden Erfindung ist daher neue Verbindungen bereitzustellen, die selektiv die PDE-4 hemmen.

Gelöst wird diese Aufgabe durch die nachfolgend gezeigten Verbindungen der allgemeinen Formeln 1a, 1b, 1c und 1d.

In bisher nur wenigen Fällen ist über biologisch aktive Derivate der allgemeinen Formel 1b berichtet worden:

So wurden von G. Wagner et al.: Pharmazie (1993), 48, 667-669, die Synthese von Thienodipyrimidinen beschrieben, welche antianaphylaktische Eigenschaften besitzen. Weiterhin wurden die von D. Briel Pharmazie (1998), 53, 227-231 dargetellten Thienodipyrimidine auf Antiulcer Aktivität getestet.

Über biologisch aktive Derivate der allgemeinen Formeln 1c und 1d wurde bislang ebenfalls wenig berichtet:

So wurden von *S. Leistner et al.: Ger. (East) (1988),* DD 258013, die Synthese von 8,9,10,11-Tetrahydropyrimido[4',5':4,5]thieno[2,3-c]isochinolin-4-onen beschrieben, welche antianaphylaktische und antiinflammatorische Eigenschaften besitzen. Die Synthese von 1,2-Dihydro-pyrano[4',3':4,5]pyrido[2,3-b]thieno[3,2-d]pyrimidin-8-onen, welche antibakterielle Eigenschaften zeigen, wurde *von Paronikyan et al.: U.S.S.R. (1988),* SU 85-3914497 beschrieben. Desweiteren wurden am Pyridin-Teil [c]-anellierte Pyrido[3',2':4,5]thieno[3,2-d]pyridin-2-carbonsäuren von *H. Vieweg et al.: Ger. (East) (1988),* DD 258018*,* und von S. *Leistner et al.: Ger. (East) (1988),* DD 258015*,* DD 258017 u. DD 258019*,* hinsichtlich ihrer potentiellen Anwendbarkeit als Arzneistoffe erwähnt.

In bisher nur wenigen Fällen ist auch über biologisch aktive Derivate der allgemeinen Formel 1a berichtet worden:

So wurden von J.M. Quintela et al.: Bioorg. Med. 6 (1998) 1911-1925, die Synthese von mehreren 2-Dimethylamino-(oder 2-H)-4-sek.amino-7-ethoxy-8-cyano-(oder 8-H)-9-phenyl- und auch das strukturanaloge 4-Ethoxy-pyrido[3',2':4,5]thieno-[3,2-d]pyrimidin-Derivat beschrieben. Einige dieser Verbindungen zeigten eine die Histamin-Freisetzung aus Mastzellen von Ratten inhibierende Wirkung. EP-A-1 323 719 beschreibt Thienopyrimidin-Verbindungen, die einen inhibierenden Effekt auf die cGMP-spezifische Phosphodiesterase haben. EP-A-1 277 738 beschreibt pharmazeutische Zusammensetzungen, die als Phosphatidylinositol3-kinase-Inhibitoren eingesetzt werden können und kondensierte Heteroarylderivate darstellen. Abdel-Rahmann et al.: Pharmazie 58 (2003) 372-377 berichteten über die Synthese von 8-Acetyl-3-amino-7-methyl-4-imino-9-subst.phenyl-pyrido[3',2':4,5]thieno-[3,2-d]-pyrimidinen mit antimikrobieller Wirksamkeit. Weiterhin wurden Derivate dieses Heterosystems u.a mit cholesterinsenkender (*C.J.Shishoo, M.B.Devani und V.S.Bhadti: Indian Patent*151.456 *(*1983*);* Chem. Abstr.: 100, 209858 (1984*);* und V.P.Arya; Drugs Future, 10, 123 (1985*)), mit analgetischer (*C.G.Dave et al.: J. Indian Chem.Soc. 66, 48 (1989*))*, mit antipyretischer *(*E.Bousquet et al.: Farmaco Ed.Sci. 40, 869(1985*); und* E. Bousquet et al.: Farmaco Ed. Sci. 39. 110 (1984*)),* mit antianaphylaktischer *(*H. Vieweg, S.Leistner, G. Wagner et al.: Patent DD 257830 *(*1988*);* Chem. Abstr.: 110, 95262p (1989*); und H.Vieweg, S.Leistner, G. Wagner et al.: East German Patent* DD 258234 *(*1988*)),* mit antiinflammatorischer (E.F.Elslager, P.W.Jacob und M.Leslic: J.Heterocyclic Chem. 9, 775 (1972*);* und M.Chaykovsky et al.: J.Med.Chem. 10, 188 (1973*);* und L.A.Radinovskaya und A.Sharanin: Khim.Geterotsikl.Soedin. 805 (1988*);* und S.Leistner et al.: Pharmazie 41, 54 (1986*));* mit klinisch effektiver antiallergischer (G.D.Madding und M.D.Thompson: J.Heterocyclic Chem. 24, 581 (1987*))* und mit potentiell antineoplastischer Wirksamkeit *(*C.C.Cheng in Progress in Medicinal Chemistry 25, 35 (1989*) beschrieben.*

Als Inhibitoren der PDE 4 und der TNFα-Freisetzung sind die hier beschrieben Derivate der allgemeinen Formeln 1a, 1b, 1c und 1d bisher jedoch völlig unbekannt.

### Detailierte Beschreibung der Erfindung

Die Erfindung betrifft Derivate der allgemeinen Formeln 1a, 1b, 1c und 1d. 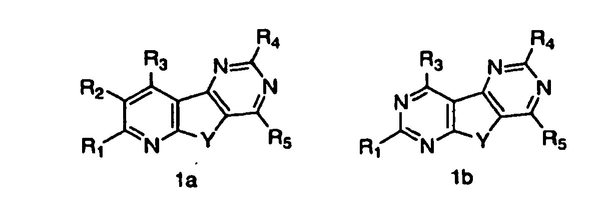 worin bedeuten:
Y S, O oder N-H
R¹
- Wasserstoff,
- C₁₋₁₀Alkyl, -C₂₋₁₂ Alkenyl, -C₂₋₁₂ Alkinyl, jeweils geradkettig, verzweigt oder cyclisch sowie ggf. ein-, zwei- oder dreifach mit unabhängig voneinander ausgewählten Resten R^{§} substituiert,
- Aralkyl- mit C₆₋₁₂Aryl und C₁₋₅ Alkyl, ggf. ein-, zwei- oder dreifach mit unabhängig voneinander ausgewählten Resten R^{§} substituiert,
- Phenyl, ggf. ein-, zwei- oder dreifach mit unabhängig voneinander ausgewählten Resten R^{§} substituiert,
- 1-Naphthyl, 2-Naphthyl
- Pyridyl-N-oxid, (ggf. mit R^{§} substituiert),
- mono- oder bicyclische gesättigte oder ein- oder mehrfach ungesättigte Heterocyclen mit 5 - 14 Ringatomen darunter 1 - 4 Heteroatome, die vorzugsweise N, O und S sind, ggf. ein-, zwei- oder dreifach mit unabhängig voneinander ausgewählten Resten R^{§} substituiert,
- C₁₋₁₀ Alkoxy, geradkettig, verzweigt oder cyclisch sowie ggf. ein-, zwei- oder dreifach mit unabhängig voneinander ausgewählten Resten R^{§} substituiert,
- Aralkyloxy- mit C₆₋₁₂Aryl und C₁₋₅ Alkyl, ggf. ein-, zwei- oder dreifach mit unabhängig voneinander ausgewählten Resten R^{§} substituiert,
- C₂₋₁₂Alkylacyl (ggf. mit R^{§} substituiert),
- Benzoyl, 1- und 2-Naphthoyl (jeweils ggf. mit R^{§} substituiert),
- Hydroxy, Sulfhydryl, Formyl, Carboxyl, CONH₂, Cyano, Rhodano, Nitro, SO₃H
- Alkylthio, Alkylsulfinyl, SO₂OAlk und Alkylsulfonyl, jeweils geradkettig, verzweigt oder cyclisch C₁₋₆ sowie ggf. ein-, zwei- oder dreifach mit unabhängig voneinander ausgewählten Resten R^{§} substituiert,
- Arylthio, Arylsulfinyl, Arylsulfonyl, Heteroarylthio Heteroarylsulfinyl und Heteroarylsulfonyl, jeweils ggf. ein-, zwei- oder dreifach mit unabhängig voneinander ausgewählten Resten R^{§} substituiert,
- , Alkoxycarbonyl, CONHAlk und CONAlk₂ ,jeweils geradkettig, verzweigt oder cyclisch C₁₋₆, sowie ggf. ein-, zwei- oder dreifach mit unabhängig voneinander ausgewählten Resten R^{§} substituiert,
- Aryloxycarbonyl, Arylcarbamoyl, Arylamido, N-Aryl,N-alkylamido, N-Aryl,N-alkylcarbamoyl, Aralkyloxycarbonyl und Aralkylcarbamoyl, mit Alkyl C₁₋₅, Aryl C₆₋₁₀ und ggf. ein-, zwei- oder dreifach mit unabhängig voneinander ausgewählten Resten R^{§} substituiert,
- Heterocyclylcarbonyl, Heterocyclylcarbamoyl, Heterocyclylamido, N-Heterocyclyl,N-alkylcarbamoyl, N-Heterocyclyl,N-alkylamido, Heterocyclylalkyloxycarbonyl und Heterocyclylkylcarbamoyl, mit einem mono- oder bicyclischen gesättigten oder ein- oder mehrfach ungesättigten Heterocyclus mit 5 - 14 Ringatomen darunter 1 - 4 Heteroatomen, die vorzugsweise N, O und S sind, ggf. ein-, zwei- oder dreifach mit unabhängig voneinander ausgewählten Resten R^{§} substituiert und Alkyl C₁₋₅,
- Chlor, Brom, Iod, Fluor,
- Amino, C₁₋₆Alkylamino, Di(C₁₋₅)alkylamino, jeweils geradkettig, verzweigt oder cyclisch sowie ggf. ein-, zwei- oder dreifach mit unabhängig voneinander ausgewählten Resten R^{§} substituiert,
- Arylamino C₆₋₁₀, ggf. ein-, zwei- oder dreifach mit unabhängig voneinander ausgewählten Resten R^{§} substituiert,
- Heterocyclylamino, mono- oder bicyclisch, gesättigt oder ein- oder mehrfach ungesättigt, mit 5 - 14 Ringatomen darunter 1 - 4 Heteroatome, die vorzugsweise N, O und S sind, ggf. ein-, zwei- oder dreifach mit unabhängig voneinander ausgewählten Resten R^{§} substituiert ,
- Arylhydrazino, ggf. ein-, zwei- oder dreifach mit unabhängig voneinander ausgewählten Resten R^{§} substituiert,
- L_{A}-A-L_{B}-B
worin bedeuten:
L_{A}:
- Einfachbindung,
- NR^{#}, O S, SO, SO₂

A:
- Phenyl, ggf. ein-, zwei- oder dreifach mit unabhängig voneinander ausgewählten Resten R^{§} substituiert
- Naphthyl, Naphtyl ein- oder mehrfach mit R^{§} substituiert
- mono- oder bicyclische gesättigte oder ein- oder mehrfach ungesättigte Heterocyclen mit 4 - 14 Ringatomen darunter 1 - 5 Heteroatome, die vorzugsweise N, O und S sind, die ein oder mehrere Sauerstoff-Atome an C, N und/oder S tragen können und ggf. ein-, zwei- oder dreifach mit unabhängig voneinander ausgewählten Resten R^{§} substituiert sind

-L_{A}-A kann ferner auch eine direkte Bindung Tricyclus- L_{B} bedeuten, wobei in diesem Fall L_{B} keine Einfachbindung ist
L_{B}:
- Einfachbindung
- NR^{#}, O, S, SO, SO₂, -CHR^{§}-, -CH₂-O-, -O-CH₂,
- folgende funktionelle Gruppen:

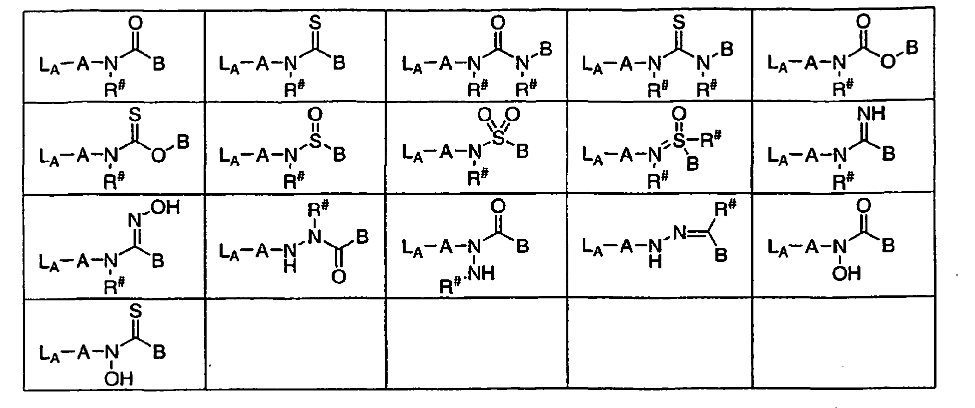

B:
- Wasserstoff
- Alkyl, ggf. ein-, zwei- oder dreifach unabhängig voneinander mit R^{§} substituiert
   - -CONH₂, -CONHAlk (Alk ggf. mit R^{§} substituiert), -CONHAryl (Aryl ggf. mit R^{§} substituiert), -CONHHetaryl (Hetaryl ggf. mit R^{§} substituiert), -COOH, -COOAlk (Alk ggf. mit R^{§} substituiert),
   - -COAlk (Alk ggf. mit R^{§} substituiert), -COAryl (Aryl ggf. mit R^{§} substituiert), -COHetaryl (Hetaryl ggf. mit R^{§} substituiert)
   - -CH₂-CONH₂ , -CH₂-CONHAlk (Alk. ggf. mit R^{§} substituiert), -CH₂-CONHAryl (Aryl ggf. mit R substituiert), -CH₂-CONHHetaryl ((Hetaryl ggf. mit R^{§} substituiert), -CH₂-COOH, -CH₂-COOAlk (Alk. ggf. mit R^{$} substituiert)
   - -CH₂-COAlk (Alk ggf. mit R^{§} substituiert),
      -CH₂-COAryl (Aryl ggf. mit R^{§} substituiert), -CH₂-COHetaryl (Hetaryl ggf. mit R^{§} substituiert)
- Phenyl, ggf. ein-, zwei- oder dreifach mit unabhängig voneinander ausgewählten Resten R^{§} substituiert,
- Naphthyl, ggf. ein-, zwei- oder dreifach mit unabhängig voneinander ausgewählten Resten R^{§} substituiert,
- mono- oder bicyclische gesättigte oder ein- oder mehrfach ungesättigte Heterocyclen mit 4 - 14 Ringatomen darunter 1 - 5 Heteroatome, die vorzugsweise N, O und S sind, die ein oder mehrere Sauerstoff-Atome an C, N und/oder S tragen können und ggf. ein-, zwei- oder dreifach mit unabhängig voneinander ausgewählten Resten R^{§} substituiert sein können,

R^{#}
- Wasserstoff, Alkyl, ggf. mit R^{§} substituiert

R²
- Wasserstoff,
- C₁₋₁₂ Alkyl,
- C₂₋₁₂ Alkenyl und C₂₋₁₂ Alkinyl,
- Benzyl, Phenyl(C₂₋₆)alkyl (ggf. mit R^{§} ein oder mehrmals, gleich oder ungleich am aromatischen und/oder aliphatischen Molekülteil substituiert);
- Phenacyl (ggf. mit R^{§} ein oder mehrmals, gleich oder ungleich am aromatischen Molekülteil substituiert);
- Carboxyl, C₁₋₄Alkoxycarbonyl, -CONH₂, -CONHAlk und CONAlk₂ (mit "Alk" jeweils C₁₋₆),
- R^{#}C(O)- (worin R^{#} dieselbe Bedeutung hat wie oben),
- Cyano, Nitro, Amino, C₁₋₆Alkylamino, Di(C₁₋₆ )alkylamino, -N=N-C₆H₅, -N=N-C₆H₄-R^{§},
- 1,3-Diphenyl-pyrazol-4-yl, Thiazolin-2-yl, Imidazolin-2-yl und 3,4,5,6-Tetrahydro-pyrimidinyl;

R³
- Wasserstoff,
- C₁₋₁₀Alkyl, -C₂₋₁₂ Alkenyl, -C₂₋₁₂ Alkinyl, jeweils geradkettig, verzweigt oder cyclisch sowie ggf. ein-, zwei- oder dreifach mit unabhängig voneinander ausgewählten Resten R^{§} substituiert,
- Aralkyl- mit C₆₋₁₂Aryl und C₁₋₅ Alkyl, ggf. ein-, zwei- oder dreifach mit unabhängig voneinander ausgewählten Resten R^{§} substituiert,
- Phenyl, ggf. ein-, zwei- oder dreifach mit unabhängig voneinander ausgewählten Resten R^{§} substituiert,
- 1-Naphthyl, 2-Naphthyl
- Pyridyl-N-oxid, (ggf. mit R^{§} substituiert),
- C₁₋₁₀ Alkoxy, geradkettig, verzweigt oder cyclisch sowie ggf. ein-, zwei- oder dreifach mit unabhängig voneinander ausgewählten Resten R^{§} substituiert,
- Aralkyloxy- mit C₆₋₁₂Aryl und C₁₋₅ Alkyl, ggf. ein-, zwei- oder dreifach mit unabhängig voneinander ausgewählten Resten R^{§} substituiert,
- C₂₋₁₂Alkylacyl (ggf. mit R^{§} substituiert),
- Benzoyl, 1- und 2-Naphthoyl (jeweils ggf. mit R^{§} substituiert),
- Hydroxy, Sulfhydryl, Formyl, Carboxyl, CONH₂, Cyano, Rhodano, Nitro, SO₃H
- Alkylthio, Alkylsulfinyl, SO₂OAlk und Alkylsulfonyl, jeweils geradkettig, verzweigt oder cyclisch C₁₋₆ sowie ggf. ein-, zwei- oder dreifach mit unabhängig voneinander ausgewählten Resten R^{§} substituiert,
- Arylthio, Arylsulfinyl, Arylsulfonyl, Heteroarylthio Heteroarylsulfinyl und Heteroarylsulfonyl, jeweils ggf. ein-, zwei- oder dreifach mit unabhängig voneinander ausgewählten Resten R^{§} substituiert,
- , Alkoxycarbonyl, CONHAlk und CONAlk₂ ,jeweils geradkettig, verzweigt oder cyclisch C₁₋₆, sowie ggf. ein-, zwei- oder dreifach mit unabhängig voneinander ausgewählten Resten R^{§} substituiert,
- Aryloxycarbonyl, Arylcarbamoyl, Arylamido, N-Aryl,N-alkylamido, N-Aryl,N-alkylcarbamoyl, Aralkyloxycarbonyl und Aralkylcarbamoyl, mit Alkyl C₁₋₅, Aryl C₆₋₁₀ und ggf. ein-, zwei- oder dreifach mit unabhängig voneinander ausgewählten Resten R^{§} substituiert,
- Chlor, Brom, Iod, Fluor,
- Amino, C₁₋₆Alkylamino, Di(C₁₋₅)alkylamino, jeweils geradkettig, verzweigt oder cyclisch sowie ggf. ein-, zwei- oder dreifach mit unabhängig voneinander ausgewählten Resten R^{§} substituiert,
- Arylamino C₆₋₁₀, ggf. ein-, zwei- oder dreifach mit unabhängig voneinander ausgewählten Resten R^{§} substituiert,
- Arylhydrazino, ggf. ein-, zwei- oder dreifach mit unabhängig voneinander ausgewählten Resten R^{§} substituiert,

R⁴
- C₂₋₁₄Alkyl, C₃₋₁₄Cycloalkyl, C₂₋₁₄Alkenyl, C₃₋₁₄Cycloalkenyl, C₂₋₁₄Alkinyl (jeweils ggf. am C-Skelett der vorgenannten aliphatischen Reste mit R§ substituiert);
- Phenyl, 4-R^{§} -Phenyl, 3-R^{§} -Phenyl, 2-R^{§}-Phenyl (jedoch ausgenommen: 2-NO₂-Phenyl), 3-R^{§},4-R^{§}-Phenyl, 3-R^{§},4-R^{§}-,5-R^{§}-Phenyl,
- 1-Naphthyl, 2-Naphthyl (jeweils ggf. mit R^{§} substituiert);
- S-Alkyl, SO-Alkyl, SO₂-Alkyl, (jeweils C₁-C₈),
   S-Alkenyl, SO-Alkenyl, SO₂-Alkenyl (jeweils C₂-C₈)
   S-Alkinyl, SO-Alkinyl, SO₂-Alkinyl (jeweils C₂-C₆)
   (ggf. jeweils am C-Skelett der vorgenannten aliphatischen Reste mit -OH, -CN, -SCN, -NO₂, Phenyl oder C₃ - C₇Cycloalkyl substituiert);
- mono-, bi- oder tricyclische gesättigter oder ein- oder mehrfach ungesättigter heterocyclischer Rest mit insgesamt 4-14 Ring-Atomen, davon 1-5 Heteroatomen, die vorzugsweise N, O, S und Se sind (jeweils ggf. mit R^{§} substituiert;
- OR⁶, worin R⁶ bedeutet
   - CH₃, C₂H₅, (CH₂)₂CH₃, (CH₂)₃CH₃, C(CH₃)₃, CH₂CH₂OH, CH₂CH₂SH, CH₂CH₂SCH₃, CH₂CF₃, CH₂CCl₃, CH₂CHF₂, CH₂CHCl₂, CH₂CH₂F, CH₂CH₂Cl, CH₂CH₂Br,
   - C₃-₇Cycloalkyl [z.B.Cylopropyl, Cylopropylmethyl, Cylobutyl, Cyclobutylmethyl, Cyclopentyl, Cyclopentylmethyl, Cylohexyl, Cyclohexylmethyl] (ggf. am C- Skelett mit R^{§} substituiert),
   - C₂-₅Alkenyl und C₂-₅Alkinyl,
   - C₃-₇ Cycloalkenyl,
   - Aryl und Heteroaryl als Reste mono-, bi- oder tricyclischer Aromaten oder Heteroaromaten mit ggf. 6-14 Ring-Atomen [z.B. Phenyl, 4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl],
   - 1-Naphthyl, 2-Naphthyl (ggf. jeweils mit R^{§} substituiert),
   - Pyridyl, Isochinolinyl, Chinolinyl, Acridinyl;
- NR⁷R⁸, worin dieser Substituent insgesamt bedeutet:
   - Morpholino, Thiomorpholino, Thiomorpholino-S,S-Dioxid, Pyrrolidino, Piperidino, 1-Piperazino, 1-Homopiperazino, 4-C₁₋₆-Alkyl-1-piperazino, 4-(2-Hydroxyethyl)-1-piperazino, 4-Benzyl-1-piperazino, 4-Aryl-1-piperazino (ggf. mit R^{§} am heterocycloaliphatischen Ring substituiert),
   - weitere Amino-Reste sekundärer, mono- oder polycyclischer cycloaliphatischer Amine mit insgesamt 5-14 Ringatomen, einschließlich der am C-Skelett mit R^{§} substituierte Vertreter;
   - Amino-Reste sekundärer aliphatischer und aromatischer Amine, R⁷R⁸NH,
   worin R⁷ und R⁸ unabhängig voneinander gleich oder ungleich sein können und bedeuten:
   - C₁-₆Alkyl, Benzyl, Phenyl, 1- und 2-Naphthyl, 2-, 3- und 4-Pyridyl, Chinolinyl, Isochinolinyl, 2-Thienyl, 2-Furyl (ggf. jeweils mit R^{§} substituiert);
   - Aminoreste primärer Amine, R⁷NH₂, ( worin R⁷ dasselbe wie oben bedeutet),
   - Amino, NH₂, mit der Einschränkung, dass dann R⁵ nur die Bedeutung von OR⁶ hat;

R⁵
- OR⁶, wobei R⁶ dasselbe wie oben bedeutet,
- NR⁷R⁸, mit gleicher Bedeutung wie oben, jedoch mit der Einschränkung, dass R⁴ dabei nicht ebenfalls die Bedeutung von NR⁷R⁸ aufweist, sowie ausgenommen Morpholino, wenn R⁴ = Aryl,
- Azido, Hydroxylamino, O-(C₁-₃ )Alkylhydroxylamino,
- N-(C₁-₃ )Alkylhydroxylamino, N,N-Di(C₁-₃ )alkylhydroxylamino,
- Hydrazino, (C₁-₄)Alkyl- und Di(C₁-₄)alkylhydrazino,
- Benzylhydrazino, Acylhydrazino, N,N-Diacylhydrazino,
- Carbamoylamino,
- 1-Imidazolyl, 1,2,4-Triazol-1yl, 1-Pyridinium,
- 1-Pyrazinium, 1-Pyridazinium, einschliesslich der alkylsubstituierten Vertreter dieser Azaheterocyclen;

R^{§}
- OH, -SH, -O-C₁₋₈Alkyl, -O-C₆₋₁₄Aryl, -S-C₁₋₄Alkyl, -S-C₆₋₁₄Aryl,
- SO-C₁₋₄Alkyl, -SO-C₆₋₁₄Aryl, -SO₂-C₁₋₄Alkyl, -SO₂-C₆₋₁₄Aryl, -SO₃H,
- OSO₂C₁₋₈Alkyl, -OSO₂C₆₋₁₄Aryl, -COOH, -COOC₁₋₈Alkyl,
- (CO)C₁₋₈Alkyl,
- COOH, -CONH₂, -CONHC₁₋₆Alkyl, -CON(C₁₋₆Alkyl)₂,
- NH₂, -NHC₁₋₆Alkyl, -N(C₁₋₆Alkyl)₂, -NHC₆₋₁₄Aryl, -NH-Hetaryl,
- N(C₆₋₁₄Aryl)₂, -N(C₁₋₆Alkyl)(C₆₋₁₄Aryl),
- C₁₋₆Alkyl, -C₂₋₁₂ Alkenyl, -C₂₋₁₂ Alkinyl, jeweils geradkettig, verzweigt oder cyclisch sowie ggf. ein-, zwei- oder dreifach unabhängig voneinander mit Halogen substituiert,
- Halogen (F, -Cl, -Br, -I),
- CH₂CH₂OH, -CH₂CH₂SH, -CH₂CH₂SCH₃,,
- Sulfamoyl, Alkylsulfamoyl, Dialkylsulfamoyl mit Alkyl C₁₋₅ ggf. substituiert mit Methoxy,
- Amidino, Hydroxyamidino
- Sulfo, Phosphono,
- -CN, -NO₂, und -SCN

Ausgenommen vom Schutz sind die folgenden Verbindungen der Formel 1(a):
I) Y = S, R¹ = OC₂H₅ , R² = CN, R³ = C₆H₅ , R⁴ = N(CH₃)₂, R⁵ = OC₂H₅
II) Y = S, R¹ = CH₃ , R² = H, R³ = CH₃ , R⁴ = Phenyl, R⁵ = Hydrazino
III) Y = S, R¹ = CH₃ , R² = H, R³ = CH₃ , R⁴ = Phenyl, R⁵ = NH-(CH₂)₂-OH
IV) Y = S, R¹ = Phenyl, R² = H, R³ = Phenyl, R⁴ = Thiopheno, R⁵ = NH-(CH₂)₂-OH
V) Y = S, R¹ = Phenyl, R² = H, R³ = Phenyl, R⁴ = Phenyl dreifach substituiert mit Methoxy, R⁵ = NH-(CH₂)₂-OH

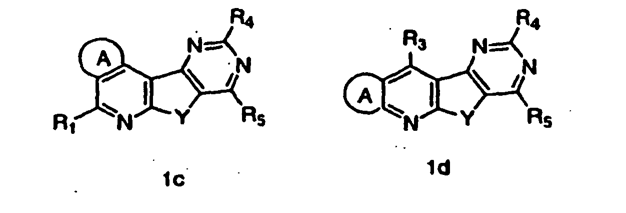

- Y: S, N-H oder O
- A: - anellierter Carbocyclus mit 5 bis 8 Ringatomen, einfach oder mehrfach ungesättigt sowie aromatisch, (ggf. mit R* substituiert und/oder ggf. mit R^{§} substituiert);
- anellierter Bicyclus mit 6 bis 11 Ringatomen, einfach oder mehrfach ungesättigt, welcher auch Stickstoff, Sauerstoff und/oder Schwefel enthalten kann (ggf. mit R* substituiert und/oder ggf. mit R^{§} substituiert);
- annelierter Heterocyclus mit 5 bis 8 Ringatomen einfach oder mehrfach ungesättigt sowie aromatisch, welcher Stickstoff, Sauerstoff und Schwefel einfach, mehrfach, gleich oder ungleich unabhängig voneinander im Cyclus enthalten kann (ggf. mit R* substituiert und/oder ggf. mit R^{§} substituiert),
- R¹ und R³: - Wasserstoff (außer wenn A ein anellierter unsubstituierter Cyclopenten-Ring kombiniert mit R^{4,} R⁵gleich -OH ist)
- C₁₋₁₀Alkyl (ggf. mit R^{§} substituiert),
- C₂₋₁₂ Alkenyl und C₂₋₁₂ Alkinyl (jeweils ggf. mit R^{§} substituiert ),
- Difluormethyl, Trifluormethyl,
- Benzyl, Phenyl-(C₂₋₆)alkyl (jeweils ggf. mit R^{§} substituiert), Phenyl (ggf. mit R^{§} substituiert), dabei inbesondere: 4-R^{§}-Phenyl, 3-R^{§}-Phenyl, 2-R^{§}-Phenyl, 3-R^{§},4-R^{§}-Phenyl, 3-R^{§},4-R^{§}-,5-R^{§}-Phenyl, 2-R^{§}, 4-R^{§}-Phenyl,
- 1-Naphthyl, 2-Naphthyl (jeweils ggf. mit R^{§} substituiert),
- C₃₋₁₄Cycloalkyl, C₃₋₁₄Cycloalkenyl (jeweils ggf. mit R^{§}-substituiert),
- mono- oder bicyclische gesättigte oder ein- oder mehrfach ungesättigte Heterocyclen mit 4 - 14 Ringatomen darunter 1 - 5 Heteroatome, die vorzugsweise N, O und S sind, die ein oder mehrere Sauerstoff-Atome an C, N und/oder S tragen können und ggf. ein-, zwei- oder dreifach mit unabhängig voneinander ausgewählten Resten R^{§} substituiert sein können,
- C₂₋₁₂Alkylacyl (ggf. mit R^{§} substituiert),
- Benzoyl, 1- und 2-Naphthoyl (jeweils ggf. mit R^{§} substituiert),
- Heterocyclylacyl [z.B. Nicotinoyl, Isonicotinoyl, 2-Picolinoyl, 2-Thienoyl, 2-Furoyl] (ggf. mit R^{§} substituiert)
- Hydroxy,
- Sulfhydryl,
- C₁₋₁₀ Alkoxy,
- Alkylthio, Alkylsulfinyl und Alkylsulfonyl (jeweils C₁₋₆)
- Formyl, Carboxyl, C₁₋₄Alkoxycarbonyl;
- CONH₂, CONHAlk und CONAlk₂ (mit "Alk" jeweils C₁₋₆),
- Cyano, Rhodano, Nitro, SO₃H, SO₂OAlk (mit "Alk": C₁₋₅),
- Chlor, Brom, Iod, Fluor,
- Amino, C₁₋₆Alkylamino, Di(C₁₋₅)alkylamino (jeweils ggf. mit R^{§} am Alkylrest substituiert),
- Morpholino, Thiomorpholino, Thiomorpholino-S-Oxid, Thiomorpholino-S,S-Dioxid, Pyrrolidino,
- Piperidino, 1-Piperazino, 4-Methyl-1-piperazino, 4-Hydroxyethyl-1-piperazino, 4-Phenyl-1-piperazino,
- Cycloalkylamino, C₃₋₁₄ Arylamino und Heteroarylamino [z.B. Phenyl-, 1- und 2-Naphthyl-, 2-, 3- oder 4-Pyridyl-, Chinolinyl-, Isochinolinyl-, Acridinyl-, Phenothiazinyl-, 2-Thienyl- und 2-Furylamino] (ggf. jeweils an den carbo- bzw. heterocyclischen Ringen mit R^{§} substituiert);
- R⁴: - C₂₋₁₄Alkyl, C₃₋₁₄Cycloalkyl, C₂₋₁₄Alkenyl, C₃₋₁₄Cycloalkenyl, C₂₋₁₄Alkinyl (jeweils ggf. am C-Skelett der vorgenannten Reste mit R^{§} substituiert);
- Benzyl, Phenyl-(C₂₋₆)alkyl (jeweils ggf. mit R^{§} substituiert), Phenyl (ggf. mit R^{§} substituiert), dabei inbesondere: 4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl, 2-R^{§}, 4-R^{§}-Phenyl,
- 1-Naphthyl, 2-Naphthyl (jeweils ggf. mit R^{§} substituiert);
- S-Alkyl (C₃-C₈), SO-Alkyl, SO₂-Alkyl, (jeweils C₁-C₈),
   S-Alkenyl, SO-Alkenyl, SO₂-Alkenyl (jeweils C₂-C₈)
   S-Alkinyl, SO-Alkinyl, SO₂-Alkinyl (jeweils C₂-C₆)
   (ggf. jeweils am C-Skelett der vorgenannten Reste mit -OH, -CN, -SCN, -NO₂, Phenyl oder C₃ - C₇Cycloalkyl substituiert);
- mono-, bi- oder tricyclischer gesättigter oder ein- oder mehrfach ungesättigter heterocyclischer Rest mit insgesamt 4-14 Ring-Atomen, davon 1-5 Heteroatomen, die vorzugsweise N, O, S und Se sind (jeweils ggf. mit R^{§} substituiert);
- OR⁶, worin R⁶ bedeutet
   - Wasserstoff
   - CH₃, C₂H₅, (CH₂)₂CH₃, (CH₂)₃CH₃, CH(CH₃)₂, CH₂CH(CH₃)₂, C(CH₃)₃, CH₂CH₂OH, CH₂CH₂SH, CH₂CH₂SCH₃, CH₂CF₃, CH₂CCl₃, CH₂CHF₂, CH₂CHCl₂, CH₂CH₂F, CH₂CH₂Cl, CH₂CH₂Br,
   - C₃-₇Cycloalkyl [z.B.Cylopropyl, Cylopropylmethyl, Cylobutyl, Cyclobutylmethyl, Cyclopentyl, Cyclopentylmethyl, Cylohexyl, Cyclohexylmethyl] (ggf. am C- Skelett mit R^{§} substituiert),
   - C₂-₅Alkenyl und C₂-₅Alkinyl,
   - C₃-₇ Cycloalkenyl,
   - Aryl und Heteroaryl als Reste mono-, bi- oder tricyclischer Aromaten oder Heteroaromaten mit ggf. 6-14 Ring-Atomen [z.B. Phenyl, 4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl],
   - 1-Naphthyl, 2-Naphthyl (ggf. jeweils mit R^{§} substituiert),
   - Pyridyl, Isochinolinyl, Chinolinyl, Acridinyl;
- NR⁷R⁸, worin dieser Substituent insgesamt bedeutet:
   - Morpholino, Thiomorpholino, Thiomorpholino-S-Oxid, Thiomorpholino-S,S-Dioxid, Pyrrolidino, Piperidino, 1-Piperazino, 1-Homopiperazino, 4-C₁₋₆₋Alkyl-1-piperazino, 4-(2-Hydroxyethyl)-1-piperazino, 4-Benzyl-1-piperazino, 4-Aryl-1-piperazino (jeweils ggf. mit R^{§} am heterocycloaliphatischen Ring substituiert),
   - weitere Amino-Reste sekundärer, mono- oder polycyclischer cycloaliphatischer Amine mit insgesamt 5-14 Ringatomen, einschließlich der am C-Skelett mit R^{§} substituierte Vertreter;
   - Amino-Reste sekundärer aliphatischer und aromatischer Amine, R⁷R⁸H,
   worin R⁷und R⁸unabhängig voneinander gleich oder ungleich sein können und bedeuten:
   - C₁-₆Alkyl, Benzyl, Phenyl, 1- und 2-Naphthyl, 2-, 3- und 4-Pyridyl, Chinolinyl, Isochinolinyl, 2-Thienyl, 2-Furyl (ggf. jeweils mit R^{§} substituiert);
   - Aminoreste primärer Amine, R⁷NH₂, (worin R⁷ dasselbe wie oben bedeutet),
   - Amino, NH₂, mit der Einschränkung, dass dann R⁵ nur die Bedeutung von -OR⁶hat;
- R⁵: - Wasserstoff,
- Heterocyclyl, fünf- bis siebengliedrig, ggf. substituiert durch R³,
- Phenyl, ggf. substituiert durch R³,
- OR⁶, wobei R⁶ dasselbe wie oben bedeutet,
- NR⁷ R⁸, mit gleicher Bedeutung wie oben
- Azido, Hydroxylamino, O-(C₁-₃)Alkylhydroxylamino,
- N-(C₁-₃)Alkylhydroxylamino, N,N-Di(C₁-₃)alkylhydroxylamino,
- Hydrazino, (C₁-₄)Alkyl- und Di(C₁-₄)alkylhydrazino,
- Benzylhydrazino, Acylhydrazino, N,N-Diacylhydrazino,
- Carbamoylamino,
- 1-Imidazolyl, 1,2,4-Triazol-1yl, 1-Pyridinium,
- 1-Pyrazinium, 1-Pyridazinium, einschliesslich der alkylsubstituierten Vertreter dieser Azaheterocyclen;
- R*: =O, =S, =N-H, =N-C₁₋₈Alkyl, =N-Aryl, =N-OH, =N-O-C₁₋₈Alkyl, =N-O-C₆₋₁₄ Aryl
- R^{§}: wie oben (bei Formel 1a bzw. 1 b) definiert
wobei folgende Verbindungen der Formel 1 (c) vom Schutz ausgenommen sind:
1,4-Dihydro-2,2-dimethyl-5-(4-morpholinyl)-10-propylthio-2H-pyrano[4",3":4',5']pyrido[3',2':4',5']thieno[3,2-d]pyrimidin-8(9H)-on,
10-Buthylthio-1,4-dihydro-2,2-dimethyl-5-(4-morpholinyl)-2H-pyrano[4",3":4',5']pyrido[3',2':4',5']thieno[3,2-d]pyrimidin-8(9H)-on
und 2-Methyl-5-thiophen-2-yl-1,2,3,4-tetrahydro-11H-7-thia-2,6,9,11-tetraaza-benzo[c]fluorene-8,10-dione
sowie pharmazeutisch verträgliche Salze, Solvate, aktive Metabolite, Tautomere und Prodrugs dieser Verbindungen,

Der oben erwähnte Ausdruck "ggf. mit R^{§} substituiert" bedeutet, daß die genannten Reste einfach, zweifach oder mehrfach, gleich oder ungleich unabhängig voneinander, substituiert sein können, wobei R^{§} folgende Bedeutung hat:
- OH, -SH, -O-C₁₋₈Alkyl, -O-C₆₋₁₄ Aryl, -S-C₁₋₄ Alkyl, -S-C₆₋₁₄Aryl,
- SO-C₁₋₄Alkyl, -SO-C₆₋₁₄Aryl, -SO₂-C₁₋₄Alkyl, -SO₂-C₆₋₁₄Aryl, -SO₃H,
- OS0₂C₁₋₈Alkyl, -OSO₂C₆₋₁₄Aryl, -COOH, -COOC₁₋₈Alkyl, -(CO)C₁₋₈Alkyl,
- COOH, -COOC_{1...8}Alkyl, -CONH₂, -CONHC₁₋₆Alkyl, -CON(C₁₋₆Alkyl)₂,
- NH₂, -NHC₁₋₆Alkyl, -N(C₁₋₆Alkyl)₂, -NHC₆₋₁₄Aryl, -NH-Hetaryl,
- N(C₆₋₁₄Aryl)₂, -N(C₁₋₆Alkyl)(C₆₋₁₄Aryl).
- CH₃, -CHF₂, -CF₃, -C₂H₅, -C(CH₃)₂, -(CH₂)₂CH₃, -(CH₂)₃CH₃, -CH₂CH₂OH,
- CH₂CH₂SH, -CH₂CH₂SCH₃, -CH₂CF₃, -CH₂CCl₃, -CH₂CHF₂, -CH₂CHCl₂,
- CH₂CH₂F, -CH₂CH₂Cl, -CH₂CH₂Br, -Cylopropyl, -Cylopropylmethyl,
- Cylobutyl, -Cyclobutylmethyl, -Cyclopentyl, -Cyclopentylmethyl,
- Cylohexyl, -Cyclohexylmethyl,
- F, -Cl, -Br, -I, -CN, -NO₂, und -SCN.

Der oben erwähnte Ausdruck "ggf. mit R* substituiert" bedeutet, daß die genannten Reste einfach oder mehrfach, gleich oder ungleich unabhängig voneinander, substituiert sein können, wobei R^{*} folgende Bedeutung hat:
=O, =S, =N-H, =N-C₁₋₈Alkyl, =N-Aryl, =N-OH, =N-O-C₁₋₈Alkyl, =N-O-C₆₋₁₄ Aryl

Die Begriffe "Alkyl, Alkenyl, Alkinyl, Alkoxy, usw.", auch in Wortzusammensetzungen wie Alkylsulfonyl, Alkylamino oder Alkoxycarbonyl usw. bedeuten sowohl die unverzweigten wie auch die verzweigten möglichen Verbindungen. Ebenso bedeuten "Alkenyl und Alkinyl" die entsprechend möglichen einfach oder mehrfach ungesättigten Verbindungen. Das gleiche gilt auch für die entsprechenden cyclischen Verbindungen.

Von den Verbindungen der allgemeinen Formel 1a sind als besondere Ausführungsform der Erfindung die Verbindungen der allgemeinen Formeln 1a (I) und 1a (II) hervorzuheben, 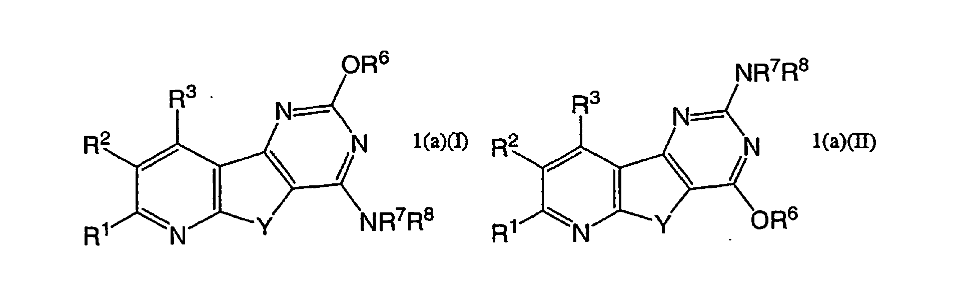 worin R¹, R², R³, R⁶, R⁷, R⁸ und Y die bezüglich Formel 1 (a) oben genannten Bedeutungen besitzen.

Von den Verbindungen der allgemeinen Formel 1c sind als besondere Ausführungsform der Erfindung die Verbindungen der allgemeinen Formeln 1 c (I) und 1 c (II) hervorzuheben, 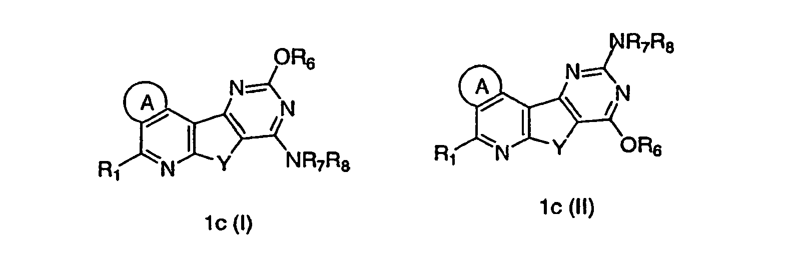 worin R¹, R⁶, R⁷ , R⁸ sowie A und Y die bezüglich Formel 1c oben genannten Bedeutungen besitzen.

Im Sinne der Erfindung gelten alle Reste als miteinander kombinierbar, soweit bei der Definition der Reste nichts anderes angegeben ist. Es sollen alle denkbaren Untergruppierungen daraus als offenbart gelten.

Die Erfindung betrifft auch physiologisch verträgliche Salze der Verbindungen der allgemeinen Formeln 1 a, 1b, 1 c und 1 d.

Die physiologisch verträglichen Salze werden auf übliche Weise durch Umsetzung basischer Verbindungen der allgemeinen Formeln 1a, 1b, 1 c und 1d mit anorganischen oder organischen Säuren, ggf. auch bei Vorliegen von Verbindungen mit aciden Eigenschaften, wenn z.B. einer der Substituenten R₁, R₂, R₃, R₄ oder R₅ in diesen Verbindungen -COOH bzw. -SO₃H bedeutet, durch Neutralisation mit anorganischen oder organischen Basen, erhalten.

Als anorganische Säuren kommen vorzugsweise Salzsäure, Schwefelsäure, Salpetersäure oder Bromwasserstoffsäure, als organische Säuren zum Beispiel Ameisensäure, Essigsäure, Propionsäure, Glykolsäure, Milchsäure, Mandelsäure, Weinsäure, Äpfelsäure, Zitronensäure, Malonsäure, Maleinsäure, Fumarsäure, Succinsäure, Alginsäure, Benzoesäure, 2-, 3- und 4-Alkyloxy- und Acyloxybenzoesäuren, Ascorbinsäure, C₁-C₃-Alkylsulfonsäuren, Benzolsulfonsäure, Nicotinsäure, Isonicotinsäure und Aminosäuren zur Anwendung.

Als anorganische Basen kommen zum Beispiel Ammoniak, Natron- und Kalilauge sowie als organische Basen Alkylamine, C₁-C₃, Pyridin, Chinolin, Isochinolin, Piperazin und -Derivate, Picoline, Chinaldin oder Pyrimidin zur Anwendung.

Weiterhin können physiologisch verträgliche Salze der Verbindungen gemäß der allgemeinen Formeln 1a, 1b, 1 c und 1d dadurch gewonnen werden, dass jene Substanzen, die als Substituenten eine tertiäre Amino-Gruppe besitzen, in prinzipiell bekannter Weise mit alkylierenden Agentien - wie zum Beispiel Alkyl- oder Aralkylhalogeniden - in die entsprechenden quarternären Ammoniumsalze übergeführt werden können.

Die Erfindung betrifft auch Solvate der Verbindungen, einschließlich der pharmazeutisch akzeptablen Salze, Säuren, Basen und Ester sowie deren aktive Metabolite und gegebenenfalls deren Tautomere gemäß der allgemeinen Formeln 1a, 1b, 1 c und 1 d einschließlich Prodrug-Formulierungen. Prodrug-Formulierungen umfassen hierbei alle jene Substanzen, die durch einfache Transformation einschließlich Hydrolyse, Oxidation, oder Reduktion entweder enzymatisch, metabolisch oder auf andere Art und Weise entstehen. Ein geeignetes Prodrug enthält beispielsweise eine Substanz der allgemeinen Formeln 1a, b, c oder d, die über einen enzymatisch spaltbaren Linker (z.B. Carbamat, Phosphat, N-Glycosid oder eine Disulfidgruppe an eine lösungsverbessernde Substanz (z.B. Tetraethylenglykol, Saccharide, Aminosäuren) gebunden ist. Ein solches Prodrug einer erfindungsgemäßen Verbindung kann einem Patienten appliziert werden, und dieses Prodrug kann in eine Substanz der allgemeinen Formeln 1a, b, c oder d transformiert werden, wodurch der gewünschte pharmakologische Effekt erzielt wird.

Die durch die erfindungsgemäßen Verbindungen behandelbaren Erkrankungen schließen alle ein, bei denen das Enzym Phosphodiesterase (PDE-4) eine Rolle spielt, z.B. Asthma bronchiale, COPD, Rheumatoide Arthritis (RA), Osteoarthritis, Multiple Sklerose, Guillain-Barré Syndrom, Mb. Crohn, Colitis ulcerosa, Psoriasis, atopische Dermatitis, allergische Ekzeme, Rhinitis allergica, allergische Konjunktivitis, systemische Sklerodermie, Graft versus host disease (GvHD), Systemischer Lupus Erythematodes (SLE), Diabetes mellitus Typ I, neurodegenerative Erkrankungen, insbesondere Mb. Alzheimer und Mb Parkinson, posttraumatisches Multiorganversagen, Toxisches Schocksyndrom, Akute Glomerulonephritis, akute und chronische Schmerzen, Arteriosklerose, Herzinfarkt, Schlaganfall, Tumorerkrankungen und hier insbesondere Tumoren des blutbildenden Systems wie z.B. Leukämien (insbesondere CLL) und Lymphome, axonale Degeneration, virale Erkrankungen und hier insbesondere retrovirale Erkrankungen wie z.B. das erworbene Immundefizienz Syndrom (AIDS) und Myastenia Gravis.

Die durch die erfindungsgemäßen Verbindungen behandelbaren Erkrankungen schließen auch die in der Veterinärmedizin auftretenden mit ein, insbesondere das Asthma bronchiale, die COPD und Dermatiditen unterschiedlicher Genese.

Die erfindungsgemäßen Verbindungen können auf verschiedenen Wegen verabreicht werden, z.B. oral, parenteral, kutan, subkutan, intravenös, intramuskulär, rektal oder inhalativ. Bevorzugt ist die orale oder inhalative Verabreichung. Die Verbindung wird einem Patienten, der einer Therapie einer unter das Indikationsspektrum der erfindungsgemäßen Verbindungen fallenden Krankheit bedarf, über einen vom Arzt zu bestimmenden Zeitraum verabreicht. Die Verbindung kann sowohl Menschen als auch anderen Säugern verabreicht werden.

Die Dosierung der erfindungsgemäßen Verbindungen wird vom Arzt anhand der patientenspezifischen Parameter wie z.B. Alter, Gewicht, Geschlecht, Schwere der Erkrankung, etc. bestimmt. Bevorzugt beträgt die Dosierung zwischen 0,001 mg/kg bis 1000 mg/kg Körpergewicht, bevorzugt 0,01 bis 500 mg/kg Körpergewicht und ganz bevorzugt 0,1 bis 100 mg/kg Körpergewicht.

Entsprechend der Art der Verabreichung wird das Medikament in geeigneter Weise formuliert, z.B. in Form von Lösungen bzw. Suspensionen, einfachen oder dragierten Tabletten, Hart- oder Weichgelatinekapseln, Pulver zur Rekonstitution vor Gebrauch, Aerosolen, Inhalationssprays, Wirkstoffpflastern, Granulaten, Suppositorien, Ovula, Injektionspräparaten, Cremes, Salben, Gels, Mikrospheren, Implantaten, die nach üblichen galenischen Verfahren hergestellt werden.

Die erfindungsgemäßen Verbindungen können gegebenenfalls zusammen mit weiteren Wirkstoffen und mit in pharmazeutischen Zusammensetzungen üblichen Exzipientien formuliert werden, z.B. je nach herzustellendem Präparat Talk, Gummi arabicum, Lactose, Stärke, Magnesiumstearat, Kakaobutter, wäßrige und nichtwäßrige Träger, Fettkörper mit tierischem oder pflanzlichem Ursprung, Paraffinderivate, Glykole (insbesondere Polyethylenglykol), verschiedene Weichmacher, Dispergiermittel oder Emulgatoren, pharmazeutisch verträgliche Gase (z.B. Luft, Sauerstoff, Kohlendioxid usw.), Konservierungsstoffe.

Zur Herstellung flüssiger Präparate können Additive wie Natriumchloridlösung, Ethanol, Sorbit, Glycerin, Olivenöl, Mandelöl, Propylenglycol, Ethylenglycol oder andere Additive, die in der Pharmazie üblich sind, verwendet werden.

Bei der Verwendung von Infusions- oder Injektionslösungen sind diese bevorzugt wäßrige Lösungen oder Suspensionen, wobei es möglich ist, diese vor Gebrauch herzustellen, beispielsweise aus lyophilisierten Präparaten, die den Wirkstoff alleine oder zusammen mit einem Träger, wie Mannit, Lactose, Glucose, Albumin und dergleichen, enthalten. Die gebrauchsfertigen Lösungen werden sterilisiert und gegebenenfalls mit Hilfsmitteln vermischt, beispielsweise mit Konservierungsstoffen, Stabilisatoren, Emulgatoren, Lösungsvermittlern, Puffern und/oder Salzen zur Regulierung des osmotischen Drucks. Die Sterilisierung kann durch Sterilfiltration durch Filter mit einer kleinen Porengröße erzielt werden, wonach die Zusammensetzung gegebenenfalls lyophilisiert werden kann. Geringe Mengen an Antibiotika können auch zugesetzt werden, um die Beibehaltung der Sterilität zu gewährleisten.

Weiter bevorzugt werden Inhalationszusammensetzungen, z.B. in Form von Aerosolen, Sprays, oder als mikronisiertes Pulver hergestellt. Dazu werden die erfindungsgemäßen Verbindungen entweder in pharmazeutisch üblichen Lösungsmitteln gelöst bzw. suspendiert und mittels Überdruck in einem bestimmten Volumen fein verteilt und inhaliert. Ein entsprechendes Vorgehen erfolgt bei den zu inhalierenden Festsubstanzen, die gleichfalls mittels Überdruck fein verteilt und inhaliert werden. Ebenfalls andere als mit Überdruck funktionierende Applikatoren sind hierbei eingeschossen.

Die Erfindung betrifft auch pharmazeutische Zubereitungen, die eine therapeutisch wirksame Menge des aktiven Inhaltsstoffs (erfindungsgemäße Verbindung der Formeln 1 a, 1 b, 1 c oder 1 d) zusammen mit organischen oder anorganischen festen oder flüssigen pharmazeutisch verträglichen Trägern, die für die beabsichtigte Verabreichung geeignet sind, und die mit den aktiven Inhaltsstoffen nicht nachteilig Wechselwirken, enthalten.

Die Erfindung betrifft auch Verfahren zur Herstellung pharmazeutischer Zubereitungen, die dadurch gekennzeichnet sind, dass die erfindungsgemäße Verbindung mit einem pharmazeutisch verträglichen Träger vermischt wird.

Die erfindungsgemäßen Verbindungen eignen sich auch im Rahmen von Kombinationstherapien mit schon bekannten Wirkstoffen zur Behandlung der oben genannten Erkrankungen. Dabei sollen überraschende Synergieeffekte zur Steigerung der therapeutischen Wirksamkeit der erfindungsgemäßen Substanzen genutzt werden. Die Kombination kann zum einen darin bestehen, eine einzige pharmazeutische Zusammensetzung anzubieten, die mindestens eine der erfindungsgemäßen Verbindungen in Kombination mit einem oder mehrere der nachfolgend genannten Wirkstoffen enthält oder dem Patienten werden gleichzeitig oder zeitlich versetzt zur erfindungsgemäßen pharmazeutischen Zusammensetzung mehrere Mittel, die einen oder mehreren der nachfolgenden Wirkstoffe enthalten, verabreicht.

Es ist bevorzugt eine oder mehrere der erfindungsgemäßen Verbindungen mit einem oder mehreren der folgenden Wirkstoffe zu kombinieren:
- β₂-Adrenoceptor Agonisten (z.B. Terbutalin, Salbutanol, Salmetanol, Fenoterol, Formoterol)
- Dinatriumcromoglycat
- Korticosteroide
- Leukotrien-Antagonisten (entweder Enzyminhibitoren [wie 5-Lipoxygenaseinhibitoren oder Arachidonsäure-Enzyminhibitoren] oder Rezeptorantagonisten) , z.B. Pramkulast, Montelukast, Zafirlukast, Zileuton
- Antihistaminika (bevorzugt solche mit Mastzellen-stabilisierenden Eigenschaften oder Leukotrien-antagonisierenden Aspekten, wie z.B. Loratadin, Astemizol, Mizolastin, Olopatadin
- Theophyllin
- Muscarinrezeptor-Antagonisten, z.B. Spiriva
- (monoklonale) Antikörper gegen TNF-alpha oder andere Wirkstoffe, die die Bildung bzw. Freisetzung von TNF-alpha oder die Aktivität von TNF-alpha hemmen (z.B. rekombinante lösliche Rezeptorkonstrukte)

Die Kombination mit Korticosteroiden, Leukotrien-Antagonisten, Antihistaminika, Theophylin, Muscarinrezeptor-Antagonisten und/oder TNF-alpha-Hemmern dient besonders dazu, den akuten zu behandelnden Krankheitszustand nicht chronisch werden zu lassen, da die erfindungsgemäßen Verbindungen und die anderen Wirkstoffe komplementäre Aspekte der der Erkrankung zugrundeliegenden pathophysiologischen Mechanismen angehen. Erfindungsgemäß sollte insbesondere die Kombination der erfindungsgemäßen Verbindungen mit β₂-Adrenoceptoragonisten und/oder Dinatriumcromogycat als Inhalationstherapie bei milden Formen von Asthma, insbesondere im Kindesalter, wirksam sein. In der Kombination mit Glucocorticoiden ergibt sich ein positiver Effekt daraus, dass weniger Glucocorticoide angewendet werden müssen und damit ein Spareffekt zu erreichen ist und die von Glucocorticoiden bekannten Nebenwirkungen vermindert werden bzw. ganz ausbleiben. Die Kombination der erfindungsgemäßen Verbindungen mit Glucocorticoiden und/oder Theophyllin hat sich insbesondere bei persistierendem Asthma bewährt.

Abhängig von der Krankheitsausprägung und den zugrunde liegenden Symptomen können die erfindungsgemäßen Verbindungen zu den anderen Wirkstoffen in der Kombination im Verhältnis von 1:10.000 bis 10.000:1, bevorzugt 1:100 bis 100:1, ganz bevorzugt 1:10 bis 10:1 vorliegen.

Die Erfindung betrifft weiterhin Verfahren zur Herstellung der erfindungsgemäßen Verbindungen.

Die erfindungsgemäßen Verfahren zur Herstellung der Verbindungen der allgemeinen Formeln 1 a, 1 b, 1 c und 1 d mit den zuvor aufgeführten Bedeutungen von R₁, R₂, R₃, R₄, R₅ und Y sind gekennzeichnet durch folgende Verfahrensweisen:
nach Schema 1 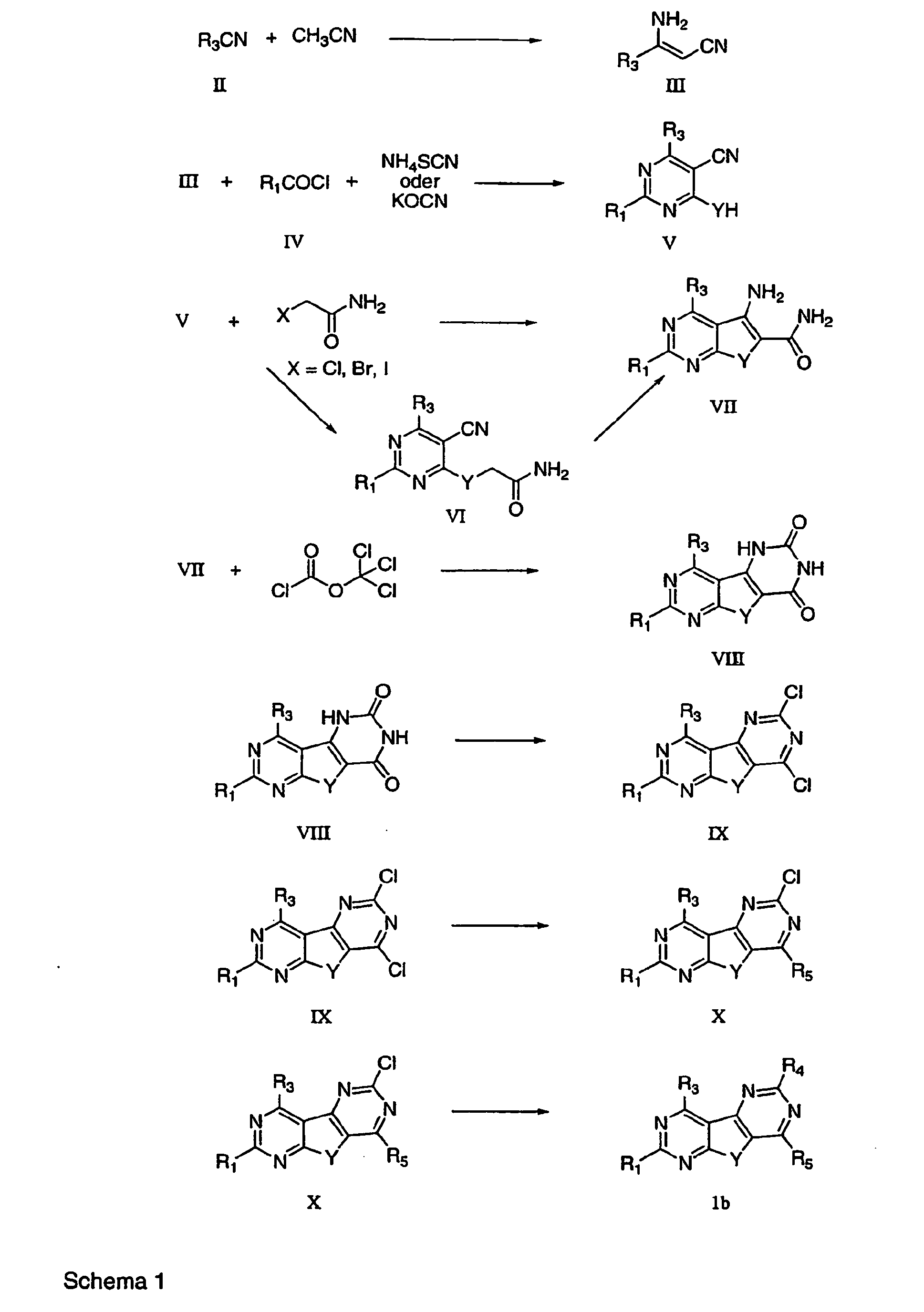
- Darstellung der 2-Aminonitrile der allgemeinen Formel III durch Umsetzung von Acetonitril mit einem Nitril der allgemeinen Formel II (mit identischer Bedeutung von R₃) in Gegenwart eines Alkoxides, vorzugsweise Kalium-*tert-*butoxid in einem geeigneten Lösungsmittel, vorzugsweise Toluol.
- Umsetzung eines Säurehalogenids der allgemeinen Formel IV (mit identischer Bedeutung von R₁) mit einem Thiocyanat für Y=S oder Cyanat für Y=O, vorzugsweise Ammoniumthiocyanat oder Kaliumcyanat, in einem geeignetem Lösungsmittel, vorzugsweise Dioxan, zum Carbonsäureisothiocyanat oder -isocyanat welches mit einem 2-Aminonitril der allgemeinen Formel III zum Pyrimidin-5-carbonitril der allgemeinen Formel V umgesetzt wird.
- Umsetzung der dargestellten Pyrimidin-5-carbonitrile der allgemeinen Formel V (mit identischer Bedeutung von R₁ und R₃ wie vorstehend) in an sich bekannter Weise mit 2-Chloracetamid in methanolischer oder ethanolischer Lösung in Gegenwart eines Natriumalkoxides, vorzugsweise Natriummethoxid oder Natriumethoxid, in die analogen 5-Aminofuro- oder 5-Aminothieno[2,3-d]pyrimidin-6-carbonsäureamide der allgemeinen Formel VII (mit identischer Bedeutung von R₁, R₃ und Y wie vorstehend).
- Die Verbindungen der allgemeinen Formel VII sind aus den Verbindungen der allgemeinen Formel V (wobei R₁, R₃ und Y die oben genannten Bedeutungen aufweisen) auch dadurch darstellbar, dass diese Verbindungen mit 2-Chloracetamid zunächst in vorzugsweise ethanolischer Lösung in Gegenwart von vorzugsweise Triethylamin oder einem sekundären cycloaliphatischen Amin wie Morpholin, Piperidin oder Pyrrolidin zu den Verbindungen der allgemeinen Formel VI (wobei R₁, R₃ und Y die oben genannten Bedeutungen aufweisen), umgesetzt werden und diese Verbindungen in einem weiteren Syntheseschritt in vorzugsweise wasserfreier ethanolischer Lösung mit einer katalytischen Menge Natriummethoxid oder Natriumethoxid durch Erhitzen unter Rückfluss gleichfalls in die oben genannten Verbindungen der allgemeinen Formel VII übergeführt werden.
- Umsetzung der Verbindungen der allgemeinen Formel VII mit Phosgenderivaten, vorzugsweise Diphosgen, in einem geeignetem Lösungsmittel, vorzugsweise Dioxan oder Toluol, zu den Verbindungen der allgemeinen Formel VIII.
- Umsetzung der tricyclischen Dipyrimidin-2,4-dione der allgemeinen Formel VIII mit einem Halogenierungsmittel, vorzugsweise Dichlorphenylphosphinoxid, Phosphortrichlorid, Phosphorpentachlorid, Phosphoroxychlorid bzw. deren Mischungen, jeweils in der Hitze, zu den tricyclischen 2,4-Dichlorpyrimidinen der allgemeinen Formel IX, worin R₁, R₃ und Y dasselbe bedeuten wie oben.
- Umsetzung der 2,4-Dichlorpyrimidine der allgemeinen Formel IX mit O-, N-, S- oder C-Nucleophilen, vorzugsweise Alkoholate, Amine und Thiolate, in Alkanolen oder gegebenenfalls aprotischen, dipolaren Lösungsmitteln unter Erwärmen, in Ausnahmefällen auch bei Raumtemperatur, zu den tricyclischen 2-Chlor-pyrimidinen der allgemeinen Formel X, worin R₁, R₃, R₅ und Y dasselbe wie oben bedeuten.
- Umsetzung der 2-Chlor-pyrimidine der allgemeinen Formel X mit O-, N-, S- oder C-Nucleophilen, vorzugsweise Alkoholate, Amine und Thiolate, in Alkanolen oder gegebenenfalls aprotischen, dipolaren Lösungsmitteln unter Erwärmen, in Ausnahmefällen auch bei Raumtemperatur, zu den Verbindungen der allgemeinen Formel 1 b, worin R₁, R₃, R₄, R₅ und Y dasselbe wie oben bedeuten.
nach Schema 2 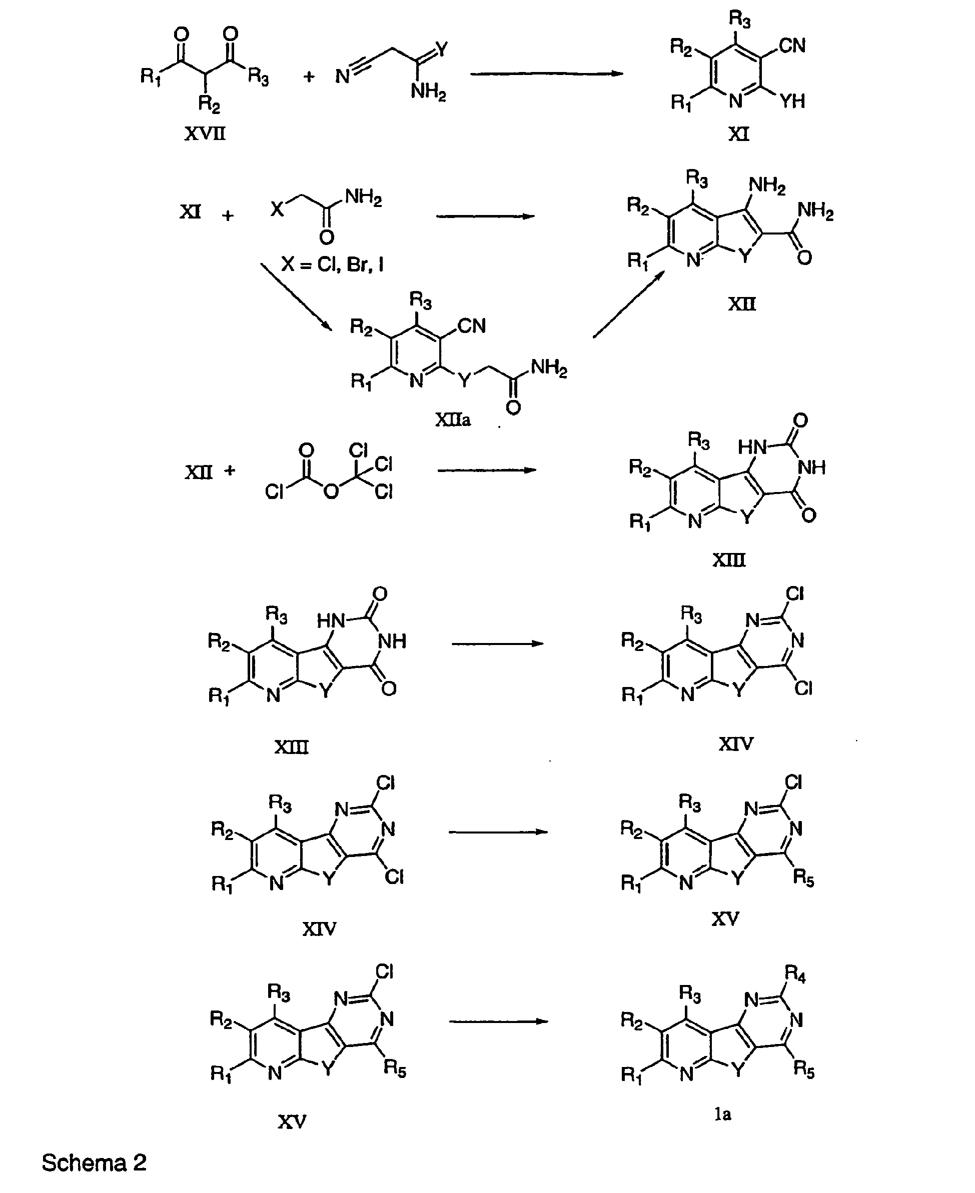
- Umsetzung eines 1,3-Diketons der allgemeinen Formel XVII (mit identischer Bedeutung von R₁, R₂, R₃) mit Cyanthioacetamid für Y=S oder Cyanacetamid für Y=O in einem geeignetem Lösungsmittel, vorzugsweise Aceton oder Ethanol, zum Pyridin-3-carbonitril der allgemeinen Formel XI.
- Umsetzung der dargestellten Pyridin-3-carbonitrile der allgemeinen Formel XI (mit identischer Bedeutung von R₁, R₂ und R₃ wie vorstehend) in an sich bekannter Weise mit 2-Chloracetamid in methanolischer oder ethanolischer Lösung in Gegenwart eines Natriumalkoxides, vorzugsweise Natriummethoxid oder Natriumethoxid, in die Carbonsäureamide der allgemeinen Formel XII (mit identischer Bedeutung von R₁, R₂, R₃ und Y wie vorstehend).
- Die Verbindungen der allgemeinen Formel XIIa sind aus den Verbindungen der allgemeinen Formel XI (wobei R₁, R₂, R₃ und Y die oben genannten Bedeutungen aufweisen) auch dadurch darstellbar, dass diese Verbindungen mit 2-Chloracetamid zunächst in vorzugsweise ethanolischer Lösung in Gegenwart von vorzugsweise Triethylamin oder einem sekundären cycloaliphatischen Amin wie Morpholin, Piperidin oder Pyrrolidin zu den Verbindungen der allgemeinen Formel XIIa (wobei R₁, R₂, R₃ und Y die oben genannten Bedeutungen aufweisen), umgesetzt werden und diese Verbindungen in einem weiteren Syntheseschritt in vorzugsweise wasserfreier ethanolischer Lösung mit einer katalytischen Menge Natriummethoxid oder Natriumethoxid durch Erhitzen unter Rückfluss gleichfalls in die oben genannten Verbindungen der allgemeinen Formel XII übergeführt werden.
- Umsetzung der Verbindungen der allgemeinen Formel XII mit Phosgenderivaten, vorzugsweise Diphosgen, in einem geeignetem Lösungsmittel, vorzugsweise Dioxan oder Toluol, zu den Verbindungen der allgemeinen Formel XIII.
- Umsetzung der tricyclischen Dipyrimidin-2,4-dione der allgemeinen Formel XIII mit einem Halogenierungsmittel, vorzugsweise Dichlorphenylphosphinoxid, Phosphortrichlorid, Phosphorpentachlorid, Phosphoroxychlorid bzw. deren Mischungen, jeweils in der Hitze, zu den tricyclischen 2,4-Dichlorpyrimidinen der allgemeinen Formel XV, worin R₁, R₂, R₃ und Y dasselbe bedeuten wie oben.
- Umsetzung der 2,4-Dichlorpyrimidine der allgemeinen Formel XIV mit O-, N-, S- oder C-Nucleophilen, vorzugsweise Alkoholate, Amine und Thiolate, in Alkanolen oder gegebenenfalls aprotischen, dipolaren Lösungsmitteln unter Erwärmen, in Ausnahmefällen auch bei Raumtemperatur, zu den tricyclischen 2-Chlor-pyrimidinen der allgemeinen Formel XV, worin R₁, R₂, R₃, R₅ und Y dasselbe wie oben bedeuten.
- Umsetzung der 2-Chlor-pyrimidine der allgemeinen Formel XV mit O-, N-, S- oder C-Nucleophilen, vorzugsweise Alkoholate, Amine und Thiolate, in Alkanolen oder gegebenenfalls aprotischen, dipolaren Lösungsmitteln unter Erwärmen, in Ausnahmefällen auch bei Raumtemperatur, zu den Verbindungen der allgemeinen Formel 1a, worin R₁, R₂, R₃, R₄, R₅ und Y dasselbe wie oben bedeuten.

Die Pyridin-3-carbonitrile der allgemeinen Formel XIX lassen sich auch durch folgende Reaktionen darstellen (Schema 3): 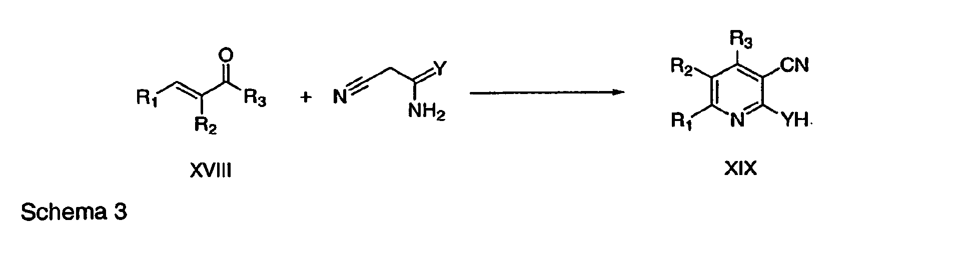
- Durch Umsetzung eines geeigneten α,β-ungesättigten Ketons der allgemeinen Formel XVIII (mit identischer Bedeutung von R₁, R₂, R₃) mit Cyanthioacetamid für Y=S oder Cyanacetamid für Y=O in einem geeignetem Lösungsmittel, vorzugsweise Aceton unter Zusatz eines Oxidationsmittels, wie beispielsweise Sauerstoff oder Schwefel, zum Pyridin-3-carbonitril der allgemeinen Formel XIX.

Die R₂-R₃-anellierten Carbonitrile lassen sich nach Schema 4 darstellen: 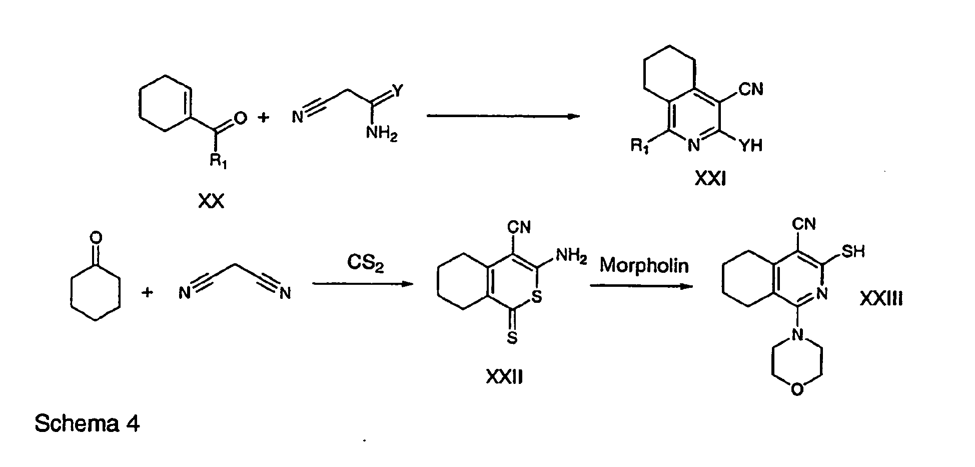
- Durch Umsetzung eines cyclischen α,β-ungesättigten Ketons der allgemeinen Formel XX mit Cyanthioacetamid für Y=S oder Cyanacetamid für Y=O in einem geeignetem Lösungsmittel, vorzugsweise Aceton unter Zusatz einer Base, vorzugsweise K₂CO₃ und eines Oxidationsmittels, wie beispielsweise Sauerstoff oder Schwefel, zum Pyridin-3-carbonitril der allgemeinen Formel XXI.
   oder
- Durch Umsetzung eines cyclischen Ketons, beispielsweise Cyclohexanon, mit Malondinitril und Schwefelkohlenstoff unter Zusatz einer geeigneten Base, vorzugsweise Triethylamin, zu den Carbonitrilen der Formel XXII in einem geeignetem Lösungsmittel, vorzugsweise Methanol und anschließender Umsetzung mit einem sekundärem Amin, beispielsweise Morpholin zu den Verbindungen der Formel XXIII.

Die nach Schema 4 dargestellten Verbindungen lassen sich anschließend analog Schema 2 zu den Verbindungen der allgemeinen Formel 1 c umsetzen.

Die R₁-R₂-anellierten Carbonitrile lassen sich nach Schema 5 darstellen: 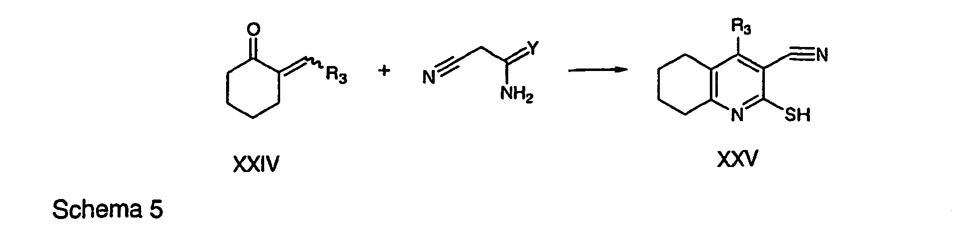
- Durch Umsetzung eines cyclischen α,β-ungesättigten Ketons der allgemeinen Formel XXIV mit Cyanthioacetamid für Y=S oder Cyanacetamid für Y=O in einem geeignetem Lösungsmittel, vorzugsweise Aceton unter Zusatz einer Base, vorzugsweise K₂CO₃ und eines Oxidationsmittels, wie beispielsweise Sauerstoff oder Schwefel zu den anellierten Carbonitrilen der Formel XXV.

Weiterhin lassen sich die Pyridin-3-carbonitrile analog zu PCT/US03/17343 wie in Schema 6 dargestellt herstellen.

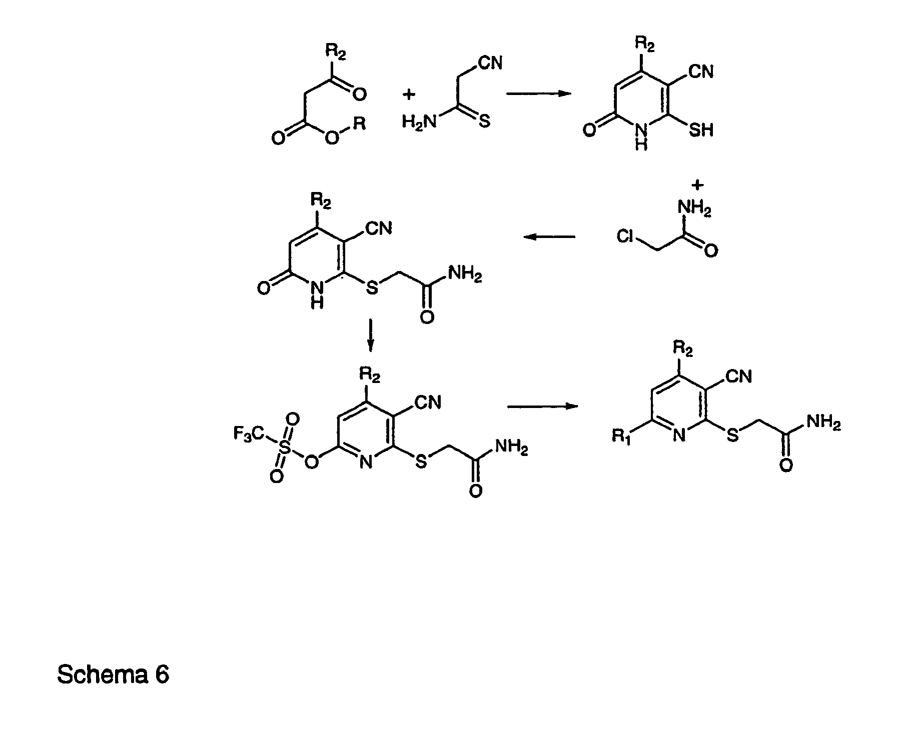

Die Verbindungen der allgemeinen Formeln 1a, 1b, 1 c und 1d sind starke Inhibitoren der Phosphodiesterase 4 und der Freisetzung von TNFalpha. Ihr therapeutisches Potential in vivo ist erwiesen durch die Inhibierung der asthmatischen Spät-Phasen Reaktion (Eosinophilie) in der Lungen-Spülflüssigkeit des Meerschweinchens und durch die Beeinflussung der Allergen-induzierten Gefäßpermeabilität in aktiv sensitierten braunen Ratten (R. Norwegicus). Detailliert sind die Ergebnisse im nachfolgenden Beispiel 23 dargestellt.

Besonders bevorzugte Verbindungen hinsichtlich der Hemmung von PDE4 sind:
4-Ethoxy-9-ethyl-7-(4-(pyrimidyl-2-yl)piperazin-1-yl)-2-piperazin-1-yl-pyrido[3',2':4,5]thieno[3,2-d]pyrimidin
4-Ethoxy-9-ethyl-7-(4-methyl-phenyl)-2-(piperazin-1-yl)-pyrido[3',2':4,5]thieno[3,2-d]pyrimidin
2-Ethoxy-9-ethyl-7-(4-methyl-phenyl)-4-(piperazin-1-yl)-pyrido[3',2':4,5]thieno[3,2-d]pyrimidin
4-Ethoxy-7-(4-ethoxy-phenyl)-9-ethyl-2-(piperazin-1-yl)-pyrido[3',2':4,5]thieno[3,2-d]pyrimidin
7-(4-Ethoxy-phenyl)-9-ethyl-2-(piperazin-1-yl)-4-*n*-propoxy-pyrido[3',2':4,5]thieno[3,2-d]pyrimidin
2-Ethoxy-7-(4-ethoxy-phenyl)-9-ethyl-4-(piperazin-1-yl)-pyrido[3',2':4,5]thieno[3,2-d]pyrimidin
4-Ethoxy-9-ethyl-7-(4-methylthio-phenyl)-2-(piperazin-1-yl)-pyrido[3',2':4,5]thieno[3,2-d]pyrimidin
4-Ethoxy-7-(4-methyl-phenyl)-9-iso-propyl-2-(piperazin-1-yl)-pyrido[3',2':4,5]thieno-[3,2-d]pyrimidin
4-Ethoxy-9-ethyl-7-[4-(4-methoxyphenoxy)phenyl]-2-(piperazin-1-yl)-pyrido[3',2':4,5] thieno[3,2-d]pyrimidin
4-Ethoxy-9-ethyl-7-(2,3-dihydrobenzo[b][1,4]dioxin-6-yl)-2-(piperazin-1-yl)-pyrido[3',2':4,5] thieno[3,2-d]pyrimidin
2-Ethoxy-9-ethyl-7-(2,3-dihydrobenzo[b][1,4]dioxin-6-yl)-4-(piperazin-1-yl)-pyrido[3',2':4,5] thieno[3,2-d]pyrimidin
4-Ethoxy-9-ethyl-7-(3,4-methylendioxyphenyl)-2-piperazin-1-yl-pyrido[3',2':4,5]thieno-[3,2-d]pyrimidin
4-Ethoxy-7-(4-methoxy-phenyl)-2-(piperazin-1-yl)-9-trifluormethyl-pyrido[3',2':4,5]thieno[3,2-d]pyrimidin
4-Ethoxy-7-(4-methyl-phenyl)-2-(piperazin-1-yl)-9-trifluormethyl-pyrido[3',2':4,5]thieno[3,2-d]pyrimidin
7-(2,3-Dihydrobenzo[b][1,4]dioxin-6-yl)-4-ethoxy-2-(piperazin-1-yl)-9-trifluormethyl-pyrido[3',2':4,5] thieno[3,2-d]pyrimidin
4-Ethoxy-7-(4-(1*H*-imidazol-1-yl)phenyl)-2-(piperazin-1-yl)-9-trifluormethyl-pyrido[3',2':4,5]thieno[3,2-d]pyrimidin
4-[4-Ethoxy-2-(piperazin-1-yl)-9-trifluormethyl-pyrido[3',2':4,5]thieno[3,2-d]pyrimidin-7-yl]-benzoesäureethylester
4-Ethoxy-2-(piperazin-1-yl)-7-(4-(piperidin-1-yl)phenyl)-9-trifluormethyl-pyrido[3',2':4,5]thieno[3,2-d]pyrimidin
4-Ethoxy-9-ethyl-7-(4-methoxyphenyl)-2-(piperazin-1-yl)-pyrido[3',2':4,5]thieno[3,2-d]pyrimidin
4-Ethoxy-7-(3,4-methylendioxyphenyl)-2-piperazin-1-yl-9-trifluormethyl-pyrido[3',2':4,5]thieno-[3,2-d]pyrimidin
4-Ethoxy-7-(4-methylthiophenyl)-2-(piperazin-1-yl)-9-trifluormethyl-pyrido[3',2':4,5]thieno[3,2-d]pyrimidin
5,6-Dihydro-11-ethoxy-3'-methoxy-9-piperazin-1-yl-7-trifluormethyl-benzo[h]pyrimido[4',5':4,5]thieno[2,3-b]chinolin
4-Ethoxy-7-(4-(1H-pyrazol-1-yl)-phenyl)-9-ethyl-2-piperazin-1-yl-pyrido[3',2':4,5]thieno[3,2-d]pyrimidin
4-Ethoxy-7-(4-iod-phenyl)-2-(piperazin-1-yl)-9-trifluormethyl-pyrido[3',2':4,5]thieno[3,2-d]pyrimidin
4-Ethoxy-7-(4-(1H-pyrazol-1-yl)-phenyl)-2-piperazin-1-yl-9-trifluormethyl-pyrido[3',2':4,5]thieno[3,2-d]pyrimidin
7-(4-Brom-phenyl)-4-ethoxy-9-ethyl-2-(piperazin-1-yl)-pyrido[3',2':4,5]thieno[3,2-d]pyrimidin
4-Ethoxy-9-ethyl-7-(4-iod-phenyl)-2-(piperazin-1-yl)-pyrido[3',2':4,5]thieno[3,2-d]pyrimidin
4-Ethoxy-7-(3-nitro-phenyl)-2-piperazin-1-yl-9-triluormethyl-pyrido[3',2':4,5]thieno[3,2-d]pyrimidin
7-(3-Cyano-phenyl)-4-ethoxy-2-piperazin-1-yl-9-trifluormethyl-pyrido[3',2':4,5]thieno[3,2-d]pyrimidin
N-(4-(4-Ethoxy-9-trifluormethyl-2-piperazin-1-yl-pyrido[3',2':4,5]thieno[3,2-d]pyrimidin-7-yl)-phenyl)-formamid
N-(4-Ethoxy-9-trifluormethyl-2-piperazin-1-yl-pyrido[3',2':4,5]thieno[3,2-d]pyrimidin-7-yl)-benzamid Trifluoracetat
N'-Hydroxy-4-(4-ethoxy-9-trifluormethyl-2-piperazin-1-yl-pyrido[3',2':4,5]thieno[3,2-d]pyrimidin-7-yl)-benzamidin Bistrifluoracetat
N-(4-(4-Ethoxy-9-trifluormethyl-2-piperazin-1-yl-pyrido[3',2':4,5]thieno[3,2-d]pyrimidin-7-yl)-phenyl)-isonicotinamid
4-Ethoxy-9-trifluormethyl-2-piperazin-1-yl-7-(4-(1H-pyrrol-1-yl)-phenyl)-pyrido[3',2':4,5]thieno[3,2-d]pyrimidin
N-(4-(4-Ethoxy-9-trifluormethyl-2-piperazin-1-yl-pyrido[3',2':4,5]thieno[3,2-d]pyrimidin-7-yl)-phenyl)-nicotinamid-N-oxid
4-(4-Ethoxy-9-trifluormethyl-2-piperazin-1-yl-pyrido[3',2':4,5]thieno[3,2-d]pyrimidin-7-yl)-phenyl-carbaminsäuremethylester
N-(4-(4-Ethoxy-9-trifluormethyl-2-piperazin-1-yl-pyrido[3',2':4,5]thieno[3,2-d]pyrimidin-7-yl)-phenyl)-nicotinamid
7-(3'-Aminobiphen-4-yl)-4-ethoxy-9-methyl-2-piperazin-1-yl-thieno[2,3-d:4,5-d']dipyrimidin
4-Ethoxy-7-(4-[3-furyl]-phenyl)-9-methyl-2-piperazin-1-yl-thieno[2,3-d:4,5-d']dipyrimidin
4-Ethoxy-7-(4-[2-furyl]-phenyl)-9-methyl-2-piperazin-1-yl-thieno[2,3-d:4,5-d']dipyrimidin
4-Ethoxy-9-methyl-2-piperazin-1-yl-7-(4-[3-pyridyl]-phenyl)-thieno[2,3-d:4,5-d']dipyrimidin
4-Ethoxy-9-methyl-2-piperazin-1-yl-7-(4-[4-pyridyl]-phenyl)-thieno[2,3-d:4,5-d']dipyrimidin
7-(4'-Aminobiphen-4-yl)-4-ethoxy-9-methyl-2-piperazin-1-yl-thieno[2,3-d:4,5-d']dipyrimidin
4-Ethoxy-9-methyl-2-piperazin-1-yl-7-(3',4',5'-trimethoxybiphen-4-yl)-thieno[2,3-d:4,5-d']dipyrimidin
4-Ethoxy-9-methyl-7-(4-phenoxy-phenyl)-2-piperazin-1-yl-thieno[2,3-d:4,5-d']dipyrimidin
2'-Amino-4-(4-ethoxy-9-methyl-2-piperazin-1-yl-thieno[2,3-d:4,5-d']dipyrimidin-7-yl)-biphenyl-4'-carbonsäuremethylester
7-(3',4'-Dimethoxybiphen-4-yl)-4-ethoxy-9-methyl-2-piperazin-1-yl-thieno[2,3-d:4,5-d']dipyrimidin
7-(4-[3,5-Dimethyl-isoxazol-4-yl]-phenyl)-4-ethoxy-9-methyl-2-piperazin-1-yl-thieno[2,3-d:4,5-d']dipyrimidin

Besonders bevorzugte Verbindung hinsichtlich der Hemmung der Freisetzung von TNF-alpha sind:
7-(3'-Aminobiphen-4-yl)-4-ethoxy-9-methyl-2-piperazin-1-yl-thieno[2,3-d:4,5-d']dipyrimidin
4-Ethoxy-7-(4-[3-furyl]-phenyl)-9-methyl-2-piperazin-1-yl-thieno[2,3-d:4,5-d']dipyrimidin
4-Ethoxy-9-methyl-2-piperazin-1-yl-7-(4-[3-pyrrolyl]-phenyl)-thieno[2,3-d:4,5-d']dipyrimidin
7-(4-[1,2-Dihydroxyethyl]-phenyl)-4-ethoxy-9-methyl-2-piperazin-1-yl-thieno[2,3-d:4,5-d']dipyrimidin
4-Ethoxy-7-(3,4-dimethoxy-phenyl)-9-methyl-2-(piperazin-1-yl)-pyrido[3',2':4,5]thieno[3,2-d]pyrimidin
4-Ethoxy-7-(4-methoxy-phenyl)-9-methyl-2-(piperazin-1-yl)-pyrido[3',2':4,5]thieno[3,2-d]pyrimidin-hydrochlorid
2-Ethoxy-7-methyl-9-phenyl-4-(piperazin-1-yl)-pyrido[3',2':4,5]thieno[3,2-d]pyrimidin-hydrochlorid
2-Ethoxy-7-(4-trifluor-methyl-phenyl)-9-methyl-4-(piperazin-1-yl)-pyrido[3',2':4,5]thieno[3,2-d]pyrimidin
2-(2-Hydroxy-ethoxy)-9-methyl-7-phenyl-4-(piperazin-1-yl)-pyrido[3',2':4,5]thieno[3,2-d]pyrimidin
8-(4-Amino-benzyl)-4-ethoxy-7,9-dimethyl-2-(piperazin-1-yl)-pyrido[3',2':4,5]thieno[3,2-d]pyrimidin
4-Ethoxy-7-(3,4-dimethoxy-phenyl)-9-methyl-2-(piperazin-1-yl)-pyrido[3',2':4,5]thieno[3,2-d]pyrimidin-hydrochlorid
4-Ethoxy-7-morpholino-9-phenyl-2-(piperazin-1-yl)-pyrido[3',2':4,5]thieno[3,2-d]pyrimidin
2-Ethoxy-7-(3,4-dimethoxy-phenyl)-9-methyl-4-(piperazin-1-yl)-pyrido[3',2':4,5]thieno[3,2-d]pyrimidin
2-Ethoxy-7-(4-methoxy-phenyl)-9-methyl-4-(piperazin-1-yl)-pyrido[3',2':4,5]thieno[3,2-d]pyrimidin
2-Ethoxy-7,9-dimethyl-8-(4-nitro-benzyl)-4-(piperazin-1-yl)-pyrido[3',2':4,5]thieno[3,2-d]pyrimidin
4-Ethoxy-7,9-diphenyl-2-(piperazin-1-yl)-pyrido[3',2':4,5]thieno[3,2-d]pyrimidin
4-Ethoxy-7-(4-methoxy-phenyl)-9-methyl-2-(piperazin-1-yl)-pyrido[3',2':4,5]thieno[3,2-d]pyrimidin
9-Methyl-7-phenyl-2-(piperazin-1-yl)-4-*n*-propoxy-pyrido[3',2':4,5]thieno[3,2-d]pyrimidin
4-Ethoxy-7,9-dimethyl-8-(4-nitro-benzyl)-2-(piperazin-1-yl)-pyrido[3',2':4,5]thieno[3,2-d]pyrimidin
4-Ethoxy-7-(4-fluor-phenyl)-9-methyl-2-(piperazin-1-yl)-pyrido[3',2':4,5]thieno[3,2-d]pyrimidin
4-Ethoxy-2-piperazin-1-yl-7-(3-pyridyl)-9-trifluormethyl-pyrido[3',2':4,5]thieno[3,2-d]pyrimidin
4-Ethoxy-7-(1-oxy-pyridin-3-yl)-2-(piperazin-1-yl)-9-trifluormethyl-pyrido[3',2':4,5]thieno[3,2-d]pyrimidin
4-Ethoxy-2-piperazin-1-yl-7-(4-pyridyl)-9-trifluormethyl-pyrido[3',2':4,5]thieno[3,2-d]pyrimidin
4-Ethoxy-7-(1-oxy-pyridin-4-yl)-2-(piperazin-1-yl)-9-trifluormethyl-pyrido[3',2':4,5]thieno[3,2-d]pyrimidin
4-Ethoxy-7-(4-(1*H*-imidazol-1-yl)phenyl)-2-(piperazin-1-yl)-9-trifluormethyl-pyrido[3',2':4,5]thieno[3,2-d]pyrimidin
4-(2-Hydroxy-ethoxy)-7-[(1H-imidazol-1-yl)phenyl]-2-(piperazin-1-yl)-9-trifluormethyl-pyrido[3',2':4,5]thieno[3,2-d]pyrimidin
8-Ethoxy-5-morpholino-10-piperazin-1-yl-pyrimido[4',5':4,5]thieno[2,3-c]-1,2,3,4-tetrahydro-isochinolin
4-(4-Ethoxy-9-trifluormethyl-2-piperazin-1-yl-pyrido[3',2':4,5]thieno[3,2-d]pyrimidin-7-yl)-benzamid Trifluoracetat
N'-Hxdroxy-4-(4-ethoxy-9-trifluormethyl-2-piperazin-1-yl-pyrido[3',2':4,5]thieno[3,2-d]pyrimidin-7-yl)-benzamidin Bistrifluoracetat
4-Ethoxy-9-trifluormethyl-2-piperazin-1-yl-7-(4-(1H-1 ,2,4-triazol-4-yl)-phenyl)-pyrido[3',2':4,5]thieno[3,2-d]pyrimidin
N-(4-(4-Ethoxy-9-trifluormethyl-2-piperazin-1-yl-pyrido[3',2':4,5]thieno[3,2-d]pyrimidin-7-yl)-phenyl)-isonicotinamid
N-(4-(4-Ethoxy-9-trifluormethyl-2-piperazin-1-yl-pyrido[3',2':4,5]thieno[3,2-d]pyrimidin-7-yl)-phenyl)-formamid
N-(4-(4-Ethoxy-9-trifluormethyl-2-piperazin-1-yl-pyrido[3',2':4,5]thieno[3,2-d]pyrimidin-7-yl)-phenyl)-nicotinamid

Von der Anmelderin wurden insbesondere die nachfolgenden Verbindungen als zytotoxisch wirksam charakterisiert:
4-Ethoxy-7-(4-[2-furyl]-phenyl)-9-methyl-2-piperazin-1-yl-thieno[2,3-d:4,5-d']dipyrimidin
4-Ethoxy-9-methyl-2-piperazin-1-yl-7-(4-[N-BOC-3-pyrrolyl]-phenyl)-thieno[2,3-d:4,5-d']dipyrimidin
4-Ethoxy-9-ethyl-7-(4-methyl-phenyl)-2-(piperazin-1-yl)-pyrido[3',2':4,5]thieno[3,2-d]pyrimidin
2-Ethoxy-7-(4-methyl-phenyl)-4-(piperazin-1-yl)-9-propyl-pyrido[3',2':4,5]thieno[3,2-d]pyrimidin
2-Ethoxy-7-(4-methyl-phenyl)-4-(piperazin-1-yl)-9-propyl-pyrido[3',2':4,5]thieno[3,2-d]pyrimidin Hydrochlorid
2-Ethoxy-9-methyl-4-(piperazin-1-yl)-7-phenyl-pyrido[3',2':4,5]furo[3,2-d]pyrimidin
7-(2,3-Dihydrobenzo[b][1,4]dioxin-6-yl)-2-ethoxy-9-ethyl-4-piperazino-pyrido[3',2':4,5] thieno[3,2-d]pyrimidin
4-Ethoxy-9-ethyl-7-(4-methoxy-phenyl)-2-piperazin-1-yl-pyrido[3',2':4,5]thieno[3,2-d]pyrimidin Hydrochlorid
4-Ethoxy-7-(4-methylthio-phenyl)-2-piperazin-1-yl-9-trifluormethyl-pyrido[3',2':4,5]thieno[3,2-d]pyrimidin
Mono-[4-Ethoxy-7-(4-methylthio-phenyl)-2-piperazin-1-yl-9-trifluormethyl-pyrido[3',2':4,5]thieno[3,2-d]pyrimidin] Citrat
7-(4-Amino-phenyl)-4-ethoxy-2-piperazin-1-yl-9-trifluormethyl-pyrido[3',2':4,5]thieno[3,2-d]pyrimidin
4-Ethoxy-9-methyl-2-piperazin-1-yl-7-(4-[4-pyridyl]-phenyl)-thieno[2,3-d:4,5-d']dipyrimidin
4-Ethoxy-9-methyl-2-piperazin-1-yl-7-(4-[3-pyridyl]-phenyl)-thieno[2,3-d:4,5-d']dipyrimidin
4-Ethoxy-9-methyl-2-piperazin-1-yl-7-(4-[3-pyrrolyl]-phenyl)-thieno[2,3-d:4,5-d']dipyrimidin
4-(4-Ethoxy-9-methyl-2-piperazin-1-yl-thieno[2,3-d:4,5-d']dipyrimidin-7-yl)-biphenyl-3'-carbonsäure Kaliumsalz
7-(4'-Aminobiphen-4-yl)-4-ethoxy-9-methyl-2-piperazin-1-yl-thieno[2,3-d:4,5-d']dipyrimidin
4-Ethoxy-9-methyl-2-piperazin-1-yl-7-(4-[5-pyrimidinyl]-phenyl)-thieno[2,3-d:4,5-d']dipyrimidin
7-(3',4'-Dimethoxybiphen-4-yl)-4-ethoxy-9-methyl-2-piperazin-1-yl-thieno[2,3-d:4,5-d']dipyrimidin
4-Ethoxy-7-(4-methoxy-phenyl)-9-methyl-2-piperazin-1-yl-pyrido[3',2':4,5]thieno[3,2-d]pyrimidin
2-Ethoxy-9-methyl-7-(3,4-methylendioxy-phenyl)-4-piperazin-1-yl-pyrido[3',2':4,5]-thieno[3,2-d]pyrimidin
4-Ethoxy-9-ethyl-7-(4-methoxy-phenyl)-2-piperazin-1-yl-pyrido[3',2':4,5]thieno[3,2-d]pyrimidin

Die nachfolgenden Tabellen geben besonders bevorzugte Verbindungen an:
- Formel 1(a), wobei Y = S und die Substituenten R¹, R², R³, R⁴, R⁵ die folgenden Bedeutungen haben:

| R₁ | R₂ | R₃ | R₄ | R₅ |
|---|---|---|---|---|
| 3,4-(MeO)₂-Ph | H | Me | Piperazin-1-yl | OEt |
| 4-MeO-Ph | H | Me | Piperazin-1-yl | OEt |
| 4-F-Ph | H | Me | Piperazin-1-yl | OEt |
| Ph | H | Me | Piperazin-1-yl | O*i*Pr |
| Ph | H | Me | Piperazin-1-yl | OCH₂CHF₂ |
| Ph | H | Ph | Piperazin-1-yl | OEt |
| Me | 4'-NO₂-Ph-CH₂ | Me | Piperazin-1-yl | OEt |
| Me | 4'-NH₂₋ Ph-CH₂ | Me | Piperazin-1-yl | OEt |
| 3,4-Methylen-dioxyphenyl | H | Me | Piperazin-1-yl | OEt |
| Ph | H | Me | Piperazin-1-yl | O*n*Pr |
| Ph | H | Me | Piperazin-1-yl | OEt |
| Ph | H | Me | Piperazin-1-yl | OCH₂CH₂O H |
| 3,4-(MeO)₂-Ph | H | Me | OEt | Piperazin-1-yl |
| 4-MeO-Ph | H | Me | OEt | Piperazin-1-yl |
| Ph | H | Me | OCH₂CHF₂ | Piperazin-1-yl |
| Me | 4'-NO₂-Ph-CH₂ | MG | OEt | Piperazin-1-yl |
| Ph | 4'-NO₂-Ph-CH₂ | Me | OEt | Piperazin-1-yl |
| 3,4-(MeO)₂-Ph | H | Me | *n*Pr | Piperazin-1-yl |
| Ph | H | Me | OCH₂CH₂O H | Piperazin-1-yl |
| Ph | H | Me | OEt | Piperazin-1-yl |
| Ph | H | Me | O*i*Pr | Piperazin-1-yl |
| 3,4-Methylen-dioxyphenyl | H | Me | OEt | Piperazin-1-yl |
| 4-(Pyrimidyl-2-yl)piperazin-1-yl)- | H | Et | Piperazin-1-yl | OEt |
| 4-Me-Ph | H | Et | Piperazin-1-yl | OEt |
| 4-EtO-Ph | H | Et | Piperazin-1-yl | OEt |
| 4-EtO-Ph | H | Et | Piperazin-1-yl | OProp |
| 4-MeS-Ph | H | Et | Piperazin-1-yl | OEt |
| 4-Me-Ph | H | Et | Piperazin-1-yl | O*i*Pr |
| 4-(4-MeOPhO)Ph | H | Et | Piperazin-1-yl | OEt |
| 2,3-Dihydro-benzo[b][1,4]-dioxin-6-yl | H | Et | Piperazin-1-yl | OEt |
| 3,4-Methylen-dioxyphenyl | H | Et | Piperazin-1-yl | OEt |
| 4-MeO-Ph | H | Et | Piperazin-1-yl | OEt |
| 4-(1H-pyrazol-1-yl)-Ph | H | Et | Piperazin-1-yl | OEt |
| 4-Br-Ph | H | Et | Piperazin-1-yl | OEt |
| 4-Cl-Ph | H | Et | Piperazin-1-yl | OEt |
| 4-I-Ph | H | Et | Piperazin-1-yl | OEt |
| Morpholin | H | Ph | Piperazin-1-yl | OEt |
| 4-MeO-Ph | H | CF₃ | Piperazin-1-yl | OEt |
| 4-Me-Ph | H | CF₃ | Piperazin-1-yl | OEt |
| 2,3-Dihydro-benzo[b][1,4]-dioxin-6-yl | H | CF₃ | Piperazin-1-yl | OEt |
| 4-Imidazol-1-yl-Ph | H | CF₃ | Piperazin-1-yl | OEt |
| 4-Imidazol-1-yl-Ph | H | CF₃ | Piperazin-1-yl | OCH₂CH₂O H |
| 4-(Piperidin-1-yl)-Ph | H | CF₃ | Piperazin-1-yl | OEt |
| 4-Morpholino-Ph | H | CF₃ | Piperazin-1-yl | OEt |
| 3,4-Methylen-dioxyphenyl | H | CF₃ | Piperazin-1-yl | OEt |
| 4-MeS-Ph | H | CF₃ | Piperazin-1-yl | OEt |
| 4-EtOOC-Ph | H | CF₃ | Piperazin-1-yl | OEt |
| 4-(1H-pyrazol-1-yl)-Ph | H | CF₃ | Piperazin-1-yl | OEt |
| 4-F-Ph | H | CF₃ | Piperazin-1-yl | OEt |
| 4-I-Ph | H | CF₃ | Piperazin-1-yl | OEt |
| 3-NO₂-Ph | H | CF₃ | Piperazin-1-yl | OEt |
| 4-NO₂-Ph | H | CF₃ | Piperazin-1-yl | OEt |
| 3-CN-Ph | H | CF₃ | Piperazin-1-yl | OEt |
| 4-CN-Ph | H | CF₃ | Piperazin-1-yl | OEt |
| 3-NH₂-Ph | H | CF₃ | Piperazin-1-yl | OEt |
| 4-NH₂-Ph | H | CF₃ | Piperazin-1-yl | OEt |
| 4-MeS-Ph | H | CF₃ | Piperazin-1-yl | OEt |
| 4-MeS(O)-Ph | H | CF₃ | Piperazin-1-yl | OEt |
| Pyridin-4-yl | H | CF₃ | Piperazin-1-yl | OEt |
| Pyridin-4-yl-N-oxid | H | CF₃ | Piperazin-1-yl | OEt |
| Pyridin-3-yl | H | CF₃ | Piperazin-1-yl | OEt |
| 4-(pyrazin-2-yl) | H | CF₃ | Piperazin-1-yl | OEt |
| 4(HCONH)-Ph | H | CF₃ | Piperazin-1-yl | OEt |
| 4-(Pyridin-4-yl-CONH)-Ph | H | CF₃ | Piperazin-1-yl | OEt |
| 4-(Pyridin-3-yl-CONH)-Ph | H | CF₃ | Piperazin-1-yl | OEt |
| 4-(Pyridin-3-yl-N-Oxid-CONH)-Ph | H | CF₃ | Piperazin-1-yl | OEt |
| 4-(1H-1,2,4-triazol-4-yl)-Ph | H | CF₃ | Piperazin-1-yl | OEt |
| 4-(1H-pyrrol-1-yl)-Ph | H | CF₃ | Piperazin-1-yl | OEt |
| 4(CH₃OOCNH )-Ph | H | CF₃ | Piperazin-1-yl | OEt |
| *iso*Pr | H | *iso*Pr | Piperazin-1-yl | OEt |
| 2,3-Dihydro-benzo[b][1,4]-dioxin-6-yl | H | Et | OEt | Piperazin-1-yl |
| 4-EtO-Ph | H | Et | OEt | Piperazin-1-yl |
| 4-Me-Ph | H | Et | OEt | Piperazin-1-yl |
| 4-Me-Ph | H | Et | OCH₂CH₂O H | Piperazin-1-yl |

- Formel 1 (a), wobei Y = O und die Substituenten R¹, R², R³, R⁴, R⁵ die folgenden Bedeutungen haben:

| R₁ | R₂ | R₃ | R₄ | R₅ |
|---|---|---|---|---|
| Ph | H | Me | Piperazin-1-yl | OEt |
| Ph | H | Me | OEt | Piperazin-1-yl |

- Formel 1(b), wobei Y = S und die Substituenten R¹, R³, R⁴, R⁵ die folgenden Bedeutungen haben:

| R₁ | R₃ | R₄ | R₅ |
|---|---|---|---|
| 3-NH₂-Ph-4-Ph | Me | Piperazin-1 -yl | OEt |
| 4-(3-furyl)-Ph | Me | Piperazin-1-yl | OEt |
| 4-(2-furyl)-Ph | Me | Piperazin-1-yl | OEt |
| 4-(3-pyridyl)-Ph | Me | Piperazin-1-yl | OEt |
| 4-(4-pyridyl)-Ph | Me | Piperazin-1-yl | OEt |
| 4-NH₂-Ph-Ph | Me | Piperazin-1-yl | OEt |
| 3,4,5-(CH₃O)₃-Ph-Ph | Me | Piperazin-1-yl | OEt |
| 4-PhO-Ph | Me | Piperazin-1-yl | OEt |
| 2-NH₂-4-CH₃OOC-Ph-Ph | Me | Piperazin-1-yl | OEt |
| 3-CH₃OOC-Ph-Ph | Me | Piperazin-1-yl | OEt |
| 3,4-(CH₃O)₂-Ph-Ph | Me | Piperazin-1-yl | OEt |
| 3,5-(CH₃)₂-isoxazol-4-yl-Ph | Me | Piperazin-1-yl | OEt |
| Phenyl | Me | Piperazin-1-yl | OEt |
| 4-Br-Ph | Me | Piperazin-1-yl | OEt |
| 3-NH₂-Ph | Me | Piperazin-1-yl | OEt |
| 4-(pyrrol-3-yl)-Ph | Me | Piperazin-1-yl | OEt |
| 4-(pyrimidin-5-yl)-Ph | Me | Piperazin-1-yl | OEt |
| 4-(pyrid-3-yl-hydroxymethyl)-Ph | Me | Piperazin-1-yl | OEt |
| Ph-Ph | Me | Piperazin-1yl | OEt |
| 4-(*iso*PropO)-Ph | Me | Piperazin-1yl | OEt |
| 4-Vinyl-Ph | Me | Piperazin-1yl | OEt |

- Formel 1(a) oder 1(b), wobei diese explizit sind:
2-(2-Hydroxyethoxy)-9-methyl-7-phenyl-4-(piperazin-1-yl)-pyrido[3',2':4,5]thieno[3,2-d]pyrimidin;
2-Ethoxy-7-(3,4-dimethoxy-phenyl)-9-methyl-4-(piperazin-1-yl)-pyrido[3',2':4,5]thieno[3,2-d]pyrimidin;
2-Ethoxy-7-(4-methoxy-phenyl)-9-methyl-4-(piperazin-1-yl)-pyrido[3',2':4,5]thieno[3,2-d]pyrimidin;
2-Ethoxy-9-ethyl-7-(4-methyl-phenyl)-4-(piperazin-1-yl)-pyrido[3',2':4,5]thieno[3,2-d]pyrimidin
2-Ethoxy-7-(4-ethoxy-phenyl)-9-ethyl-4-(piperazin-1-yl)-pyrido[3',2':4,5]thieno[3,2-d]pyrimidin
2-Ethoxy-9-ethyl-7-(2,3-dihydrobenzo[b][1,4]dioxin-6-yl)-4-(piperazin-1-yl)-pyrido[3',2':4,5] thieno[3,2-d]pyrimidin
4-Ethoxy-9-trifluormethyl-7-(pyrazin-2-yl)-2-piperazino-pyrido[3',2':4,5]thieno[3,2-d]pyrimidin
4-Ethoxy-9-ethyl-7-(4-(pyrimidyl-2-yl)piperazin-1-yl)-2-piperazin-1-yl-pyrido[3',2':4,5]thieno[3,2-d]pyrimidin
4-Ethoxy-7-morpholino-9-phenyl-2-(piperazin-1-yl)-pyrido[3',2':4,5]thieno[3,2-d]pyrimidin;
4-Ethoxy-7-phenyl-9-methyl-2-(piperazin-1-yl)-pyrido[3',2':4,5]furo[3,2-d]pyrimidin;
4-Ethoxy-7,9-dimethyl-8-(4-nitro-benzyl)-2-(piperazin-1-yl)-pyrido[3',2':4,5]thieno[3,2-d]pyrimidin;
4-Ethoxy-7-(3,4-dimethoxy-phenyl)-9-methyl-2-(piperazin-1-yl)-pyrido[3',2':4,5]thieno[3,2-d]pyrimidin;
4-Ethoxy-9-ethyl-7-(4-methyl-phenyl)-2-(piperazin-1-yl)-pyrido[3',2':4,5]thieno[3,2-d]pyrimidin
4-Ethoxy-7-(4-fluorphenyl)-9-methyl-2-(piperazin-1-yl)-pyrido[3',2':4,5]furo[3,2-d]pyrimidin
7-(4-Ethoxy-phenyl)-9-ethyl-2-(piperazin-1-yl)-4-*n*-propoxy-pyrido[3',2':4,5]thieno[3,2-d]pyrimidin
4-Ethoxy-7-(4-ethoxy-phenyl)-9-ethyl-2-(piperazin-1-yl)-pyrido[3',2':4,5]thieno[3,2-d]pyrimidin
4-Ethoxy-7-(4-(pyrimidin-2-yl)piperazin-1-yl)-9-ethyl-2-(piperazin-1-yl)-pyrido[3',2':4,5]thieno[3,2-d]pyrimidin
4-Ethoxy-9-ethyl-7-(4-methylthio-phenyl)-2-(piperazin-1-yl)-pyrido[3',2':4,5]thieno[3,2-d]pyrimidin
4-Ethoxy-7-(4-methyl-phenyl)-9-*iso*-propyl-2-(piperazin-1-yl)-pyrido[3',2':4,5]thieno-[3,2-d]pyrimidin
4-Ethoxy-9-ethyl-7-[4-(4-methoxyphenoxy)phenyl]-2-(piperazin-1-yl)-pyrido[3',2':4,5] thieno[3,2-d]pyrimidin
4-Ethoxy-9-ethyl-7-(2,3-dihydrobenzo[b][1,4]dioxin-6-yl)-2-(piperazin-1-yl)-pyrido[3',2':4,5] thieno[3,2-d]pyrimidin
4-Ethoxy-9-ethyl-7-(3,4-methylendioxyphenyl)-2-piperazin-1-yl-pyrido[3',2':4,5]thieno-[3,2-d]pyrimidin
4-Ethoxy-7-(4-methoxy-phenyl)-2-(piperazin-1-yl)-9-trifluormethyl-pyrido[3',2':4,5]thieno[3,2-d]pyrimidin
4-Ethoxy-7-(4-methyl-phenyl)-2-(piperazin-1-yl)-9-trifluormethyl-pyrido[3',2':4,5]thieno[3,2-d]pyrimidin
7-(2,3-Dihydrobenzo[b][1,4]dioxin-6-yl)-4-ethoxy-2-(piperazin-1-yl)-9-trifluormethyl-pyrido[3',2':4,5] thieno[3,2-d]pyrimidin
4-Ethoxy-7-(4-(1*H*-imidazol-1-yl)phenyl)-2-(piperazin-1-yl)-9-trifluormethyl-pyrido[3',2':4,5]thieno[3,2-d]pyrimidin
4-[4-Ethoxy-2-(piperazin-1-yl)-9-trifluormethyl-pyrido[3',2':4,5]thieno[3,2-d]pyrimidin-7-yl]-benzoesäureethylester
4-Ethoxy-2-(piperazin-1-yl)-7-(4-(piperidin-1-yl)phenyl)-9-trifluormethyl-pyrido[3',2':4,5]thieno[3,2-d]pyrimidin
4-Ethoxy-9-ethyl-7-(4-methoxyphenyl)-2-(piperazin-1-yl)-pyrido[3',2':4,5]thieno[3,2-d]pyrimidin
4-Ethoxy-7-(3,4-methylendioxyphenyl)-2-piperazin-1-yl-9-trifluormethyl-pyrido[3',2':4,5]thieno-[3,2-d]pyrimidin
4-Ethoxy-7-(4-methylthiophenyl)-2-(piperazin-1-yl)-9-trifluormethyl-pyrido[3',2':4,5]thieno[3,2-d]pyrimidin
4-Ethoxy-7-(4-(1H-pyrazol-1-yl)-phenyl)-9-ethyl-2-piperazin-1-yl-pyrido[3',2':4,5]thieno[3,2-d]pyrimidin
4-Ethoxy-7-(4-iod-phenyl)-2-(piperazin-1-yl)-9-trifluormethyl-pyrido[3',2':4,5]thieno[3,2-d]pyrimidin
4-Ethoxy-7-(4-(1H-pyrazol-1-yl)-phenyl)-2-piperazin-1-yl-9-trifluormethyl-pyrido[3',2':4,5]thieno[3,2-d]pyrimidin
7-(4-Brom-phenyl)-4-ethoxy-9-ethyl-2-(piperazin-1-yl)-pyrido[3',2':4,5]thieno[3,2-d]pyrimidin
4-Ethoxy-9-ethyl-7-(4-iod-phenyl)-2-(piperazin-1-yl)-pyrido[3',2':4,5]thieno[3,2-d]pyrimidin
4-Ethoxy-7-(3-nitro-phenyl)-2-piperazin-1-yl-9-triluormethyl-pyrido[3',2':4,5]thieno[3,2-d]pyrimidin
7-(3-Cyano-phenyl)-4-ethoxy-2-piperazin-1-yl-9-trifluormethyl-pyrido[3',2':4,5]thieno[3,2-d]pyrimidin
7-(4-Cyano-phenyl)-4-ethoxy-2-piperazin-1-yl-9-trifluormethyl-pyrido[3',2':4,5]thieno[3,2-d]pyrimidin
7-(3-Amino-phenyl)-4-ethoxy-2-piperazin-1-yl-9-trifluormethyl-pyrido[3',2':4,5]thieno[3,2-d]pyrimidin
7-(4-Amino-phenyl)-4-ethoxy-2-piperazin-1-yl-9-trifluormethyl-pyrido[3',2':4,5]thieno[3,2-d]pyrimidin
4-Ethoxy-9-methyl-7-(4-methylsulfinyl-phenyl)-2-(piperazin-1-yl)-pyrido[3',2':4,5]thieno[3,2-d]pyrimidin
N-(4-(4-Ethoxy-9-trifluormethyl-2-piperazin-1-yl-pyrido[3',2':4,5]thieno[3,2-d]pyrimidin-7-yl)-phenyl)-formamid
N-(4-(4-Ethoxy-9-trifluormethyl-2-piperazin-1-yl-pyrido[3',2':4,5]thieno[3,2-d]pyrimidin-7-yl)-phenyl)-isonicotinamid
4-Ethoxy-9-trifluormethyl-2-piperazin-1-yl-7-(4-(1H-pyrrol-1-yl)-phenyl)-pyrido[3',2':4,5]thieno[3,2-d]pyrimidin
N-(4-(4-Ethoxy-9-trifluormethyl-2-piperazin-1-yl-pyrido[3',2':4,5]thieno[3,2-d]pyrimidin-7-yl)-phenyl)-nicotinamid-N-oxid
4-(4-Ethoxy-9-trifluormethyl-2-piperazin-1-yl-pyrido[3',2':4,5]thieno[3,2-d]pyrimidin-7-yl)-phenyl-carbaminsäuremethylester
N-(4-(4-Ethoxy-9-trifluormethyl-2-piperazin-1-yl-pyrido[3',2':4,5]thieno[3,2-d]pyrimidin-7-yl)-phenyl)-nicotinamid
N-(4-Ethoxy-9-trifluormethyl-2-piperazin-1-yl-pyrido[3',2':4,5]thieno[3,2-d]pyrimidin-7-yl)-benzamid Trifluoracetat
N'-Hydroxy-4-(4-ethoxy-9-trifluormethyl-2-piperazin-1-yl-pyrido[3',2':4,5]thieno[3,2-d]pyrimidin-7-yl)-benzamidin Bistrifluoracetat
7-(3'-Aminobiphen-4-yl)-4-ethoxy-9-methyl-2-piperazin-1-yl-thieno[2,3-d:4,5-d']dipyrimidin
4-Ethoxy-7-(4-[3-furyl]-phenyl)-9-methyl-2-piperazin-1-yl-thieno[2,3-d:4,5-d']dipyrimidin
4-Ethoxy-7-(4-[2-furyl]-phenyl)-9-methyl-2-piperazin-1-yl-thieno[2,3-d:4,5-d']dipyrimidin
4-Ethoxy-9-methyl-2-piperazin-1-yl-7-(4-[3-pyridyl]-phenyl)-thieno[2,3-d:4,5-d']dipyrimidin
4-Ethoxy-9-methyl-2-piperazin-1-yl-7-(4-[4-pyridyl]-phenyl)-thieno[2,3-d:4,5-d']dipyrimidin
7-(4'-Aminobiphen-4-yl)-4-ethoxy-9-methyl-2-piperazin-1-yl-thieno[2,3-d:4,5-d']dipyrimidin
4-Ethoxy-9-methyl-2-piperazin-1-yl-7-(3',4',5'-trimethoxybiphen-4-yl)-thieno[2,3-d:4,5-d']dipyrimidin
4-Ethoxy-9-methyl-7-(4-phenoxy-phenyl)-2-piperazin-1-yl-thieno[2,3-d:4,5-d']dipyrimidin
2'-Amino-4-(4-ethoxy-9-methyl-2-piperazin-1-yl -thieno[2,3-d:4,5-d']dipyrimidin-7-yl)-biphenyl-4'-carbonsäuremethylester
7-(3',4'-Dimethoxybiphen-4-yl)-4-ethoxy-9-methyl-2-piperazin-1-yl-thieno[2,3-d:4,5-d']dipyrimidin

- Formel 1(c) oder 1(d), wobei diese explizit sind:
10-Ethoxy-5-morpholino-8-piperazin-1-yl-pyrimido[4',5':4,5]thieno[2,3-c]-1,2,3,4-tetrahydro-isochinolin
7-Ethoxy-2,3-dihydro-4-morpholino-9-piperazin-1yl-1H-cyclopenta[4',5']pyrido[3',2':4,5]thieno[3,2-d]pyrimidin
8-Ethoxy-5-piperidino-10-piperazin-1-yl-pyrimido[4',5':4,5]thieno[2,3-c]-1,2,3,4-tetrahydro-isochinolin-hydrochlorid
8-Ethoxy-5-morpholino-10-piperazin-1-yl-pyrimido[4',5':4,5]thieno[2,3-c]-1,2,3,4-tetrahydro-isochinolin-hydrochlorid
8-Ethoxy-5-phenyl-10-piperazin-1-yl-pyrimido[4',5':4,5]thieno[2,3-c]-1,2,3,4-tetrahydro-isochinolin-hydrochlorid
10-Ethoxy-5-phenyl-8-piperazin-1-yl-pyrimido[4',5':4,5]thieno[2,3-c]-1,2,3,4-tetrahydro-isochinolin
8-Ethoxy-5-morpholino-10-piperazin-1-yl-pyrimido[4',5':4,5]thieno[2,3-c]-1,2,3,4-tetrahydro-isochinolin
7-Ethoxy-2,3,4,5-tetrahydro-4-morpholino-9-piperazin-1yl-1H-cyclohepta[4',5']pyrido[3',2':4,5]thieno[3,2-d]pyrimidin

Die Erfindung wird weiter anhand der nachfolgenden Beispiele beschrieben.

### Beispiele:

### Beispiel 1

### Die Synthese von 4-Ethoxy-9-methyl-7-(4-iso-propoxy-phenyl)-2-piperazin-1-yl-thieno[2,3-d:4,5-d']dipyrimidin

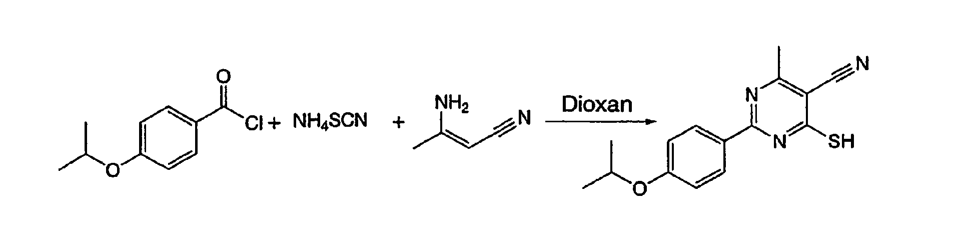

5.5 g (28 mmol) 4-*iso*-Propoxybenzoylchlorid und 2.5 g (33 mmol) Ammoniumthiocyanat werden in 100 ml Dioxan suspendiert und 15 min unter Rückfluss erhitzt. Anschließend werden 3.0 g (36 mmol) (E/Z)-3-Amino-crotonsäurenitril zugegeben. Das Reaktionsgemisch wird 3 h unter Rückfluss erhitzt. Danach wird das Gemisch mit 500 ml Eiswasser gemischt und über Nacht stehen gelassen, anschließend filtriert und getrocknet. Man erhält 7.3 g (92 %) 6-Mercapto-4-methyl-2-(4-*iso*-propoxy-phenyl)-pyrimidin-5-carbonitril.

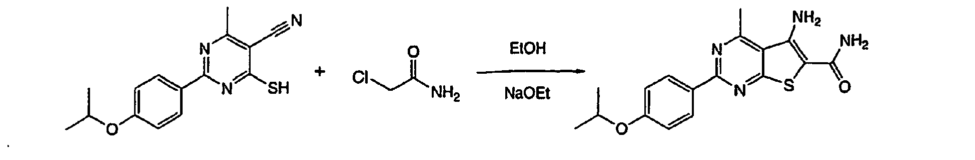

4.0 g (14 mmol) 6-Mercapto-4-methyl-2-(4-*iso*-propoxy-phenyl)-pyrimidin-5-carbonitril und 1.6 g (17 mmol) 2-Chloracetamid werden in 100 ml Ethanol suspendiert und mit 1.9 g (28 mmol) Natriumethylat versetzt. Das Reaktionsgemisch wird 3 h unter Rückfluss erhitzt. Es wird filtriert, mit Ethanol und Wasser gewaschen und getrocknet. Man erhält 1.5 g (31 %) 5-Amino-4-methyl-2-(4-*iso*-propoxy-phenyl)-thieno[2,3-d]pyrimidin-6-carbonsäureamid.

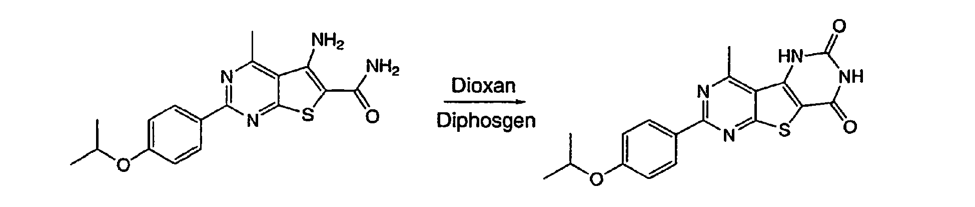

1.0 g (3 mmol) 5-Amino-4-methyl-2-(4-*iso*-propoxy-phenyl)-thieno[2,3-d]pyrimidin-6-carbonsäureamid warden in 200 ml Dioxan suspendiert, nach Zugabe von 0.73 ml (6 mmol) Diphosgen wird 2 h unter Rückfluß erhitzt. Nach dem Abkühlen auf Raumtemperatur wurden vorsichtig 20 ml Wasser zugegeben und im Anschluss der Niederschlag abgesaugt. Man erhält 0.8 g (68 %) 9-Methyl-7-(4-*iso*-propoxy-phenyl)-thieno[2,3-d:4,5-d']dipyrimidin-2,4(1*H*,3*H*)-dion.

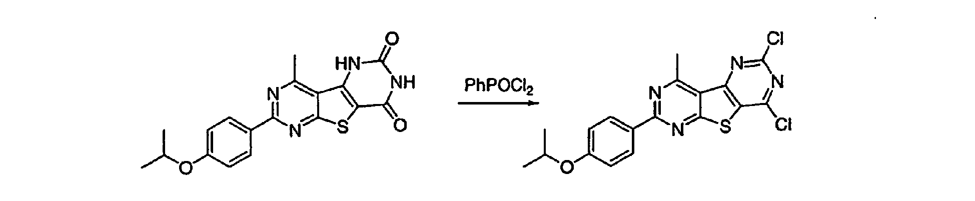

1.0 g (2.7 mmol) 9-Methyl-7-(4-*iso*-propoxy-phenyl)-thieno[2,3-d:4,5-d']dipyrimidin-2,4(1*H*,3*H*)-dion und 2.3 ml (16.3 mmol) Dichlorphenylphosphinoxid werden 6 h auf 185 °C erhitzt. Nach Abkühlung auf RT wird auf 40 g Eiswasser gegossen und mit gesättigter Natriumhydrogencarbonat-Lösung neutralisiert. Der Niederschlag wird abgesaugt, mit 300 ml Wasser gewaschen und getrocknet. Man erhält 0.9 g (82 %) 2,4-Dichlor-9-methyl-7-(4-*iso*-propoxy-phenyl)-thieno[2,3-d:4,5-d']dipyrimidin.

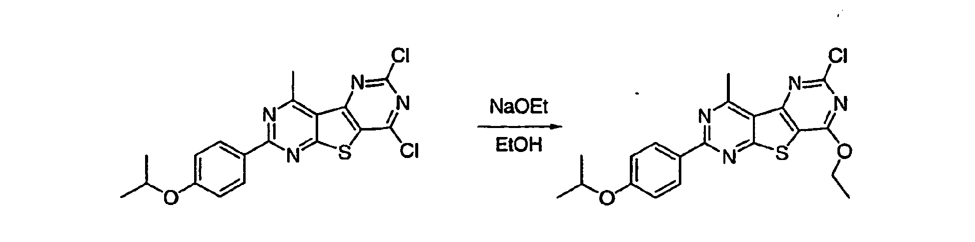

0.9 g (2.2 mmol) 2,4-Dichlor-9-methyl-7-(4-*iso*-propoxy-phenyl)-thieno[2,3-d:4,5-d']dipyrimidin werden in 50 ml Ethanol suspendiert und mit 0.46 g (6.7 mol) Natriumethylat versetzt. Das Gemisch wird 4 h bei RT gerührt. Anschließend wird der Niederschlag abgesaugt, mit Ethanol und Wasser gewaschen und getrocknet. Man erhält nach Flash-Chromatographie (CH₂Cl₂) 0.47 g (51 %) 2-Chlor-4-ethoxy-9-methyl-7-(4-*iso*-propoxy-phenyl)-thieno[2,3-d:4,5-d']dipyrimidin.

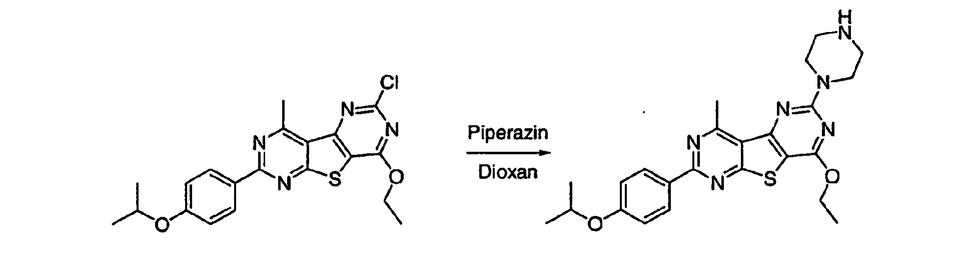

0.1 g (0.24 mmol) 2-Chlor-4-ethoxy-9-methyl-7-(4-*iso*-propoxy-phenyl)-thieno[2,3-d:4,5-d']dipyrimidin und 83 mg (0.96 mmol) Piperazin werden in 40 ml Dioxan 4 h unter Rückfluss erhitzt. Anschließend wird das Lösungsmittel entfernt und der Rückstand in Wasser suspendiert und abgesaugt. Nach Flash-Chromatographie (Chloroform/Ethanol 10:2) erhält man 20 mg (18 %) der Titelsubstanz.

Die nach Beispiel 1 dargestellten Verbindungen sind in Tabelle 1 zusammengefasst

**Tabelle 1**

| Struktur | M [g/mol] | MS (ESI) [m/z] | Smp. [°C] | Reinheit [%] |
|---|---|---|---|---|
| 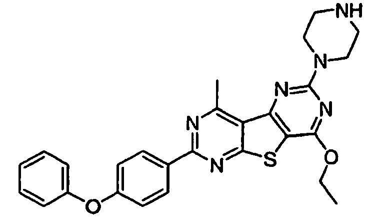 | 498.60 | 499 [M+H]⁺ | 188 - 189 | >99 |
| 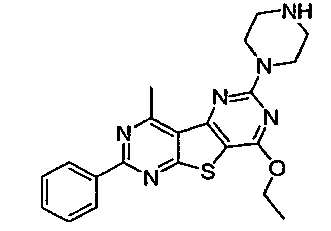 | 406.50 | 407 [M+H]⁺ | 195 | 98 |
| 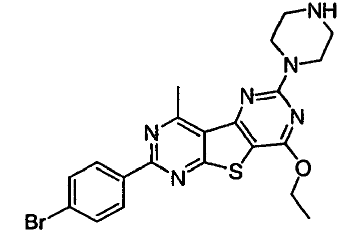 | 485.40 | 485, 487 [M+H]⁺ | 210 - 211 | 97 |
| 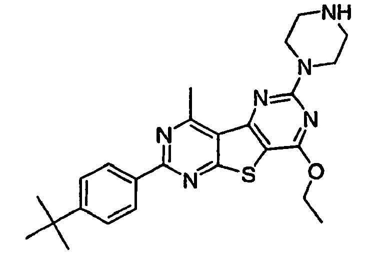 | 562.61 | 563 [M+H]⁺ | 144 | >99 |
| 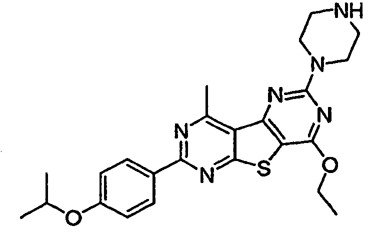 | 464.58 | 465 [M+H]⁺ | 194 - 195 | 98 |
| 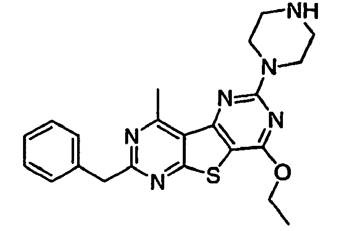 | 420.53 | 421 [M+H]⁺ | 170 - 171 | 98 |
| 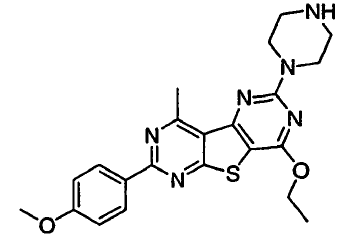 | 436.53 | 437 [M+H]⁺ | 179 | >99 |
| 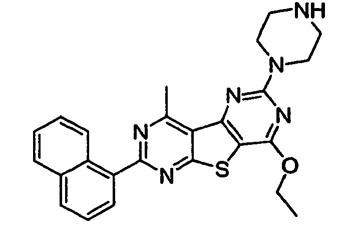 | 456.56 | 457 [M+H]⁺ | 148 - 149 | 98 |
| 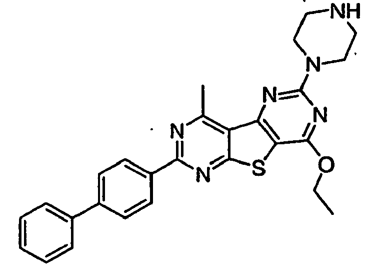 | 482.60 | 483 [M+H]⁺ | 217 | >99 |
| 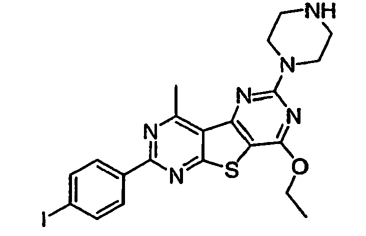 | 532.40 | 533 [M+H]⁺ | 273- 276 | >99 |

### Beispiel 2

### Die Synthese von 2-Ethoxy-9-methyl-4-piperazin-1-yl-7-phenyl-thieno[2,3-d:4,5-d']dipyrimidin

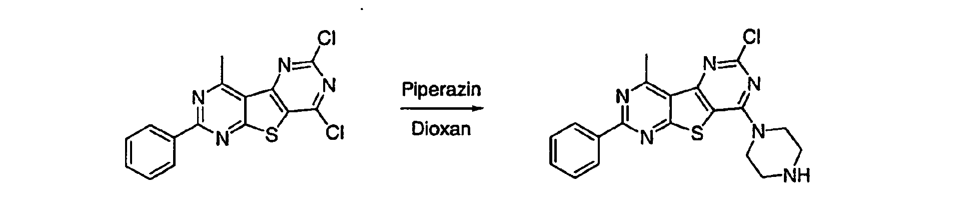

3.0 g (8.6 mmol) 2,4-Dichlor-9-methyl-7-phenyl-thieno[2,3-d:4,5-d']dipyrimidin und 2.0 g (23.2 mmol) Piperazin werden in 40 ml Tetrahydrofuran 6 h bei RT gerührt. Anschließend wird das Lösungsmittel entfernt, der Rückstand in Wasser suspendiert, abgesaugt, mit 40 ml Wasser gewaschen und getrocknet. Man erhält 1.5 g (44 %) 2-Chlor-9-methyl-4-piperazin-1-yl-7-phenyl-thieno[2,3-d:4,5-d']dipyrimidin.

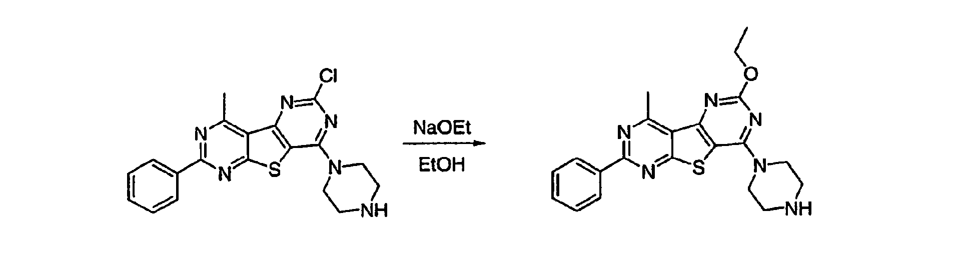

1.30 g (3.3 mmol) 2-Chlor-9-methyl-4-piperazin-1-yl-7-phenyl-thieno[2,3-d:4,5-d']di-pyrimidin und 0.67 g (9.9 mmol) Natriumethylat werden in 30 ml Ethanol 3 h unter Rückfluss erhitzt. Anschließend wird das Lösungsmittel entfernt und der Rückstand in wenig Wasser suspendiert. Die wässrige Suspension wird mit Dichlormethan extrahiert und das Lösungsmittel entfernt. Man erhält 0.90 g (68%) der Titelsubstanz. ESI-MS [m/z]: 407, Smp.: >310 °C

### Beispiel 3

### Die Synthese von 7-(4-[3'-Aminobiphenyl)-4-ethoxy-9-methyl-2-piperazin-1-yl-thieno[2,3-d:4,5-d']dipyrimidin

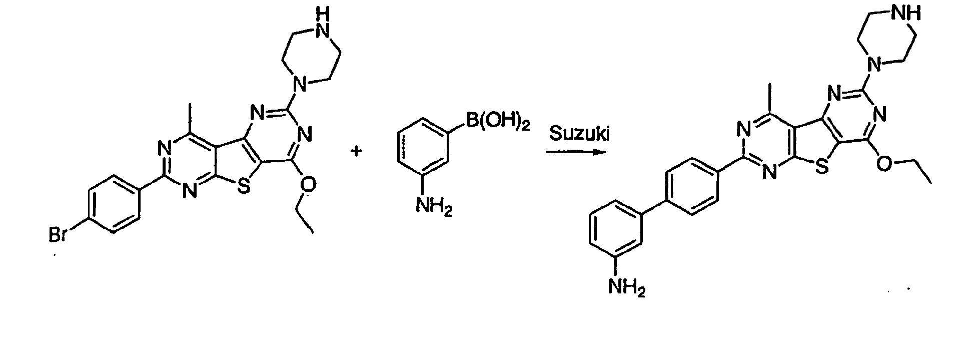

0.20 g (0.41 mmol) 7-(4-Bromphenyl)-4-ethoxy-9-methyl-2-piperazin-1-yl-thieno[2,3-d:4,5-d']dipyrimidin (analog Beispiel 1 dargestellt), 0.09 g (0.67 mmol) 3-Aminophenylboronsäure und 1 ml 1 M Cs₂CO₃ wurden in 50 ml Isopropanol/Toluol (1:4) suspendiert, mit Stickstoff 15 min entgast. Anschließen wurde unter Stickstoffatmosphäre mit 10 mg Tetrakis(triphenyl)phosphin)palladium versetzt und mittels Mikrowelle (130 W) 3 h bei 100 °C gehalten. Die flashchromatographische Reinigung (Chloroform/Methanol, 8.5:1.5) lieferte 150 mg (73%) der Titelsubstanz.

Folgende Verbindungen wurden nach Beispiel 3 erhalten (Tabelle 2)

**Tabelle2**

| Struktur | M [g/mol] | MS (ESI) [m/z] | Smp. [°C] | Reinheit [%] |
|---|---|---|---|---|
| 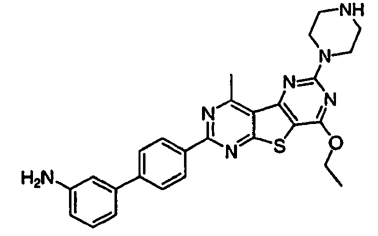 | 497.61 | 498 [M+H]⁺ | 225 | >99 |
| 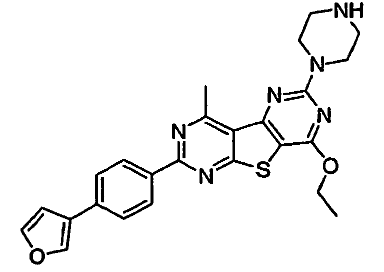 | 472.56 | 473 [M+H]⁺ | 250 - 251 | >99 |
| 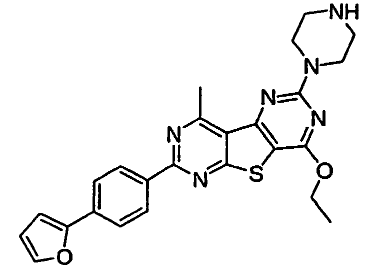 | 472.56 | 473 [M+H]⁺ | 220 - 221 | 98 |
| 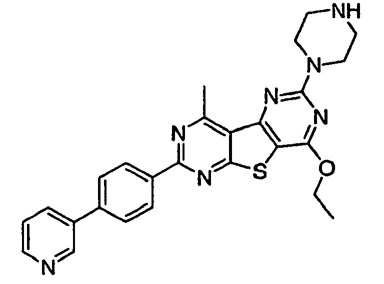 | 483.59 | 484 [M+H]⁺ 242 [M+2H]²⁺ | >300 | >99 |
| 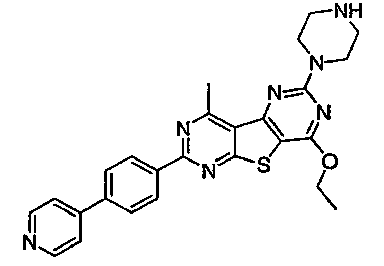 | 483.59 | 484 [M+H]⁺ 242 [M+2H]²⁺ | 227 | >99 |
| 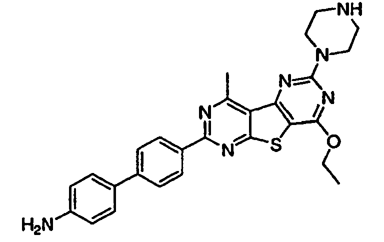 | 497.61 | 498 [M+H]⁺ 250 [M+2H]²⁺ | >300 | >99 |
| 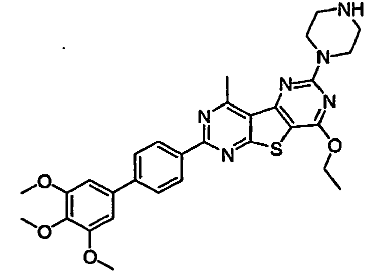 | 572.68 | 573 [M+H]⁺ | n.d. | >99 |
| 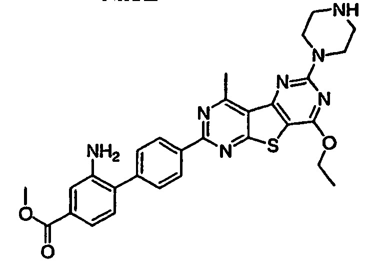 | 555.65 | 556 [M+H]⁺ 279 [M+2H]²⁺ | 208 - 209 | 98 |
| 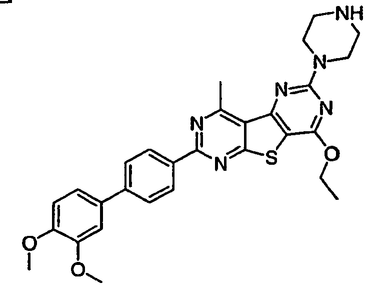 | 542.65 | 543 [M+H]⁺ | 210 - 211 | 97 |
| 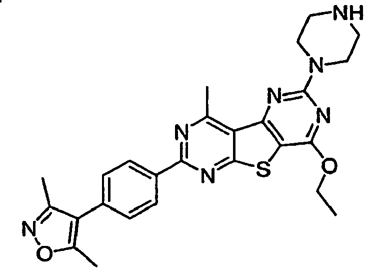 | 501.60 | 502 [M+H]⁺ | n.d. | >99 |
| 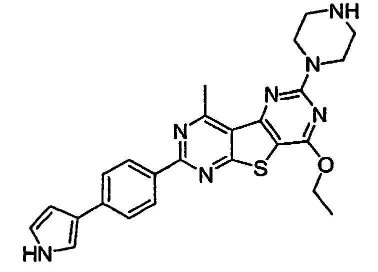 | 471.58 | 472 [M+H]⁺ | >300 | 98 |
| 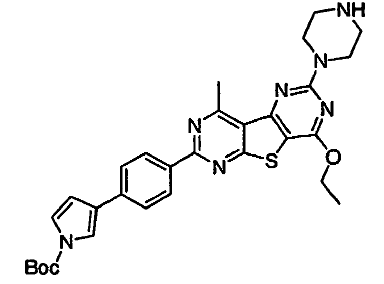 | 571.69 | 572 [M+H]⁺ | 179 | >99 |
| 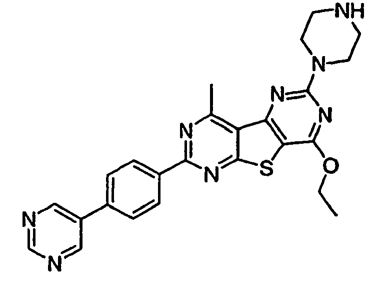 | 484.58 | 485 [M+H]⁺ 243 [M+2H]²⁺ | 246 | >99 |
| 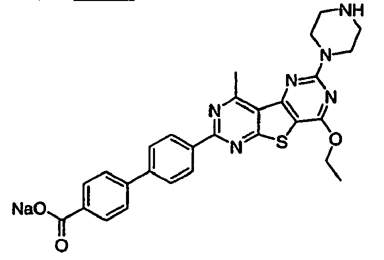 | 526.61 | 527 [M+H]⁺ | 267- 269 | 95 |
| 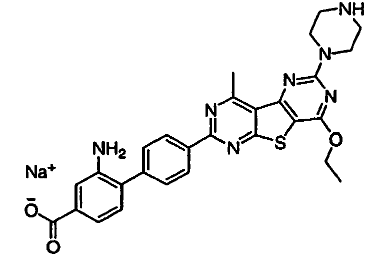 | 563.61 | 542 [M+H]⁺ | >300 | 98 |
| 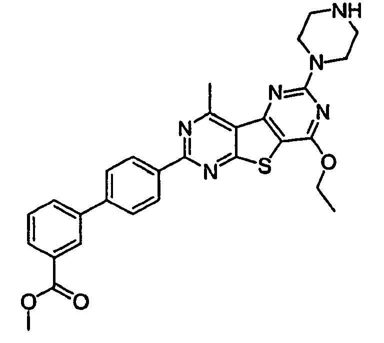 | 540.64 | 541 [M+H]⁺ | 239 | >99 |
| 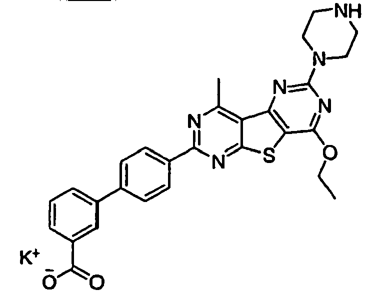 | 564.70 | 527 [M+H]⁺ | >300 | >99 |
| 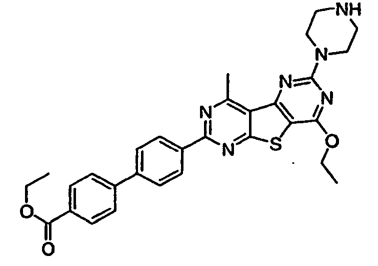 | 554.66 | 555 [M+H]⁺ | 211 | >99 |

### Beispiel 4

### Die Synthese von 4-Ethoxy-9-methyl-2-piperazin-1-yl-7-(4-vinylphenyl)-thieno[2,3-d:4,5-d']dipyrimidin

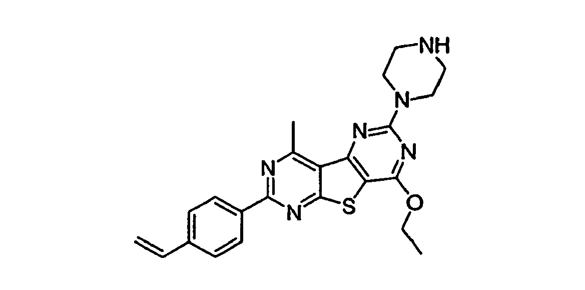

Die Titelsubstanz wurde durch die Kupplung von 7-(4-Bromphenyl)-4-ethoxy-9-methyl-2-piperazin-1-yl-thieno[2,3-d:4,5-d']dipyrimidin mit 2,4,6-Trivinylcyclotriboroxan-pyridin-Komplex analog Beispiel 3 hergestellt. ESI-MS [m/z]: 433, Smp.: 165 - 166 °C

### Beispiel 5

### Die Synthese von 7-(4-[1,2-Dihydroxyethyl]-phenyl)-4-ethoxy-9-methyl-2-piperazin-1-yl-thieno[2,3-d:4,5-d']dipyrimidin

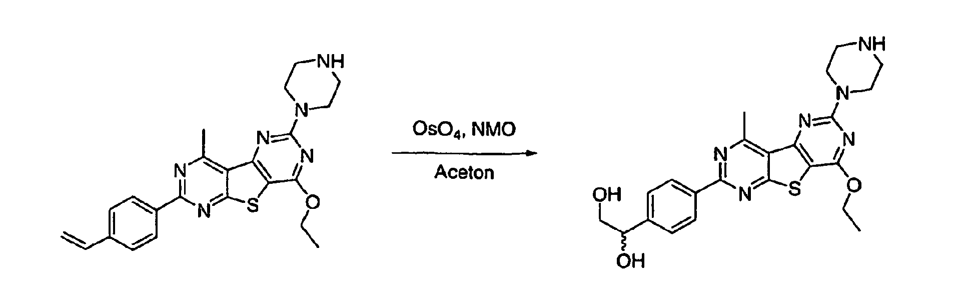

0.20 g (0.46 mmol) 4-Ethoxy-9-methyl-2-piperazin-1-yl-7-(4-vinylphenyl)-thieno[2,3-d:4,5-d']dipyrimidin und 0.16 g (1.39 mmol) N-Methylmorpholin-N-oxid-Monohydrat wurden in 50 ml Aceton gelöst. Anschließen wurden 0.3 ml (4% wässrige) OsO₄-Lösung zugegeben und 24 h bei Raumtemperatur gerührt. Nachfolgend wurden 100 ml 1 M NaCO₃-Lösung zugegeben und mit Dichlormethan extrahiert. Nach flashchromatographischer Reinigung (Chloroform/Methanol, 8.5:1.5) erhält man 100 mg (46%) der Titelverbindung. ESI-MS [m/z]: 466, Smp.: >300 °C

### Beispiel 6

### Die Synthese von 4-Ethoxy-7-(4-[{2E,4E}-1-hydroxyhexa-2,4-dienyl]phenyl)-9-methyl-2-piperazin-1-yl-thieno[2,3-d:4,5-d']dipyrimidin

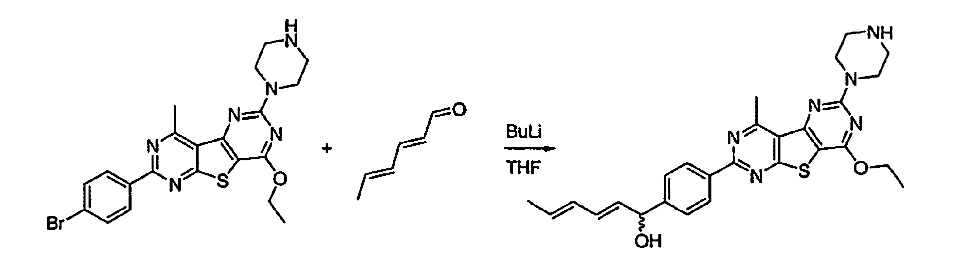

200 mg (0.4 mmol) 7-(4-Bromphenyl)-4-ethoxy-9-methyl-2-piperazin-1-yl-thieno[2,3-d:4,5-d']dipyrimidin wurden bei -80 °C in 50 ml THF mit 0.48 ml (1.2 mmol) 2.5 M BuLi-Lösung versetzt. Nach 15 min bei dieser Temperatur wurden 112 mg (1.2 mmol) (2*E*,4*E*)-Hexadienal zugegeben. Anschliend ließ man die Reaktionsmischung auf -10 °C erwärmen und beendete die Reaktion durch Zugabe von 10 ml gesätt. NaHCO₃. Das organische Lösungsmittel wurde abgezogen und die wässrige Phase mit CH₂Cl₂ extrahiert. Man erhielt nach flashchromatographischer Reinigung (CH₂Cl₂/Methanol, 8:2) 130 mg (63 %) der Titelverbindung. ESI-MS [m/z]: 503, Smp.: 149 - 150 °C

Folgende Verbindung wurde analog dargestellt:
4-Ethoxy-7-(4-[hydroxy{pyridin-3-yl}methyl]-phenyl)-9-methyl-2-piperazin-1-yl-thieno[2,3-d:4,5-d']dipyrimidin 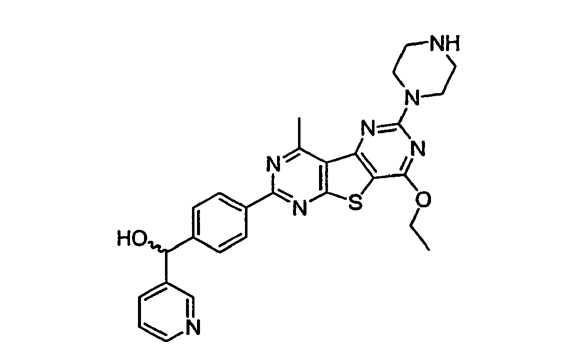
ESI-MS [m/z]: 514, Smp.: 170 -171 °C

### Beispiel 7

### Die Synthese von 7-(4-n-Butoxy-phenyl)-4-ethoxy-9-methyl-2-piperazin-1-yl-thieno[2,3-d:4,5-d']dipyrimidin

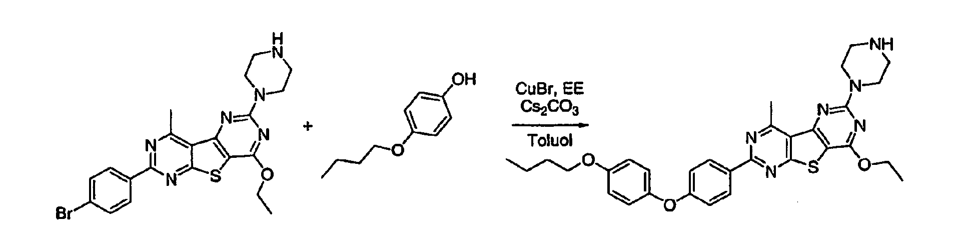

100 mg (0.21 mmol) 7-(4-Bromphenyl)-4-ethoxy-9-methyl-2-piperazin-1-yl-thieno[2,3-d:4,5-d']dipyrimidin, 51 mg (0.31 mmol) 4-n-Butoxyphenol, 134 mg (0.41 mmol) Cs₂CO₃, 1 µl (5 mol%) Essigester und 1.5 mg (5 mol%) CuBr wurden in 5 ml abs. Toluol suspendiert und 17 h in einer Mirkowelle bei 180 °C erhitzt. Anschließend wurde das Lösungsmittel entfernt und der Rückstand flashchromatographisch gereinigt. Man erhält 4 mg (3 %) der Titelsubstanz. ESI-MS [m/z]: 571

### Beispiel 8

### Die Synthese von 8-Ethoxy-5-morpholino-10-piperazin-1-yl-pyrimido[4',5':4,5]-thieno[2,3-c]-1,2,3,4-tetrahydro-isochinolin

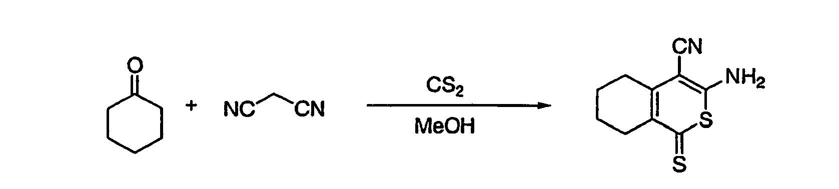

Zu einer Lösung von 19.60 g (0.2 mol) Cyclohexanon, 13.20 g (0.2 mol) Malonsäuredinitril, 60 ml Schwefelkohlenstoff und 60 ml Methanol wurden 12.0 ml Triethylamin zugetroft. Die Lösung wurde 24 h bei RT gerührt, wobei das Produkt als orangefarbener kristalliner Niederschlag ausfiel. Anschließend wurde filtriert, mit Methanol gewaschen und getrocknet. Man erhält 32 g (72 %) 3-Amino-l-thioxo-5,6,7,8-tetrahydro-1*H*-isothiocumarin-4-carbonitril.

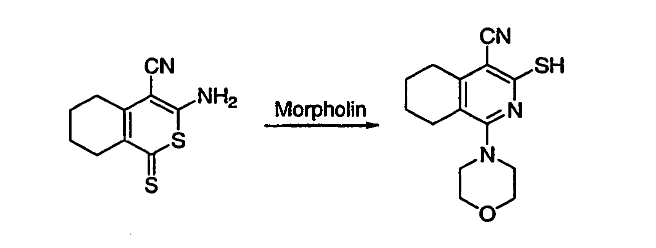

22.23 g (0.1 mol) 3-Amino-1-thioxo-5,6,7,8-tetrahydro-1*H*-isothiocumarin-4-carbonitril wurden in 100 ml Morpholin suspendiert und auf 100°C Badtemperatur bis zur Beendigung der H₂S-Entwicklung (4-6 h) erhitzt. Nach Abkühlung des Reaktionsgemischs wurde filtriert, mit Ethanol gewaschen und getrocknet. Man erhält 23 g (83 %) 3-Mercapto-1-morpholino-5,6,7,8-tetrahydroisochinolin-4-carbonitril.

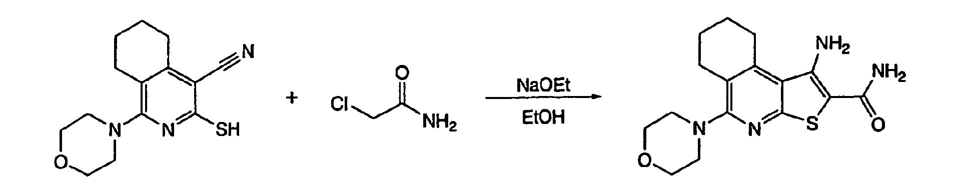

4.00 g (20 mmol) 3-Mercapto-1-morpholino-5,6,7,8-tetrahydroisochinolin-4-carbonitril und 1.80 g (20 mmol) 2-Chloracetamid werden in 50 ml Ethanol suspendiert und mit 1.50 g (40 mmol) Natriumethylat versetzt. Das Reaktinonsgemisch wird 3 h unter Rückfluss gehalten. Es wird filtriert und mit Ethanol, Wasser gewaschen und getrocknet. Man erhält 4.00 g (80 %) 1-Amino-5-morpholino-thieno[2,3-c]-6,7,8,9-tetrahydro-isochinolin-2-carbonsäureamid.

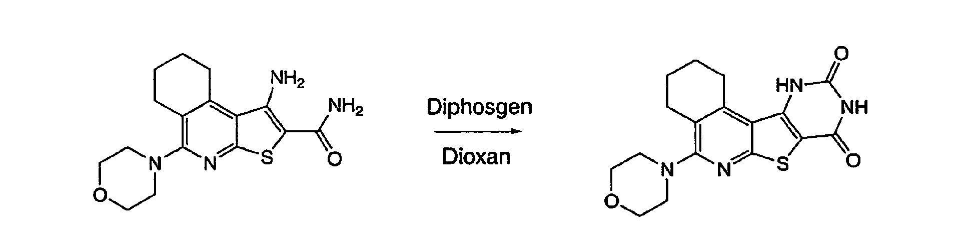

3.0 g (9 mmol) 1-Amino-5-morpholino-thieno[2,3-c]-6,7,8,9-tetrahydro-isochinolin-2-carbonsäureamid werden in 100 ml Dioxan suspendiert, anschließend werden 1.10 ml (8 mmol) Chlorameisensäure-trichlormethylester zugegeben und 3 h unter Rückfluss erhitzt. Der Niederschlag wird filtriert und getrocknet. Man erhält 3.0 g (93 %) 5-Morpholino-pyrimido[4',5':4,5]thieno[2,3-c]-1,2,3,4-tetrahydro-isochinolin-8,10(9H,11H)-dion.

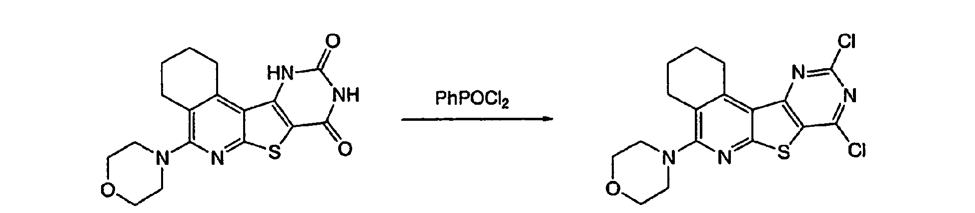

3.0 g (8 mmol) 5-Morpholino-pyrimido[4',5':4,5]thieno[2,3-c]-1,2,3,4-tetrahydroisochinolin-8,10(9H,11H)-dion und 20 ml (140 mmol) Dichlorphenylphosphinoxid werden 6 h bei 193°C erhitzt. Anschließend wird auf 40 g Eiswasser gegossen und mit gesättigter Natriumhydrogencarbonat-Lösung neutralisiert. Der Niederschlag wird abgesaugt, mit 100 ml Wasser gewaschen und getrocknet. Man erhält 2.0 g (63 %) 8,10-Dichlor-5-morpholino-pyrimido[4',5':4,5]thieno[2,3-c]-1,2,3,4-tetrahydroisochinolin.

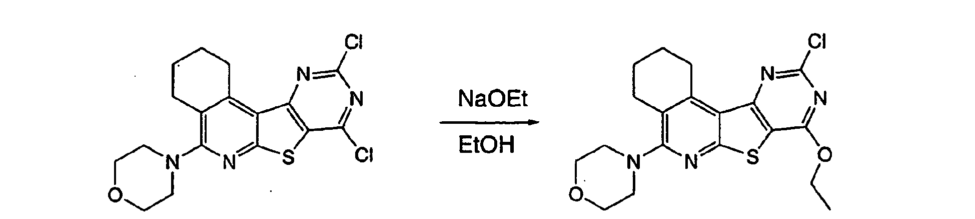

0.50 g (1 mmol) 8,10-Dichlor-5-morpholino-pyrimido[4',5':4,5]thieno[2,3-c]-1,2,3,4-tetrahydro-isochinolin werden in 40 ml Ethanol suspendiert und mit 0.12 g (2 mmol) NaOEt versetzt. Das Gemisch wird 24 h bei RT gerührt. Anschließend wird der Niederschlag abgesaugt, mit Ethanol gewaschen und getrocknet. Man erhält 3.9 g (96 %) 10-Chlor-8-ethoxy-5-morpholino-pyrimido[4',5':4,5]thieno[2,3-c]-1,2,3,4-tetrahydro-isochinolin.

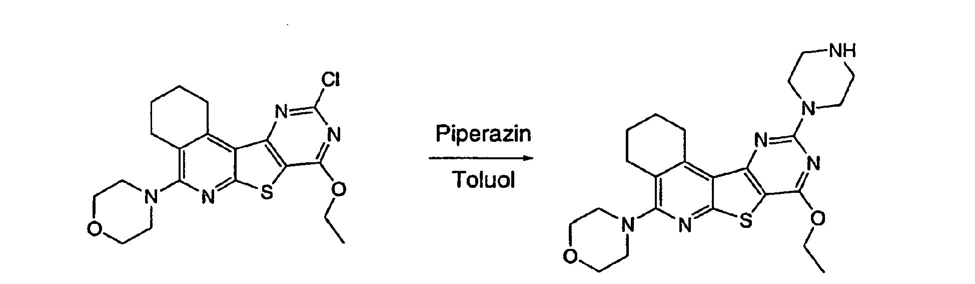

0.10 g (0.25 mmol) 10-Chlor-8-ethoxy-5-morpholino-pyrimido[4',5':4,5]thieno[2,3-c]-1,2,3,4-tetrahydro-isochinolin und 0.12 g (1.4 mmol) Piperazin werden in 20 ml Toluol 4 h unter Rückfluss erhitzt. Anschließend wird das Lösungsmittel entfernt und der Rückstand in Dichlormethan suspendiert. Die organische Phase wird mit gesättigter Natriumcarbonat-Lösung gewaschen, über Natriumsulfat getrocknet und das Lösungsmittel entfernt. Der Rückstand wird durch Flash-Chromatographie (Chloroform/Ethanol 10:2) gereinigt. Man erhält 0.11 g (97 %) der Titelverbindung.

Folgende Verbindungen wurden nach Beispiel 8 erhalten (Tabelle 3)

**Tabelle3**

| **Struktur** | **M** **[g/mol]** | **ESI MS** **[m/z]** | **Smp.** **[°C]** |
|---|---|---|---|
| 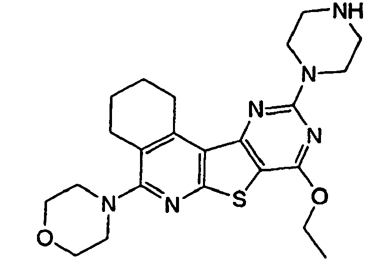 | 454.22 | 455 [M+H]⁺ | 181-184 |
| 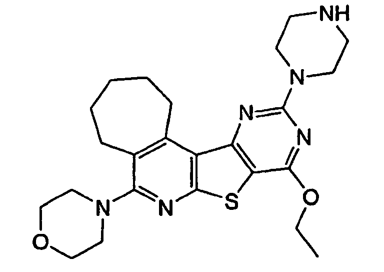 | 468.23 | 469 [M+H]⁺ | 320 Zers. |
| 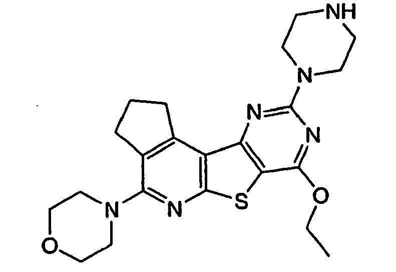 | 440.56 | 441 [M+H]⁺ | 151-159 |
| 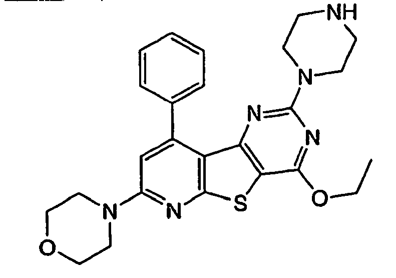 | 476.59 | 477 | 244 |
| 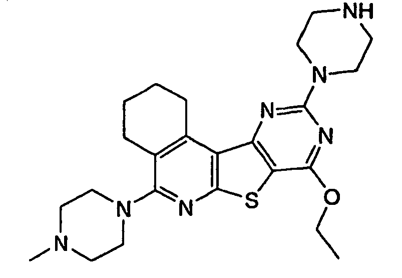 | 467.63 | 468 | n.d. |
| 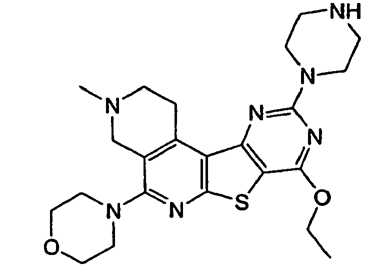 | 469.60 | 470 | n.d. |

### Beispiel 9

### Die Synthese von 10-Ethoxy-5-morpholino-8-piperazin-1-yl-pyrimido[4',5':4,5]-thieno[2,3-c]-1,2,3,4-tetrahydro-isochinolin

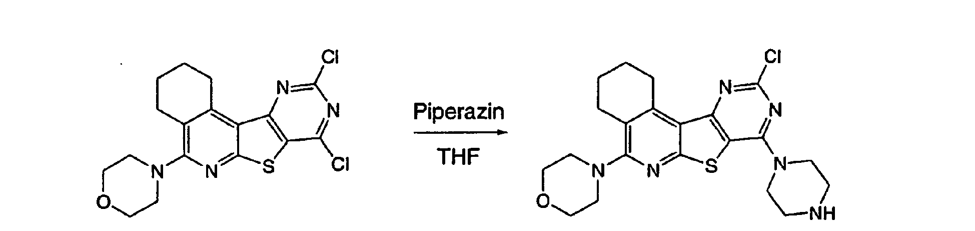

0.30 g (0.76 mmol) 8,10-Dichlor-5-morpholino-pyrimido[4',5':4,5]thieno[2,3-c]-1,2,3,4-tetrahydro-isochinolin und (1 mmol) Piperazin werden in 40 ml Tetrahydrofuran 6 h bei RT gerührt. Anschließend wird das Lösungsmittel entfernt, der Rückstand in Wasser suspendiert, abgesaugt, mit ca. 40 ml Wasser gewaschen und getrocknet. Man erhält 0.3 g (89 %) 10-Chlor-8-piperazin-1-yl-5-morpholino-pyrimido[4',5':4,5]-thieno-[2,3-c]-1,2,3,4-tetrahydro-isochinolin

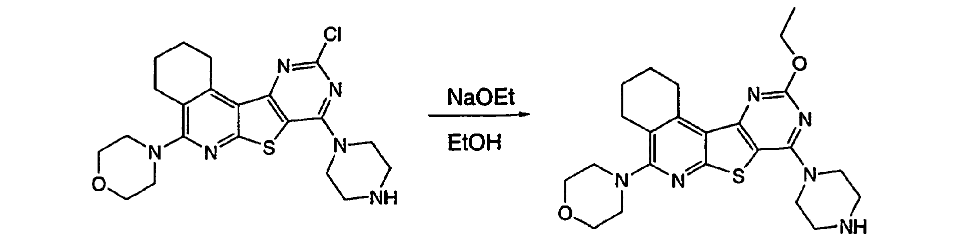

0.30 g (0.44 mmol) 10-Chlor-8-piperazin-1-yl-5-morpholino-pyrimido[4',5':4,5]thieno-[2,3-c]-1,2,3,4-tetrahydro-isochinolin und 0.30 g (4.40 mmol) Natriumethylat werden in 10 ml Ethanol 3 h unter Rückfluss erhitzt. Anschließend wird das Lösungsmittel entfernt und der Rückstand in wenig Wasser suspendiert. Die wässrige Suspension wird mit Dichlormethan extrahiert, die organische Phase über Natriumsulfat getrocknet und das Lösungsmittel entfernt. Man erhält 0.21 g (97%) der Titelsubstanz. ESI-MS [m/z]: 455, Smp.: 123 - 126

### Beispiel 10

### Die Synthese von 4-Ethoxy-7-(3,4-dimethoxyphenyl)-9-methyl-2-piperazin-1-yl-pyrido[3',2':4,5]thieno[3,2-d]pyrimidin

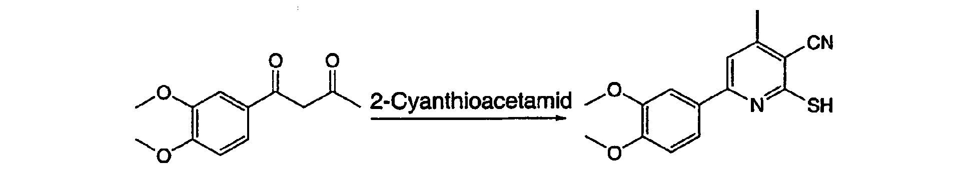

5.0 g (22.5 mmol) 1-(3,4-Dimethoxyphenyl)butan-1,3-dion, 2.9 g (29.2 mmol) Cyanthioacetamid und 4.0 g (29.2 mmol) Kaliumcarbonat werden bei Raumtemperatur 21 h in 170 ml Aceton gerührt. Anschließend wird das Lösungsmittel entfernt und der Rückstand in Ethanol/Wasser (1:1) gerade gelöst. Nach Ansäuern mit Eisessig wird der erhaltene Niederschlag abgesaugt, mit wenig Wasser gewaschen, trocken gesaugt und i. Vak. bei 50 °C getrocknet. Man erhält 6.34 g (98 %) 2-Mercapto-6-(3,4-dimethoxyphenyl)-4-methyl-pyridin-3-carbonitril.

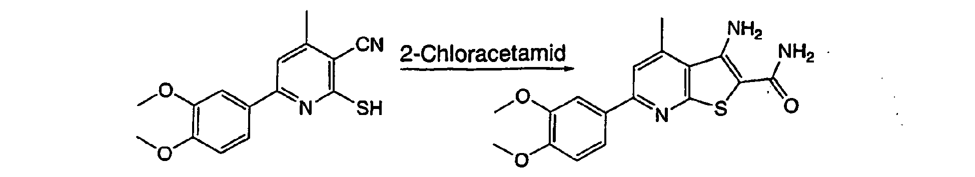

2.1 g (31.4 mmol) Natriumethylat werden in 40 ml abs. Ethanol gelöst. Es werden 3.0 g (10.5 mmol) 2-Mercapto-6-(3,4-dimethoxyphenyl)-4-methyl-pyridin-3-carbonitril und 1.2 g (12.6 mmol) 2-Chloracetamid zugegeben und 1 h unter Rückfluß erhitzt. Nach dem Abkühlen werden 10 ml Wasser zugegeben und der Niederschlag abgesaugt, trocken gesaugt und i. Vak. bei 50 °C getrocknet. Man erhält 3.4 g (94 %) 3-Amino-6-(3,4-dimethoxyphenyl)-4-methyl-thieno[2,3-*b*]pyridin-2-carbonsäureamid.

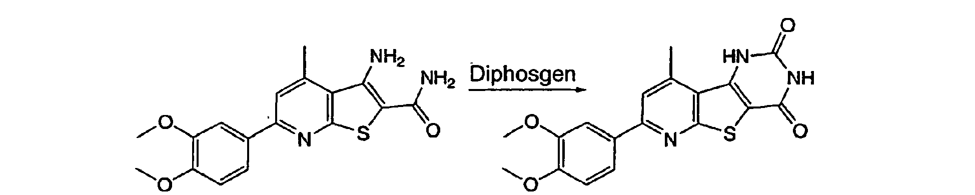

3.0 g (8.7 mmol) 3-Amino-6-(3,4-dimethoxyphenyl)-4-methyl-thieno[2,3-*b*]pyridin-2-carbonsäureamid werden in 100 ml abs. Dioxan suspendiert. Anschließend werden 1.1 ml (8.7 mmol) Chlorameisensäure-trichlormethylester (Diphosgen) zugegeben und 3 h unter Rückfluß erhitzt. Nach Abkühlen wird der Niederschlag abgesaugt und anschließend in 50 ml Wasser suspendiert. Die Suspension wird mit konz. Ammoniak-Lösung auf pH 8 eingestellt und der Niederschlag abgesaugt, mit wenig Wasser gewaschen, trocken gesaugt und i. Vak. bei 50 °C getrocknet. Man erhält 3.08 g (96 %) 7-(3,4-Dimethoxyphenyl)-9-methyl-pyrido[3',2':4,5]thieno[3,2-*d*]pyrimidin-2,4-dion.

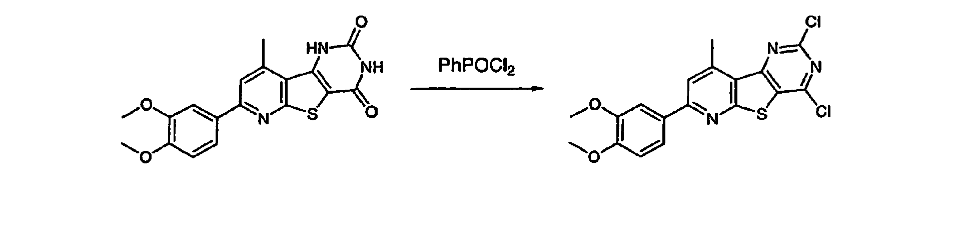

3.0 g (8.1 mmol) 7-(3,4-Dimethoxyphenyl)-9-methyl-pyrido[3',2':4,5]thieno[3,2-*d*]-pyrimidin-2,4-dion und 6.8 ml (48.7 mmol) Dichlorphenylphosphinoxid werden 6 h bei 200 °C erhitzt. Anschließend wird auf ca. 20 g Eiswasser gegossen und mit gesättigter Natriumhydrogencarbonat-Lösung neutralisiert. Der Niederschlag wird abgesaugt, mit ca. 100 ml Wasser gewaschen, trocken gesaugt und i. Vak. bei 50 °C getrocknet. Man erhält 3.08 g (94 %) 2,4-Dichlor-7-(3,4-dimethoxyphenyl)-9-methyl-pyrido[3',2':4,5]thieno[3,2-*d*]pyrimidin.

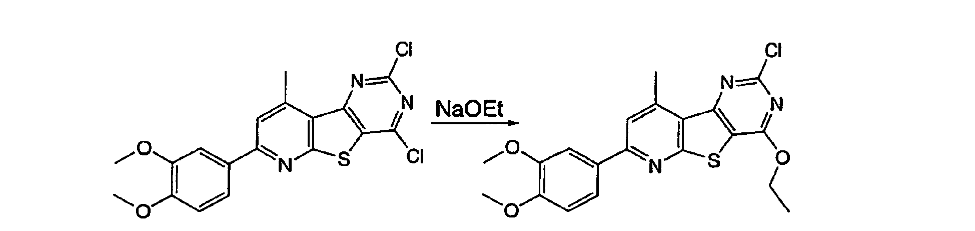

1.5 g (3.7 mmol) 2,4-Dichlor-7-(3,4-dimethoxyphenyl)-9-methyl-pyrido[3',2':4,5]-thieno[3,2-*d*]pyrimidin und 0.7 g (11.1 mmol) Natriumethylat werden in 50 ml abs. Ethanol 24 h bei RT gerührt. Anschließend wird der Niederschlag abgesaugt, mit ca. 40 ml Wasser gewaschen, trocken gesaugt und i. Vak. bei 50 °C getrocknet. Man erhält 1.38 g (90 %) 2-Chlor-4-ethoxy-7-(3,4-dimethoxyphenyl)-9-methyl-pyrido[3',2':4,5]thieno[3,2-*d*]pyrimidin.

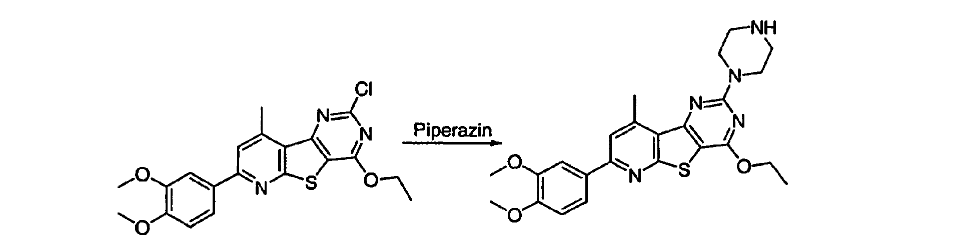

1.0 g (2.4 mmol) 2-Chlor-4-ethoxy-7-(3,4-dimethoxyphenyl)-9-methyl-pyrido-[3',2':4,5]thieno[3,2-*d*]pyrimidin und 0.83 g (9.6 mmol) Piperazin werden in 20 ml Toluol 4 h unter Rückfluß erhitzt. Anschließend wird das Lösungsmittel entfernt und der Rückstand in Dichlormethan suspendiert. Die organische Phase wird mit gesättigter Natriumcarbonat-Lösung gewaschen, über Natriumsulfat getrocknet und das Lösungsmittel entfernt. Der Rückstand wird durch Flash-Chromatographie (Ethanol/Chloroform 1:5) gereinigt. Man erhält 0.98 g (87 %) der Titelsubstanz.

Folgende Verbindungen wurden nach Beispiel 10 erhalten (Tabelle 4)

**Tabelle 4**

| **Struktur** | **M** **[g/mol]** | **ESI MS** **[m/z]** [M+H]⁺ | **Schmp.** **[°C]** |
|---|---|---|---|
| 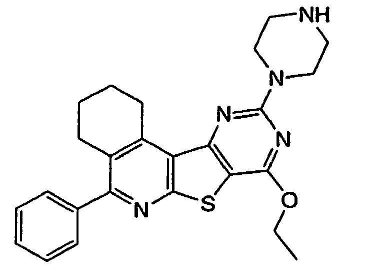 | 445.58 | 446 | 200-203 |
| 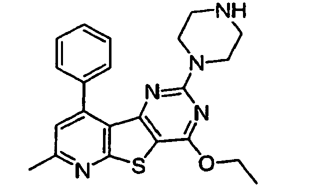 | 405.52 | 406 | n.d. |
| 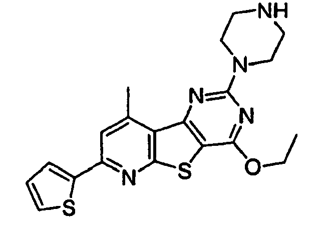 | 411.54 | 412 | n.d. |
| 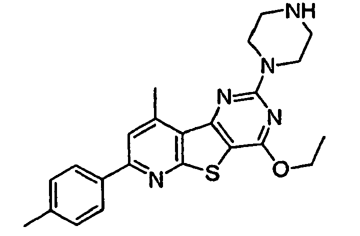 | 419.54 | 420 | n.d. |
| 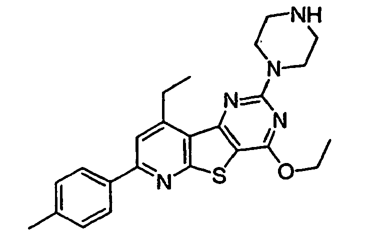 | 433.57 | 434 | n.d. |
| 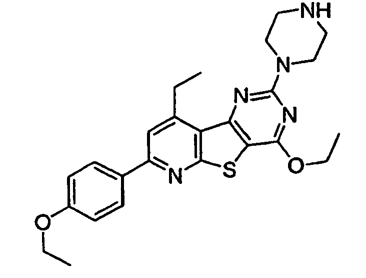 | 463.60 | 464 | n.d. |
| 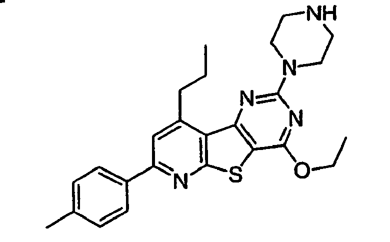 | 447.60 | 448 | n.d. |
| 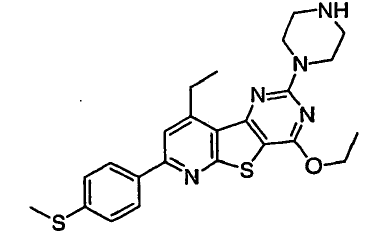 | 465.63 | 466 | n.d. |
| 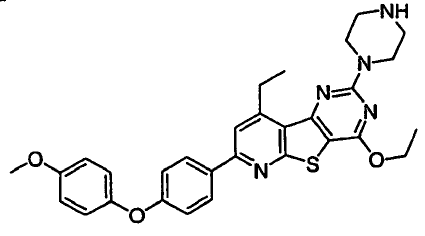 | 541.66 | 542 | 166 |
| 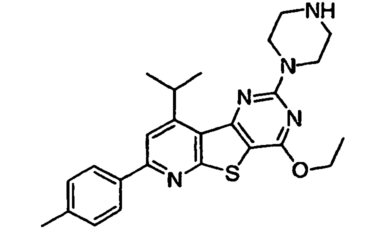 | 447.60 | 44.8 | n.d. |
| 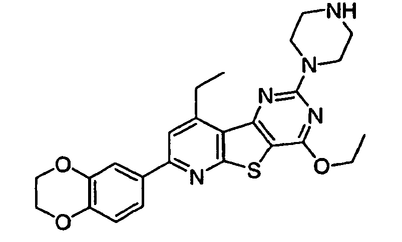 | 477.57 | 478 | n.d. |
| 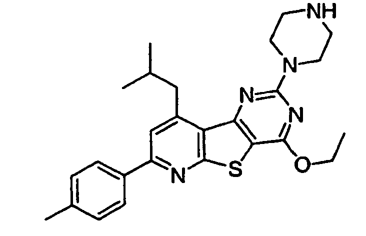 | 461.62 | 462 | n.d. |
| 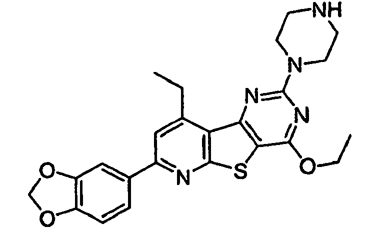 | 463.55 | 464 | n.d. |
| 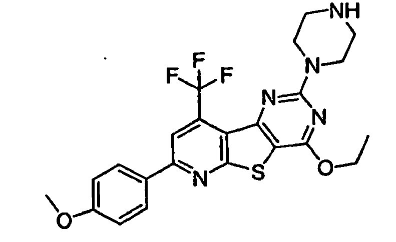 | 489.51 | 490 | n.d. |
| 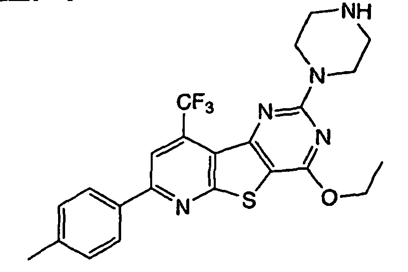 | 473.51 | 474 | n.d. |
| 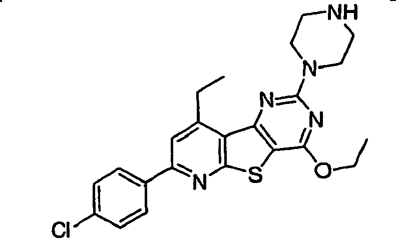 | 453.99 | 454 | n.d. |
| 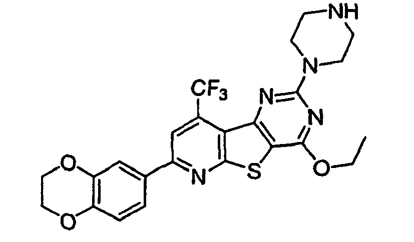 | 517.52 | 518 | n.d. |
| 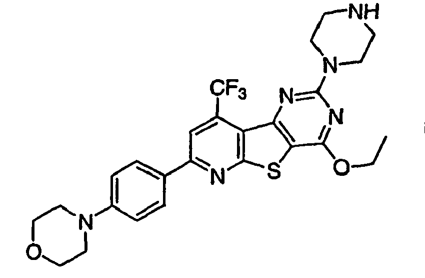 | 544.59 | 545 | n.d. |
| 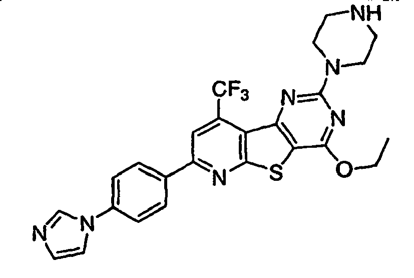 | 525.55 | 526 | 216 - 222 |
| 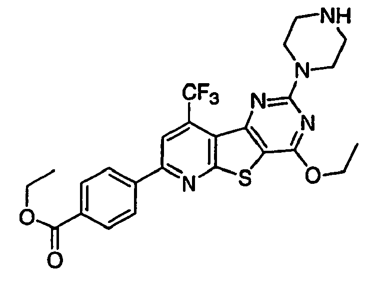 | 531.60 | 532 | n.d. |
| 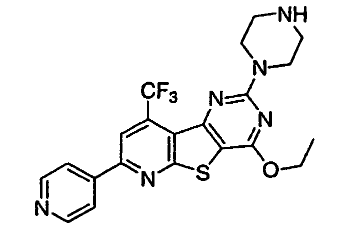 | 460.47 | 461 | 185 - 187 |
| 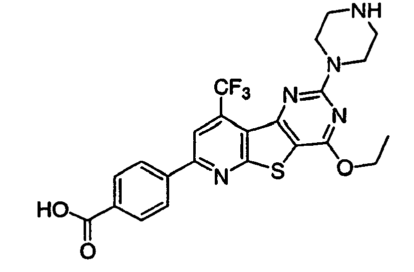 | 503.50 | 504 | n.d. |
| 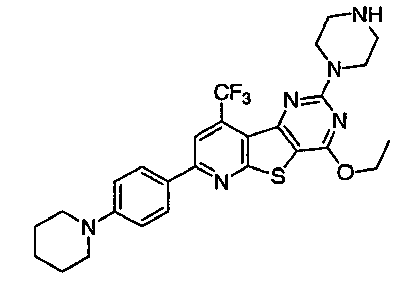 | 542.62 | 543 | 204 - 205 |
| 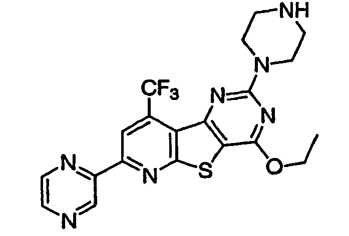 | 461.46 | 462 | n.d. |
| 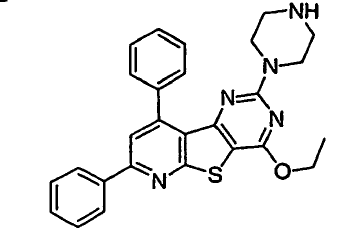 | 467.59 | 468 | 193 - 210 |
| 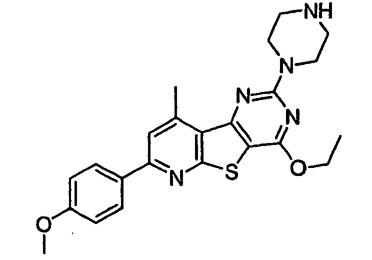 | 435.54 | 436 | 77 - 82 |
| 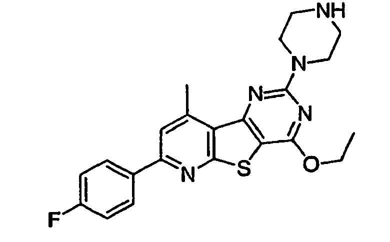 | 423.51 | 424 | 220 |
| 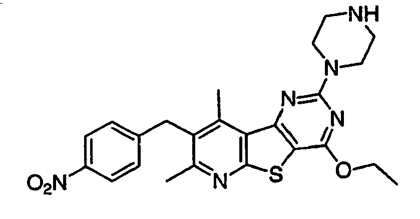 | 478.57 | 479 | 207 - 217 |
| 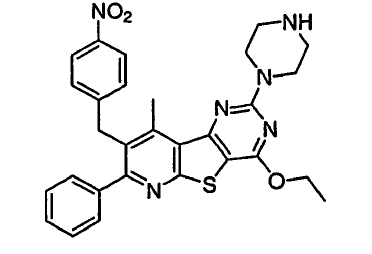 | 540.64 | 541 | n.d. |
| 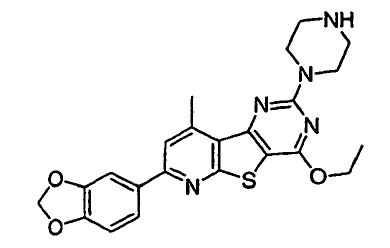 | 446.53 | 447 | n.d. |
| 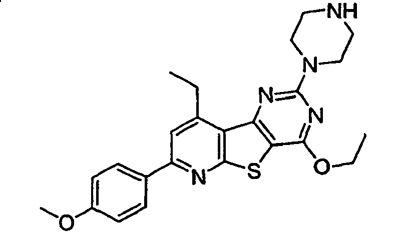 | 449.57 | 450 | n.d. |
| 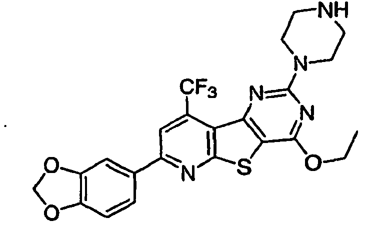 | 503.50 | 504 | n.d. |
| 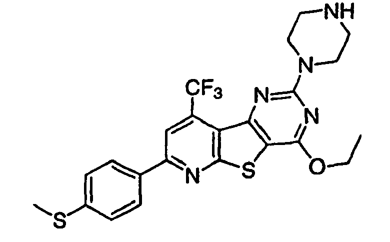 | 697.70 | 698 | 178 - 181 |
| 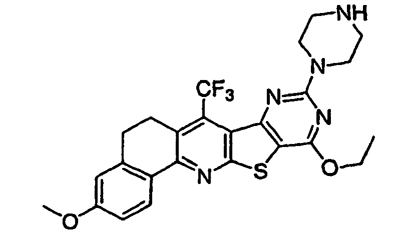 | 515.55 | 516 | n.d. |
| 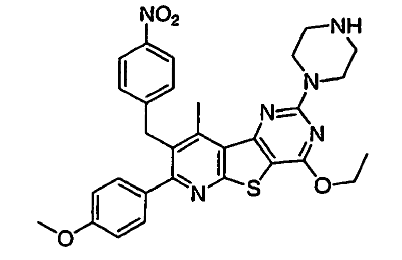 | 570.66 | 571 | n.d. |
| 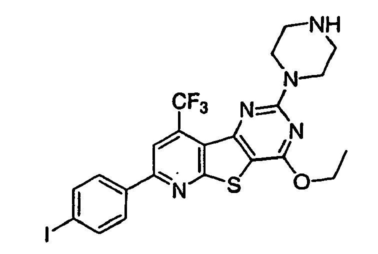 | 585.38 | 586 | 133 |
| 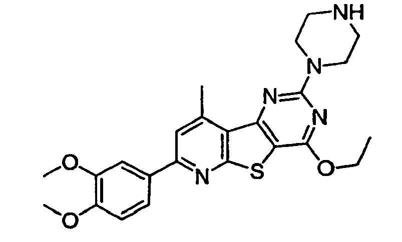 | 465.57 | 466 | n.d. |
| 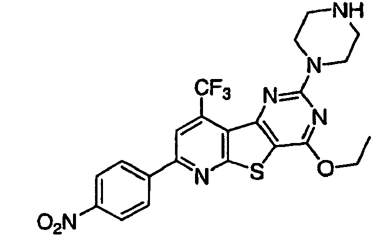 | 504.48 | 505 | 229 - 233 |
| 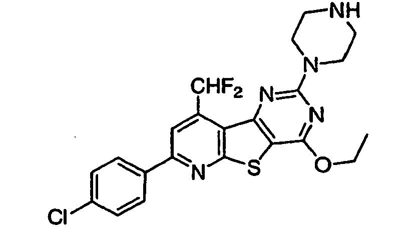 | 475.94 | 476 | n.d. |
| 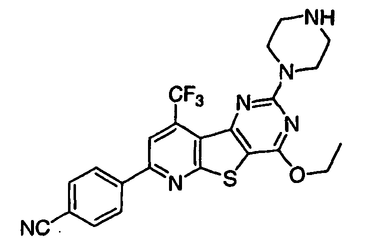 | 484.50 | 485 | n.d. |
| 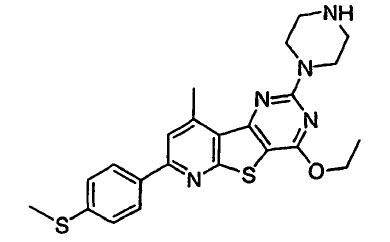 | 451.61 | 452 | n.d. |
| 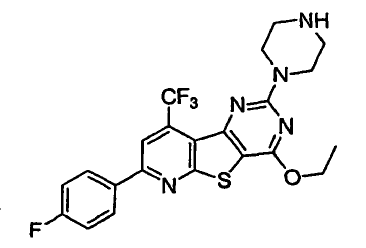 | 477.48 | 478 | n.d. |
| 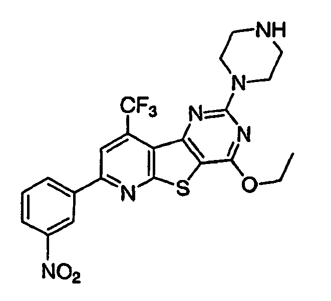 | 504.49 | 505 | n.d. |
| 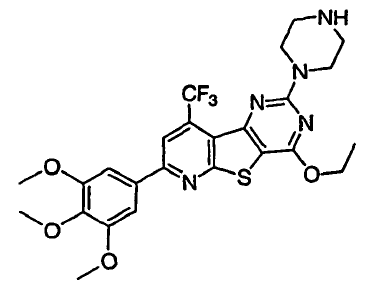 | 549.57 | 550 | n.d. |
| 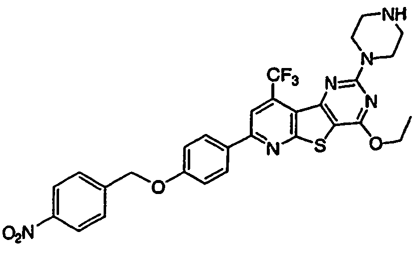 | 610.61 | 611 | n.d. |
| 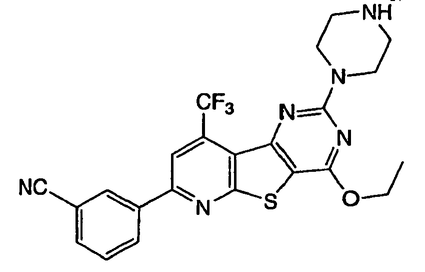 | 484.50 | 485 | n.d. |
| 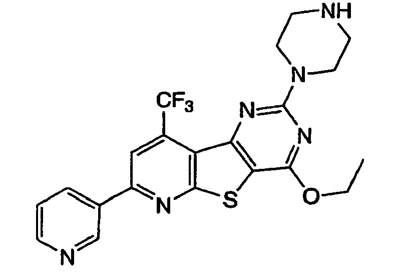 | 460.47 | 461 | 219 - 221 |
| 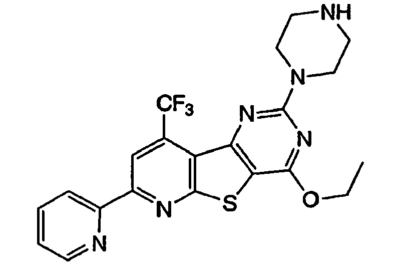 | 460.47 | 461 | n.d. |
| 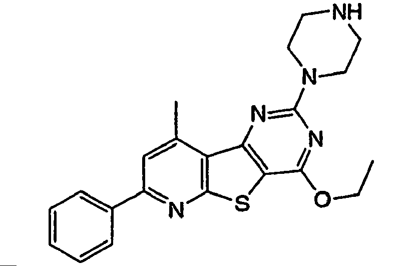 | 405.52 | 406 | n.d. |

### Beispiel 11

### Die Synthese von 2-Ethoxy-7-(3,4-dimethoxyphenyl)-9-methyl-4-piperazin-1-yl-pyrido[3',2':4,5]thieno[3,2-d]pyrimidin

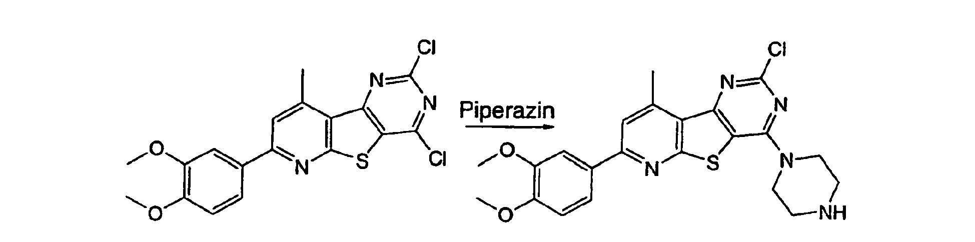

0.5 g (1.2 mmol) 2,4-Dichlor-7-(3,4-dimethoxyphenyl)-9-methyl-pyrido[3',2':4,5]-thieno[3,2-*d*]pyrimidin, 0.13 g (1.5 mmol) Piperazin und 0.2 ml (1.5 mmol) Triethylamin werden in 20 ml abs. Tetrahydrofuran 6 h bei RT gerührt. Anschließend wird das Lösungsmittel entfernt, der Rückstand in Wasser suspendiert, abgesaugt, mit ca. 40 ml Wasser gewaschen, trocken gesaugt und i. Vak. bei 50 °C getrocknet. Man erhält 0.49 g (90 %) 2-Chlor-7-(3,4-dimethoxyphenyl)-9-methyl-4-piperazin-1-yl-pyrido[3',2':4,5]thieno[3,2-*d*]pyrimidin.

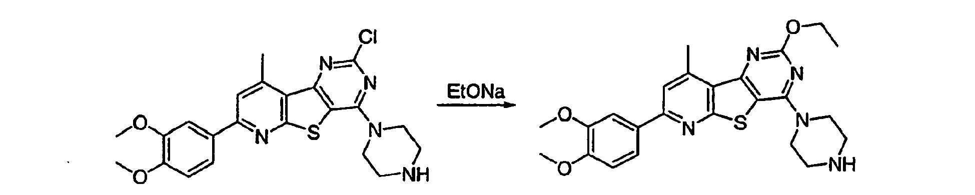

0.1 g (0.22 mmol) 2-Chlor-7-(3,4-dimethoxyphenyl)-9-methyl-4-piperazin-1-yl-pyrido-[3',2':4,5]thieno[3,2-*d*]pyrimidin und 0.22 ml (0.44 mmol) Kaliumethylat (2M THF) werden in 10 ml abs. Dioxan 3 h unter Rückfluß erhitzt. Anschließend wird das Lösungsmittel entfernt und der Rückstand in wenig Wasser suspendiert. Die wässrige Suspension wird mit Dichlormethan extrahiert, die organische Phase über Natriumsulfat getrocknet und das Lösungsmittel entfernt. Man erhält 50 mg (49 %) der Titelsubstanz.

Folgende Verbindungen wurden nach Beispiel 11 erhalten (Tabelle 5)

**Tabelle 5**

| **Struktur** | **M** **[g/mol]** | **ESI MS** **[m/z]** [M+H]⁺ | **Smp.** **[°C]** |
|---|---|---|---|
| 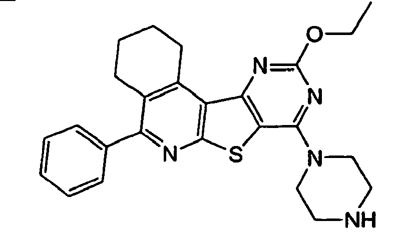 | 445.58 | 446 | 197-199 |
| 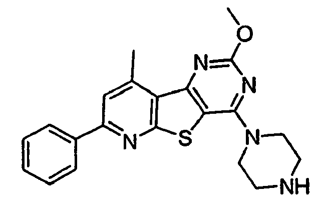 | 391.49 | 392 | 175 - 179 |
| 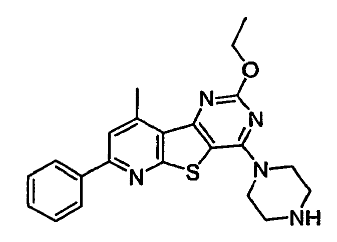 | 405.51 | 406 | 185 - 188 |
| 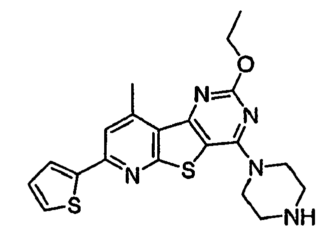 | 411.54 | 412 | n.d. |
| 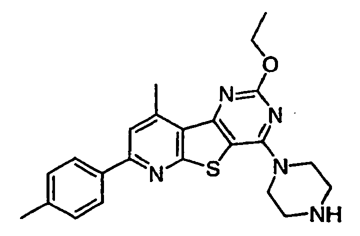 | 419.54 | 420 | n.d. |
| 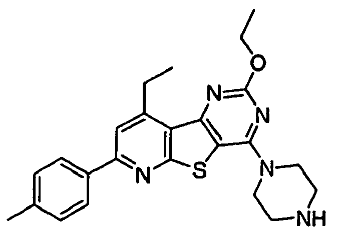 | 433.57 | 434 | n.d. |
| 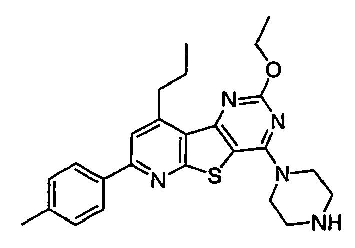 | 447.60 | 448 | n.d. |
| 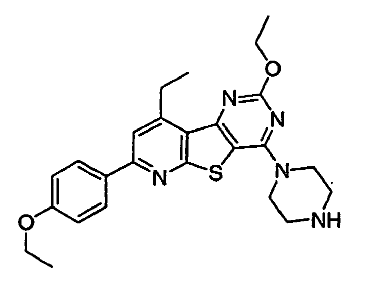 | 463.60 | 464 | n.d. |
| 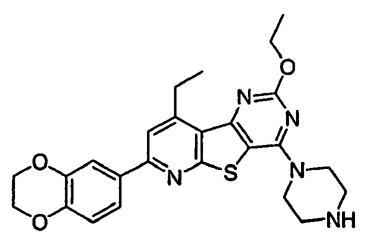 | 477.58 | 478 | 235 |
| 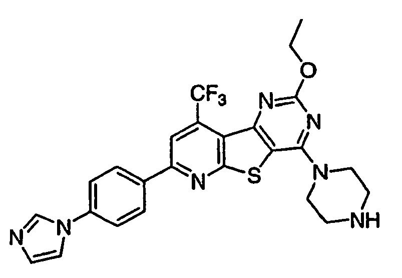 | 525.55 | 526 | >400 |
| 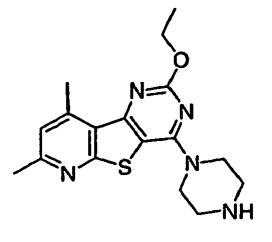 | 329.42 | 330 | 210 - 220 |
| 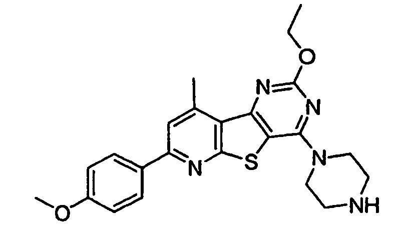 | 435.54 | 436 | 191 - 194 |
| 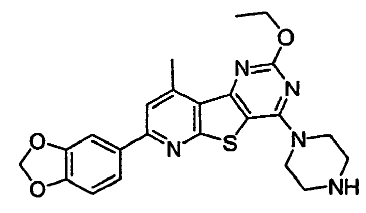 | 449.53 | 450 | n.d. |
| 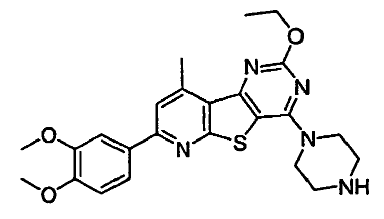 | 465.57 | 466 | n.d. |
| 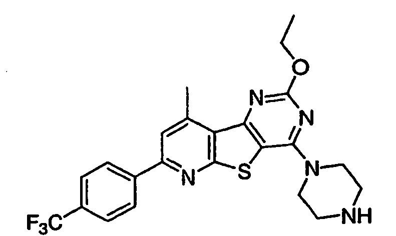 | 473.51 | 474 | n.d. |

### Beispiel 12

### Die Synthese von 4-Ethoxy-2-piperazin-1-yl-7,8,9,10-tetrahydro-11-thiophen-2-yl-pyrimido[4',5':4,5]thieno[2,3-b]chinolin

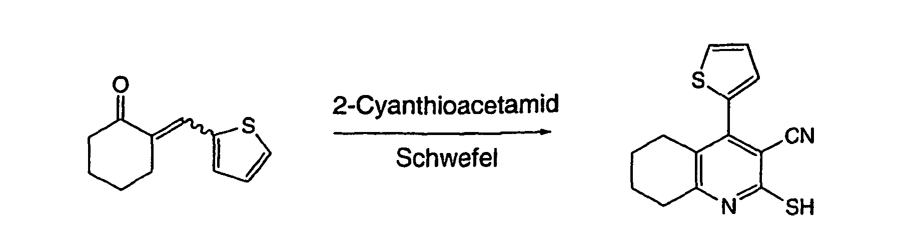

12.68 g (65.9 mmol) 2-Thiophen-2-yl-methylen-cyclohexanon, 7.00 g (70.0 mmol) Cyanthioacetamid und 10.00 g (72.4 mmol) Kaliumcarbonat werden in 100 ml Aceton 8 h unter Rückfluß erhitzt. Anschließend werden 1.05 g (32.8 mmol) Schwefel zugesetzt und 12 h unter Rückfluß erhitzt. Das Lösungsmittel wird i. Vak. entfernt und der Rückstand in 600 ml Ethanol/Wasser (1:5) suspendiert. Nach Ansäuern mit Eisessig wird der erhaltene Niederschlag abgesaugt, mit 200 ml Wasser gewaschen, trocken gesaugt und i. Vak. bei 50 °C getrocknet. Man erhält 16.50 g (92 %) 5,6,7,8-Tetrahydro-2-mercapto-4-thiophen-2-yl-chinolin-3-carbonitril.

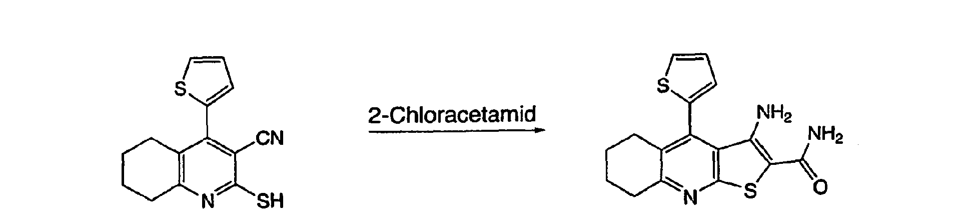

3.07 g (11.3 mmol) Tetrahydro-2-mercapto-4-thiophen-2-yl-chinolin-3-carbonitril, 1.26 g (13.5 mmol) Chloracetamid und 3.2 g (47.0 mmol) Natriumethylat werden in 70 ml abs. Ethanol 5 h unter Rückfluß erhitzt. Nach Abkühlen werden 100 ml Wasser zugegeben und der Niederschlag abgesaugt, trocken gesaugt und i. Vak. bei 50 °C getrocknet. Man erhält 1.9 g (51 %) 3-Amino-5,6,7,8-tetrahydro-4-thiophen-2-yl-thieno[2,3-b]chinolin-2-carbonsäureamid.

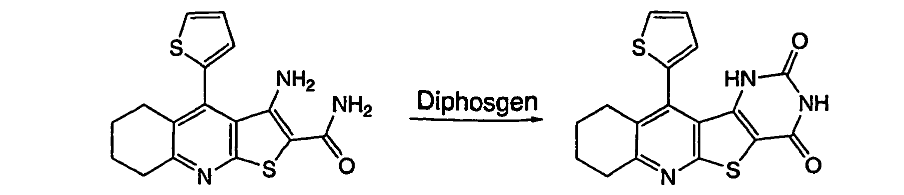

Die Umsetzung von 3-Amino-5,6,7,8-tetrahydro-4-thiophen-2-yl-thieno[2,3-b]chinolin-2-carbonsäureamid mit Diphosgen zu 7,8,9,10-Tetrahydro-11-thiophen-2-yl-pyrimido[4',5':4,5]thieno[2,3-b]chinolin-2,4-dion erfolgt analog Beispiel 1.
Dabei werden aus 1.87 g (5.7 mmol) Edukt 1.06 g (52 %) Produkt erhalten.

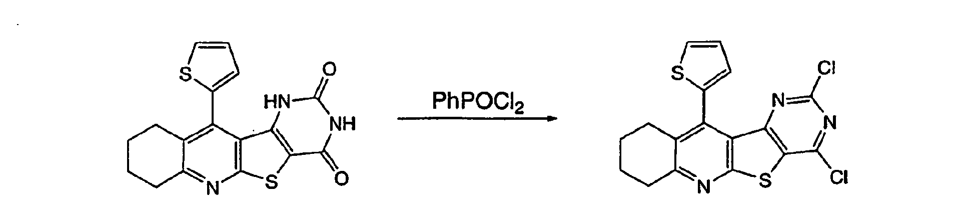

Die Umsetzung von 7,8,9,10-Tetrahydro-11-thiophen-2-yl-pyrimido[4',5':4,5]thieno-[2,3-b]chinolin-2,4-dion mit Dichlorphenylphosphinoxid zu 2,4-Dichlor-7,8,9,10-tetrahydro-11-thiophen-2-yl-pyrimido[4',5':4,5]thieno[2,3-b]chinolin erfolgt analog Beispiel 1.
Dabei werden aus 1.03 g (2.9 mmol) Edukt 1.10 g (97 %) Produkt erhalten.

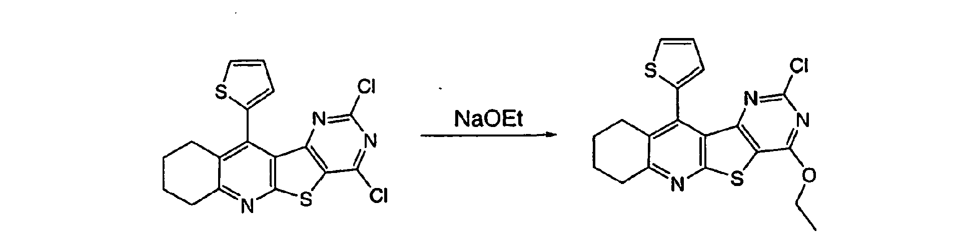

Die Umsetzung von 2,4-Dichlor-7,8,9,10-tetrahydro-11-thiophen-2-yl-pyrimido-[4',5':4,5]thieno[2,3-b]chinolin mit Natriumethylat zu 2-Chlor-4-ethoxy-7,8,9,10-tetrahydro-11-thiophen-2-yl-pyrimido[4',5':4,5]thieno[2,3-b]chinolin erfolgt analog Beispiel 1.
Dabei werden aus 0.50 g (1.4 mmol) Edukt 0.36 g (64 %) Produkt erhalten

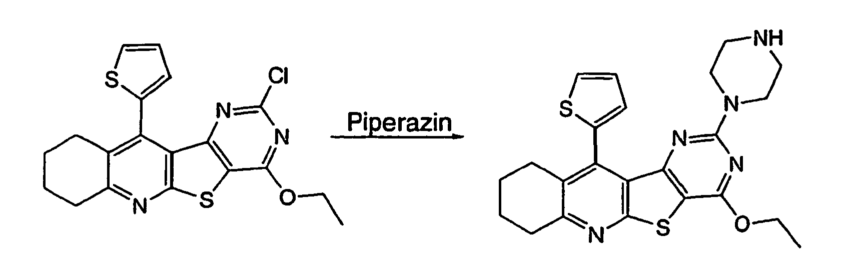

Die Umsetzung von 2-Chlor-4-ethoxy-7,8,9,10-tetrahydro-11-thiophen-2-yl-pyrimido[4',5':4,5]thieno[2,3-b]chinolin mit Piperazin zu 4-Ethoxy-2-piperazin-1-yl-7,8,9,10-tetrahydro-11-thiophen-2-yl-pyrimido[4',5':4,5]thieno[2,3-b]chinolin erfolgt analog Beispiel 1.
Dabei werden aus 0.36 g (0.9 mmol) Edukt 0.23 g (57 %) Produkt erhalten.

Folgende Verbindungen wurden nach Beispiel 12 erhalten (Tabelle 6)

**Tabelle 6**

| **Struktur** | **M** **[g/mol]** | **ESI MS** **[m/z]** [M+H]⁺ | **Smp.** **[°C]** |
|---|---|---|---|
| 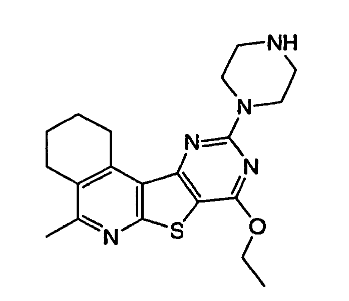 | 465.47 | 466 | 167 - 171 |
| 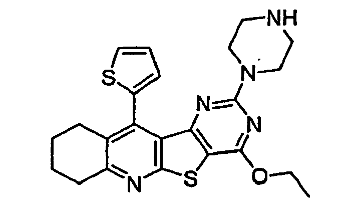 | 451.61 | 452 | n.d. |
| 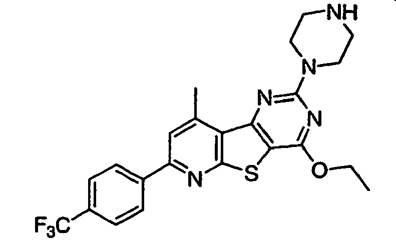 | 473.51 | 474 | n.d. |
| 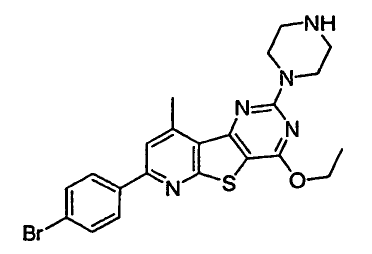 | 484.41 | 485 | n.d. |
| 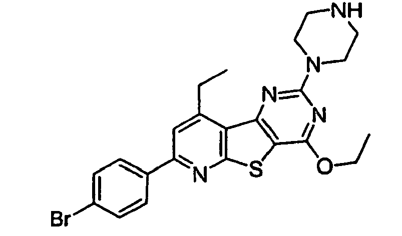 | 498.43 | 499 | n.d. |
| 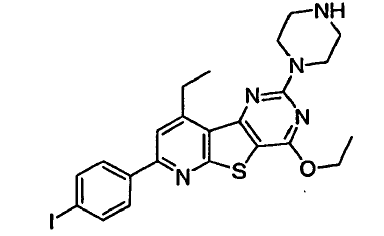 | 545.43 | 546 | n.d. |

### Beispiel 13

### Die Synthese von 2-Ethylthio-9-methyl-7-phenyl-4-piperazin-1-yl-pyrido-[3',2':4,5]thieno[3,2-d]pyrimidin

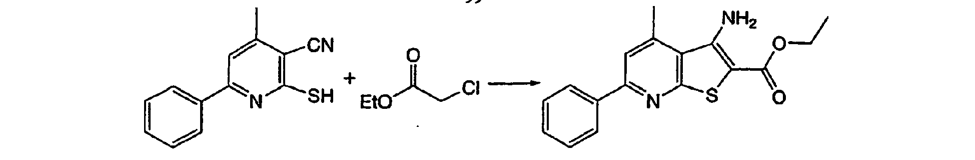

6.45 g (28.5 mmol) 2-Mercapto-4-methyl-6-phenyl-pyridin-3-carbonitril werden in 100 ml abs. Ethanol vorgelegt und 5.3 ml (50.0 mmol) 2-Chloressigsäureethylester sowie 10 ml (72.1 mmol) Triethylamin hinzugefügt. Es wird 1 h unter Rückfluß erhitzt und der nach dem Abkühlen ausgefallene Niederschlag abgesaugt, mit wenig Wasser und Ethanol gewaschen, trocken gesaugt und i. Vak. bei 50 °C getrocknet. Anschließend wird der Niederschlag in einer Lösung aus 0.14 g (1.6 mmol) Natriumethylat in abs. Ethanol 1 h unter Rückfluß erhitzt. Der Niederschlag wird abgesaugt, mit 30 ml Wasser und 15 ml Ethanol gewaschen, trocken gesaugt und i. Vak. bei 50 °C getrocknet. Man erhält 5.0 g (91 %) 3-Amino-4-methyl-6-phenyl-thieno[2,3-*b*]pyridin-2-carbonsäureethylester.

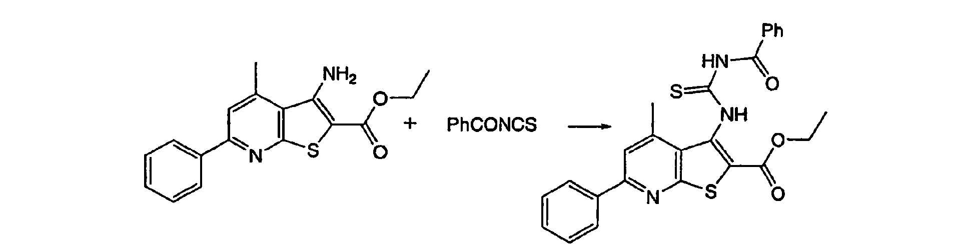

Zu 2.78 g (8.9 mmol) 3-Amino-4-methyl-6-phenyl-thieno[2,3-*b*]pyridin-2-carbonsäure-ethylester in 10 ml Aceton werden 5 ml (13.3 mmol) acetonischer Benzoylisothiocyanat-Lösung (2 M) gegeben und 2 h unter Rückfluß erhitzt. Der nach Abkühlen ausgefallene Niederschlag wird abgesaugt, mit wenig Wasser und Ethanol gewaschen, trocken gesaugt und i. Vak. bei 50 °C getrocknet. Man erhält 3.93 g (93 %) 3-(3-Benzoylthioureido)-4-methyl-6-phenyl-thieno[2,3-*b*]pyridin-2-carbon-säureethylester.

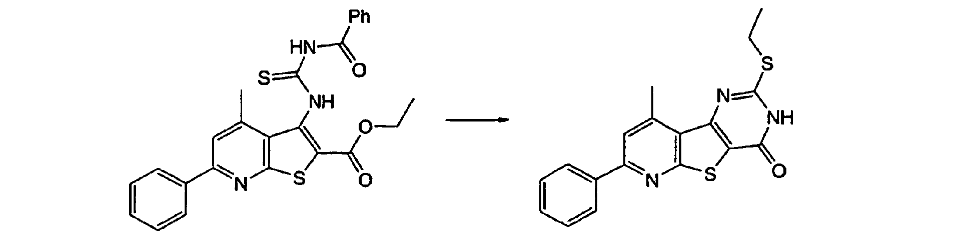

Zu 2.00 g (4.2 mmol) 3-(3-Benzoylthioureido)-4-methyl-6-phenyl-thieno[2,3-*b*]pyridin-2-carbonsäureethylester in 25 ml Ethanol werden 10.5 ml (10.5 mmol) 1 M Natriumhydroxid-Lösung gegeben und 30 min unter Rückfluß erhitzt. Nach Zugabe von 10 ml *N*,*N*-Dimethylformamid, 5 ml (5 mmol) Natriumhydroxid-Lösung (1 M) und 0,50 ml lodethan (6.2 mmol) wird 30 min bei RT gerührt. Der nach Ansäuern mit verd. Salzsäure ausgefallene Niedeschlag wird abgesaugt, mit Wasser gewaschen, trocken gesaugt und i. Vak. bei 50 °C getrocknet. Man erhält 1.23 g (83 %) 2-Ethylthio-9-methyl-7-phenyl-pyrido[3',2':4,5]thieno[3,2-*d*]pyrimidin-4-on.

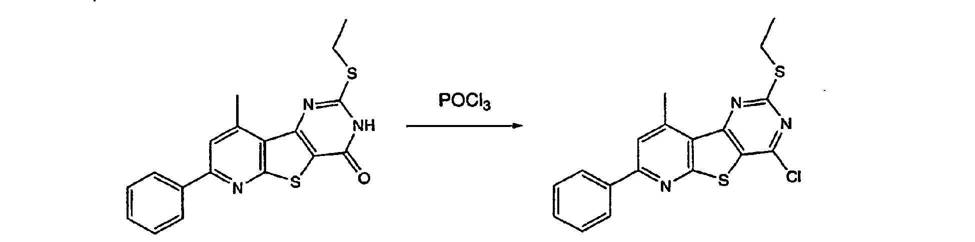

1.23 g (3.5 mol) 2-Ethylthio-9-methyl-7-phenyl-pyrido[3',2':4,5]thieno[3,2-*d*]pyrimidin-4-on werden in 10 ml (10.9 mol) Phosphoroxychlorid 6 h unter Rückfluß erhitzt. Anschließend wird auf ca. 100 g Eiswasser gegossen und mit gesättigter. Natriumhydrogencarbonat-Lösung neutralisiert. Der Niederschlag wird abgesaugt, mit ca. 100 ml Wasser gewaschen, getrocknet und durch Flash-Chromatographie (Dichlormethan/Petrolether 5:1) gereinigt. Man erhält 0.29 g (22 %) 4-Chlor-2-ethylthio-9-methyl-7-phenyl-pyrido[3',2':4,5]thieno[3,2-*d*]pyrimidin.

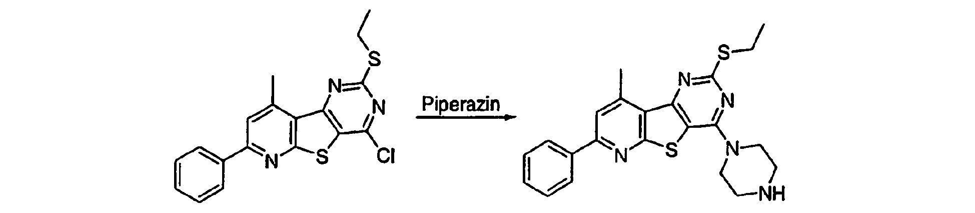

0.63 g (1.7 mmol) 4-Chlor-2-ethylthio-9-methyl-7-phenyl-pyrido[3',2':4,5]thieno[3,2-*d*]pyrimidin werden mit 0.29 g (3.4 mmol) Piperazin in 10 ml Tetrahydrofuran 1 h bei RT gerührt. Der Niederschlag wird abgesaugt, mit wenig Wasser gewaschen und trocken gesaugt. Anschließend wird der Niederschlag in ca. 300 ml Dichlormethan suspendiert, ausgerührt, filtriert und das Lösungsmittel des Filtrats entfernt. Man erhält 0.67 g (93 %) der Titelsubstanz. ESI-MS [m/z]: 422, Smp.: 150 -154 °C

### Beispiel 14

### Die Synthese von 2-Ethylsulfinyl-9-methyl-7-phenyl-4-piperazin-1-yl-pyrido-[3',2':4,5]thieno[3,2-d]pyrimidin

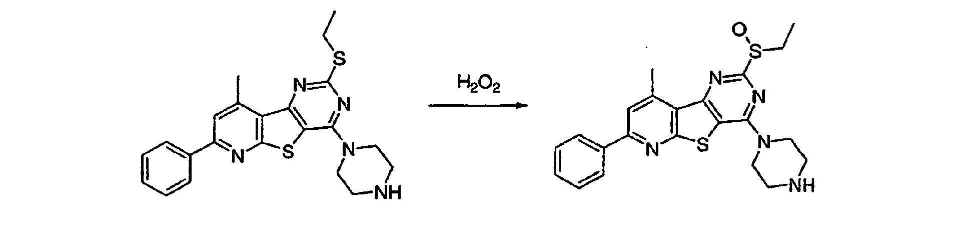

85 mg (0.2 mmol) 2-Ethylthio-9-methyl-7-phenyl-4-piperazin-1-yl-pyrido[3',2':4,5]-thieno[3,2-*d*]pyrimidin, 3 ml Eisessig und 0.04 ml Wasserstoffperoxid (60 %) werden 48 h bei RT gerührt. Nach Entfernen des Lösungsmittels wird der Rückstand durch Flash-Chromatographie (Methanol/Dichlormethan 1:20 - 1:3) gereinigt. Man erhält 50 mg (57 %) der Titelsubstanz. ESI-MS [m/z]: 438, Smp.: 165 - 170 °C

Folgende Verbindung wurde analog dargestellt:

### 4-Ethoxy-7-(4-methylsulfinyl-phenyl)-2-piperazin-1-yl-9-trifluormethyl-pyrido-[3',2':4,5]thieno[3,2-d]pyrimidin

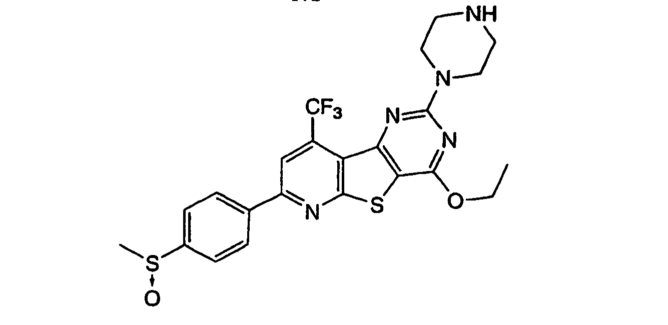

ESI-MS [m/z]: 522

### Beispiel 15

### Die Synthese von 2-Ethylsulfonyl-9-methyl-7-phenyl-4-piperazin-1-yl-pyrido-[3',2':4,5]thieno[3,2-d]pyrimidin

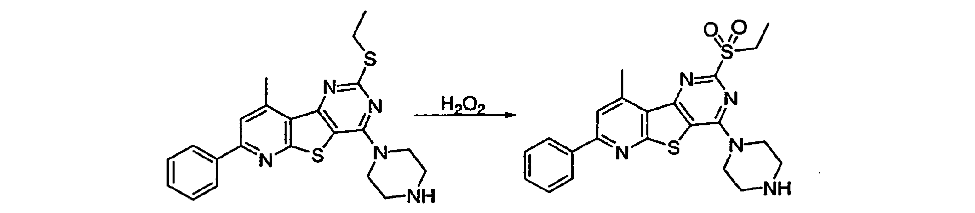

85 mg (0.2 mmol) 2-Ethylthio-9-methyl-7-phenyl-4-piperazin-1-yl-pyrido[3',2':4,5]-thieno[3,2-*d*]pyrimidin, 3 ml Eisessig und 0.12 ml Wasserstoffperoxid (60 %) werden 96 h bei RT gerührt. Nach Entfernen des Lösungsmittels wird der Rückstand durch Flash-Chromatographie (Methanol/Dichlormethan 1:20 - 1:10) gereinigt. Man erhält 16 mg (18 %) der Titelsubstanz. ESI-MS [m/z]: 454, Smp.: 190 °C Zers.

### Beispiel16

### Die Synthese von 7-(3,4-Dimethoxyphenyl)-9-methyl-4-piperazin-1-yl-2-n-propyl-pyrido[3',2':4,5]thieno[3,2-d]pyrimidin

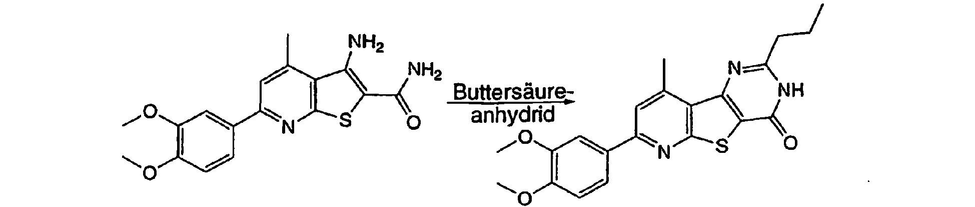

Es werden 1.10 g (3.2 mmol) 3-Amino-6-(3,4-dimethoxyphenyl)-4-methyl-thieno[2,3-*b*]pyridin-2-carbonsäureamid und 10.0 ml (89.1 mmol) Buttersäureanhydrid in 10 ml Toluol 6 h unter Rückfluß erhitzt. Nach Abkühlen wird der Niederschlag abgesaugt, mit ca. 5 ml Ethanol gewaschen und trocken gesaugt. Der Rückstand wird in 5.0 ml Natriumethylat-Lösung (21 %) 6 h unter Rückfluß erhitzt. Nach Abkühlen wird mit HCl (10%) unter Kühlung auf pH 6 angesäuert. Anschließend wird der Niederschlag abgesaugt, mit ca. 30 ml Wasser gewaschen, trocken gesaugt und i. Vak. bei 50 °C getrocknet. Man erhält 0.59 g (47 %) 7-(3,4-Dimethoxyphenyl)-9-methyl-2-propyl-pyrido[3',2':4,5]thieno-[3,2-*d*]pyrimidin-4-on

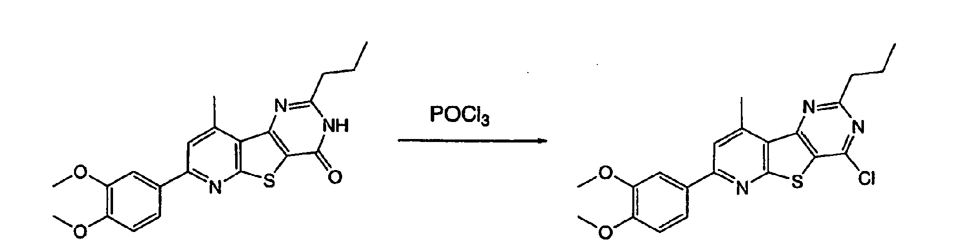

0.57 g (1.4 mmol) 7-(3,4-Dimethoxyphenyl)-9-methyl-2-propyl-pyrido[3',2':4,5]thieno-[3,2-*d*]pyrimidin-4-on werden in 8 ml (86.4 mmol) Phosphoroxychlorid 9 h unter Rückfluß erhitzt. Anschließend wird auf ca. 50 g Eiswasser gegossen. Der Niederschlag wird abgesaugt, mit ca. 250 ml Wasser gewaschen, trocken gesaugt und i. Vak. bei 50 °C getrocknet. Man erhält 0.58 g (96 %) 4-Chlor-7-(3,4-dimethoxyphenyl)-9-methyl-2-propyl-pyrido[3',2':4,5]thieno[3,2-*d*]pyrimidin.

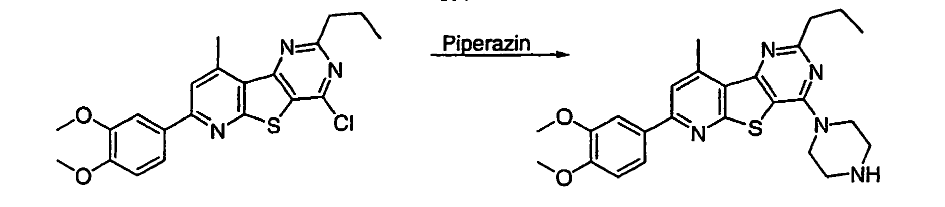

0.42 g (1.0 mmol) 4-Chlor-7-(3,4-dimethoxyphenyl)-9-methyl-2-propyl-pyrido-[3',2':4,5]thieno[3,2-*d*]pyrimidin und 0.37 g (4.3 mmol) Piperazin in 8 ml Tetrahydrofuran werden 4 h bei RT gerührt. Nach Entfernen des Lösungsmittels wird der Rückstand in 10 ml Wasser suspendiert und abgesaugt. Der Rückstand wird in ca. 50 ml Dichlormethan suspendiert und filtriert. Das Filtrat wird mit insgesammt 30 ml Wasser gewaschen, über Natriumsulfat getrocknet und das Lösungsmittel entfernt. Der Rückstand wird aus Essigsäureethylester umkristallisiert. Man erhält 0.17 g (37 %) der Titelsubstanz.

Folgende Verbindungen wurden nach Beispiel 16 erhalten (Tabelle 7)

**Tabelle7**

| **Struktur** | **M** **[g/mol]** | **ESI MS** **[m/z]** | **Smp.** **[°C]** |
|---|---|---|---|
| 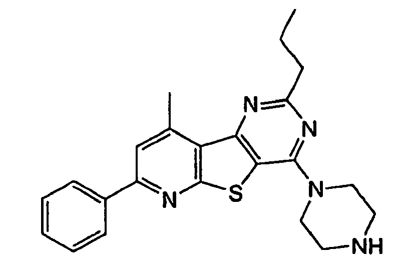 | 403.54 | 404 [M+H]⁺ | 162 - 163 |
| 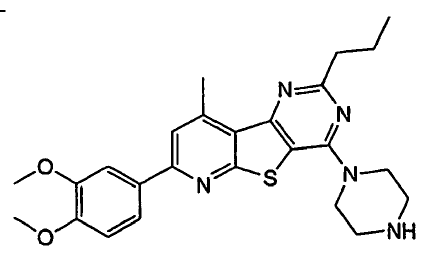 | 463.60 | 464 [M+H]⁺ | 130 - 132 |
| 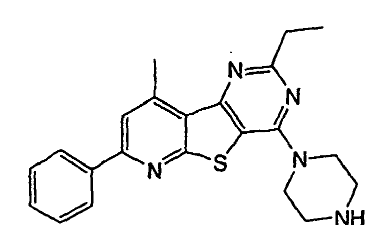 | 389.52 | 390 [M+H]⁺ | 155-158 |

### Beispiel 17

### Die Synthese von 4-Ethoxy-9-trifluormethyl-7-(1-oxy-pyridin-4-yl)-2-piperazino-pyrido[3',2':4,5]-thieno[3,2-d]pyrimidin

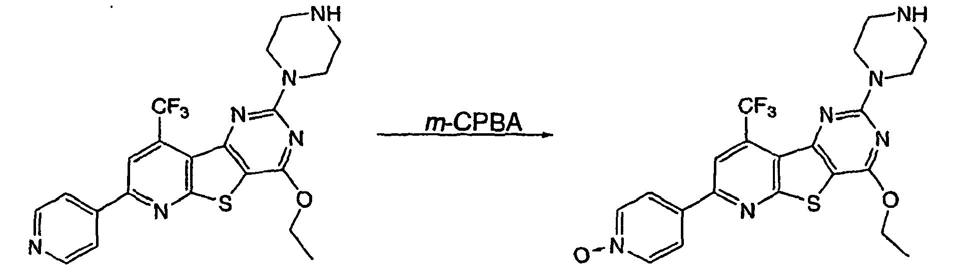

0.4 g (1 mmol) 4-Ethoxy-9-trifluormethyl-7-(pyridin-4-yl)-2-piperazino-pyrido[3',2':4,5]-thieno[3,2-d]pyrimidin werden in 10 ml Dichlormethan mit 0.5 g *m*-Chlorperbenzoesäure 3-5 h im Ultraschallbad erwärmt. Es werden 250 ml Dichlormethan zugegeben, mit Natriumhydrogensulfit-Lösung und anschließend mit Natriumhydrogencarbonat-Lösung gewaschen. Die organische Phase wird über Na₂SO₄ getrocknet und das Lösungsmittel entfernt. Man erhält 0.4 g (97 %) der Titelsubstanz.

Folgende Verbindungen wurden nach Beispiel 17 erhalten (Tabelle 8)

**Tabelle8**

| **Struktur** | **M** **[g/mol]** | **ESI MS** **[m/z]** | **Smp.** **[°C]** |
|---|---|---|---|
| 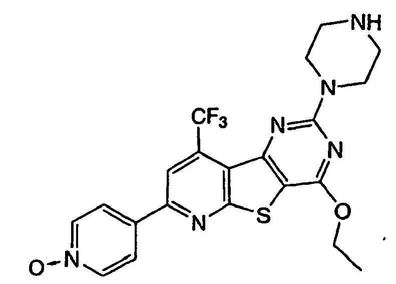 | 476.47 | 477 [M+H]⁺ | 215 |
| 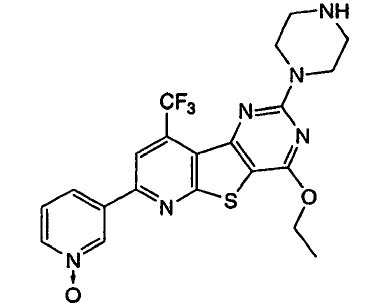 | 476.47 | 477 [M+H]⁺ | 261 |
| 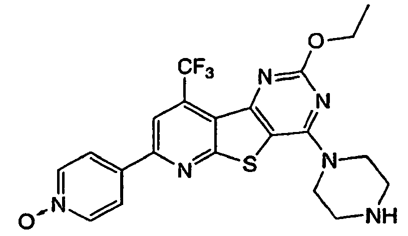 | 476.47 | 477 [M+H]⁺ | 250 - 251 |

### Beispiel 18

### Die Synthese von 2-Ethoxy-9-methyl-7-phenyl-4-piperazin-1-yl-pyrido[3',2':4,5]-furo[3,2-d]pyrimidin

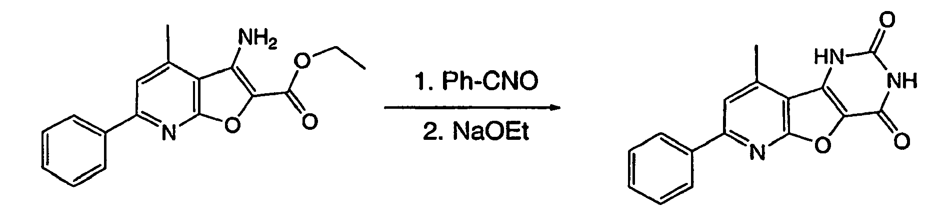

Zu 1.0 g (3.4 mmol) 3-Amino-4-methyl-6-phenyl-furo[2,3-*b*]pyridin-2-carbonsäure-ethylester (beschrieben in Wagner G., Prantz J. *Pharmazie* **1990,** *45*, 213-214) in 20 ml abs. Aceton werden 1.0 g (6.8 mmol) Benzoylisocyanat gegeben und 30 Minuten bei Raumtemperatur gerührt. Das Lösungsmittel wird entfernt und der nach Zugabe von Wasser ausgefallene Niederschlag abgesaugt und getrocknet. Der erhaltene Niederschlag wird mit 0.46 g (6.8 mmol) NaOEt und 20 ml Ethanol 15 min unter Rückfluss erhitzt. Anschließend werden 20 ml Wasser zugegeben, der Niederschlag abgesaugt, mit wenig Ethanol gewaschen und getrocknet. Man erhält 0.85 g (85 %) 9-Methyl-7-phenyl-pyrido[3',2':4,5]furo[3,2-*d*]pyrimidin-2,4-dion

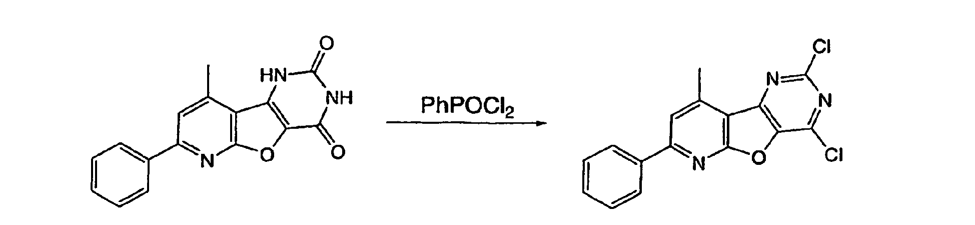

0.85 g (2.9 mmol) 9-Methyl-7-phenyl-pyrido[3',2':4,5]furo[3,2-*d*]pyrimidin-2,4-dion und 2.4 ml (17.4 mmol) Dichlorphenylphosphinoxid werden 6 h bei 180 °C erhitzt. Anschließend wird auf ca. 20 g Eiswasser gegossen und mit gesättigter Natriumhydrogencarbonat-Lösung neutralisiert. Der Niederschlag wird abgesaugt, mit ca. 100 ml Wasser gewaschen, trocken gesaugt und i. Vak. bei 50 °C getrocknet. Man erhält 0.88 g (92 %) 2,4-Dichlor-9-methyl-7-phenyl-pyrido[3',2':4,5]furo[3,2-*d*]pyrimidin.

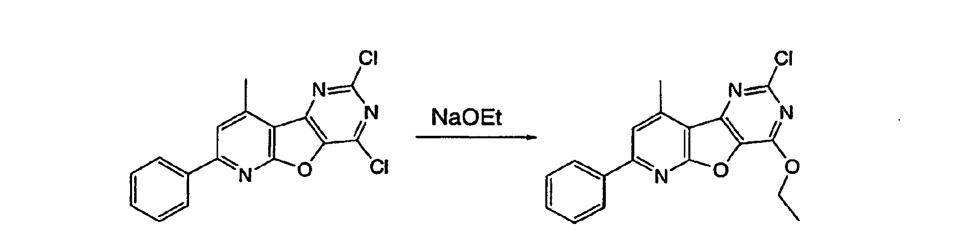

0.2 g (0.6 mmol) 2,4-Dichlor-9-methyl-7-phenyl-pyrido[3',2':4,5]furo[3,2-*d*]pyrimidin und 0.12 g (1.8 mmol) Natriumethylat werden in 50 ml abs. Ethanol 24 h bei RT gerührt. Anschließend wird der Niederschlag abgesaugt, mit ca. 40 ml Wasser gewaschen und getrocknet. Nach Flashchromatographie erhält man 94 mg (46 %) 2-Chlor-4-ethoxy-9-methyl-7-phenyl-pyrido[3',2':4,5]furo[3,2-*d*]pyrimidin.

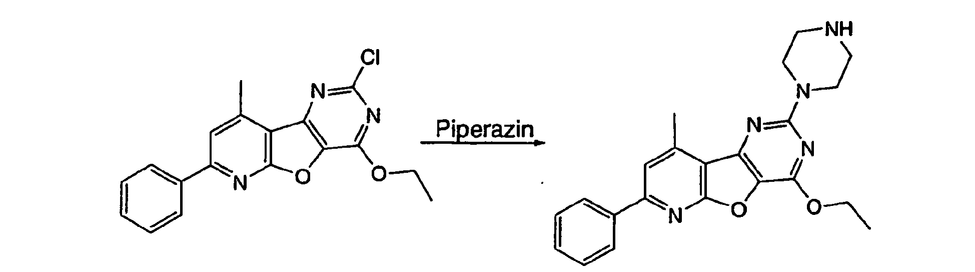

90 mg (0.26 mmol) 2-Chlor-4-ethoxy-9-methyl-7-phenyl-pyrido[3',2':4,5]furo[3,2-*d*]pyrimidin und 91 mg (1.1 mmol) Piperazin werden in 10 ml Toluol 8 h unter Rückfluß erhitzt. Anschließend wird das Lösungsmittel entfernt und der Rückstand in Dichlormethan suspendiert. Die organische Phase wird mit gesättigter Natriumcarbonat-Lösung gewaschen, über Natriumsulfat getrocknet und das Lösungsmittel entfernt. Der Rückstand wird durch Flash-Chromatographie (Ethanol/Chloroform 1:5) gereinigt. Man erhält 52 mg (51 %) der Titelsubstanz. ESI-MS [m/z]: 390, Smp.: 131 - 132 °C

### Beispiel 19

### 2-Ethoxy-9-methyl-7-phenyl-4-piperazin-1-yl-pyrido[3',2':4,5]furo[3,2-d]pyrimidin wurde analog Beispiel 11 erhalten.

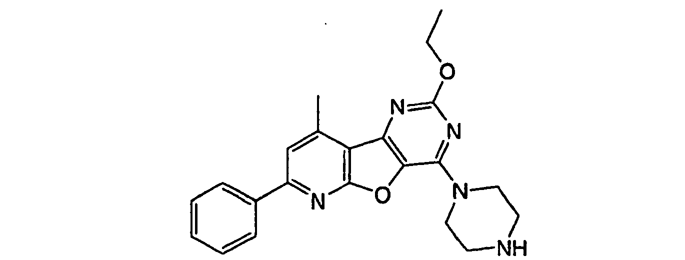

MS [m/z]: 390, Smp.: 175 °C

### Beipspiel 20

### 4-Ethoxy-9-trifluormethyl-2-piperazin-1-yl-7-(4-(1H-pyrazol-1-yl)-phenyl)-pyrido-[3',2':4,5]thieno[3,2-d]pyrimidin

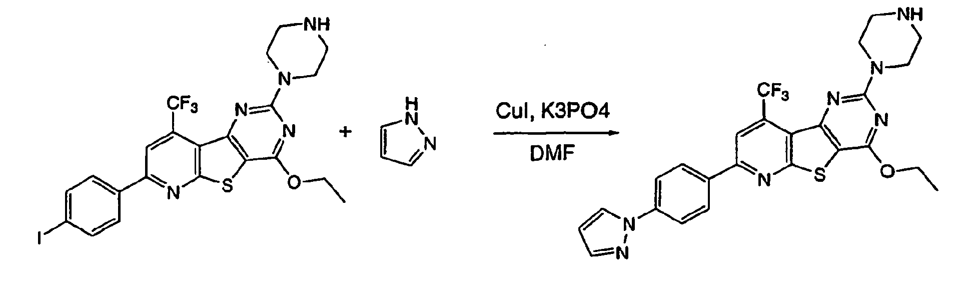

Unter Stickstoff-Atmosphäre werden 0.30 g (0.5 mmol) 4-Ethoxy-9-trifluormethyl-7-(4-iod-phenyl)-2-piperazin-1-yl-pyrido[3',2':4,5]thieno[3,2-d]pyrimidin, 0.14 g (2.1 mmol) Pyrrazol, 0.22 g (1.0 mmol) Kaliumphosphat-tribasisch, 0.06 g (0.3 mmol) Kupfer(I)-iodid und 0.014 g (0.1 mmol) (1*R*,2*R*)-N,N'-Dimethyl-1,2-cyclohexandiamin in 5 ml N,N-Dimethylformamid (trocken) 24 h bei 110°C gerührt. Das Lösungsmittel wird i. Vak. entfernt und der Rückstand in 20 ml Wasser suspendiert, abgesaugt, mit 100 ml Wasser gewaschen und trocken gesaugt. Nach Reinigung mittels Flash-Chromatographie (Methanol/Dichlormethan 1:10) werden 0.04 g (15%) 4-Ethoxy-9-trifluormethyl-2-piperazin-1-yl-7-(4-(1H-pyrazol-1-yl)-phenyl)-pyrido[3',2':4,5]thieno-[3,2-d]pyrimidin erhalten.

Folgende Verbindungen wurden nach Beispiel 20 erhalten (Tabelle 9)

**Tabelle9**

| **Struktur** | **M** **[g/mol]** | **ESI MS** **[m/z]** [M+H]⁺ | **Smp.** **[°C]** |
|---|---|---|---|
| 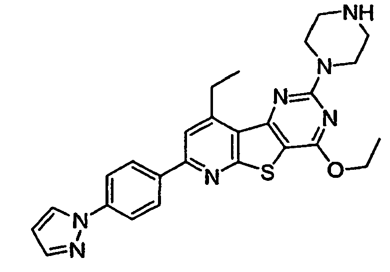 | 485.60 | 486 | 189 |
| 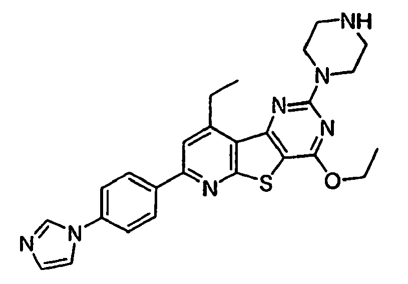 | 485.60 | 486 | 202 - 203 |
| 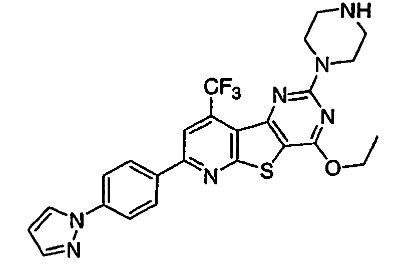 | 525.54 | 527 | 235 - 236 |
| 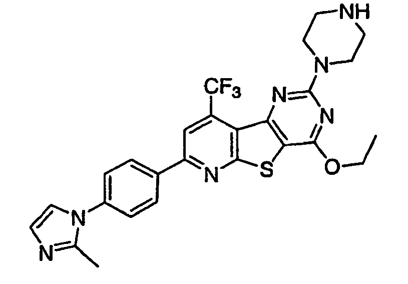 | 539.57 | 540 | 232 - 233 |
| 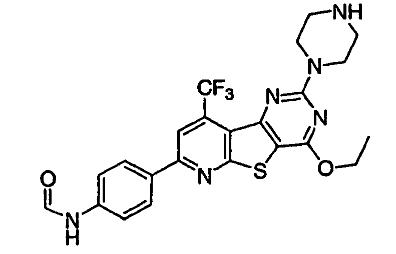 | 502.51 | 503 | 257 |
| 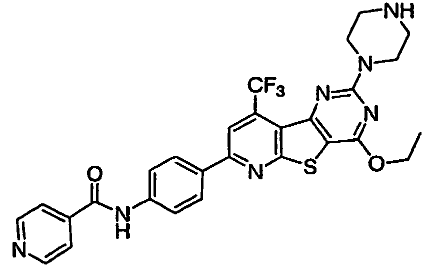 | 579.60 | 580 | 209 |
| 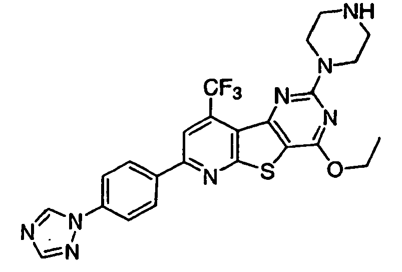 | 526.54 | 527 | 269 - 270 |
| 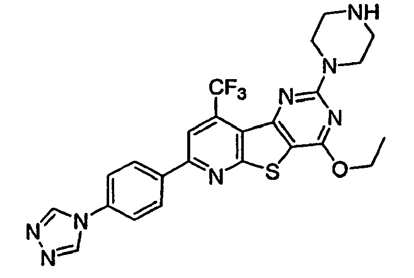 | 526.54 | 527 | 252 - 253 |
| 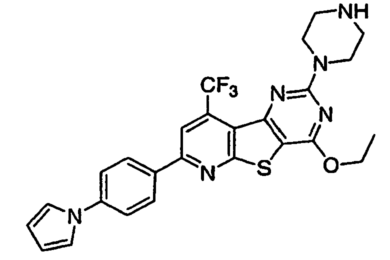 | 524.56 | 525 | 223 - 224 |

### Beispiel 21

### Die Synthese von 8-(4-Aminobenzyl)-4-ethoxy-7,9-dimethyl-2-piperazin-1-yl-pyrido[3',2':4,5]thleno[3,2-d]pyrimidin

Zu 0.36 g (0.8 mmol) 4-Ethoxy-7,9-dimethyl-8-(4-nitrobenzyl)-2-piperazin-1-yl-pyrido[3',2':4,5]thieno[3,2-*d*]pyrimidin in 3 ml Ethanol werden 40 mg 10 % Pd/C und 0.5 ml (10 mmol) Hydraziniumhydroxid gegeben. Es wird 10 h unter Rückfluß erhitzt, das Lösungsmittel entfernt und der Rückstand mit 50 ml Chloroform aufgenommen. Nach Filtrieren wird das Lösungsmittel entfernt und der Rückstand aus Chloroform / Cyclohexan umkristallisiert. Man erhält 0.16 g (45 %) der Titelsubstanz.

Folgende Verbindungen wurden nach Beispiel 21 erhalten (Tabelle 10)

**Tabelle10**

| **Struktur** | **M** **[g/mol]** | **ESI MS** **[m/z]** [M+H]⁺ | **Smp.** **[°C]** |
|---|---|---|---|
| 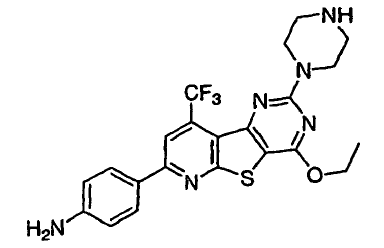 | 474.50 | 475 | 229 - 233 |
| 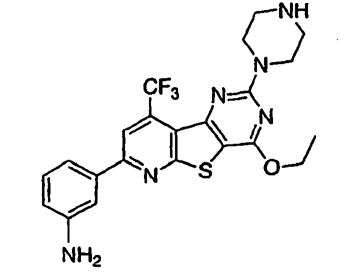 | 474.50 | 475 | n.d. |
| 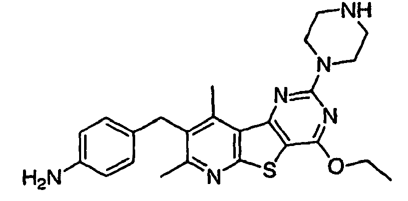 | 448.58 | 449 | 195 - 202 |

### Beispiel 22

### Die Synthese von 4-Ethoxy-9-ethyl-7-(4-(pyrimidyl-2-yl)piperazin-1-yl)-2-piperazin-1-yl-pyrido[3',2':4,5]thieno[3,2-d]pyrimidin

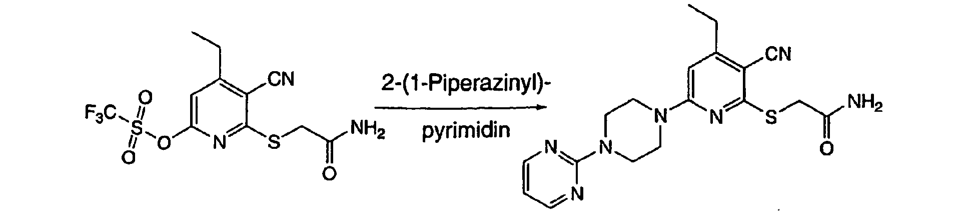

1.6 g (4.34 mmol) 6-(Carbamoylmethylthio)-5-cyano-4-ethylpyridin-2-yl-trifluormethansulfonat, zugänglich z.B. analog US 2004/0180922, werden mit 1.45 g (8.8 mmol) 2-(1-Piperazinyl)pyrimidin in 20 ml trockenem Dioxan 5 h bei RT gerührt. Das Produkt wird mit 500 Vol% Wasser gefällt. Nach Waschen (Wasser) und Trocknen im Vakuum über Nacht werden 1.58 g (95 %) 2-(3-Cyano-4-ethyl-6-(4-(pyrimidyl-2-yl)piperazin-1-yl)pyridin-2-ylthio)acetamid als graues Pulver erhalten.

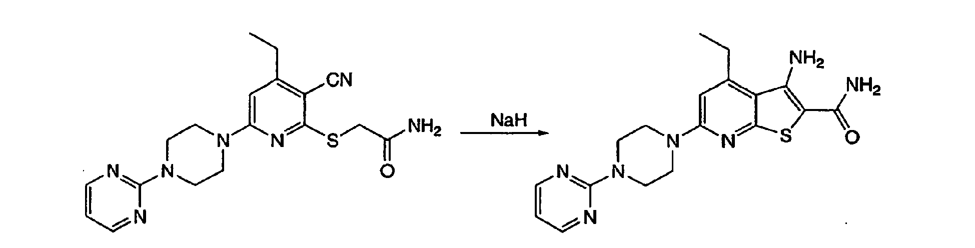

1.58 g (4.13 mmol) 2-(3-Cyano-4-ethyl-6-(4-pyrimidyl-2-yl)piperazin-1-yl)pyridin-2-ylthio)acetamid werden in 25 ml trockenem DMF suspendiert und mit 100 mg NaH bei Raumtemperatur 3 h gerührt. Nach vorsichtigem Eingießen der Suspension in 300 ml Wasser wird das Produkt abgesaugt, mit Wasser gewaschen und im Vakuum getrocknet. Es werden 780 mg (45 %) 3-Amino-4-ethyl-6-(4-(pyrimidin-2-yl)piperazin-1-yl)thieno[2,3-b]pyridin-2-carboxamid als ockerfarbenes Pulver erhalten.

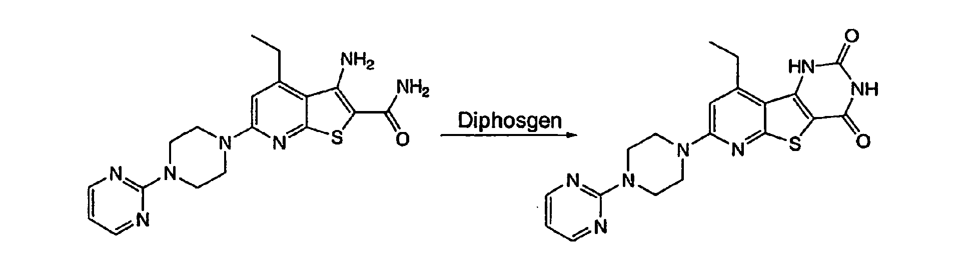

Die Umsetzung des 3-Amino-4-ethyl-6-(4-(pyrimidin-2-yl)piperazin-1-yl)thieno[2,3-b]pyridin-2-carboxamid mit Diphosgen zum 9-Ethyl-7-(4-(pyrimidin-2-yl)piperazin-1-yl)pyrido[3',2':4,5]thieno[3,2-*d*]pyrimidin-2,4-dion erfolgt analog Beispiel 1.
Dabei werden aus 900 mg (2.35 mmol) Edukt 740 mg (77 %) Rohprodukt erhalten, welches ohne weitere Reinigung der Chlorierung unterworfen wird.

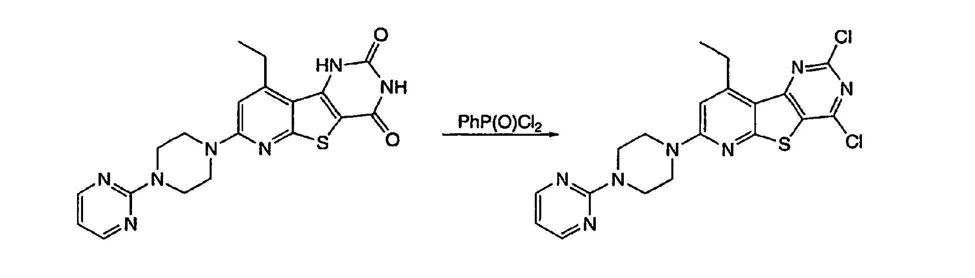

1.5 g (3.7 mmol) (9-Ethyl-7-(4-(pyrimidin-2-yl)piperazin-1-yl)pyrido[3',2':4,5]thieno-[3,2-*d*]pyrimidin-2,4-dion werden analog Beispiel 1 mit Dichlorphenylphosphinoxid umgesetzt. Der nach 30 h bei 140 °C erhaltene Rückstand wird flashchromatographisch gereinigt (Methylenchlorid/ Methanol). Es werden 400 mg (24 %) 2,4-Dichlor-9-ethyl-7-(4-(pyrimidin-2-yl)piperazin-1-yl)pyrido[3',2':4,5]thieno[3,2-*d*]pyrimidin als farbloser Feststoff erhalten.

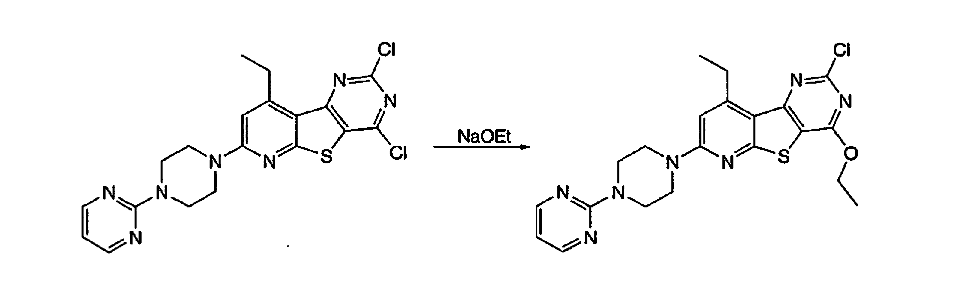

Aus 200 mg (0.45 mmol) der Dichlorverbindung werden analog Beispiel 1 180 mg Produktgemisch erhalten, welches ohne das 2-Chlor-4-ethoxy-9-ethyl-7-(4-(pyrimidin-2-yl)piperazin-1-yl)pyrido[3',2':4,5]thieno[3,2-*d*]pyrimidin zu isolieren weiter umgesetzt wird.

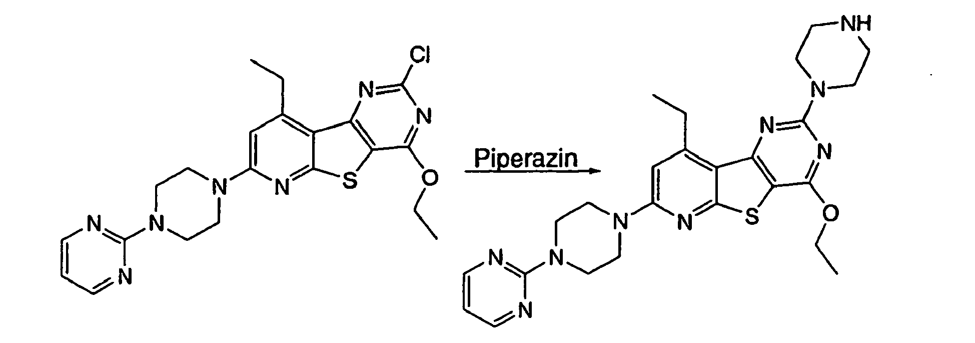

Aus 170 mg 2-Chlor-4-ethoxy-9-ethyl-7-(4-(pyrimidin-2-yl)piperazin-1-yl)pyrido-[3',2':4,5]thieno[3,2-*d*]pyrimidin werden analog Beispiel 1 nach flashchromatografischer Aufreinigung 30 mg (13% bzgl. der Dichlorverbindung) als gelbes Pulver erhalten.

Die nach Beispiel 22 dargestellten Verbindungen sind in Tabelle 11 zusammengefasst.

**Tabelle 11**

| Struktur | M [g/mol] | MS (ESI) [m/z] | Smp. [°C] | Reinheit [%] |
|---|---|---|---|---|
| 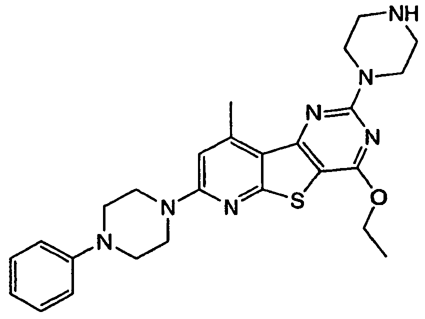 | 489.64 | 490 [M+H]⁺ | 141 | >96 |
| 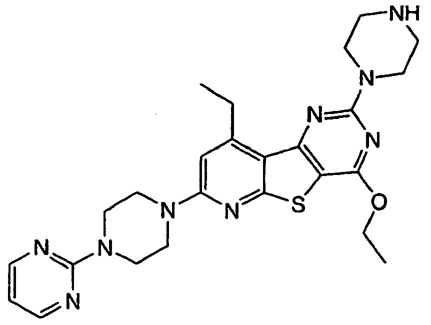 | 505.64 | 506 [M+H]⁺ | 191 - 192 | >96 |
| 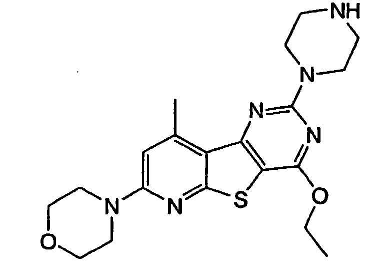 | 414.52 | 415 [M+H]⁺ | 153 - 154 | >95 |
| 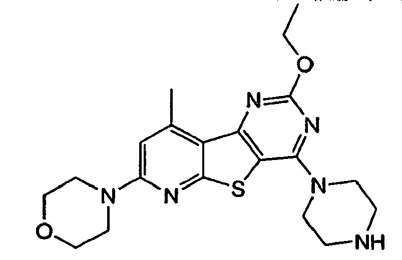 | 414.52 | 415 [M+H]⁺ | 270 | >96 |
| 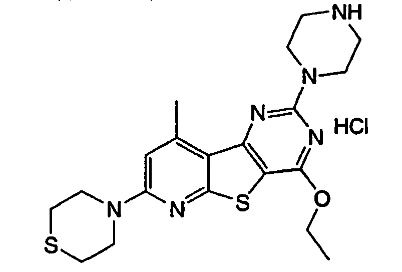 | 467.05 | 431 [M-CI]⁺ | 333 | 96 |

### Beispiel 23

### Untersuchungen zur PDE4A-Hemmung und Hemmung der TNFα-Freisetzung

### Methodenbeschreibung

Die Verbindungen der allgemeinen Formeln 1a, 1b, 1c und 1d sind starke Inhibitoren der Phosphodiesterase 4 und/oder der Freisetzung von TNFα.

Die Volllängen-Formen der Phosphodiesterasen wurden aus zytosolischen Präparationen der humanen monozytischen Zellinien U937 oder HL60 erhalten und durch Anionen-Austauschchromatographie an einer Poros HQ Säule (starker Anionen-Austauscher) entsprechend einer adaptierten Methode von Lavan B. E., Lakey T., Houslay M. D. Biochemical Pharmacology, (1989), 38(22), 4123-4136*., und* Silver P. J et al., Eur. J. Pharmacol. (1988) 150: 85-94*, and* T. J. Torphy et al., J. Pharm. Exp. Ther. (1992), 263: 1195-1205 teilweise gereinigt.

Humane rekombinante PDE-4A katalytische Domäne, die die Aminosäuren 342 - 704 umfasst, wurde in Escherichia coli kloniert und exprimiert, wie beschrieben durch W. Richter et al., Protein Expression and Purification (2000) 19: 375-383*.* Die anderen Phosphodiesterasen wurden entweder aus Zellinien humanen Ursprungs gewonnen (TPH1 Monozyten Linie für PDE-1, MCF7 für PDE-5), oder in SF9 Insektenzellen rekombinant exprimiert (für PDE-3A, PDE-3B, PDE-7A und PDE-4D), entsprechend einer adaptierten Methode von Luckow, V. A. et al., in: Recombinant DNA Technology&Applications., (1991) eds. Prokop, Bajpai, R. K.&Ho, C. S., pp 97-152*.*

Die Phosphodiesterase-Aktivität wurde wie beschrieben durch den Hersteller Amersham Biosciences und durch Horton J.K and Baxendale P. M., Methods Mol. Biol. (1995) 41: 91-105 mittels des SPA-Assays bestimmt.

Die Reaktionsmischungen enthielten 100 mM Tris-HCl (pH 7.5), 5 mM MgCl₂, 3.8 mM 2-Mercaptoethanol, und 1 µM cAMP oder cGMP als Substrat, die Inhibitoren in unterschiedlichen Konzentrationen und ferner weitere Komponenten, die für die Detektion der individuellen Isoenzyme benötigt wurden (s. unten). Die Reaktion wurde durch Zugabe des Enzyms gestartet. Das Arbeitsvolumen beträgt 100 µl in den Wells einer weißen 96-Well-Mikrotiterplatte. Die Testsubstanzen wurden als Stock-Lösungen in DMSO hergestellt. Die DMSO-Konzentration im Reaktionsansatz war 1 % v/v. Bei dieser DMSO-Konzentration wird die PDE-Aktivität nicht beeinträchtigt. Nach Start der Reaktion durch Zugabe von Enzym, wurden die Proben bei 22 °C für 20 min inkubiert. Die Reaktion wurde gestoppt durch Zugabe von SPA-Bead Suspension, die nach Hersteller vorgegebene Zinksulfatkonzentration enthält. Danach lässt man die Beads für 30 min sedimentieren und wertet die Platten am Lumineszenz Mikrotiterplattenreader Fluostar Optima (BMG Labtechologies) aus.

[³H]-cAMP wurde als Substrat für die Bestimmung der Aktivität von PDE-2, -3, -4 und -7 eingesetzt und [³H]-cGMP für die Bestimmung der Aktivität von PDE-5. Die unspezifischen Enzymaktivitäten wurden in Anwesenheit von 100 µM Rolipram (für PDE-4) und in Anwesenheit von 100 µM IBMX (für PDE-3 und -5) bestimmt und von den Testwerten subtrahiert. Die Inkubationsproben des PDE-3 Assays enthalten 10 µM Rolipram, um mögliche Kontaminationen durch PDE-4 zu inhibieren. PDE-2-Aktivität wird unter Verwendung eines SPA-Assays von Amersham Biosciences in Gegenwart des PDE-2 Aktivators 5 µM cGMP getestet.

IC₅₀-Werte im Bereich von 10⁻⁹ bis 10⁻⁵ M wurden für die in der Erfindung beschriebenen Inhibitoren bezüglich ihrer inhibitorischen Wirkung auf Phosphodiesterase 4 beobachtet. Die Selektivität zu den PDE's 2, 3 und 5 weist einen Faktor von 100 bis 10 000 auf.

Die Stimulierung isolierter Leukozyten für die Freisetzung von Zytokinen kann auf verschiedenen Wegen erfolgen. Lipopolysaccharide (LPS) stellen einen Stimulus für die Untersuchung der Freisetzung von TNFα dar. LPS ist Bestandteil bakterieller Zellwände und wird beim Abtöten der Bakterien (durch Antibiotika oder das natürliche Immunsystem) freigesetzt. LPS stimuliert insbesondere die Aktivität phagozytierender Leukozyten (Gewebsmakrophagen, Granulozyten, Monozyten) und verursacht die Infiltration von Leukozyten vom peripheren Blut in das betroffene Gewebe. Ein Zytokin von besonderer Bedeutung für diese Mechanismen ist TNFα, das in großen Mengen durch die betroffenen Zellen sezerniert wird. Hauptquelle dabei sind Monozyten und Makrophagen. TNFα initiiert und prolongiert den Entzündungsprozess im Zusammenspiel mit anderen Mediatoren.

Für die Untersuchung des Effektes auf die LPS-induzierte TNFα-Freisetzung wurde eine Methode verwendet, die von Marx, D., Tassabehji, M., Heer, S., Hüttenbrink, K.-B., and I. Szelenyi, Pulmonary Pharmacology & Therapeutics (2002) 15, 7-15 beschrieben wurde. Die Methode in Kürze: Humanes Blut wurde von verschiedenen Spendern entnommen, durch Zusatz von 10 mM Na-Citrat ungerinnbar gemacht und 1:5 mit RPMI 1640 Zellkulturmedium verdünnt. Die Testsubstanzen wurden den Blutproben in verschiedenen Konzentrationen zugefügt. 15 min später wurden die Leukozyten durch Zusatz von Lipopolysacchariden (LPS) aus Salmonella abortus equi in einer Endkonzentration von 1 µg/ml stimuliert. Nach Inkubation der Testansätze für 24 h bei 37 °C und unter 5% CO₂ in wassergesättigter Luft, wurde das Blut zentrifugiert und die Konzentration an TNFα im zellfreien Überstand unter Verwendung eines käuflichen ELISA (BD Biosciences) nach Angaben des Herstellers exakt vermessen.

Die Verbindungen der allgemeinen Formeln 1 a, 1 b, 1 c und 1 d sind starke Inhibitoren der Phosphodiesterase 4 und der Freisetzung von TNFalpha. Ihr therapeutisches Potential in vivo ist erwiesen durch die Inhibierung der asthmatischen Spät-Phasen Reaktion (Eosinophilie) in der Lungen-Spülflüssigkeit des Meerschweinchens und durch die Beeinflussung der Allergen-induzierten Gefäßpermeabilität in aktiv sensitierten braunen Ratten (R. Norwegicus).

Die nachfolgende Aufzählung beinhaltet erfindungsgemäße Substanzen der allgemeinen Formeln 1 a, 1b, 1 c und 1 d, die im PDE-4A-Hemmassay einen IC₅₀-Wert <2 nM aufweisen:
4-Ethoxy-9-ethyl-7-(4-(pyrimidyl-2-yl)piperazin-1-yl)-2-piperazin-1-yl-pyrido[3',2':4,5]thieno[3,2-d]pyrimidin
4-Ethoxy-9-ethyl-7-(4-methyl-phenyl)-2-(piperazin-1-yl)-pyrido[3',2':4,5]thieno[3,2-d]pyrimidin
2-Ethoxy-9-ethyl-7-(4-methyl-phenyl)-4-(piperazin-1-yl)-pyrido[3',2':4,5]thieno[3,2-d]pyrimidin
4-Ethoxy-7-(4-ethoxy-phenyl)-9-ethyl-2-(piperazin-1-yl)-pyrido[3',2':4,5]thieno[3,2-d]pyrimidin
7-(4-Ethoxy-phenyl)-9-ethyl-2-(piperazin-1-yl)-4-*n*-propoxy-pyrido[3',2':4,5]thieno[3,2-d]pyrimidin
2-Ethoxy-7-(4-ethoxy-phenyl)-9-ethyl-4-(piperazin-1-yl)-pyrido[3',2':4,5]thieno[3,2-d]pyrimidin
4-Ethoxy-9-ethyl-7-(4-methylthio-phenyl)-2-(piperazin-1-yl)-pyrido[3',2':4,5]thieno[3,2-d]pyrimidin
4-Ethoxy-7-(4-methyl-phenyl)-9-*iso*-propyl-2-(piperazin-1-yl)-pyrido[3',2':4,5]thieno-[3,2-d]pyrimidin
4-Ethoxy-9-ethyl-7-[4-(4-methoxyphenoxy)phenyl]-2-(piperazin-1-yl)-pyrido[3',2':4,5] thieno[3,2-d]pyrimidin
4-Ethoxy-9-ethyl-7-(2,3-dihydrobenzo[b][1,4]dioxin-6-yl)-2-(piperazin-1-yl)-pyrido[3',2':4,5]thieno[3,2-d]pyrimidin
2-Ethoxy-9-ethyl-7-(2,3-dihydrobenzo[b][1,4]dioxin-6-yl)-4-(piperazin-1-yl)-pyrido[3',2':4,5]thieno[3,2-d]pyrimidin
4-Ethoxy-9-ethyl-7-(3,4-methylendioxyphenyl)-2-piperazin-1-yl-pyrido[3',2':4,5]thieno-[3,2-d]pyrimidin
4-Ethoxy-7-(4-methoxy-phenyl)-2-(piperazin-1-yl)-9-trifluormethyl-pyrido[3',2':4,5]thieno[3,2-d]pyrimidin
4-Ethoxy-7-(4-methyl-phenyl)-2-(piperazin-1-yl)-9-trifluormethyl-pyrido[3',2':4,5]thieno[3,2-d]pyrimidin
7-(2,3-Dihydrobenzo[b][1,4]dioxin-6-yl)-4-ethoxy-2-(piperazin-1-yl)-9-trifluormethyl-pyrido[3',2':4,5]thieno[3,2-d]pyrimidin
4-Ethoxy-7-(4-(1*H*-imidazol-1-yl)phenyl)-2-(piperazin-1-yl)-9-trifluormethyl-pyrido[3',2':4,5]thieno[3,2-d]pyrimidin
4-[4-Ethoxy-2-(piperazin-1-yl)-9-trifluormethyl-pyrido[3',2':4,5]thieno[3,2-d]pyrimidin-7-yl]-benzoesäureethylester
4-Ethoxy-2-(piperazin-1-yl)-7-(4-(piperidin-1-yl)phenyl)-9-trifluormethyl-pyrido[3',2':4,5]thieno[3,2-d]pyrimidin
4-Ethoxy-9-ethyl-7-(4-methoxyphenyl)-2-(piperazin-1-yl)-pyrido[3',2':4,5]thieno[3,2-d]pyrimidin
4-Ethoxy-7-(3,4-methylendioxyphenyl)-2-piperazin-1-yl-9-trifluormethyl-pyrido[3',2':4,5]thieno-[3,2-d]pyrimidin
4-Ethoxy-7-(4-methylthiophenyl)-2-(piperazin-1-yl)-9-trifluormethyl-pyrido[3',2':4,5]thieno[3,2-d]pyrimidin
5,6-Dihydro-11-ethoxy-3'-methoxy-9-piperazin-1-yl-7-trifluormethyl-benzo[h]pyrimido[4',5':4,5]thieno[2,3-b]chinolin
4-Ethoxy-7-(4-(1H-pyrazol-1-yl)-phenyl)-9-ethyl-2-piperazin-1-yl-pyrido[3',2':4,5]thieno[3,2-d]pyrimidin
4-Ethoxy-7-(4-iod-phenyl)-2-(piperazin-1-yl)-9-trifluormethyl-pyrido[3',2':4,5]thieno[3,2-d]pyrimidin
4-Ethoxy-7-(4-(1H-pyrazol-1-yl)-phenyl)-2-piperazin-1-yl-9-trifluormethyl-pyrido[3',2':4,5]thieno[3,2-d]pyrimidin
7-(4-Brom-phenyl)-4-ethoxy-9-ethyl-2-(piperazin-1-yl)-pyrido[3',2':4,5]thieno[3,2-d]pyrimidin
4-Ethoxy-9-ethyl-7-(4-iod-phenyl)-2-(piperazin-1-yl)-pyrido[3',2':4,5]thieno[3,2-d]pyrimidin
4-Ethoxy-7-(3-nitro-phenyl)-2-piperazin-1-yl-9-triluormethyl-pyrido[3',2':4,5]thieno[3,2-d]pyrimidin
7-(3-Cyano-phenyl)-4-ethoxy-2-piperazin-1-yl-9-trifluormethyl-pyrido[3',2':4,5]thieno[3,2-d]pyrimidin
N-(4-(4-Ethoxy-9-trifluormethyl-2-piperazin-1-yl-pyrido[3',2':4,5]thieno[3,2-d]pyrimidin-7-yl)-phenyl)-formamid
N-(4-Ethoxy-9-trifluormethyl-2-piperazin-1-yl-pyrido[3',2':4,5]thieno[3,2-d]pyrimidin-7-yl)-benzamid Trifluoracetat
N'-Hydroxy-4-(4-ethoxy-9-trifluormethyl-2-piperazin-1-yl-pyrido[3',2':4,5]thieno[3,2-d]pyrimidin-7-yl)-benzamidin Bistrifluoracetat
N-(4-(4-Ethoxy-9-trifluormethyl-2-piperazin-1-yl-pyrido[3',2':4,5]thieno[3,2-d]pyrimidin-7-yl)-phenyl)-isonicotinamid
4-Ethoxy-9-trifluormethyl-2-piperazin-1-yl-7-(4-(1H-pyrrol-1-yl)-phenyl)-pyrido[3',2':4,5]thieno[3,2-d]pyrimidin
N-(4-(4-Ethoxy-9-trifluormethyl-2-piperazin-1-yl-pyrido[3',2':4,5]thieno[3,2-d]pyrimidin-7-yl)-phenyl)-nicotinamid-N-oxid
4-(4-Ethoxy-9-trifluormethyl-2-piperazin-1-yl-pyrido[3',2':4,5]thieno[3,2-d]pyrimidin-7-yl)-phenyl-carbaminsäuremethylester
N-(4-(4-Ethoxy-9-trifluormethyl-2-piperazin-1-yl-pyrido[3',2':4,5]thieno[3,2-d]pyrimidin-7-yl)-phenyl)-nicotinamid
7-(3'-Aminobiphen-4-yl)-4-ethoxy-9-methyl-2-piperazin-1-yl-thieno[2,3-d:4,5-d']dipyrimidin
4-Ethoxy-7-(4-[3-furyl]-phenyl)-9-methyl-2-piperazin-1-yl-thieno[2,3-d:4,5-d']dipyrimidin
4-Ethoxy-7-(4-[2-furyl]-phenyl)-9-methyl-2-piperazin-1-yl-thieno[2,3-d:4,5-d']dipyrimidin
4-Ethoxy-9-methyl-2-piperazin-1-yl-7-(4-[3-pyridyl]-phenyl)-thieno[2,3-d:4,5-d']dipyrimidin
4-Ethoxy-9-methyl-2-piperazin-1-yl-7-(4-[4-pyridyl]-phenyl)-thieno[2,3-d:4,5-d']dipyrimidin
7-(4'-Aminobiphen-4-yl)-4-ethoxy-9-methyl-2-piperazin-1-yl-thieno[2,3-d:4,5-d']dipyrimidin
4-Ethoxy-9-methyl-2-piperazin-1-yl-7-(3',4',5'-trimethoxybiphen-4-yl)-thieno[2,3-d:4,5-d']dipyrimidin
4-Ethoxy-9-methyl-7-(4-phenoxy-phenyl)-2-piperazin-1-yl-thieno[2,3-d:4,5-d']dipyrimidin
2'-Amino-4-(4-ethoxy-9-methyl-2-piperazin-1-yl-thieno[2,3-d:4,5-d']dipyrimidin-7-yl)-biphenyl-4'-carbonsäuremethylester
7-(3',4'-Dimethoxybiphen-4-yl)-4-ethoxy-9-methyl-2-piperazin-1-yl-thieno[2,3-d:4,5-d']dipyrimidin
7-(4-[3,5-Dimethyl-isoxazol-4-yl]-phenyl)-4-ethoxy-9-methyl-2-piperazin-1-yl-thieno[2,3-d:4,5-d']dipyrimidin

Die nachfolgende Aufzählung beinhaltet erfindungsgemäße Substanzen der allgemeinen Formeln 1 a, 1b, 1 c und 1 d, die im PDE-4A-Hemmassay einen IC₅₀-Wert von 2 - 50 nM aufweisen:
4-Ethoxy-9-methyl-2-piperazin-1-yl-7-(4-[3-pyrrolyl]-phenyl)-thieno[2,3-d:4,5-d']dipyrimidin
7-(4-[1,2-Dihydroxyethyl]-phenyl)-4-ethoxy-9-methyl-2-piperazin-1-yl-thieno[2,3-d:4,5-d']dipyrimidin
4-Ethoxy-9-methyl-7-phenyl-2-piperazin-1-yl-thieno[2,3-d:4,5-d']dipyrimidin
4-Ethoxy-9-methyl-2-piperazin-1-yl-7-(4-vinyl-phenyl)-thieno[2,3-d:4,5-d']dipyrimidin
4-Ethoxy-9-methyl-2-piperazin-1-yl-7-(4-[N-BOC-3-pyrrolyl]-phenyl)-thieno[2,3-d:4,5-d']dipyrimidin
7-(4-Brom-phenyl)-4-ethoxy-9-methyl-2-piperazin-1-yl-thieno[2,3-d:4,5-d']dipyrimidin
7-(4-*tert*-Butyl-phenyl)-4-ethoxy-9-methyl-2-piperazin-1-yl-thieno[2,3-d:4,5-d']dipyrimidin
4-Ethoxy-9-methyl-2-piperazin-1-yl-7-(4-*iso*-propoxy-phenyl)-thieno[2,3-d:4,5-d']dipyrimidin
4-Ethoxy-9-methyl-2-piperazin-1-yl-7-(4-[5-pyrimidinyl]-phenyl)-thieno[2,3-d:4,5-d']dipyrimidin
7-(4-[4-*n*-Butoxy-phenoxy]phenyl)-4-ethoxy-9-methyl-2-piperazin-1-yl-thieno[2,3-d:4,5-d']dipyrimidin
7-Benzyl-4-ethoxy-9-methyl-2-piperazin-1-yl-thieno[2,3-d:4,5-d']dipyrimidin
4-Ethoxy-7-(4-methoxy-phenyl)-9-methyl-2-piperazin-1-yl-thieno[2,3-d:4,5-d']dipyrimidin
4-Ethoxy-9-methyl-7-(1-naphthyl)-2-piperazin-1-yl-thieno[2,3-d:4,5-d']dipyrimidin
7-(4-Biphenyl)-4-ethoxy-9-methyl-2-piperazin-1-yl-thieno[2,3-d:4,5-d']dipyrimidin
4-Ethoxy-7-(4-[hydroxyl{3-pyridyl}methyl]-phenyl)-9-methyl-2-piperazin-1-yl-thieno[2,3-d:4,5-d']dipyrimidin
4-(4-Ethoxy-9-methyl-2-piperazin-1-yl-thieno[2,3-d:4,5-d']dipyrimidin-7-yl)-biphenyl-4'-carbonsäure Natriumsalz
2'-Amino-4-(4-ethoxy-9-methyl-2-piperazin-1-yl-thieno[2,3-d:4,5-d']dipyrimidin-7-yl)-biphenyl-4'-carbonsäure Natriumsalz
4-(4-Ethoxy-9-methyl-2-piperazin-1-yl-thieno[2,3-d:4,5-d']dipyrimidin-7-yl)-biphenyl-3'-carbonsäuremethylester
4-(4-Ethoxy-9-methyl-2-piperazin-1-yl-thieno[2,3-d:4,5-d']dipyrimidin-7-yl)-biphenyl-3'-carbonsäure Kaliumsalz
4-(4-Ethoxy-9-methyl-2-piperazin-1-yl-thieno[2,3-d:4,5-d']dipyrimidin-7-yl)-biphenyl-4'-carbonsäureethylester
4-Ethoxy-9-methyl-7-morpholino-2-(piperazin-1-yl)-pyrido[3',2':4,5]thieno[3,2-d]pyrimidin
2-Ethoxy-9-methyl-7-morpholino-4-(piperazin-1-yl)-pyrido[3',2':4,5]thieno[3,2-d]pyrimidin
4-Ethoxy-9-ethyl-7-(4-phenyl-piperazin-1-yl)-2-piperazin-1-yl-pyrido[3',2':4,5]thieno[3,2-d]pyrimidin
4-Ethoxy-9-methyl-7-thiomorpholino-2-(piperazin-1-yl)-pyrido[3',2':4,5]thieno[3,2-d]pyrimidin-hydrochlorid
2-Ethoxy-9-ethyl-7-(4-methyl-phenyl)-4-(piperazin-1-yl)-pyrido[3',2':4,5]thieno[3,2-d]pyrimidin
2-(2-Hydroxy-ethoxy)-9-ethyl-7-(4-methyl-phenyl)-4-(piperazin-1-yl)-pyrido[3',2':4,5]thieno[3,2-d]pyrimidin
7-(4-Ethoxy-phenyl)-9-ethyl-4-(piperazin-1-yl)-2-*n*-propoxy-pyrido[3',2':4,5]thieno[3,2-d]pyrimidin
9-*iso*-Butyl-4-ethoxy-7-(4-methyl-phenyl)-2-(piperazin-1-yl)-pyrido[3',2':4,5]thieno-[3,2-d]pyrimidin
7-(4-Chlor-phenyl)-9-ethyl-4-ethoxy-2-(piperazin-1-yl)-pyrido[3',2':4,5]thieno[3,2-d]pyrimidin
4-Ethoxy-7-(4-morpholinophenyl)-2-(piperazin-1-yl)-9-trifluormethyl-pyrido[3',2':4,5]thieno[3,2-d]pyrimidin
4-Ethoxy-2-(piperazin-1-yl)-7-(4-pyridyl)-9-trifluormethyl-pyrido[3',2':4,5]thieno[3,2-d]pyrimidin
4-Ethoxy-7-(1-oxy-pyridin-4-yl)-2-(piperazin-1-yl)-9-trifluormethyl-pyrido[3',2':4,5]thieno[3,2-d]pyrimidin
4-(2-Hydroxy-ethoxy)-7-[(1H-imidazol-1-yl)phenyl]-2-(piperazin-1-yl)-9-trifluormethyl-pyrido[3',2':4,5]thieno[3,2-d]pyrimidin
4-Ethoxy-9-methyl-7-(3,4-methylendioxyphenyl)-2-piperazin-1-yl-pyrido[3',2':4,5]thieno-[3,2-d]pyrimidin
2-Ethoxy-9-methyl-7-(3,4-methylendioxyphenyl)-4-piperazin-1-yl-pyrido[3',2':4,5]thieno-[3,2-d]pyrimidin
4-Ethoxy-7-(4-methoxy-phenyl)-9-methyl-8-(4-nitro-benzyl)-2-(piperazin-1-yl)-pyrido[3',2':4,5]thieno[3,2-d]pyrimidin
7-(4-Brom-phenyl)-4-ethoxy-9-methyl-2-(piperazin-1-yl)-pyrido[3',2':4,5]thieno[3,2-d]pyrimidin
4-Ethoxy-7-(4-nitro-phenyl)-2-piperazin-1-yl-9-trifluormethyl-pyrido[3',2':4,5]thieno[3,2-d]pyrimidin
7-(4-Amino-phenyl)-4-ethoxy-2-piperazin-1-yl-9-trifluormethyl-pyrido[3',2':4,5]thieno[3,2-d]pyrimidin
9-Difluormethyl-7-(4-chlor-phenyl)-4-ethoxy-2-piperazin-1-yl-pyrido[3',2':4,5]thieno[3,2-d]pyrimidin
7-(4-Cyano-phenyl)-4-ethoxy-2-piperazin-1-yl-9-trifluormethyl-pyrido[3',2':4,5]thieno[3,2-d]pyrimidin
4-Ethoxy-9-methyl-7-(4-methylthio-phenyl)-2-piperazin-1-yl-pyrido[3',2':4,5]thieno[3,2-d]pyrimidin
4-Ethoxy-7-(4-fluor-phenyl)-2-piperazin-1-yl-9-trifluormethyl-pyrido[3',2':4,5]thieno[3,2-d]pyrimidin
4-Ethoxy-2-piperazin-1-yl-9-trifluormethyl-7-(3,4,5-trimethoxy-phenyl)-pyrido[3',2':4,5]thieno[3,2-d]pyrimidin
7-(3-Amino-phenyl)-4-Ethoxy-2-piperazin-1-yl-9-trifluormethyl-pyrido[3',2':4,5]thieno[3,2-d]pyrimidin
4-Ethoxy-7-[4-(4-nitrobenryloxy)-phenyl]-2-piperazin-1-yl-9-trifluormethyl-pyrido[3',2':4,5]thieno[3,2-d]pyrimidin
4-Ethoxy-2-piperazin-1-yl-7-(3-pyridyl)-9-trifluormethyl-pyrido[3',2':4,5]thieno[3,2-d]pyrimidin
4-Ethoxy-7-(1-oxy-pyridin-3-yl)-2-(piperazin-1-yl)-9-trifluormethyl-pyrido[3',2':4,5]thieno[3,2-d]pyrimidin
4-Ethoxy-2-piperazin-1-yl-7-(2-pyridyl)-9-trifluormethyl-pyrido[3',2':4,5]thieno[3,2-d]pyrimidin
2-Ethoxy-7-phenyl-9-methyl-4-(piperazin-1-yi)-pyrido[3',2':4,5]furo[3,2-d]pyrimidin
9-Methyl-7-phenyl-4-piperazin-1-yl-2-*n*-propoxy-pyrido[3',2':4,5]thieno[3,2-d]pyrimidin
9-Methyl-7-phenyl-2-piperazin-1-yl-4-(1,1,1-trifluorethoxy)-pyrido[3',2':4,5]thieno[3,2-d]pyrimidin
4-Ethoxy-7-morpholino-9-phenyl-2-(piperazin-1-yl)-pyrido[3',2':4,5]thieno[3,2-d]pyrimidin
4-Ethoxy-7-(4-trifluor-methyl-phenyl)-9-methyl-2-(piperazin-1-yl)-pyrido[3',2':4,5]thieno[3,2-d]pyrimidin
8-(4-Amino-benzyl)-4-ethoxy-7,9-dimethyl-2-(piperazin-1-yl)-pyrido[3',2':4,5]thieno[3,2-d]pyrimidin
4-(2,2-Difluor-ethoxy)-9-methyl-7-phenyl-2-(piperazin-1-yl)-pyrido[3',2':4,5]thieno[3,2-d]pyrimidin
9-Methyl-7-phenyl-2-(piperazin-1-yl)-4-*n*-propoxy-pyrido[3',2':4,5]thieno[3,2-d]pyrimidin
4-Ethoxy-7-thien-2-yl-9-trifluor-methyl-2-(piperazin-1-yl)-pyrido[3',2':4,5]thieno[3,2-d]pyrimidin
2-Ethoxy-7-(4-trifluor-methyl-phenyl)-9-methyl-4-(piperazin-1-yl)-pyrido[3',2':4,5]thieno[3,2-d]pyrimidin
2-(2-Hydroxy-ethoxy)-9-methyl-7-phenyl-4-(piperazin-1-yl)-pyrido[3',2':4,5]thieno[3,2-d]pyrimidin
2-Ethoxy-7-methyl-9-phenyl-4-(piperazin-1-yl)-pyrido[3',2':4,5]thieno[3,2-d]pyrimidin
4-Ethoxy-7-(4-methoxy-phenyl)-9-methyl-2-(piperazin-1-yl)-pyrido[3',2':4,5]thieno[3,2-d]pyrimidin
4-Ethoxy-7-phenyl-9-methyl-2-(piperazin-1-yl)-pyrido[3',2':4,5]furo[3,2-d]pyrimidin
2-(2,2-Difluor-ethoxy)-9-methyl-7-phenyl-4-(piperazin-1-yl)-pyrido[3',2':4,5]thieno[3,2-d]pyrimidin
2-Ethoxy-7,9-di-*iso*-propyl-4-(piperazin-1-yl)-pyrido[3',2':4,5]thieno[3,2-d]pyrimidin
7-(3,4-Dimethoxy-phenyl)-9-methyl-4-(piperazin-1-yl)-2-*n*-propyl-pyrido[3',2':4,5]thieno[3,2-d]pyrimidin
2-Ethoxy-9-methyl-7-(4-methyl-phenyl)-4-(piperazin-1-yl)-pyrido[3',2':4,5]thieno[3,2-d]pyrimidin
9-Methyl-7-phenyl-2-(piperazin-1-yl)-4-*iso*-propoxy-pyrido[3',2':4,5]thieno[3,2-d]pyrimidin
4-(2-Hydroxy-ethoxy)-9-methyl-7-phenyl-2-(piperazin-1-yl)-pyrido[3',2':4,5]thieno[3,2-d]pyrimidin
2-Ethoxy-7-thien-2-yl-9-trifluor-methyl-4-(piperazin-1-yl)-pyrido[3',2':4,5]thieno[3,2-d]pyrimidin
2-Ethoxy-7-(3,4-dimethoxy-phenyl)-9-methyl-4-(piperazin-1-yl)-pyrido[3',2':4,5]thieno[3,2-d]pyrimidin
2-Ethoxy-7-(4-methoxy-phenyl)-9-methyl-4-(piperazin-1-yl)-pyrido[3',2':4,5]thieno[3,2-d]pyrimidin
2-Ethoxy-9-methyl-7-thien-2-yl-9-methyl-4-(piperazin-1-yl)-pyrido[3',2':4,5]thieno[3,2-d]pyrimidin
4-Ethoxy-9-methyl-7-(4-methyl-phenyl)-2-(piperazin-1-yl)-pyrido[3',2':4,5]thieno[3,2-d]pyrimidin
4-Ethoxy-7,9-dimethyl-8-(4-nitro-benzyl)-2-(piperazin-1-yl)-pyrido[3',2':4,5]thieno[3,2-d]pyrimidin
4-Ethoxy-7-(4-fluor-phenyl)-9-methyl-2-(piperazin-1-yl)-pyrido[3',2':4,5]thieno[3,2-d]pyrimidin
2-Ethoxy-7,9-dimethyl-8-(4-nitro-benzyl)-4-(piperazin-1-yl)-pyrido[3',2':4,5]thieno[3,2-d]pyrimidin
4-Ethoxy-7,9-diphenyl-2-(piperazin-1-yl)-pyrido[3',2':4,5]thieno[3,2-d]pyrimidin
4-Ethoxy-7-methyl-9-phenyl-2-(piperazin-1-yl)-pyrido[3',2':4,5]thieno[3,2-d]pyrimidin
2-Ethoxy-9-trifluor-methyl-7-phenyl-4-(piperazin-1-yl)-pyrido[3',2':4,5]thieno[3,2-d]pyrimidin
4-Ethoxy-7,9-di-*iso*-propyl-2-(piperazin-1-yl)-pyrido[3',2':4,5]thieno[3,2-d]pyrimidin
9-Methyl-7-phenyl-4-(piperazin-1-yl)-2-*iso*-propoxy-pyrido[3',2':4,5]thieno[3,2-d]pyrimidin
9-Methyl-7-phenyl-4-(piperazin-1-yl)-2-*n*-propyl-pyrido[3',2':4,5]thieno[3,2-d]pyrimidin
4-Ethoxy-9-methyl-8-(4-nitro-benzyl)-7-phenyl-2-(piperazin-1-yl)-pyrido[3',2':4,5]thieno[3,2-d]pyrimidin
4-Ethoxy-9-methyl-7-thien-2-yl-2-(piperazin-1-yl)-pyrido[3',2':4,5]thieno[3,2-d]pyrimidin
4-Ethoxy-9-trifluor-methyl-7-phenyl-2-(piperazin-1-yl)-pyrido[3',2':4,5]thieno[3,2-d]pyrimidin
4-(2,2-Difluor-ethoxy)-9-methyl-7-phenyl-2-(piperazin-1-yl)-pyrido[3',2':4,5]thieno-[3,2-d]pyrimidin
4-Ethoxy-7-(3,4-dimethoxy-phenyl)-9-methyl-2-(piperazin-1-yl)-pyrido[3',2':4,5]thieno-[3,2-d]pyrimidin
8-Ethoxy-5-morpholino-10-piperazin-1-yl-pyrimido[4',5':4,5]thieno[2,3-c]-1,2,3,4-tetrahydro-isochinolin
8-Ethoxy-5-(N-methyl-piperazin-1-yl)-10-piperazin-1-yl-pyrimido[4',5':4,5]thieno[2,3-c]-1,2,3,4-tetrahydro-isochinolin
7-Ethoxy-2,3-dihydro-4-morpholino-9-piperazin-1yl-1H-cyclopenta[4',5']pyrido[3',2':4,5]thieno[3,2-d]pyrimidin
8-Ethoxy-5-piperidino-10-piperazin-1-yl-pyrimido[4',5':4,5]thieno[2,3-c]-1,2,3,4-tetrahydro-isochinolin-hydrochlorid
8-Ethoxy-5-phenyl-10-piperazin-1-yl-pyrimido[4',5':4,5]thieno[2,3-c]-1,2,3,4-tetrahydro-isochinolin-hydrochlorid
10-Ethoxy-5-phenyl-8-piperazin-1-yl-pyrimido[4',5':4,5]thieno[2,3-c]-1,2,3,4-tetrahydro-isochinolin
8-Ethoxy-3-methyl-5-morpholino-10-piperazin-1-yl-pyrimido[4',5':4,5]thieno[2,7]-1,2,3,4-tetrahydro-naphthyridin
9-Ethoxy-6-morpholino-11-(piperazin-1-yl)-2,3,4,5-tetrahydro-1*H-*cyclohepta[4',5']pyrido[3',2':4,5]thieno[3,2-d]pyrimidin

Von ausgewählten Verbindungen der allgemeinen Formeln 1 a, 1b, 1 c und 1 d wurde die TNFα-Freisetzungs-Hemmung bestimmt. Die nachfolgende Auflistung beinhaltet erfindungsgemäße Substanzen, die im TNFα-Hemmassay einen IC₅₀-Wert von <2 µM aufweisen:
7-(3'-Aminobiphen-4-yl)-4-ethoxy-9-methyl-2-piperazin-1-yl-thieno[2,3-d:4,5-d']dipyrimidin
4-Ethoxy-7-(4-[3-furyl]-phenyl)-9-methyl-2-piperazin-1-yl-thieno[2,3-d:4,5-d']dipyrimidin
4-Ethoxy-9-methyl-2-piperazin-1-yl-7-(4-[3-pyrrolyl]-phenyl)-thieno[2,3-d:4,5-d']dipyrimidin
7-(4-[1,2-Dihydroxyethyl]-phenyl)-4-ethoxy-9-methyl-2-piperazin-1-yl-thieno[2,3-d:4,5-d']dipyrimidin
4-Ethoxy-7-(3,4-dimethoxy-phenyl)-9-methyl-2-(piperazin-1-yl)-pyrido[3',2':4,5]thieno[3,2-d]pyrimidin
4-Ethoxy-7-(4-methoxy-phenyl)-9-methyl-2-(piperazin-1-yl)-pyrido[3',2':4,5]thieno[3,2-d]pyrimidin-hydrochlorid
2-Ethoxy-7-methyl-9-phenyl-4-(piperazin-1-yl)-pyrido[3',2':4,5]thieno[3,2-d]pyrimidin-hydrochlorid
2-Ethoxy-7-(4-trifluor-methyl-phenyl)-9-methyl-4-(piperazin-1-yl)-pyrido[3',2':4,5]thieno[3,2-d]pyrimidin
2-(2-Hydroxy-ethoxy)-9-methyl-7-phenyl-4-(piperazin-1-yl)-pyrido[3',2':4,5]thieno[3,2-d]pyrimidin
8-(4-Amino-benzyl)-4-ethoxy-7,9-dimethyl-2-(piperazin-1-yl)-pyrido[3',2':4,5]thieno[3,2-d]pyrimidin
4-Ethoxy-7-(3,4-dimethoxy-phenyl)-9-methyl-2-(piperazin-1-yl)-pyrido[3',2':4,5]thieno[3,2-d]pyrimidin-hydrochlorid
4-Ethoxy-7-morpholino-9-phenyl-2-(piperazin-1-yl)-pyrido[3',2':4,5]thieno[3,2-d]pyrimidin
2-Ethoxy-7-(3,4-dimethoxy-phenyl)-9-methyl-4-(piperazin-1-yl)-pyrido[3',2':4,5]thieno[3,2-d]pyrimidin
2-Ethoxy-7-(4-methoxy-phenyl)-9-methyl-4-(piperazin-1-yl)-pyrido[3',2':4,5]thieno[3,2-d]pyrimidin
2-Ethoxy-7,9-dimethyl-8-(4-nitro-benzyi)-4-(piperazin-1-yl)-pyrido[3',2':4,5]thieno[3,2-d]pyrimidin
4-Ethoxy-7,9-diphenyl-2-(piperazin-1-yl)-pyrido[3',2':4,5]thieno[3,2-d]pyrimidin
4-Ethoxy-7-(4-methoxy-phenyl)-9-methyl-2-(piperazin-1-yl)-pyrido[3',2':4,5]thieno[3,2-d]pyrimidin
9-Methyl-7-phenyl-2-(piperazin-1-yl)-4-*n*-propoxy-pyrido[3',2':4,5]thieno[3,2-d]pyrimidin
4-Ethoxy-7,9-dimethyl-8-(4-nitro-benzyl)-2-(piperazin-1-yl)-pyrido[3',2':4,5]thieno[3,2-d]pyrimidin
4-Ethoxy-7-(4-fluor-phenyl)-9-methyl-2-(piperazin-1-yl)-pyrido[3',2':4,5]thieno[3,2-d]pyrimidin
4-Ethoxy-2-piperazin-1-yl-7-(3-pyridyl)-9-trifluormethyl-pyrido[3',2':4,5]thieno[3,2-d]pyrimidin
4-Ethoxy-7-(1-oxy-pyridin-3-yl)-2-(piperazin-1-yl)-9-trifluormethyl-pyrido[3',2':4,5]thieno[3,2-d]pyrimidin
4-Ethoxy-2-piperazin-1-yl-7-(4-pyridyl)-9-trifluormethyl-pyrido[3',2':4,5]thieno[3,2-d]pyrimidin
4-Ethoxy-7-(1-oxy-pyridin-4-yl)-2-(piperazin-1-yl)-9-trifluormethyl-pyrido[3',2':4,5]thieno[3,2-d]pyrimidin
4-Ethoxy-7-(4-(1*H*-imidazol-1-yl)phenyl)-2-(piperazin-1-yl)-9-trifluormethyl-pyrido[3',2':4,5]thieno[3,2-d]pyrimidin
4-(2-Hydroxy-ethoxy)-7-[(1H-imidazol-1-yl)phenyl]-2-(piperazin-1-yl)-9-trifluormethyl-pyrido[3',2':4,5]thieno[3,2-d]pyrimidin
8-Ethoxy-5-morpholino-10-piperazin-1-yl-pyrimido[4',5':4,5]thieno[2,3-c]-1,2,3,4-tetrahydro-isochinolin
4-(4-Ethoxy-9-trifluormethyl-2-piperazin-1-yl-pyrido[3',2':4,5]thieno[3,2-d]pyrimidin-7-yl)-benzamid Trifluoracetat
N'-Hxdroxy-4-(4-ethoxy-9-trifluormethyl-2-piperazin-1-yl-pyrido[3',2':4,5]thieno[3,2-d]pyrimidin-7-yl)-benzamidin Bistrifluoracetat
4-Ethoxy-9-trifluormethyl-2-piperazin-1-yl-7-(4-(1H-1,2,4-triazol-4-yl)-pheriyl)-pyrido[3',2':4,5]thieno[3,2-d]pyrimidin
N-(4-(4-Ethoxy-9-trifluormethyl-2-piperazin-1-yl-pyrido[3',2':4,5]thieno[3,2-d]pyrimidin-7-yl)-phenyl)-isonicotinamid
N-(4-(4-Ethoxy-9-trifluormethyl-2-piperazin-1-yl-pyrido[3',2':4,5]thieno[3,2-d]pyrimidin-7-yl)-phenyl)-formamid
N-(4-(4-Ethoxy-9-trifluormethyl-2-piperazin-1-yl-pyrido[3',2':4,5]thieno[3,2-d]pyrimidin-7-yl)-phenyl)-nicotinamid

### Beispiel 24

### Einsatz von PDE-Inhibitoren für die axonale Regeneration

Eine Anzahl erfindungsgemäßer Verbindungen wurde bezüglich der Indikation "axonale Degeneration" getestet. Axonale Regeneration, d.h. funktionelle Wiederherstellung von Nervenbahnen (Axonen), ist überall dort ein Therapieziel, wo es infolge mechanischer Einwirkungen zu massiven Verletzungen des Zentralnervensystems (Gehirn und Rückmark) gekommen ist. Gegenwärtig existieren keinerlei erfolgreiche Behandlungstechniken, um Verletzungen des Zentralnervensystems mit Durchtrennung der Nervenbahnen wiederherzustellen. Chirurgische Eingriffe bei Rückmarksverletzungen beschränken sich auf die Wiederherstellung der normalen Form der Wirbelsäule und gegebenenfalls auf die Freilegung des Rückmarkkanals zur Unterstützung der Selbst-Regeneration der Nervenbahnen. Die meisten schweren ZNS-Verletzungen enden jedoch mit lebenslangem Funktionsverlust des ganzen Körperbereichs.

Ein völlig neuartiger Ansatz zur Regeneration durchtrennter Nervenbahnen geht von der topischen Anwendung von PDE-4 Inhibitoren aus. Dabei wird der Umstand genutzt, dass das axonale Nervenwachstum über eine cAMP-abhängige Signaltransduktionskaskade eingeleitet wird. Überschreitet der intrazelluläre Spiegel an cAMP einen bestimmten Schwellenwert, so wird Neuritenwachstum induziert.

Pharmakologisch kann eine ausreichende Erhöhung des intrazellulären cAMP-Spiegels durch Inhibierung der Phosphodiesterase-4 erreicht werden, desjenigen Enzyms, das für den Abbau von cAMP hauptsächlich verantwortlich ist.

Der erfolgreiche Einsatz des Prototyp PDE-4 Inhibitors Rolipram in Ratten zur funktionellen Wiederherstellung Rückmarks nach experimenteller Nervenverletzung wurde jüngst in zwei Arbeiten beschrieben (Nikulina,E., Tidwell,J.L., Dai,H.N., Bregman,B.S., and Filbin,M.T. (2004), Proc. Nat. Acad. Sci. USA 101(23), pp. 8786-8790; Pearse,D.D., Pereira,F.C., Marcillo,A.E., Bates,M.L., Berrocal,Y.A., Filbin,M.T. and Bunge,M.B. (2004), Nat. Med. 10(6), pp. 610-616).

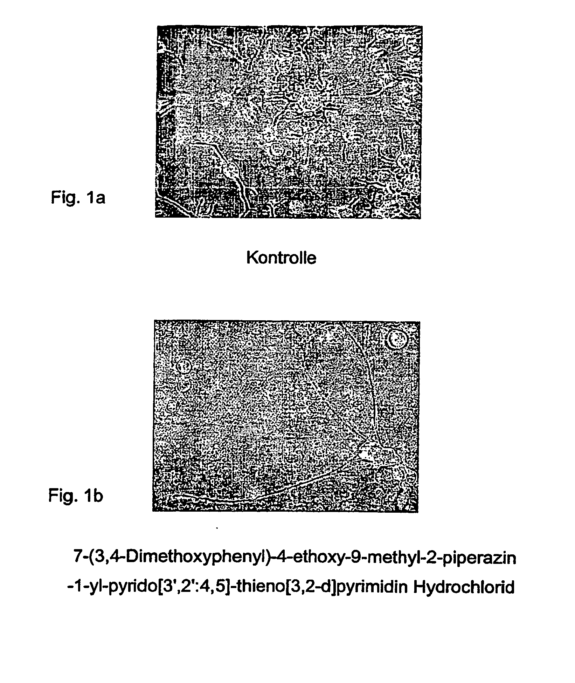

Die Erfinder haben einige erfindungsgemäße PDE4-Inhibitoren auf die Induktion von Axon-Wachstum an der Maus-Neuroblastomzellinie N2a getestet. Dabei zeigte sich, dass insbesondere die Substanzen 4-Ethoxy-9-ethyl-7-(3,4-methylendioxyphenyl)-2-piperazin-1-yl-pyrido[3',2':4,5]thieno[3,2-d]pyrimidin und 7-(3,4-Dimethoxyphenyl)-4-ethoxy-9-methyl-2-piperazin-1-yl-pyrido[3',2':4,5]thieno[3,2-d]pyrimidin Hydrochlorid in der Lage waren, bei Konzentrationen von 1 µM im Testansatz das Auswachsen langer Fortsätze ("Axone") mit bis zu 300 µM Länge nach 72 h zu induzieren (Fig. 1a und 1b).

### Beispiel 25

### Einsatz von PDE4-Inhibitoren für die Induktion von Apoptose bei chronisch lymphatischer Leukämie (CLL)

Desweiteren wurden von den Erfindern erfindungsgemäße Verbindungen als PDE4-Inhibitoren auf die Induktion einer zytotoxischen Reaktion, d.h. Auslösung von Apoptose, bei chronisch lymphatischer Leukämie (CLL) getestet.

Die Phosphodiesterase 4 stellt nach neueren Erkenntnissen auch ein valides therapeutisches Target bei der Behandlung der CLL dar.

Die chronisch lymphatische Leukämie der B-Zellen ist eine selten heilbare, klonale Erkrankung, die auf Chemotherapeutika (Zytostatika) anspricht. Viele Patienten entwickeln jedoch gegenüber diesen Medikamenten eine sog. Chemoresistenz.

Seit den 70er Jahren beobachtete man, dass eine Erhöhung des intrazellulären zyklischen Adenosinmonophosphates (cAMP) zu einer selektiven Apoptoseinduktion in den malignen CLL Zellen, im Vergleich zu gesunden T und B Lymphozyten, führte (V. Daniel et al. Proc Natl Acad Sci 70: 76; 1973). Membran-durchgängige cAMP Analoga (dibutyryl cAMP), Adenylatezyklase Aktivatoren (Forskolin) und Inhibitoren der Phosphodiesterase PDE-3 (Cilostamide) und PDE4 (Rolipram), allein oder in Kombination, führten zu einer Caspase-abhängigen Apoptose in CLL Lymphozyten. Rolipram Behandlung unterdrückt die Expression des anti-Apoptose Mitglieds der Bcl-2 Familie und induziert das pro-Apoptoseprotein Bax (E Moon et al. Clin Cancer Res 8: 589 (2002); SB Welsch et al. Leukemia 15: 1564 (2001)).

Um der Frage nachzugehen, ob die selektiven PDE-4 Inhibitoren der Anmelderin gleichfalls in der Lage sind, eine selektive Apoptose der CLL Lymphozyten zu induzieren, wurden die erfindungsgemäßen Verbindungen bei Patienten mit einer primären CLL in einer Konzentration von 1 µM getestet.

Als Positivkontrolle wurde Fludarabine, das bei der CLL als Standard-Chemotherapeutikum Anwendung findet, mitgeführt. Mit Fludarabine konnte eine Zytotoxizität bis zu maximal 60% unter den gleichen Versuchsbedingungen, in einem cAMP-unabhängigen Weg, erreicht werden.

Die Zytotoxizität der CLL Zellen wurde mittels eines kommerziell erhältlichen Formazan Reduktions-Assays (MTT Test) nach 24 h Inkubation des Patientenblutes mit den Wirkstoffen bestimmt.

Die Phosphodiesterase Aktivität der rekombinanten humanen PDE-4A, PDE-4B und PDE-4D, die in sf9 Insektenzellen exprimiert wurden, wurde mit Hilfe eines Scintillations-Proximity Assays entsprechend den Herstellerangaben bestimmt. Lipopolysaccharide (LPS)-abhängige Freisetzung des Tumor Nekrose Faktor-α (TNFα) durch mononukleare Zellen in verdünntem humanen Vollblut, wurde mittels eines Sandwich TNFα ELISA bestimmt.

Die folgende Auflistung zeigt eine Auswahl der erfindungsgemäßen Substanzen, die in 60% -85 % der Zellen eine Zytotoxizität auslösen:
4-Ethoxy-7-(4-[2-furyl]-phenyl)-9-methyl-2-piperazin-1-yl-thieno[2,3-d:4,5-d']dipyrimidin
4-Ethoxy-9-methyl-2-piperazin-1-yl-7-(4-[N-BOC-3-pyrrolyl]-phenyl)-thieno[2,3-d:4,5-d']dipyrimidin
4-Ethoxy-9-ethyl-7-(4-methyl-phenyl)-2-(piperazin-1-yl)-pyrido[3',2':4,5]thieno[3,2-d]pyrimidin
2-Ethoxy-7-(4-methyl-phenyl)-4-(piperazin-1-yl)-9-propyl-pyrido[3',2':4,5]thieno[3,2-d]pyrimidin
2-Ethoxy-7-(4-methyl-phenyl)-4-(piperazin-1-yl)-9-propyl-pyrido[3',2':4,5]thieno[3,2-d]pyrimidin Hydrochlorid
2-Ethoxy-9-methyl-4-(piperazin-1-yl)-7-phenyl-pyrido[3',2':4,5]furo[3,2-d]pyrimidin
7-(2,3-Dihydrobenzo[b][1,4]dioxin-6-yl)-2-ethoxy-9-ethyl-4-piperazino-pyrido[3',2':4,5] thieno[3,2-d]pyrimidin
4-Ethoxy-9-ethyl-7-(4-methoxy-phenyl)-2-piperazin-1-yl-pyrido[3',2':4,5]thieno[3,2-d]pyrimidin Hydrochlorid
4-Ethoxy-7-(4-methylthio-phenyl)-2-piperazin-1-yl-9-trifluormethyl-pyrido[3',2':4,5]thieno[3,2-d]pyrimidin
Mono-[4-Ethoxy-7-(4-methylthio-phenyl)-2-piperazin-1-yl-9-trifluormethyl-pyrido[3',2':4,5]thieno[3,2-d]pyrimidin] Citrat
7-(4-Amino-phenyl)-4-ethoxy-2-piperazin-1-yl-9-trifluormethyl-pyrido[3',2':4,5]thieno[3,2-d]pyrimidin
4-Ethoxy-9-methyl-2-piperazin-1-yl-7-(4-[4-pyridyl]-phenyl)-thieno[2,3-d:4,5-d']dipyrimidin
4-Ethoxy-9-methyl-2-piperazin-1-yl-7-(4-[3-pyridyl]-phenyl)-thieno[2,3-d:4,5-d']dipyrimidin
4-Ethoxy-9-methyl-2-piperazin-1-yl-7-(4-[3-pyrrolyl]-phenyl)-thieno[2,3-d:4,5-d']dipyrimidin
4-(4-Ethoxy-9-methyl-2-piperazin-1-yl-thieno[2,3-d:4,5-d']dipyrimidin-7-yl)-biphenyl-3'-carbonsäure Kaliumsalz
7-(4'-Aminobiphen-4-yl)-4-ethoxy-9-methyl-2-piperazin-1-yl-thieno[2,3-d:4,5-d']dipyrimidin
4-Ethoxy-9-methyl-2-piperazin-1-yl-7-(4-[5-pyrimidinyl]-phenyl)-thieno[2,3-d:4,5-d']dipyrimidin
7-(3',4'-Dimethoxybiphen-4-yl)-4-ethoxy-9-methyl-2-piperazin-1-yl-thieno[2,3-d:4,5-d']dipyrimidin
4-Ethoxy-7-(4-methoxy-phenyl)-9-methyl-2-piperazin-1-yl-pyrido[3',2':4,5]thieno[3,2-d]pyrimidin
2-Ethoxy-9-methyl-7 -(3,4-methylendioxy-phenyl)-4-piperazin-1-yl-pyrido[3 ',2 ':4,5]-thieno[3,2-d]pyrimidin
4-Ethoxy-9-ethyl-7-(4-methoxy-phenyl)-2-piperazin-1-yl-pyrido[3',2':4,5]thieno[3,2-d]pyrimidin

## Patentansprüche

1. Verbindungen der allgemeinen Formeln 1 a, 1 b, 1 c oder 1d 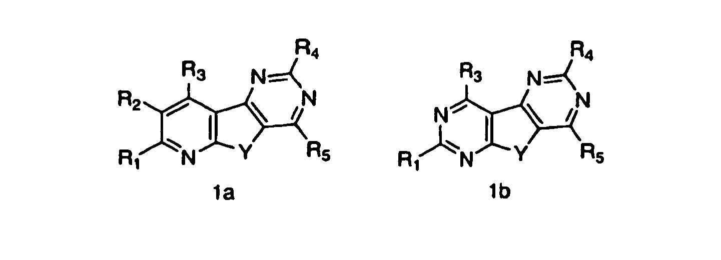 worin bedeuten:
Y S, O oder N-H
R¹
- Wasserstoff,
- C₁₋₁₀Alkyl, -C₂₋₁₂ Alkenyl, -C₂₋₁₂ Alkinyl, jeweils geradkettig, verzweigt oder cyclisch sowie ggf. ein-, zwei- oder dreifach mit unabhängig voneinander ausgewählten Resten R^{§} substituiert,
- Aralkyl- mit C₆₋₁₂Aryl und C₁₋₅ Alkyl, ggf. ein-, zwei- oder dreifach mit unabhängig voneinander ausgewählten Resten R^{§} substituiert,
- Phenyl, ggf. ein-, zwei- oder dreifach mit unabhängig voneinander ausgewählten Resten R^{§} substituiert,
- 1-Naphthyl, 2-Naphthyl
- Pyridyl-N-oxid, (ggf. mit R^{§} substituiert),
- mono- oder bicyclische gesättigte oder ein- oder mehrfach ungesättigte Heterocyclen mit 5 - 14 Ringatomen darunter 1 - 4 Heteroatome, die vorzugsweise N, O und S sind, ggf. ein-, zwei- oder dreifach mit unabhängig voneinander ausgewählten Resten R^{§} substituiert,
- C₁₋₁₀ Alkoxy, geradkettig, verzweigt oder cyclisch sowie ggf. ein-, zwei- oder dreifach mit unabhängig voneinander ausgewählten Resten R^{§} substituiert,
- Aralkyloxy- mit C₆₋₁₂Aryl und C₁₋₅ Alkyl, ggf. ein-, zwei- oder dreifach mit unabhängig voneinander ausgewählten Resten R^{§} substituiert,
- C₂₋₁₂Alkylacyl (ggf. mit R^{§} substituiert),
- Benzoyl, 1- und 2-Naphthoyl (jeweils ggf. mit R^{§} substituiert),
- Hydroxy, Sulfhydryl, Formyl, Carboxyl, CONH₂, Cyano, Rhodano, Nitro, SO₃H
- Alkylthio, Alkylsulfinyl, SO₂OAlk und Alkylsulfonyl, jeweils geradkettig, verzweigt oder cyclisch C₁₋₆ sowie ggf. ein-, zwei- oder dreifach mit unabhängig voneinander ausgewählten Resten R^{§} substituiert,
- Arylthio, Arylsulfinyl, Arylsulfonyl, Heteroarylthio Heteroarylsulfinyl und Heteroarylsulfonyl, jeweils ggf. ein-, zwei- oder dreifach mit unabhängig voneinander ausgewählten Resten R^{§} substituiert,
- , Alkoxycarbonyl, CONHAlk und CONAlk₂ ,jeweils geradkettig, verzweigt oder cyclisch C₁₋₆, sowie ggf. ein-, zwei- oder dreifach mit unabhängig voneinander ausgewählten Resten R^{§} substituiert,
- Aryloxycarbonyl, Arylcarbamoyl, Arylamido, N-Aryl,N-alkylamido, N-Aryl,N-alkylcarbamoyl, Aralkyloxycarbonyl und Aralkylcarbamoyl, mit Alkyl C₁₋₅, Aryl C₆₋₁₀ und ggf. ein-, zwei- oder dreifach mit unabhängig voneinander ausgewählten Resten R^{§} substituiert,
- Heterocyclylcarbonyl, Heterocyclylcarbamoyl, Heterocyclylamido, N-Heterocyclyl,N-alkylcarbamoyl, N-Heterocyclyl,N-alkylamido, Heterocyclylalkyloxycarbonyl und Heterocyclylkylcarbamoyl, mit einem mono- oder bicyclischen gesättigten oder ein- oder mehrfach ungesättigten Heterocyclus mit 5 - 14 Ringatomen darunter 1 - 4 Heteroatomen, die vorzugsweise N, O und S sind, ggf. ein-, zwei- oder dreifach mit unabhängig voneinander ausgewählten Resten R^{§} substituiert und Alkyl C₁₋₅,
- Chlor, Brom, Iod, Fluor,
- Amino, C₁₋₆Alkylamino, Di(C₁₋₅)alkylamino, jeweils geradkettig, verzweigt oder cyclisch sowie ggf. ein-, zwei- oder dreifach mit unabhängig voneinander ausgewählten Resten R^{§} substituiert,
- Arylamino C₆₋₁₀, ggf. ein-, zwei- oder dreifach mit unabhängig voneinander ausgewählten Resten R^{§} substituiert,
- Heterocyclylamino, mono- oder bicyclisch, gesättigt oder ein- oder mehrfach ungesättigt, mit 5 - 14 Ringatomen darunter 1 - 4 Heteroatome, die vorzugsweise N, O und S sind, ggf. ein-, zwei- oder dreifach mit unabhängig voneinander ausgewählten Resten R^{§} substituiert ,
- Arylhydrazino, ggf. ein-, zwei- oder dreifach mit unabhängig voneinander ausgewählten Resten R^{§} substituiert,
- L_{A}-A-L_{B}-B
worin bedeuten:
L_{A}:
- Einfachbindung,
- NR^{#}, O, S, SO, SO₂
A:
- Phenyl, ggf. ein-, zwei- oder dreifach mit unabhängig voneinander ausgewählten Resten R^{§} substituiert
- Naphthyl, Naphtyl ein- oder mehrfach mit R^{§} substituiert
- mono- oder bicyclische gesättigte oder ein- oder mehrfach ungesättigte Heterocyclen mit 4 - 14 Ringatomen darunter 1 - 5 Heteroatome, die vorzugsweise N, O und S sind, die ein oder mehrere Sauerstoff-Atome an C, N und/oder S tragen können und ggf. ein-, zwei- oder dreifach mit unabhängig voneinander ausgewählten Resten R^{§} substituiert sind
-L_{A}-A kann ferner auch eine direkte Bindung Tricyclus- L_{B} bedeuten, wobei in diesem Fall L_{B} keine Einfachbindung ist
L_{B}:
- Einfachbindung
- NR^{#}, O, S, SO, SO₂, -CHR^{§}-, -CH₂-O-, -O-CH₂,
- folgende funktionelle Gruppen: 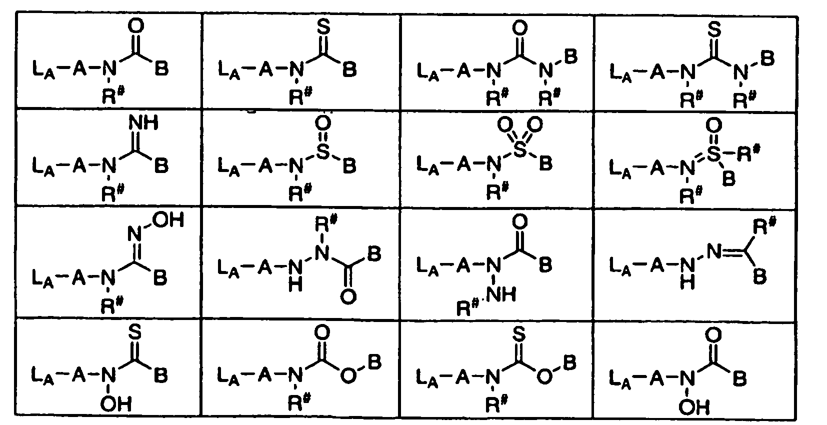
B:
- Wasserstoff
- Alkyl, ggf. ein-, zwei- oder dreifach unabhängig voneinander mit R^{§} substituiert
- -CONH₂, -CONHAlk (Alk ggf. mit R^{§} substituiert), -CONHAryl (Aryl ggf. mit R^{§} substituiert), -CONHHetaryl (Hetaryl ggf. mit R^{§} substituiert), -COOH, -COOAlk (Alk ggf. mit R^{§} substituiert),
- -COAlk (Alk ggf. mit R^{§} substituiert), -COAryl (Aryl ggf. mit-R^{§} substituiert), -COHetaryl (Hetaryl ggf. mit R^{§} substituiert)
- -CH₂-CONH₂ , -CH₂-CONHAlk (Alk. ggf. mit R^{§} substituiert), -CH₂-CONHAryl (Aryl ggf. mit R^{§} substituiert), -CH₂-CONHHetaryl ((Hetaryl ggf. mit R^{§} substituiert), -CH₂-COOH, -CH₂-COOAlk (Alk. ggf. mit R^{$} substituiert)
- -CH₂-COAlk (Alk ggf. mit R^{§} substituiert),
-CH₂-COAryl (Aryl ggf. mit R^{§} substituiert), -CH₂-COHetaryl (Hetaryl ggf. mit R^{§} substituiert)
- Phenyl, ggf. ein-, zwei- oder dreifach mit unabhängig voneinander ausgewählten Resten R^{§} substituiert,
- Naphthyl, ggf. ein-, zwei- oder dreifach mit unabhängig voneinander ausgewählten Resten R^{§} substituiert,
- mono- oder bicyclische gesättigte oder ein- oder mehrfach ungesättigte Heterocyclen mit 4 - 14 Ringatomen darunter 1 - 5 Heteroatome, die vorzugsweise N, O und S sind, die ein oder mehrere Sauerstoff-Atome an C, N und/oder S tragen können und ggf. ein-, zwei- oder dreifach mit unabhängig voneinander ausgewählten Resten R^{§} substituiert sein können,
R^{#}
- Wasserstoff, Alkyl, ggf. mit R^{§} substituiert
R²
- Wasserstoff,
- C₁₋₁₂ Alkyl,
- C₂₋₁₂ Alkenyl und C₂₋₁₂ Alkinyl,
- Benzyl, Phenyl(C₂₋₆)alkyl (ggf. mit R^{§} ein oder mehrmals, gleich oder ungleich am aromatischen und/oder aliphatischen Molekülteil substituiert);
- Phenacyl (ggf. mit R^{§} ein oder mehrmals, gleich oder ungleich am aromatischen Molekülteil substituiert);
- Carboxyl, C₁₋₄ Alkoxycarbonyl, -CONH₂, -CONHAlk und CONAlk₂ (mit "Alk" jeweils C₁₋₆),
- R^{#}C(O)- (worin R^{#} dieselbe Bedeutung hat wie oben),
- Cyano, Nitro, Amino, C₁₋₆Alkylamino, Di(C₁₋₆ )alkylamino, -N=N-C₆H₅, -N=N-C₆H₄-R^{§},
- 1,3-Diphenyl-pyrazol-4-yl, Thiazolin-2-yl, Imidazolin-2-yl und 3,4,5,6-Tetrahydro-pyrimidinyl;
R³
- Wasserstoff,
- C₁₋₁₀Alkyl, -C₂₋₁₂ Alkenyl, -C₂₋₁₂ Alkinyl, jeweils geradkettig, verzweigt oder cyclisch sowie ggf. ein-, zwei- oder dreifach mit unabhängig voneinander ausgewählten Resten R^{§} substituiert,
- Trifluormethyl
- Aralkyl- mit C₆₋₁₂Aryl und C₁₋₅ Alkyl, ggf. ein-, zwei- oder dreifach mit unabhängig voneinander ausgewählten Resten R^{§} substituiert,
- Phenyl, ggf. ein-, zwei- oder dreifach mit unabhängig voneinander ausgewählten Resten R^{§} substituiert,
- 1-Naphthyl, 2-Naphthyl
- Pyridyl-N-oxid, (ggf. mit R^{§} substituiert),
- C₁₋₁₀ Alkoxy, geradkettig, verzweigt oder cyclisch sowie ggf. ein-, zwei- oder dreifach mit unabhängig voneinander ausgewählten Resten R^{§} substituiert,
- Aralkyloxy- mit C₆₋₁₂Aryl und C₁₋₅ Alkyl, ggf. ein-, zwei- oder dreifach mit unabhängig voneinander ausgewählten Resten R^{§} substituiert,
- C₂₋₁₂Alkylacyl (ggf. mit R^{§} substituiert),
- Benzoyl, 1- und 2-Naphthoyl (jeweils ggf. mit R^{§} substituiert),
- Hydroxy, Sulfhydryl,- Formyl, Carboxyl, CONH₂, Cyano, Rhodano, Nitro, SO₃H
- Alkylthio, Alkylsulfinyl, SO₂OAlk und Alkylsulfonyl, jeweils geradkettig, verzweigt oder cyclisch C₁₋₆ sowie ggf. ein-, zwei- oder dreifach mit unabhängig voneinander ausgewählten Resten R^{§} substituiert,
- Arylthio, Arylsulfinyl, Arylsulfonyl, Heteroarylthio, Heteroarylsulfinyl und Heteroarylsulfonyl, jeweils ggf. ein-, zwei- oder dreifach mit unabhängig voneinander ausgewählten Resten R^{§} substituiert,
- , Alkoxycarbonyl, CONHAlk und CONAlk₂ ,jeweils geradkettig, verzweigt oder cyclisch C₁₋₆, sowie ggf. ein-, zwei- oder dreifach mit unabhängig voneinander ausgewählten Resten R^{§} substituiert,
- Aryloxycarbonyl, Arylcarbamoyl, Arylamido, N-Aryl,N-alkylamido, N-Aryl,N-alkylcarbamoyl, Aralkyloxycarbonyl und Aralkylcarbamoyl, mit Alkyl C₁₋₅, Aryl C₆₋₁₀ und ggf. ein-, zwei- oder dreifach mit unabhängig voneinander ausgewählten Resten R^{§} substituiert,
- Chlor, Brom, lod, Fluor,
- Amino, C₁₋₆Alkylamino, Di(C₁₋₅)alkylamino, jeweils geradkettig, verzweigt oder cyclisch sowie ggf. ein-, zwei- oder dreifach mit unabhängig voneinander ausgewählten Resten R^{§} substituiert,
- Arylamino C₆₋₁₀, ggf. ein-, zwei- oder dreifach mit unabhängig voneinander ausgewählten Resten R^{§} substituiert,
- Arylhydrazino, ggf. ein-, zwei- oder dreifach mit unabhängig voneinander ausgewählten Resten R^{§} substituiert,
R⁴
- C₂₋₁₄Alkyl, C₃₋₁₄Cycloalkyl, C₂₋₁₄Alkenyl, C₃₋₁₄Cycloalkenyl, C₂₋₁₄Alkinyl (jeweils ggf. am C-Skelett der vorgenannten aliphatischen Reste mit R§ substituiert);
- Phenyl, 4-R^{§} -Phenyl, 3-R^{§} -Phenyl, 2-R^{§}-Phenyl (jedoch ausgenommen: 2-NO₂-Phenyl), 3-R^{§},4-R^{§}-Phenyl, 3-R^{§},4-R^{§},5-R^{§}-Phenyl,
- 1-Naphthyl, 2-Naphthyl (jeweils ggf. mit R^{§} substituiert);
- S-Alkyl, SO-Alkyl, SO₂-Alkyl, (jeweils C₁-C₈),
S-Alkenyl, SO-Alkenyl, SO₂-Alkenyl (jeweils C₂-C₈)
S-Alkinyl, SO-Alkinyl, SO₂-Alkinyl (jeweils C₂-C₆)
(ggf. jeweils am C-Skelett der vorgenannten aliphatischen Reste mit -OH, -CN, -SCN, -NO₂, Phenyl oder C₃ - C₇-Cycloalkyl substituiert);
- mono-, bi- oder tricyclische gesättigter oder ein- oder mehrfach ungesättigter heterocyclischer Rest mit insgesamt 4-14 Ring-Atomen, davon 1-5 Heteroatomen, die vorzugsweise N, O, S und Se sind (jeweils ggf. mit R^{§} substituiert;
- OR⁶, worin R⁶ bedeutet
- CH₃, C₂H₅, (CH₂)₂CH₃, (CH₂)₃CH₃, C(CH₃)₃, CH₂CH₂OH, CH₂CH₂SH, CH₂CH₂SCH₃, CH₂CF₃, CH₂CCl₃, CH₂CHF₂, CH₂CHCl₂, CH₂CH₂F, CH₂CH₂Cl, CH₂CH₂Br,
- C₃-₇Cycloalkyl [z.B.Cylopropyl, Cylopropylmethyl, Cylobutyl, Cyclobutylmethyl, Cyclopentyl, Cyclopentylmethyl, Cylohexyl, Cyclohexylmethyl] (ggf. am C- Skelett mit R^{§} substituiert),
- C₂-₅Alkenyl und C₂-₅Alkinyl,
- C₃-₇ Cycloalkenyl,
- Aryl und Heteroaryl als Reste mono-, bi- oder tricyclischer Aromaten oder Heteroaromaten mit ggf. 6-14 Ring-Atomen [z.B. Phenyl, 4-R^{§} -Phenyl 3-R^{§} -Phenyl, 2-R^{§}-Phenyl, 3-R^{§},4-R^{§}-Phenyl, 3-R^{§},4-R^{§}-,5-R^{§}-Phenyl],
- 1-Naphthyl, 2-Naphthyl (ggf. jeweils mit R^{§} substituiert),
- Pyridyl, lsochinolinyl, Chinolinyl, Acridinyl;
- NA⁷R⁸, worin dieser Substituent insgesamt bedeutet:
- Morpholino, Thiomorpholino, Thiomorpholino-S,S-Dioxid, Pyrrolidino, Piperidino, 1-Piperazino, 1-Homopiperazino, 4-C₁₋₆₋Alkyl-1-piperazino, 4-(2-Hydroxyethyl)-1-piperazino, 4-Benzyl-1-piperazino, 4-Aryl-1-piperazino (ggf. mit R^{§} am heterocycloaliphatischen Ring substituiert),
- weitere Amino-Reste sekundärer, mono- oder polycyclischer cycloaliphatischer Amine mit insgesamt 5-14 Ringatomen, einschließlich der am C-Skelett mit R^{§} substituierte Vertreter;
- Amino-Reste sekundärer aliphatischer und aromatischer Amine, R⁷R⁸NH,
worin R⁷ und R⁸ unabhängig voneinander gleich oder ungleich sein können und bedeuten:
- C₁-₆Alkyl, Benzyl, Phenyl, 1- und 2-Naphthyl, 2-, 3- und 4-Pyridyl, Chinolinyl, Isochinolinyl, 2-Thienyl, 2-Furyl (ggf. jeweils mit R^{§} substituiert);
- Aminoreste primärer Amine, **R**⁷NH₂, ( worin R⁷ dasselbe wie oben bedeutet),
- Amino, NH₂, mit der Einschränkung, dass dann R⁵ nur die Bedeutung von OR⁶ hat;
R⁵
- OR⁶, wobei R⁶ dasselbe wie oben bedeutet,
- NR⁷R⁸, mit gleicher Bedeutung wie oben, jedoch mit der Einschränkung, dass R⁴ dabei nicht ebenfalls die Bedeutung von NR⁷R⁸ aufweist, sowie ausgenommen Morpholino, wenn R⁴ = Aryl,
- Azido, Hydroxylamino, O-(C₁-₃ )Alkylhydroxylamino,
- N-(C₁-₃)Alkylhydroxylamino. N,N-Di(C₁-₃)alkylhydroxylamino,
- Hydrazino, (C₁-₄)Alkyl- und Di(C₁-₄)alkylhydrazino.
- Benzylhydrazino, Acylhydrazino, N,N-Diacylhydrazino,
- Carbamoylamino,
- 1-Imidazolyl, 1,2,4-Triazol-1yl, 1-Pyridinium,
- 1-Pyrazinium, 1-Pyridazinium, einschliesslich der alkylsubstituierten Vertreter dieser Azaheterocyclen;
R^{§}
- OH, -SH, -O-C₁₋₈Alkyl. -O-C₆₋₁₄ Aryl, -S-C₁₋₄ Alkyl, -S-C₆₋₁₄Aryl,
- SO-C₁₋₄Alkyl, -SO-C₆₋₁₄Aryl, -SO₂-C₁₋₄Alkyl, -SO₂-C₆₋₁₄Aryl, -SO₃H,
- OSO₂C₁₋₈Alkyl, -OSO₂C₆₋₁₄Aryl, -COOH, -COOC₁₋₈Alkyl,
- (CO)C₁₋₈Alkyl,
- COOH, -CONH₂, -CONHC₁₋₆Alkyl, -CON(C₁₋₆Alkyl)₂,
- NH₂, -NHC₁₋₆Alkyl, -N(C₁₋₆Alkyl)₂, -NHC₆₋₁₄Aryl, -NH-Hetaryl,
- N(C₆₋₁₄Aryl)₂, -N(C₁₋₆Alkyl)(C₆₋₁₄Aryl),
- C₁₋₆Alkyl, -C₂₋₁₂ Alkenyl, -C₂₋₁₂ Alkinyl, jeweils geradkettig, verzweigt oder cyclisch sowie ggf. ein-, zwei- oder dreifach unabhängig voneinander mit Halogen substituiert,
- Halogen (F, -Cl, -Br, -I),
- CH₂CH₂OH, -CH₂CH₂SH, -CH₂CH₂SCH₃,
- Sulfamoyl, Alkylsulfamoyl, Dialkylsulfamoyl mit Alkyl C₁₋₅ ggf. substituiert mit Methoxy,
- Amidino, Hydroxyamidino
- Sulfo, Phosphono,
- -CN, -NO₂, und -SCN
Ausgenommen vom Schutz sind folgende Verbindungen der Formel 1(a):
I) Y = S, R¹ = OC₂H₅, R² = CN, R³ = C₆H₅, R⁴ = N(CH₃)₂, R⁵ = OC₂H₅
II) Y = S, R¹ = CH₃, R² = H, R³ = CH₃, R⁴ = Phenyl, R⁵ = Hydrazino
III) Y = S, R¹ = CH₃, R² = H, R³ = CH₃, R⁴ = Phenyl, R⁵ = NH-(CH₂)₂-OH
IV) Y = S, R¹ = Phenyl, R² = H, R³ = Phenyl, R⁴ = Thiopheno, R⁵ = NH-(CH₂)₂-OH
V) Y = S, R¹ = Phenyl, R² = H, R³ = Phenyl, R⁴ = Phenyl dreifach substituiert mit Methoxy, R⁵ = NH-(CH₂)₂-OH 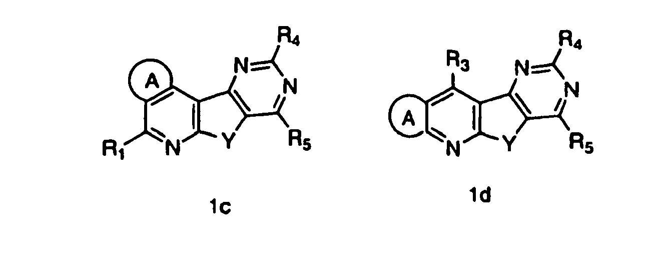
Y S, N-H oder O
A
- anellierter Carbocyclus mit 5 bis 8 Ringatomen, einfach oder mehrfach ungesättigt sowie aromatisch, (ggf. mit R* substituiert und/oder ggf. mit R^{§} substituiert);
- anellierter Bicyclus mit 6 bis 11 Ringatomen, einfach oder mehrfach ungesättigt, welcher auch Stickstoff, Sauerstoff und/oder Schwefel enthalten kann (ggf. mit R* substituiert und/oder ggf. mit R^{§} substituiert);
- annelierter Heterocyclus mit 5 bis 8 Ringatomen einfach oder mehrfach ungesättigt sowie aromatisch, welcher Stickstoff, Sauerstoff und Schwefel einfach, mehrfach, gleich oder ungleich unabhängig voneinander im Cyclus enthalten kann (ggf. mit R* substituiert und/oder ggf. mit R^{§} substituiert),
R¹ und R³
- Wasserstoff (außer wenn A ein anellierter unsubstituierter Cyclopenten-Ring kombiniert mit R⁴' R⁵gleich -OH ist)
- C₁₋₁₀Alkyl (ggf. mit R^{§} substituiert).
- C₂₋₁₂ Alkenyl und C₂₋₁₂ Alkinyl (jeweils ggf. mit R^{§} substituiert ),
- Difluormethyl, Trifluormethyl,
- Benzyl, Phenyl-(C₂₋₆)alkyl (jeweils ggf. mit R^{§} substituiert), Phenyl (ggf. mit R^{§} substituiert), dabei inbesondere:
4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl, 2-R^{§}, 4-R^{§}-Phenyl,
- 1-Naphthyl, 2-Naphthyl (jeweils ggf. mit R^{§} substituiert),
- C₃₋₁₄Cycloalkyl, C₃₋₁₄Cycloalkenyl (jeweils ggf. mit R^{§}-substituiert),
- mono- oder bicyclische gesättigte oder ein- oder mehrfach ungesättigte Heterocyclen mit 4 - 14 Ringatomen darunter 1 - 5 Heteroatome, die vorzugsweise N, O und S sind, die ein oder mehrere Sauerstoff-Atome an C, N und/oder S tragen können und ggf. ein-, zwei- oder dreifach mit unabhängig voneinander ausgewählten Resten R^{§} substituiert sein können,
- C₂₋₁₂Alkylacyl (ggf. mit R^{§} substituiert),
- Benzoyl, 1- und 2-Naphthoyl (jeweils ggf. mit R^{§} substituiert),
- Heterocyclylacyl [z.B. Nicotinoyl, Isonicotinoyl, 2-Picolinoyl, 2-Thienoyl, 2-Furoyl] (ggf. mit R^{§} substituiert)
- Hydroxy,
- Sulfhydryl,
- C₁₋₁₀ Alkoxy,
- Alkylthio, Alkylsulfinyl und Alkylsulfonyl (jeweils C₁₋₆)
- Formyl, Carboxyl, C₁₋₄Alkoxycarbonyl;
- CONH₂, CONHAlk und CONAlk₂ (mit "Alk" jeweils C₁₋₆),
- Cyano, Rhodano, Nitro, SO₃H, SO₂OAlk (mit "Alk": C₁₋₅),
- Chlor, Brom, Iod, Fluor,
- Amino, C₁₋₆Alkylamino, Di(C₁₋₅)alkylamino (jeweils ggf. mit R^{§} am Alkylrest substituiert),
- Morpholino, Thiomorpholino, Thiomorpholino-S-Oxid, Thiomorpholino-S,S-Dioxid, Pyrrolidino,
- Piperidino, 1-Piperazino, 4-Methyl-1-piperazino, 4-Hydroxyethyl-1-piperazino, 4-Phenyl-1-piperazino,
- Cycloalkylamino, C₃₋₁₄ Arylamino und Heteroarylamino [z.B. Phenyl-, 1- und 2-Naphthyl-, 2-, 3- oder 4-Pyridyl-, Chinolinyl-, Isochinolinyl-, Acridinyl-, Phenothiazinyl-, 2-Thienyl- und 2-Furylamino] (ggf. jeweils an den carbo- bzw. heterocyclischen Ringen mit R^{§} substituiert);
R⁴
- C₂₋₁₄Alkyl, C₃₋₁₄Cycloalkyl, C₂₋₁₄Alkenyl, C₃₋₁₄Cycloalkenyl, C₂₋₁₄Alkinyl (jeweils ggf. am C-Skelett der vorgenannten Reste mit R^{§} substituiert);
- Benzyl, Phenyl-(C₂₋₆)alkyl (jeweils ggf. mit R^{§} substituiert), Phenyl (ggf. mit R^{§} substituiert), dabei inbesondere: 4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl, 2-R^{§}, 4-R^{§}-Phenyl,
- 1-Naphthyl, 2-Naphthyl (jeweils ggf. mit R^{§} substituiert);
- S-Alkyl (C₃-C₈), SO-Alkyl, SO₂-Alkyl, (jeweils C₁-C₈),
S-Alkenyl, SO-Alkenyl, SO₂-Alkenyl (jeweils C₂-C₈)
S-Alkinyl, SO-Alkinyl, SO₂-Alkinyl (jeweils C₂-C₆)
(ggf. jeweils am C-Skelett der vorgenannten Reste mit -OH, -CN, -SCN, -NO₂, Phenyl oder C₃ - C₇Cycloalkyl substituiert);
- mono-, bi- oder tricyclischer gesättigter oder ein- oder mehrfach ungesättigter heterocyclischer Rest mit insgesamt 4-14 Ring-Atomen, davon 1-5 Heteroatomen, die vorzugsweise N, O, S und Se sind (jeweils ggf. mit R^{§} substituiert);
- OR⁶, worin R⁶ bedeutet
- Wasserstoff
- CH₃, C₂H₅, (CH₂)₂CH₃, (CH₂)₃CH₃, CH(CH₃)₂, CH₂CH(CH₃)₂, C(CH₃)₃, CH₂CH₂OH, CH₂CH₂SH, CH₂CH₂SCH₃, CH₂CF₃, CH₂CCl₃, CH₂CHF₂, CH₂CHCl₂, CH₂CH₂F, CH₂CH₂Cl, CH₂CH₂Br,
- C₃-₇Cycloalkyl [z.B.Cylopropyl, Cylopropylmethyl, Cylobutyl, Cyclobutylmethyl, Cyclopentyl, Cyclopentylmethyl, Cylohexyl, Cyclohexylmethyl] (ggf. am C- Skelett mit R^{§} substituiert),
- C₂-₅Alkenyl und C₂-₅Alkinyl,
- C₃-₇ Cycloalkenyl,
- Aryl und Heteroaryl als Reste mono-, bi- oder tricyclischer Aromaten oder Heteroaromaten mit ggf. 6-14 Ring-Atomen [z.B. Phenyl, 4-R^{§} -Phenyl 3-R^{§} -Phenyl, 2-R^{§}-Phenyl, 3-R^{§},4-R^{§}-Phenyl, 3-R^{§},4-R^{§}-,5-R^{§}-Phenyl],
- 1-Naphthyl, 2-Naphthyl (ggf. jeweils mit R^{§} substituiert),
- Pyridyl, Isochinolinyl, Chinolinyl, Acridinyl;
- NR⁷R⁸, worin dieser Substituent insgesamt bedeutet:
- Morpholino, Thiomorpholino, Thiomorpholino-S-Oxid, Thiomorpholino-S,S-Dioxid, Pyrrolidino, Piperidino, 1-Piperazino, 1-Homopiperazino, 4-C₁₋₆₋Alkyl-1-piperazino, 4-(2-Hydroxyethyl)-1-piperazino, 4-Benzyl-1-piperazino, 4-Aryl-1-piperazino (jeweils ggf. mit R^{§} am heterocycloaliphatischen Ring substituiert),
- weitere Amino-Reste sekundärer, mono- oder polycyclischer cycloaliphatischer Amine mit insgesamt 5-14 Ringatomen, einschließlich der am C-Skelett mit R^{§} substituierte Vertreter;
- Amino-Reste sekundärer aliphatischer und aromatischer Amine, R⁷R⁸H,
worin R⁷und R⁸unabhängig voneinander gleich oder ungleich sein können und bedeuten:
- C₁-₆Alkyl, Benzyl, Phenyl, 1- und 2-Naphthyl, 2-, 3- und 4-Pyridyl, Chinolinyl, Isochinolinyl, 2-Thienyl, 2-Furyl (ggf. jeweils mit R^{§} substituiert);
- Aminoreste primärer Amine, R⁷NH₂, (worin R⁷ dasselbe wie oben bedeutet),
- Amino, NH₂, mit der Einschränkung, dass dann R⁵ nur die Bedeutung von -OR⁶hat ;
R⁵
- Wasserstoff,
- Heterocyclyl, fünf- bis siebengliedrig, ggf. substituiert durch R³,
- Phenyl, ggf. substituiert durch R³,
- OR⁶, wobei R⁶ dasselbe wie oben bedeutet,
- NR⁷ R⁸, mit gleicher Bedeutung wie oben
- Azido, Hydroxylamino, O-(C₁-₃)Alkylhydroxylamino,
- N-(C₁-₃)Alkylhydroxylamino, N,N-Di(C₁-₃)alkylhydroxylamino,
- Hydrazino, (C₁-₄)Alkyl- und Di(C₁-₄)alkylhydrazino,
- Benzylhydrazino, Acylhydrazino, N,N-Diacylhydrazino,
- Carbamoylamino,
- 1-Imidazolyl, 1,2,4-Triazol-1yl, 1-Pyridinium,
- 1-Pyrazinium, 1-Pyridazinium, einschliesslich der alkylsubstituierten Vertreter dieser Azaheterocyclen;
R* =O, =S, =N-H, =N-C₁₋₈Alkyl, =N-Aryl, =N-OH, =N-Q-C₁₋₈Alkyl, =N-O-C₆₋₁₄ Aryl
R^{§} wie oben (bei Formel 1a bzw. 1 b) definiert
wobei folgende Verbindungen der Formel 1 (c) vom Schutz ausgenommen sind:
1,4-Dihydro-2,2-dimethyl-5-(4-morpholinyl)-10-propylthio-2H-pyrano[4",3":4',5']pyrido[3',2':4',5']thieno[3,2-d]pyrimidin-8(9H)-on,
10-Buthylthio-1,4-dihydro-2,2-dimethyl-5-(4-morpholinyl)-2H-pyrano[4",3":4',5']pyrido[3',2':4',5']thieno[3,2-d]pyrimidin-8(9H)-on
und 2-Methyl-5-thiophen-2-yl-1,2,3,4-tetrahydro-11H-7-thia-2,6,9,11-tetraaza-benzo[c]fluorene-8,10-dione
sowie pharmazeutisch verträgliche Salze, Solvate, und, Tautomere dieser Verbindungen,

2. Verbindung nach Anspruch 1, mit der allgemeinen Formel 1(a)(I) oder 1(a)(II): 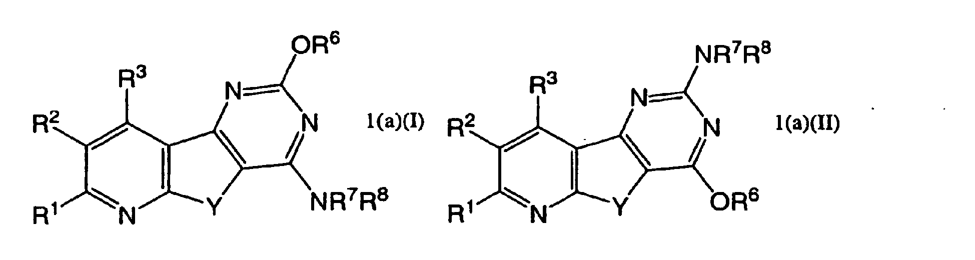 worin R¹, R², R³, R⁶, R⁷, R⁸ und Y die bezüglich Formel 1(a) oben genannten Bedeutungen besitzen.

3. Verbindung nach Anspruch 1 mit der Formel 1 (a), wobei Y = S und die Substituenten R¹, R², R³, R⁴, R⁵ die folgenden Bedeutungen haben:
| R₁ | R₂ | R₃ | R₄ | R₅ |
|---|---|---|---|---|
| 3,4-(MeO)₂-Ph | H | Me | Piperazin-1-yl | OEt |
| 4-MeO-Ph | H | Me | Piperazin-1-yl | OEt |
| 4-F-Ph | H | Me | Piperazin-1-yl | OEt |
| Ph | H | Me | Piperazin-1-yl | O/Pr |
| Ph | H | Me | Piperazin-1-yl | OCH₂CHF₂ |
| Ph | H | Ph | Piperazin-1-yl | OEt |
| Me | 4'-NO₂-Ph-CH₂ | Me | Piperazin-1-yl | OEt |
| Me | 4'-NH₂-Ph-CH₂ | Me | Piperazin-1-yl | OEt |
| 3,4-Methylen-dioxyphenyl | H | Me | Piperazin-1-yl | OEt |
| Ph | H | Me | Piperazin-1-yl | O*n*Pr |
| Ph | H | Me | Piperazin-1-yl | OEt |
| Ph | H | Me | Piperazin-1-yl | OCH₂CH₂O H |
| 3,4-(MeO)₂-Ph | H | Me | OEt | Piperazin-1-yl |
| 4-MeO-Ph | H | Me | OEt | Piperazin-1-yl |
| Ph | H | Me | OCH₂CHF₂ | Piperazin-1-yl |
| Me | 4'-NO₂-Ph-CH₂ | Me | OEt | Piperazin-1-yl |
| Ph | 4'-NO₂-Ph-CH₂ | Me | OEt | Piperazin-1-yl |
| 3,4-(MeO)₂-Ph | H | Me | *n*Pr | Piperazin-1-yl |
| Ph | H | Me | OCH₂CH₂O H | Piperazin-1-yl |
| Ph | H | Me | OEt | Piperazin-1-yl |
| Ph | H | Me | O*i*Pr | Piperazin-1-yl |
| 3,4-Methylen-dioxyphenyl | H | Me | OEt | Piperazin-1-yl |
| 4-(Pyrimidyl-2-yl)piperazin-1-yl)- | H | Et | Piperazin-1-yl | OEt |
| 4-Me-Ph | H | Et | Piperazin-1-yl | OEt |
| 4-EtO-Ph | H | Et | Piperazin-1-yl | OEt |
| 4-EtO-Ph | H | Et | Piperazin-1-yl | OProp |
| 4-Me-Ph | H | Et | Piperazin-1-yl | OEt O*i*Pr |
| 4-(4-MeOPhO)Ph | H | Et | Piperazin-1-yl | OEt |
| 2,3-Dihydro-benzo[b][1,4]-dioxin-6-yl | H | Et | Piperazin-1-yl | OEt |
| 3,4-Methylen-dioxyphenyl | H | Et | Piperazin-1-yl | OEt |
| 4-MeO-Ph | H | Et | Piperazin-1-yl | OEt |
| 4-(1H-pyrazol-1-yl)-Ph | H | Et | Piperazin-1-yl | OEt |
| 4-Br-Ph | H | Et | Piperazin-1-yl | OEt |
| 4-Cl-Ph | H | Et | Piperazin-1-yl | OEt |
| 4-I-Ph | H | Et | Piperazin-1-yl | OEt |
| Morpholin | H | Ph | Piperazin-1-yl | OEt |
| 4-MeO-Ph | H | CF₃ | Piperazin-1-yl | OEt |
| 4-Me-Ph | H | CF₃ | Piperazin-1-yl | OEt |
| 2,3-Dihydro-benzo[b][1,4]-dioxin-6-yl | H | CF₃ | Piperazin-1-yl | OEt |
| 4-Imidazol-1-yl-Ph | H | CF₃ | Piperazin-1-yl | OEt |
| 4-Imidazol-1-yl-Ph | H | CF₃ | Piperazin-1-yl | OCH₂CH₂O H |
| 4-(Piperidin-1-yl)-Ph | H | CF₃ | Piperazin-1-yl | OEt |
| 4-Morpholino-Ph | H | CF₃ | Piperazin-1-yl | OEt |
| 3,4-Methylen-dioxyphenyl | H | CF₃ | Piperazin-1-yl | OEt |
| 4-MeS-Ph | H | CF₃ | Piperazin-1-yl | OEt |
| 4-EtOOC-Ph | H | CF₃ | Piperazin-1-yl | OEt |
| 4-(1H-pyrazol-1-yl)-Ph | H | CF₃ | Piperazin-1-yl | OEt |
| 4-F-Ph | H | CF₃ | Piperazin-1-yl | OEt |
| 4-I-Ph | H | CF₃ | Piperazin-1-yl | OEt |
| 3-NO₂-Ph | H | CF₃ | Piperazin-1-yl | OEt |
| 4-NO₂-Ph | H | CF₃ | Piperazin-1-yl | OEt |
| 3-CN-Ph | H | CF₃ | Piperazin-1-yl | OEt |
| 4-CN-Ph | H | CF₃ | Piperazin-1-yl | OEt |
| 3-NH₂-Ph | H | CF₃ | Piperazin-1-yl | OEt |
| 4-NH₂-Ph | H | CF₃ | Piperazin-1-yl | OEt |
| 4-MeS-Ph | H | CF₃ | Piperazin-1-yl | OEt |
| 4-MeS(O)-Ph | H | CF₃ | Piperazin-1-yl | OEt |
| Pyridin-4-yl | H | CF₃ | Piperazin-1 -yl | OEt |
| Pyridin-4-yl-N-oxid | H | CF₃ | Piperazin-1-yl | OEt |
| Pyridin-3-yl | H | CF₃ | Piperazin-1-yl | OEt |
| 4-(Pyrazin-2-yl) | H | CF₃ | Piperazin-1-yl | OEt |
| 4(HCONH)-Ph | H | CF₃ | Piperazin-1-yl | OEt |
| 4-(Pyridin-4-yl-CONH)-Ph | H | CF₃ | Piperazin-1-yl | OEt |
| 4-(Pyridin-3-yl-CONH)-Ph | H | CF₃ | Piperazin-1-yl | OEt |
| 4-(Pyridin-3-yl-N-Oxid-CONH)-Ph | H | CF₃ | piperazin-1-yl | OEt |
| 4-(1H-1,2,4-triazol-4-yl)-Ph | H | CF₃ | Piperazin-1-yl | OEt |
| 4-(1H-pyrrol-1-yl)-Ph | H | CF₃ | Piperazin-1-yl | OEt |
| 4(CH₃OOCNH )-Ph | H | CF₃ | Piperazin-1-yl | OEt |
| *iso*Pr | H | *iso*Pr | Piperazin-1-yl | OEt |
| 2,3-Dihydro-benzo[b][1,4]-dioxin-6-yl | H | Et | OEt | Piperazin-1-yl |
| 4-EtO-Ph | H | Et | OEt | Piperazin-1-yl |
| 4-Me-Ph | H | Et | OEt | Piperazin-1-yl |
| 4-Me-Ph | H | Et | OCH₂CH₂O H | Piperazin-1-yl |

4. Verbindung nach Anspruch 1 mit der Formel 1 (a), wobei Y = O und die Substituenten R¹, R², R³, R⁴, R⁵ die folgenden Bedeutungen haben:
| R₁ | R₂ | R₃ | R₄ | R₅ |
|---|---|---|---|---|
| Ph | H | Me | Piperazin-1-yl | OEt |
| Ph | H | Me | OEt | Piperazin-1-yl |

5. Verbindung nach Anspruch 1 mit der Formel 1 (b), wobei Y = S und die Substituenten R¹, R³, R⁴, R⁵ die folgenden Bedeutungen haben:
| R₁ | R₃ | R₄ | R₅ |
|---|---|---|---|
| 3-NH₂-Ph-4-Ph | Me | Piperazin-1-yl | OEt |
| 4-(3-furyl)-Ph | Me | Piperazin-1-yl | OEt |
| 4-(2-furyl)-Ph | Me | Piperazin-1-yl | OEt |
| 4-(3-pyridyl)-Ph | Me | Piperazin-1-yl | OEt |
| 4-(4-pyridyl)-Ph | Me | Piperazin-1yl | OEt |
| 4-NH2-Ph-Ph | Me | Piperazin-1yl | OEt |
| 3,4,5-(CH₃O)₃-Ph-Ph | Me | Piperazin-1-yl | OEt |
| 4-PhO-Ph | Me | Piperazin-1 yl | OEt |
| 2-NH₂-4-CH₃OOC-Ph-Ph | Me | Piperazin-1-yl | OEt |
| 3-CH₃OOC-Ph-Ph | Me | Piperazin-1-yl | OEt |
| 3,4-(CH₃O)₂-Ph-Ph | Me | Piperazin-1-yl | OEt |
| 3,5-(CH₃)₂-isoxazol-4-yl-Ph | Me | Piperazin-1-yl | OEt |
| Phenyl | Me | Piperazin-1-yl | OEt |
| 4-Br-Ph | Me | Piperazin-1-yl | OEt |
| 3-NH2-Ph | Me | Piperazin-1-yl | OEt |
| 4-(pyrrol-3-yl)-Ph | Me | Piperaizin-1-yl | OEt |
| 4-(pyrimidin-5-yl)-Ph | Me | Piperazin-1-yl | OEt |
| 4-(pyrid-3-yl-hydroxymethyl)-Ph | Me | Piperazin-1-yl | OEt |
| Ph-Ph | Me | Piperazin-1- | OEt |
| 4-(*iso*PropO)-Ph | Me | Piperazin-1-yl | OEt |
| 4-Vinyl-Ph | Me | Piperazin-1-yl | OEt |

6. Verbindung nach Anspruch 1 mit der Formel 1(a) oder 1 (b), wobei diese sind:
2-(2-Hydroxyethoxy)-9-methyl-7-phenyl-4-(piperazin-1-yl)-pyrido[3',2':4,5]thieno[3,2-d]pyrimidin;
2-Ethoxy-7-(3,4-dimethoxy-phenyl)-9-methyl-4-(piperazin-1-yl)-pyrido[3',2':4,5]thieno[3,2-d]pyrimidin;
2-Ethoxy-7-(4-methoxy-phenyl)-9-methyl-4-(piperazin-1-yl)-pyrido[3',2':4,5]thieno[3,2-d]pyrimidin;
2-Ethoxy-9-ethyl-7-(4-methyl-phenyl)-4-(piperazin-1-yl)-pyrido[3',2':4,5]thieno[3,2-d]pyrimidin
2-Ethoxy-7-(4-ethoxy-phenyl)-9-ethyl-4-(piperazin-1-yl)-pyrido[3',2':4,5]thieno[3,2-d]pyrimidin
2-Ethoxy-9-ethyl-7-(2,3-dihydrobenzo[b][1,4]dioxin-6-yl)-4-(piperazin-1-yl)-pyrido[3',2':4,5] thieno[3,2-d]pyrimidin
4-Ethoxy-9-trifluormethyl-7-(pyrazin-2-yl)-2-piperazino-pyrido[3',2':4,5]thieno[3,2-d]pyrimidin
4-Ethoxy-9-ethyl-7-(4-(pyrimidyl-2-yl)piperazin-1-yl)-2-piperazin-1-yl-pyrido[3',2':4,5]thieno[3,2.d]pyrimidin
4-Ethoxy-7-morpholino-9-phenyl-2-(piperazin-1-yl)-pyrido[3',2':4,5]thieno[3,2-d]pyrimidin;
4-Ethoxy-7-phenyl-9-methyl-2-(piperazin-1-yl)-pyrido[3',2':4,5]furo[3,2-d]pyrimidin;
4-Ethoxy-7,9-dimethyl-8-(4-nitro-benzyl)-2-(piperazin-1-yl)-pyrido[3',2':4,5]thieno[3,2-d]pyrimidin;
4-Ethoxy-7-(3,4-dimethoxy-phenyl)-9-methyl-2-(piperazin-1-yl)-pyrido[3',2':4,5]thieno[3,2-d]pyrimidin;
4-Ethoxy-9-ethyl-7-(4-methyl-phenyl)-2-(piperazin-1-yl)-pyrido[3',2':4,5]thieno[3,2-d]pyrimidin
4-Ethoxy-7-(4-fluorphenyl)-9-methyl-2-(piperazin-1-yl)-pyrido[3',2':4,5]furo[3,2-d]pyrimidin
7-(4-Ethoxy-phenyl)-9-ethyl-2-(piperazin-1-yl)-4-*n*-propoxy-pyrido[3',2':4,5]thieno[3,2-d]pyrimidin
4-Ethoxy-7-(4-ethoxy-phenyl)-9-ethyl-2-(piperazin-1-yl)-pyrido[3',2':4,5]thieno[3,2-d]pyrimidin
4-Ethoxy-7-(4-(pyrimidin-2-yl)piperazin-1-yl)-9-ethyl-2-(piperazin-1-yl)-pyrido[3',2':4,5]thieno[3,2-d]pyrimidin
4-Ethoxy-9-ethyl-7-(4-methylthio-phenyl)-2-(piperazin-1-yl)-pyrido[3',2':4,5]thieno[3,2-d]pyrimidin
4-Ethoxy-7-(4-methyl-phenyl)-9-iso-propyl-2-(piperazin-1-yl)-pyrido[3',2':4,5]thieno-[3,2-d]pyrimidin
4-Ethoxy-9-ethyl-7-[4-(4-methoxyphenoxy)phenyl]-2-(piperazin-1-yl)-pyrido[3',2':4,5] thieno[3,2-d]pyrimidin
4-Ethoxy-9-ethyl-7-(2,3-dihydrobenzo[b][1,4]dioxin-6-yl)-2-(piperazin-1-yl)-pyrido[3',2':4,5] thieno[3,2-d]pyrimidin
4-Ethoxy-9-ethyl-7-(3,4-methylendioxyphenyl)-2-piperazin-1-yl-pyrido[3',2':4,5]thieno-[3,2-d]pyrimidin
4-Ethoxy-7-(4-methoxy-phenyl)-2-(piperazin-1-yl)-9-trifluormethyl-pyrido[3',2':4,5]thieno[3,2-d]pyrimidin
4-Ethoxy-7-(4-methyl-phenyl)-2-(piperazin-1-yl)-9-trifluormethyl-pyrido[3',2':4,5]thieno[3,2-d]pyrimidin
7-(2,3-Dihydrobenzo[b][1,4]dioxin-6-yl)-4-ethoxy-2-(piperazin-1-yl)-9-trifluormethyl-pyrido[3',2':4,5] thieno[3,2-d]pyrimidin
4-Ethoxy-7-(4-(1*H*-imidazol-1-yl)phenyl)-2-(piperazin-1-yl)-9-trifluormethyl-pyrido[3',2':4,5]thieno[3,2-d]pyrimidin
4-[4-Ethoxy-2-(piperazin-1-yl)-9-trifluormethyl-pyrido[3',2':4,5]thieno[3,2-d]pyrimidin-7-yl-benzoesäureethylester
4-Ethoxy-2-(piperazin-1-yl)-7-(4-(piperidin-1-yl)phenyl)-9-trifluormethyl-pyrido[3',2':4,5]thieno[3,2-d]pyrimidin
4-Ethoxy-9-ethyl-7-(4-methoxyphenyl)-2-(piperazin-1-yl)-pyrido[3',2':4,5]thieno[3,2-d]pyrimidin
4-Ethoxy-7-(3,4-methylendioxyphenyl)-2-piperazin-1-yl-9-trifluormethyl-pyrido[3',2':4,5]thieno-[3,2-d]pyrimidin
4-Ethoxy-7-(4-methylthiophenyl)-2-(piperazin-1-yl)-9-trifluormethyl-pyrido[3',2':4,5]thieno[3,2-d]pyrimidin
4-Ethoxy-7-(4-(1H-pyrazol-1-yl)-phenyl)-9-ethyl-2-piperazin-1-yl-pyrido[3',2':4,5]thieno[3,2-d]pyrimidin
4-Ethoxy-7-(4-iod-phenyl)-2-(piperazin-1-yl)-9-trifluormethyl-pyrido[3',2':4,5]thieno[3,2-d]pyrimidin
4-Ethoxy-7-(4-(1H-pyrazol-1-yl)-phenyl)-2-piperazin-1-yl-9-trifluormethyl-pyrido[3',2':4,5]thieno[3,2-d]pyrimidin
7-(4-Brom-phenyl)-4-ethoxy-9-ethyl-2-(piperazin-1-yl)-pyrido[3',2':4,5]thieno[3,2-d]pyrimidin
4-Ethoxy-9-ethyl-7-(4-iod-phenyl)-2-(piperazin-1-yl)-pyrido[3',2':4,5]thieno[3,2-d]pyrimidin
4-Ethoxy-7-(3-nitro-phenyl)-2-piperazin-1-yl-9-triluormethyl-pyrido[3',2':4,5]thieno[3,2-d]pyrimidin
7-(3-Cyano-phenyl)-4-ethoxy-2-piperazin-1-yl-9-trifluormethyl-pyrido[3',2':4,5]thieno[3,2-d]pyrimidin
7-(4-Cyano-phenyl)-4-ethoxy-2-piperazin-1-yl-9-trifluormethyl-pyrido[3',2':4,5]thieno[3,2-d]pyrimidin
7-(3-Aminp-phenyl)-4-ethoxy-2-piperazin-1-yl-9-trifluormethyl-pyrido[3',2':4,5]thieno[3,2-d]pyrimidin
7-(4-Amino-phenyl)-4-ethoxy-2-piperazin-1-yl-9-trifluormethyl-pyrido[3',2':4,5]thieno[3,2-d]pyrimidin
4-Ethoxy-9-methyl-7-(4-methylsulfinyl-phenyl)-2-(piperazin-1-yl)-pyrido[3',2':4,5]thieno[3,2-d]pyrimidin
N-(4-(4-Ethoxy-9-trifluormethyl-2-piperazin-1-yl-pyrido[3',2':4,5]thieno[3,2-d]pyrimidin-7-yl)-phenyl)-formamid
N-(4-(4-Ethoxy-9-trifluormethyl-2-piperazin-1-yl-pyrido[3',2':4,5]thieno[3,2-d]pyrimidin-7-yl)-phenyl)-isonicotinamid
4-Ethoxy-9-trifluormethyl-2-piperazin-1-yl-7-(4-(1H-pyrrol-1-yl)-phenyl)-pyrido[3',2':4,5]thieno[3,2-d]pyrimidin
N-(4-(4-Ethoxy-9-trifluormethyl-2-piperazin-1-yl-pyrido[3',2':4,5]thieno[3,2-d]pyrimidin-7-yl)-phenyl)-nicotinamid-N-oxid
4-(4-Ethoxy-9-trifluormethyl-2-piperazin-1-yl-pyrido[3',2':4,5]thieno[3,2-d]pyrimidin-7-yl)-phenyl-carbaminsäuremethylester
N-(4-(4-Ethoxy-9-trifluormethyl-2-piperazin-1-yl-pyrido[3',2':4,5]thieno[3,2-d]pyrimidin-7-yl)-phenyl)-nicotinamid
N-(4-Ethoxy-9-trifluormethyl-2-piperazin-1-yl-pyrido[3',2':4,5]thieno[3,2-d]pyrimidin-7-yl)-benzamid Trifluoracetat
N'-Hydroxy-4-(4-ethoxy-9-trifluormethyl-2-piperazin-1-yl-pyrido[3',2':4,5]thieno[3,2-d]pyrimidin-7-yl)-benzamidin Bistrifluoracetat
7-(3'-Aminobiphen-4-yl)-4-ethoxy-9-methyl-2-piperazin-1-yl-thieno[2,3-d:4,5-d']dipyrimidin
4-Ethoxy-7-(4-[3-furyl]-phenyl)-9-methyl-2-piperazin-1-yl-thieno[2,3-d:4,5-d']dipyrimidin
4-Ethoxy-7-(4-[2-furyl]-phenyl)-9-methyl-2-piperazin-1-yl-thieno[2,3-d:4,5-d']dipyrimidin
4-Ethoxy-9-methyl-2-piperazin-1-yl-7-(4-[3-pyridyl]-phenyl)-thieno[2,3-d:4,5-d']dipyrimidin
4-Ethoxy-9-methyl-2-piperazin-1-yl-7-(4-[4-pyridyl]-phenyl)-thieno[2,3-d:4,5-d']dipyrimidin
7-(4'-Aminobiphen-4-yl)-4-ethoxy-9-methyl-2-piperazin-1-yl-thieno[2,3-d:4,5-d']dipyrimidin
4-Ethoxy-9-methyl-2-piperazin-1-yl-7-(3',4',5'-trimethoxybiphen-4-yl)-thieno[2,3-d:4,5-d']dipyrimidin
4-Ethoxy-9-methyl-7-(4-phenoxy-phenyl)-2-piperazin-1-yl-thieno[2,3-d:4,5-d']dipyrimidin
2'-Amino-4-(4-ethoxy-9-methyl-2-piperazin-1-yl -thieno[2,3-d:4,5-d']dipyrimidin-7-yl)-biphenyl-4'-carbonsäuremethylester
7-(3',4'-Dimethoxybiphen-4-yl)-4-ethoxy-9-methyl-2-piperazin-1-yl-thieno[2,3-d:4,5-d']dipyrimidin

7. Verbindung nach Anspruch 1 mit der Formel 1(c) (I) oder 1(c) (II): 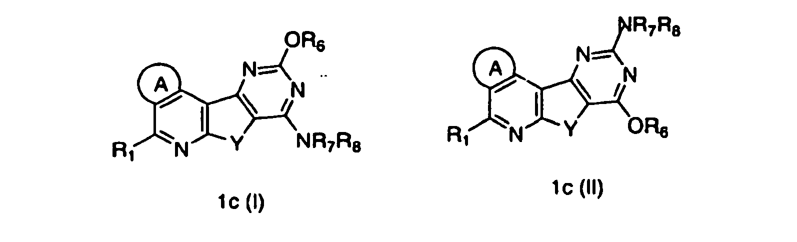 worin R¹, R⁶, R⁷, R⁸sowie A und Y die bezüglich Formel 1 (c) oben genannten Bedeutungen besitzen.

8. Verbindung nach Anspruch 1 mit der Formel 1(c) oder 1(d), wobei diese aus der Gruppe ausgewählt ist:
10-Ethoxy-5-morpholino-8-piperazin-1-yl-pyrimido[4',5':4,5]thieno[2,3-c]-1,2,3,4-tetrahydro-isochinolin
7-Ethoxy-2,3-dihydro-4-morpholino-9-piperazin-1yl-1H-cyclopenta[4',5']pyrido[3',2':4,5]thieno[3,2-d]pyrimidin
8-Ethoxy-5-piperidino-10-piperazin-1-yl-pyrimido[4',5':4,5]thieno[2,3-c]-1,2,3,4-tetrahydro-isochinolin-hydrochlorid
8-Ethoxy-5-morpholino-10-piperazin-1-yl-pyrimido[4',5':4,5]thieno[2,3-c]-1,2,3,4-tetrahydro-isochinolin-hydrochlorid
8-Ethoxy-5-phenyl-10-piperazin-1-yl-pyrimido[4',5':4,5]thieno[2,3-c]-1,2,3,4-tetrahydro-isochinolin-hydrochlorid
10-Ethoxy-5-phenyl-8-piperazin-1-yl-pyrimido[4',5':4,5]thieno[2,3-c]-1,2,3,4-tetrahydro-isochinolin
8-Ethoxy-5-morpholino-10-piperazin-1-yl-pyrimido[4',5':4,5]thieno[2,3-c]-1,2,3,4-tetrahydro-isochinolin
7-Ethoxy-2,3,4,5-tetrahydro-4-morpholino-9-piperazin-1yl-1H-cyclohepta[4',5']pyrido[3',2':4,5]thieno[3,2-d]pyrimidin

9. Pharmazeutische Zusammensetzung enthaltend mindestens eine der Verbindungen nach einem der Ansprüche 1-8 sowie ggf. pharmazeutisch verträgliche Hilfsstoffe und/oder Träger.

10. Pharmazeutische Zusammensetzung nach Anspruch 9 enthaltend zusätzlich einen oder mehrere der folgenden Wirkstoffe:
- β₂-Adrenoceptor Agonisten
- Dinatriumcromoglycat
- Korticosteroide
- Leukotrien-Antagonisten (entweder Enzym-Inhibitoren [wie 5-Lipoxygenaseinhibitoren oder Arachidonsäure-Enzyminhibitoren] oder Rezeptorantagonisten) ,
- Antihistaminika (bevorzugt solche mit Mastzellen-stabilisierenden Eigenschaften oder Leukotrien-antagonisierenden Aspekten)
- Theophyllin
- Muscarinrezeptor-Antagonisten
- (monoklonale) Antikörper gegen TNF-alpha oder andere Wirkstoffe, die die Bildung bzw. Freisetzung von TNF-alpha oder die Aktivität von TNF-alpha hemmen

11. Verwendung mindestens einer der Verbindungen nach einem der Ansprüche 1-8 zur Herstellung eines Arzneimittels zur Inhibierung des Enzyms Phosphodiesterase 4 (PDE4) und/oder TNF alpha-Freisetzung.

12. Verwendung nach Anspruch 11 zur Behandlung von Asthma bronchiale, COPD, Rheumatoider Arthritis (RA), Osteoarthritis, Multipler Sklerose, Guillain-Barré Syndrom, Mb. Crohn, Colitis ulcerosa, Psoriasis, atopischer Dermatitis, allergische Ekzeme, Rhinitis allergica, allergische Konjunktivitis, systemische Sklerodermie, Graft versus host disease (GvHD), Systemischer Lupus Erythematodes (SLE), Diabetes mellitus Typ I, neurodegenerative Erkrankungen, insbesondere Mb. Alzheimer und Mb. Parkinson, posttraumatisches Multiorganversagen, Toxisches Schocksyndrom, Akute Glomerulonephritis, akute und chronische Schmerzen, Arteriosklerose, Herzinfarkt, Schlaganfall, Tumorerkrankungen, axonaler Degeneration, virale Erkrankungen.

13. Verwendung nach Anspruch 12, wobei die Tumorerkrankung Leukämie ist.

14. Verwendung nach Anspruch 13, wobei die Tumorerkrankung CLL ist.

## Claims

1. Compounds of general formulae 1a, 1b, 1c and 1d, 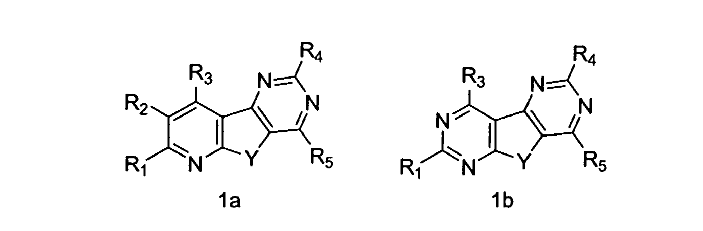 wherein:
Y is S, O or N-H
R¹ is
- hydrogen,
- C₁₋₁₀ alkyl, -C₂₋₁₂ alkenyl, -C₂₋₁₂ alkinyl, each straight-chain, branched or cyclic and, where appropriate, substituted singly, doubly or triply with independently selected residues R^{§},
- aralkyl- having C₆₋₁₂ aryl and C₁₋₅ alkyl, substituted, where appropriate, singly, doubly or triply with independently selected residues R^{§},
- phenyl, substituted, where appropriate, singly, doubly or triply with independently selected residues R^{§},
- 1-naphthyl, 2-naphthyl,
- pyridyl-N-oxide, (substituted, where appropriate, with R^{§}),
- monocyclic or bicyclic, saturated or singly or multiply unsaturated heterocycles having 5 - 14 ring atoms, including 1
- 4 heteroatoms which are preferably N, O and S, substituted, where appropriate, singly, doubly or triply with independently selected residues R^{§},
- C₁₋₁₀ alkoxy, straight-chain, branched or cyclic as well as substituted, where appropriate, singly, doubly or triply with independently selected residues R^{§},
- aralkyloxy- having C₆₋₁₂ aryl and C₁₋₅ alkyl, substituted, where appropriate, singly, doubly or triply with independently selected residues R^{§},
- C₂₋₁₂ alkylacyl (substituted, where appropriate, with R^{§}),
- benzoyl, 1- and 2-naphthoyl (each substituted, where appropriate, with R^{§} ),
- hydroxy, sulfhydryl, formyl, carboxyl, CONH₂, cyano, rhodano, nitro, SO₃H,
- alkylthio, alkylsulfinyl, SO₂OAlk and alkylsulfonyl, each straight-chain, branched or cyclic C₁₋₆, and substituted, where appropriate, singly, doubly or triply with independently selected residues R^{§},
- arylthio, arylsulfinyl, arylsulfonyl, heteroarylthio, heteroarylsulfinyl and heteroarylsulfonyl, each substituted, where appropriate, singly, doubly or triply with independently selected residues R^{§},
- alkoxycarbonyl, CONHAlk and CONAlk₂, each straight-chain, branched or cyclic C₁₋₆, and substituted, where appropriate, singly, doubly or triply with independently selected residues R^{§},
- aryloxycarbonyl, arylcarbamoyl, arylamido, N-aryl,N-alkylamido, N-aryl,N-alkylcarbamoyl, aralkyloxycarbonyl and aralkylcarbamoyl, with alkyl C₁₋₅, aryl C₆₋₁₀ and substituted, where appropriate, singly, doubly or triply with independently selected residues R^{§},
- heterocyclylcarbonyl, heterocyclylcarbamoyl, heterocyclyl-amido, N-heterocyclyl,N-alkylcarbamoyl, N-heterocyclyl,N-alkylamido, heterocyclylalkyloxycarbonyl and heterocyclyl-alkylcarbamoyl, with a monocyclic or bicyclic, saturated or singly or multiply unsaturated heterocycle having 5 - 14 ring atoms including 1 - 4 heteroatoms which are preferably N, O and S, substituted, where appropriate, singly, doubly or triply with independently selected residues R^{§} and alkyl C₁₋₅,
- chlorine, bromine, iodine, fluorine,
- amino, C₁₋₆ alkylamino, di(C₁₋₅)alkylamino, each straight-chain, branched or cyclic and substituted, where appropriate, singly, doubly or triply with independently selected residues R^{§},
- arylamino C₆₋₁₀, substituted, where appropriate, singly, doubly or triply with independently selected residues R^{§},
- heterocyclylamino, monocyclic or bicyclic, saturated or singly or multiply unsaturated, having 5 - 14 ring atoms including 1 - 4 heteroatoms which are preferably N, O and S, substituted, where appropriate, singly, doubly or triply with independently selected residues R^{§},
- arylhydrazino, substituted, where appropriate, singly, doubly or triply with independently selected residues R^{§},
- L_{A}-A-L_{B}-B
wherein:
L_{A} is
- single bond,
- NR^{#}, O, S, SO, SO₂
A is
- phenyl, substituted, where appropriate, singly, doubly or triply with independently selected residues R^{§}
- naphthyl, naphthyl substituted singly or multiply with R^{§}
- monocyclic or bicyclic, saturated or singly or multiply unsaturated heterocycles having 4 - 14 ring atoms including 1 - 5 heteroatoms which are preferably N, O and S which may carry one or more oxygen atoms on C, N and/or S and are substituted, where appropriate, singly, doubly or triply with independently selected residues R^{§}
-L_{A}-A can also be a direct bond tricycle-L_{B}, in this case L_{B} being a single bond
L_{B} is
- single bond
- NR^{#}, O, S, SO, SO₂, -CHR^{§}-, -CH₂-O-, -O-CH₂,
- the following functional groups: 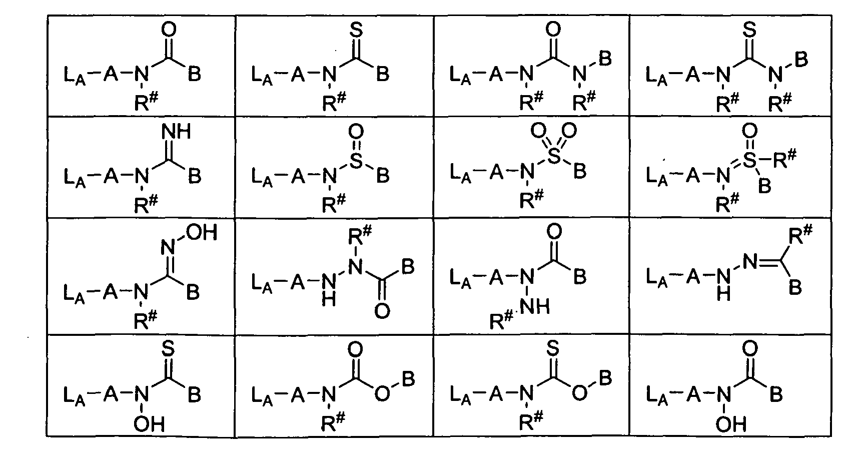
B is
- hydrogen
- alkyl, substituted, where appropriate, singly, doubly or triply independently with R^{§}
- -CONH₂, -CONHAlk (Alk substituted, where appropriate, with R^{§}), -CONHAryl (aryl substituted, where appropriate, with R^{§}), -CONHHetaryl (hetaryl substituted, where appropriate, with R^{§}), -COOH,
- COOAlk (Alk substituted, where appropriate, with R^{§}),
- -COAlk (Alk substituted, where appropriate, with R^{§}),
- COAryl (aryl substituted, where appropriate, with R^{§}),
- COHetaryl (Hetaryl substituted, where appropriate, with R^{§})
- -CH₂-CONH₂ , -CH₂-CONHAlk (Alk substituted, where appropriate, with R^{§}), -CH₂-CONHAryl (Aryl substituted, where appropriate, with R^{§}), -CH₂-CONHHetaryl (hetaryl substituted, where appropriate, with R^{§}), -CH₂-COOH,
- CH₂-COOAlk (Alk substituted, where appropriate, with R^{§})
- -CH₂-COAlk (Alk substituted, where appropriate, with R^{§}), -CH₂-COAryl (aryl substituted, where appropriate, with R^{§}), -CH₂-COHetaryl (hetaryl substituted, where appropriate, with R^{§})
- phenyl, substituted, where appropriate, singly, doubly or triply with independently selected residues R^{§},
- naphthyl, substituted, where appropriate, singly, doubly or triply with independently selected residues R^{§},
- monocyclic or bicyclic, saturated or singly or multiply unsaturated heterocycles having 4 - 14 ring atoms including 1 - 5 heteroatoms which are preferably N, O and S which may carry one or more oxygen atoms on C, N and/or S and may be substituted, where appropriate, singly, doubly or triply with independently selected residues R^{§},
R^{#} - hydrogen, alkyl, substituted, where appropriate, with R^{§}
R² is
- hydrogen,
- C₁₋₁₂ alkyl,
- C₂₋₁₂ alkenyl and C₂₋₁₂ alkinyl,
- benzyl, phenyl(C₂₋₆)alkyl (substituted, where appropriate, once or several times equally or unequally with R^{§} at the aromatic and/or aliphatic molecule portion);
- phenacyl (substituted, where appropriate, with R^{§} once or several times equally or unequally at the aromatic molecule portion);
- carboxyl, C₁₋₄ alkoxycarbonyl, -CONH₂, -CONHAlk and CONAlk₂ ("Alk" each being C₁₋₆),
- R^{#}C(O)- (wherein R^{#} is as defined above),
- cyano, nitro, amino, C₁₋₆ alkylamino, di(C₁₋₆)alkylamino, -N=N-C₆H₅, -N=N-C₆H₄-R^{§},
- 1,3-diphenyl-pyrazol-4-yl, thiazolin-2-yl, imidazolin-2-yl and 3,4,5,6-tetrahydro-pyrimidinyl;
R³ is
- hydrogen,
- C₁₋₁₀ alkyl, -C₂₋₁₂ alkenyl, -C₂₋₁₂ alkinyl, each straight-chain, branched or cyclic and substituted, where appropriate, singly, doubly or triply with independently selected residues R^{§},
- trifluoromethyl,
- aralkyl- with C₆₋₁₂ aryl and C₁₋₅ alkyl, substituted, where appropriate, singly, doubly or triply with independently selected residues R^{§},
- phenyl, substituted, where appropriate, singly, doubly or triply with independently selected residues R^{§},
- 1-naphthyl, 2-naphthyl,
- pyridyl-N-oxide, (substituted, where appropriate, with R^{§}),
- C₁₋₁₀ alkoxy, straight-chain, branched or cyclic and substituted, where appropriate, singly, doubly or triply with independently selected residues R^{§},
- aralkyloxy- with C₆₋₁₂ aryl and C₁₋₅ alkyl, substituted, where appropriate, singly, doubly or triply with independently selected residues R^{§},
- C₂₋₁₂ alkylacyl (substituted, where appropriate, with R^{§}),
- benzoyl, 1- and 2-naphthoyl (each substituted, where appropriate, with R^{§}),
- hydroxy, sulfhydryl, formyl, carboxyl, CONH₂, cyano, rhodano, nitro, SO₃H
- alkylthio, alkylsulfinyl, SO₂OAlk and alkylsulfonyl, each straight-chain, branched or cyclic C₁₋₆ and substituted, where appropriate, singly, doubly or triply with independently selected residues R^{§},
- arylthio, arylsulfinyl, arylsulfonyl, heteroarylthio, heteroarylsulfinyl and heteroarylsulfonyl, each substituted, where appropriate, singly, doubly or triply with independently selected residues R^{§},
- alkoxycarbonyl, CONHAlk and CONAlk₂, each straight-chain, branched or cyclic C₁₋₆, and substituted, where appropriate, singly, doubly or triply with independently selected residues R^{§},
- aryloxycarbonyl, arylcarbamoyl, arylamido, N-aryl,N-alkylamido, N-aryl,N-alkylcarbamoyl, aralkyloxycarbonyl and aralkylcarbamoyl, with alkyl C₁₋₅, aryl C₆₋₁₀ and substituted, where appropriate, singly, doubly or triply with independently selected residues R^{§},
- chlorine, bromine, iodine, fluorine,
- amino, C₁₋₆ alkylamino, di(C₁₋₅)alkylamino, each straight-chain, branched or cyclic and substituted, where appropriate, singly, doubly or triply with independently selected residues R^{§},
- arylamino C₆₋₁₀, substituted, where appropriate, singly, doubly or triply with independently selected residues R^{§},
- arylhydrazino, substituted, where appropriate, singly, doubly or triply with independently selected residues R^{§},
R⁴ is
- C₂₋₁₄ alkyl, C₃₋₁₄ cycloalkyl, C₂₋₁₄ alkenyl, C₃₋₁₄ cycloalkenyl, C₂₋₁₄ alkinyl (each substituted, where appropriate, on the C-skeleton of the above mentioned aliphatic residues with R§);
- phenyl, 4-R^{§}-phenyl, 3-R^{§}-phenyl, 2-R^{§}-phenyl (with the exception of: 2-NO₂-phenyl), 3-R^{§},4-R^{§}-phenyl, 3-R^{§},4-R^{§}-,5-R^{§}-phenyl,
- 1-naphthyl, 2-naphthyl (each substituted, where appropriate, with R^{§});
- S-alkyl, SO-alkyl, SO₂-alkyl, (each C₁-C₈),
S-alkenyl, SO-alkenyl, SO₂-alkenyl (each C₂-C₈)
S-alkinyl, SO-alkinyl, SO₂-alkinyl (each C₂-C₆)
(each substituted, where appropriate, on the C-skeleton of the above mentioned aliphatic residues with -OH, -CN, -SCN, -NO₂, phenyl or C₃-C₇ cycloalkyl);
- monocyclic, bicyclic or tricyclic, saturated or singly or mulitply unsaturated heterocyclic residue having a total of 4 - 14 ring atoms including 1-5 heteroatoms which are preferably N, O, S and Se (each substituted, where appropriate, with R^{§});
- OR⁶, wherein R⁶ is
- CH₃, C₂H₅, (CH₂)₂CH₃, (CH₂)₃CH₃, C(CH₃)₃, CH₂CH₂OH, CH₂CH₂SH, CH₂CH₂SCH₃, CH₂CF₃, CH₂CCl₃, CH₂CHF₂, CH₂CHCl₂, CH₂CH₂F, CH₂CH₂Cl, CH₂CH₂Br,
- C₃₋₇ cycloalkyl [e.g. cyclopropyl, cyclopropylmethyl, cyclobutyl, cyclobutylmethyl, cyclopentyl, cyclopentylmethyl, cyclohexyl, cyclohexylmethyl] (substituted, where appropriate, on the C-skeleton with R^{§}),
- C₂₋₅ alkenyl and C₂₋₅ alkinyl,
- C₃₋₇ cycloalkenyl,
- aryl and heteroaryl as residues of monocyclic, bicyclic or tricyclic aromatic compounds or heteroaromatic compounds with 6 - 14 ring atoms, where appropriate [e.g. phenyl, 4-R^{§}-phenyl, 3-R^{§}-phenyl, 2-R^{§}-phenyl, 3-R^{§},4-R^{§}-phenyl, 3-R^{§},4-R^{§}-,5-R^{§}-phenyl],
- 1-naphthyl, 2-naphthyl (each substituted, where appropriate, with R^{§}),
- pyridyl, isoquinolinyl, quinolinyl, acridinyl;
- NR⁷R⁸, wherein this substituent is altogether:
- morpholino, thiomorpholino, thiomorpholino-S,S-dioxide, pyrrolidino, piperidino, 1-piperazino, 1-homopiperazino, 4-C₁₋₆-alkyl-1-piperazino, 4-(2-hydroxyethyl)-1-piperazino, 4-benzyl-1-piperazino, 4-aryl-1-piperazino (substituted, where appropriate, with R^{§} on the heterocycloaliphatic ring),
- further amino residues of secondary, monocyclic or polycyclic cycloaliphatic amines having a total of 5 - 14 ring atoms, including the representatives substituted with R^{§} on the C-skeleton;
- amino residues of secondary aliphatic and aromatic amines, R⁷R⁸NH,
wherein R⁷ and R⁸ may independently be equal or unequal and represent:
- C₁₋₆ alkyl, benzyl, phenyl, 1- and 2-naphthyl, 2-, 3- and 4-pyridyl, quinolinyl, isoquinolinyl, 2-thienyl, 2-furyl (each substituted, where appropriate, with R^{§});
- amino residues of primary amines, R⁷NH₂, (wherein R⁷ is the same as above),
- amino, NH₂, with the limitation that in this case R⁵ only has the meaning of OR⁶;
R⁵ is
- OR⁶, wherein R⁶ is as defined above,
- NR⁷R⁸, which is as defined above but with the limitation that R⁴ does not have the meaning of NR⁷R⁸, and with the exception of morpholino when R⁴ = aryl,
- azido, hydroxylamino, O-(C₁₋₃)alkylhydroxylamino,
- N-(C₁₋₃)alkylhydroxylamino, N,N-di(C₁₋₃)alkylhydroxylamino,
- hydrazino, (C₁₋₄)alkyl- and di(C₁₋₄)alkylhydrazino,
- benzylhydrazino, acylhydrazino, N,N-diacylhydrazino,
- carbamoylamino,
- 1-imidazolyl, 1,2,4-triazol-lyl, 1-pyridinium,
- 1-pyrazinium, 1-pyridazinium, including the alkylsubstituted representatives of these azaheterocycles;
R^{§} is
- OH, -SH, -O-C₁₋₈ alkyl, -O-C₆₋₁₄ aryl, -S-C₁₋₄ alkyl, -S-C₆₋₁₄ aryl,
- SO-C₁₋₄ alkyl, -SO-C₆₋₁₄ aryl, -SO₂-C₁₋₄ alkyl, -SO₂-C₆₋₁₄ aryl,
- SO₃H,
- OSO₂C₁₋₈ alkyl, -OSO₂C₆₋₁₄ aryl, -COOH, -COOC₁₋₈ alkyl,
- (CO)C₁₋₈ alkyl,
- COOH, -CONH₂, -CONHC₁₋₆ alkyl, -CON(C₁₋₆ alkyl)₂,
- NH₂, -NHC₁₋₆ alkyl, -N(C₁₋₆ alkyl)₂, -NHC₆₋₁₄ aryl, -NH-hetaryl,
- N(C₆₋₁₄ aryl)₂, -N(C₁₋₆ alkyl)(C₆₋₁₄ aryl),
- C₁₋₆ alkyl, -C₂₋₁₂ alkenyl, -C₂₋₁₂ alkinyl, each straight-chain, branched or cyclic and independently substituted, where appropriate, singly, doubly or triply with halogen,
- halogen (F, -Cl, -Br, -I),
- CH₂CH₂OH, -CH₂CH₂SH, -CH₂CH₂SCH₃,
- sulfamoyl, alkylsulfamoyl, dialkylsulfamoyl with alkyl C₁₋₅ substituted, where appropriate, with methoxy,
- amidino, hydroxyamidino
- sulfo, phosphono,
- -CN, -NO₂, and -SCN
The following compounds of formula 1(a) are not protected:
I) Y = S, R¹ = OC₂H₅ , R² = CN, R³ = C₆H₅ , R⁴ = N(CH₃)₂, R⁵ = OC₂H₅
II) Y = S, R¹ = CH₃ , R² = H, R³ = CH₃ , R⁴ = Phenyl, R⁵ = Hydrazino
III) Y = S, R¹ = CH₃ , R² = H, R³ = CH₃ , R⁴ = Phenyl, R⁵ = NH-(CH₂)₂-OH
IV) Y = S, R¹ = Phenyl, R² = H, R³ = Phenyl, R⁴ = Thiopheno, R⁵ = NH-(CH₂)₂-OH
V) Y = S, R¹ = Phenyl, R² = H, R³ = Phenyl, R⁴ = Phenyl triply substituted with methoxy, R⁵ = NH-(CH₂)₂-OH 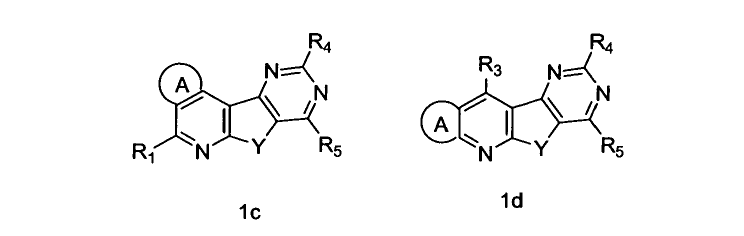
Y is
- S, N-H or O,
A is
- anellated carbocycle having 5 to 8 ring atoms, singly or multiply unsaturated and aromatic (substituted, where appropriate, with R* and/or substituted, where appropriate, with R^{§});
- anellated bicycle having 6 to 11 ring atoms, singly or mulitply unsaturated, which may also contain nitrogen, oxygen and/or sulfur (substituted, where appropriate, with R* and/or substituted, where appropriate, with R^{§});
- annelated heterocycle having 5 to 8 ring atoms, singly or multiply unsaturated and aromatic, which may independently contain nitrogen, oxygen and sulfur singly, multiply, equally or unequally in the cycle (substituted, where appropriate, with R* and/or substituted, where appropriate, with R^{§}),
R¹ and R³ are
- hydrogen (except when A is an anellated unsubstituted cyclopentene ring combined with R⁴, R⁵ equals -OH)
- C₁₋₁₀ alkyl (substituted, where appropriate, with R^{§}),
- C_{2-!2} alkenyl and C_{2-!2} alkinyl (each substituted, where appropriate, with R^{§}),
- difluoromethyl, trifluoromethyl,
- benzyl, phenyl-(C₂₋₆)alkyl (each substituted, where appropriate, with R^{§}),
phenyl (substituted, where appropriate, with R^{§}), in particular: 4-R^{§}-phenyl, 3-R^{§}-phenyl, 2-R^{§}-phenyl, 3-R^{§},4-R^{§}-phenyl, 3-R^{§},4-R^{§}-,5-R^{§}-phenyl, 2-R^{§}, 4-R^{§}-phenyl,
- 1-naphthyl, 2-naphthyl (each substituted, where appropriate, with R^{§}),
- C₃₋₁₄ cycloalkyl, C₃₋₁₄ cycloalkenyl (each substituted, where appropriate, with R^{§}),
- monocyclic or bicyclic, saturated or singly or multiply unsaturated heterocycles having 4 - 14 ring atoms including 1
- 5 heteroatoms which are preferably N, O and S which may carry one or more oxygen atoms on C, N and/or S and, where appropriate, can be substituted singly, doubly or triply with independently selected residues R^{§},
- C₂₋₁₂ alkylacyl (substituted, where appropriate, with R^{§}),
- benzoyl, 1- and 2-naphthoyl (each substituted, where appropriate, with R^{§}),
- heterocyclylacyl [e.g. nicotinoyl, isonicotinoyl, 2-picolinoyl, 2-thienoyl, 2-furoyl] (substituted, where appropriate, with R^{§})
- hydroxy,
- sulfhydryl,
- C₁₋₁₀ alkoxy,
- alkylthio, alkylsulfinyl and alkylsulfonyl (each C₁₋₆)
- formyl, carboxyl, C₁₋₄ alkoxycarbonyl;
- CONH₂, CONHAlk and CONAlk₂ (with "Alk" each C₁₋₆),
- cyano, rhodano, nitro, SO₃H, SO₂OAlk (with "Alk": C₁₋₅),
- chlorine, bromine; iodine, fluorine,
- amino, C₁₋₆ alkylamino, di(C₁₋₅)alkylamino (each substituted, where appropriate, with R^{§} on the alkyl residue),
- morpholino, thiomorpholino, thiomorpholino-S-oxide, thiomorpholino-S,S-dioxide, pyrrolidino,
- piperidino, 1-piperazino, 4-methyl-1-piperazino, 4-hydroxyethyl-1-piperazino, 4-phenyl-1-piperazino,
- cycloalkylamino, C₃₋₁₄ arylamino and heteroarylamino [e.g. phenyl-, 1- and 2-naphthyl-, 2-, 3- or 4-pyridyl-, quinolinyl-, isoquinolinyl-, acridinyl-, phenothiazinyl-, 2-thienyl- and 2-furylamino] (each substituted, where appropriate, on the carbocyclic and/or heterocyclic rings with R^{§});
R⁴ is
- C₂₋₁₄ alkyl, C₃₋₁₄ cycloalkyl, C₂₋₁₄ alkenyl, C₃₋₁₄ cycloalkenyl, C₂₋₁₄ alkinyl (each substituted, where appropriate, with R^{§} on the C-skeleton of the above mentioned residues);
- benzyl, phenyl-(C₂₋₆)alkyl (each substituted, where appropriate, with R^{§}),
phenyl (substituted, where appropriate, with R^{§}), in particular:
4-R^{§}-phenyl, 3-R^{§}-phenyl, 2-R^{§}-phenyl, 3-R^{§},4-R^{§}-phenyl, 3-R^{§},4-R^{§}-,5-R^{§}-phenyl, 2-R^{§}, 4-R^{§}-phenyl,
- 1-naphthyl, 2-naphthyl (each substituted, where appropriate, with R^{§});
- S-alkyl (C₃-C₈), SO-alkyl, SO₂-alkyl, (each C₁-C₈),
S-alkenyl, SO-alkenyl, SO₂-alkenyl (each C₂-C₈)
S-alkinyl, SO-alkinyl, SO₂-alkinyl (each C₂-C₆)
(each substituted, where appropriate, with -OH, -CN, -SCN, -NO₂, phenyl or C₃ - C₇ cycloalkyl on the C-skeleton of the above-mentioned residues);
- monocyclic, bicyclic or tricyclic, saturated or singly or multiply unsaturated heterocyclic residue having a total of 4 - 14 ring atoms including 1 - 5 heteroatoms which are preferably N, O, S and Se (each substituted, where appropriate, with R^{§});
- OR⁶, wherein R⁶ is
- hydrogen
- CH₃, C₂H₅, (CH₂)₂CH₃, (CH₂)₃CH₃, CH(CH₃)₂, CH₂CH(CH₃)₂, C(CH₃)₃, CH₂CH₂OH, CH₂CH₂SH, CH₂CH₂SCH₃, CH₂CF₃, CH₂CCl₃, CH₂CHF₂, CH₂CHCl₂, CH₂CH₂F, CH₂CH₂Cl, CH₂CH₂Br,
- C₃₋₇ cycloalkyl [e.g. cyclopropyl, cyclopropylmethyl, cyclobutyl, cyclobutylmethyl, cyclopentyl, cyclopentylmethyl, cyclohexyl, cyclohexylmethyl] (substituted, where appropriate, with R^{§} on the C-skeleton),
- C₂₋₅ alkenyl and C₂₋₅ alkinyl,
- C₃₋₇ cycloalkenyl,
- aryl and heteroaryl as residues of monocyclic, bicyclic or tricyclic aromatic compounds or heteroaromatic compounds with optionally 6 - 14 ring atoms [e.g. phenyl, 4-R^{§}-phenyl, 3-R^{§}-phenyl, 2-R^{§}-phenyl, 3-R^{§},4-R^{§}-phenyl, 3-R^{§},4-R^{§}-,5-R^{§}-phenyl],
- 1-naphthyl, 2-naphthyl (each substituted, where appropriate, with R^{§}),
- pyridyl, isoquinolinyl, quinolinyl, acridinyl;
- NR⁷R⁸, wherein this substitutent is altogether:
- morpholino, thiomorpholino, thiomorpholino-S-oxide, thiomorpholino-S,S-dioxide, pyrrolidino, piperidino, 1-piperazino, 1-homopiperazino, 4-C₁₋₆ alkyl-1-piperazino, 4-(2-hydroxyethyl)-1-piperazino, 4-benzyl-1-piperazino, 4-aryl-1-piperazino (each substituted, where appropriate, with R^{§} on the heterocycloaliphatic ring),
- further amino residues of secondary, monocyclic or polycyclic cycloaliphatic amines having a total of 5 - 14 ring atoms, including the representatives substituted with R^{§} on the C-skeleton;
- amino residues of secondary aliphatic and aromatic amines, R⁷R⁸H,
wherein R⁷ and R⁸ may be independently equal or unequal and represent:
- C₁₋₆ alkyl, benzyl, phenyl, 1- and 2-naphthyl, 2-, 3-and 4-pyridyl, quinolinyl, isoquinolinyl, 2-thienyl, 2-furyl (each substituted, where appropriate, with R^{§});
- amino residues of primary amines, R⁷NH₂, (wherein R⁷ is as defined above),
- amino, NH₂, with the limitation that in this case R⁵ only has the meaning of -OR⁶;
R⁵ is
- hydrogen,
- heterocyclyl, five-membered to seven-membered, substituted, where appropriate, by R³,
- phenyl, substituted, where appropriate, by R³,
- OR⁶, wherein R⁶ is as defined above,
- NR⁷ R⁸, with the same meaning as above
- azido, hydroxylamino, O-(C₁₋₃ )alkylhydroxylamino,
- N-(C₁₋₃)alkylhydroxylamino, N,N-di(C₁₋₃)alkylhydroxylamino,
- hydrazino, (C₁₋₄)alkyl- and di(C₁₋₄)alkylhydrazino,
- benzylhydrazino, acylhydrazino, N,N-diacylhydrazino,
- carbamoylamino,
- 1-imidazolyl, 1,2,4-triazol-lyl, 1-pyridinium,
- 1-pyrazinium, 1-pyridazinium, including the alkyl-substituted representatives of these azaheterocycles;
R* =O, =S, =N-H, =N-C₁₋₈ alkyl, =N-Aryl, =N-OH, =N-O-C₁₋₈ alkyl,
=N-O-C₆-₁₄ aryl
R^{§} as defined above (in formula 1a or 1b)
wherein the following compounds of formula 1 (c) are not protected:
1,4-dihydro-2,2-dimethyl-5-(4-morpholinyl)-10-propylthio-2H-pyrano[4",3":4',5']pyrido[3',2':4',5']thieno[3,2-d]pyrimidin-8(9H)-one,
10-buthylthio-1,4-dihydro-2,2-dimethyl-5-(4-morpholinyl)-2H-pyrano[4",3":4',5']pyrido[3',2':4',5']thieno[3,2-d]pyrimidin-8(9H)-one
and 2-methyl-5-thiophen-2-yl-1,2,3,4-tetrahydro-11H-7-thia-2,6,9,11-tetraaza-benzo[c]fluorene-8,10-dione
and pharmaceutically compatible salts, solvates, and tautomers of these compounds.

2. The compound according to claim 1, comprising general formula 1(a)(I) or 1(a)(II): 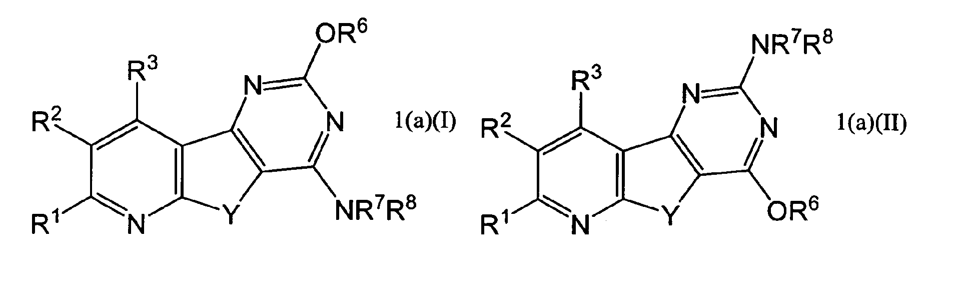 wherein R¹, R², R³, R⁶, R⁷, R⁸ and Y have the meanings mentioned above with respect to formula 1(a).

3. The compound according to claim 1, comprising formula 1(a), wherein Y = S and substituents R¹, R², R³, R⁴, R⁵ are as defined below:
| R₁ | R₂ | R₃ | R₄ | R₅ |
|---|---|---|---|---|
| 3,4-(MeO)₂-Ph | H | Me | Piperazin-1-yl | OEt |
| 4-MeO-Ph | H | Me | Piperazin-1-yl | OEt |
| 4-F-Ph | H | Me | Piperazin-1-yl | OEt |
| Ph | H | Me | Piperazin-1-yl | O*i*Pr |
| Ph | H | Me | Piperazin-1-yl | OCH₂CHF₂ |
| Ph | H | Ph | Piperazin-1-yl | OEt |
| Me | 4'-NO₂-Ph-CH₂ | Me | Piperazin-1-yl | OEt |
| Me | 4'-NH₂-Ph-CH₂ | Me | Piperazin-1-yl | OEt |
| 3,4-Methylene-dioxyphenyl | H | Me | Piperazin-1-yl | OEt |
| Ph | H | Me | Piperazin-1-yl | O*n*Pr |
| Ph | H | Me | Piperazin-1-yl | OEt |
| Ph | H | Me | Piperazin-1-yl | OCH₂CH₂O H |
| 3,4-(MeO)₂-Ph | H | Me | OEt | Piperazin-1-yl |
| 4-MeO-Ph | H | Me | OEt | Piperazin-1-yl |
| Ph | H | Me | OCH₂CHF₂ | Piperazin-1-yl |
| Me | 4'-NO₂-Ph-CH₂ | Me | OEt | Piperazin-1-yl |
| Ph | 4'-NO₂-Ph-CH₂ | Me | OEt | Piperazin-1-yl |
| 3,4-(MeO)₂-Ph | H | Me | *n*Pr | Piperazin-1-yl |
| Ph | H | Me | OCH₂CH₂O H | Piperazin-1-yl |
| Ph | H | Me | OEt | Piperazin-1-yl |
| Ph | H | Me | O*i*Pr | Piperazin-1-yl |
| 3,4-Methylene-dioxyphenyl | H | Me | OEt | Piperazin-1-yl |
| 4-(Pyrimidyl-2-yl)piperazin-1-yl)- | H | Et | Piperazin-1-yl | OEt |
| 4-Me-Ph | H | Et | Piperazin-1-yl | OEt |
| 4-EtO-Ph | H | Et | Piperazin-1-yl | OEt |
| 4-EtO-Ph | H | Et | Piperazin-1-yl | OProp |
| 4-MeS-Ph | H | Et | Piperazin-1-yl | OEt |
| 4-Me-Ph | H | Et | Piperazin-1-yl | O*i*Pr |
| 4-(4-MeOPhO)Ph | H | Et | Piperazin-1-yl | OEt |
| 2,3-Dihydro-benzo[b][1,4]-dioxin-6-yl | H | Et | Piperazin-1-yl | OEt |
| 3,4-Methylene-dioxyphenyl | H | Et | Piperazin-1-yl | OEt |
| 4-MeO-Ph | H | Et | Piperazin-1-yl | OEt |
| 4-(1H-pyrazol-1-yl)-Ph | H | Et | Piperazin-1-yl | OEt |
| 4-Br-Ph | H | Et | Piperylzin-1-yl | OEt |
| 4-Cl-Ph | H | Et | Piperazin-1-yl | OEt |
| 4-I-Ph | H | Et | Piperazin-1-yl | OEt |
| Morpholine | H | Ph | Piperazin-1-yl | OEt |
| 4-MeO-Ph | H | CF₃ | Piperazin-1-yl | OEt |
| 4-Me-Ph | H | CF₃ | Piperazin-1-yl | OEt |
| 2,3-Dihydro-benzo[b][1,4]-dioxin-6-yl | H | CF₃ | Piperazin-1-yl | OEt |
| 4-lmidazol-1-yl-Ph | H | CF₃ | Piperazin-1-yl | OEt |
| 4-Imidazol-1-yl-Ph | H | CF₃ | Piperazin-1-yl | OCH₂CH₂O |
| 4-(Piperidin-1-yl)-Ph | H | CF₃ | Piperazin-1-yl | OEt |
| 4-Morpholino-Ph | H | CF₃ | Piperazin-1-yl | OEt |
| 3,4-Methylene-dioxyphenyl | H | CF₃ | Piperazin-1-yl | OEt |
| 4-MeS-Ph | H | CF₃ | Piperazin-1-yl | OEt |
| 4-EtOOC-Ph | H | CF₃ | Piperazin-1-yl | OEt |
| 4-(1H-pyrazol-1-yl)-Ph | H | CF₃ | Piperazin-1-yl | OEt |
| 4-F-Ph | H | CF₃ | Piperazin-1-yl | OEt |
| 4-I-Ph | H | CF₃ | Piperazin-1-yl | OEt |
| 3-NO₂-Ph | H | CF₃ | Piperazin-1-yl | OEt |
| 4-NO₂-Ph | H | CF₃ | Piperazin-1-yl | OEt |
| 3-CN-Ph | H | CF₃ | Piperazin-1-yl | OEt |
| 4-CN-Ph | H | CF₃ | Piperazin-1-yl | OEt |
| 3-NH₂-Ph | H | CF₃ | Piperazin-1-yl | OEt |
| 4-NH₂-Ph | H | CF₃ | Piperazin-1-yl | OEt |
| 4-MeS-Ph | H | CF₃ | Piperazin-1-yl | OEt |
| 4-MeS(O)-Ph | H | CF₃ | Piperazin-1-yl | OEt |
| Pyridin-4-yl | H | CF₃ | Piperazin-1-yl | OEt |
| Pyridin-4-yl-N-oxide | H | CF₃ | Piperazin-1-yl | OEt |
| Pyridin-3-yl | H | CF₃ | Piperazin-1-yl | OEt |
| 4-(Pyrazin-2-yl) | H | CF₃ | Piperazin-1-yl | OEt |
| 4(HCONH)-Ph | H | CF₃ | Piperazin-1-yl | OEt |
| 4-(Pyridin-4-yl-CONH)-Ph | H | CF₃ | Piperazin-1-yl | OEt |
| 4-(Pyridin-3-yl-CONH)-Ph | H | CF₃ | Piperazin-1-yl | OEt |
| 4-(Pyridin-3-yl-N-Oxide-CONH)-Ph | H | CF₃ | Piperazin-1-yl | OEt |
| 4-(1H-1,2,4-triazol-4-yl)-Ph | H | CF₃ | Piperazin-1-yl | OEt |
| 4-(1H-pyrrol-1-yl)-Ph | H | CF₃ | Piperazin-1-yl | OEt |
| 4(CH₃OOCNH )-Ph | H | CF₃ | Piperazin-1-yl | OEt |
| *iso*Pr | H | *iso*Pr | Piperazin-1-yl | OEt |
| 2,3-Dihydro-benzo[b][1,4]-dioxin-6-yl | H | Et | OEt | Piperazin-1-yl |
| 4-EtO-Ph | H | Et | OEt | Piperazin-1-yl |
| 4-Me-Ph | H | Et | OEt | Piperazin-1-yl |
| 4-Me-Ph | H | Et | OCH₂CH₂O H | Piperazin-1-yl |

4. The compound according to claim 1, comprising formula 1(a), wherein Y = O and substituents R¹, R², R³, R⁴, R⁵ are as defined below:
| R₁ | R₂ | R₃ | R₄ | R₅ |
|---|---|---|---|---|
| Ph | H | Me | Piperazin-1-yl | OEt |
| Ph | H | Me | OEt | Piperazin-1-yl |

5. The compound according to claim 1, comprising formula 1(b), wherein Y = S and substituents R¹, R³, R⁴, R⁵ are as defined below:
| R₁ | R₃ | R₄ | R₅ |
|---|---|---|---|
| 3-NH₂-Ph-4-Ph | Me | Piperazin-1-yl | OEt |
| 4-(3-furyl)-Ph | Me | Piperazin-1-yl | OEt |
| 4-(2-furyl)-Ph | Me | Piperazin-1-yl | OEt |
| 4-(3-pyridyl)-Ph | Me | Piperazin-1-yl | OEt |
| 4-(4-pyridyl)-Ph | Me | Piperazin-1-yl | OEt |
| 4-NH₂-Ph-Ph | Me | Piperazin-1-yl | OEt |
| 3,4,5-(CH₃O)₃-Ph-Ph | Me | Piperazin-1-yl | OEt |
| 4-PhO-Ph | Me | Piperazin-1-yl | OEt |
| 2-NH₂-4-CH₃OO-Ph-Ph | Me | Piperazin-1-yl | OEt |
| 3-CH₃OOC-Ph-Ph | Me | Piperazin-1-yl | OEt |
| 3,4-(CH₃O)₂-Ph-Ph | Me | Piperazin-1-yl | OEt |
| 3,5-(CH₃)₂-isoxazol-4-yl-Ph | Me | Piperazin-1-yl | OEt |
| Phenyl | Me | Piperazin-1-yl | OEt |
| 4-Br-Ph | Me | Piperazin-1-yl | OEt |
| 3-NH₂-Ph | Me | Piperazin-1-yl | OEt |
| 4-(pyrrol-3-yl)-Ph | Me | Piperazin-1-yl | OEt |
| 4-(pyrimidin-5-yl)-Ph | Me | Piperazin-1-yl | OEt |
| 4-(pyrid-3-yl-hydroxymethyl)-Ph | Me | Piperazin-1-yl | OEt |
| Ph-Ph | Me | Piperazin-1-yl | OEt |
| 4-(*iso*PropO)-Ph | Me | Piperazin-1-yl | OEt |
| 4-Vinyl-Ph | Me | Piperazin-1-yl | OEt |

6. The compound according to claim 1, comprising formula 1(a) or 1(b), which are:
2-(2-Hydroxyethoxy)-9-methyl-7-phenyl-4-(piperazin-1-yl)-pyrido[3',2':4,5]thieno[3,2-d]pyrimidine
2-Ethoxy-7-(3,4-dimethoxy-phenyl)-9-methyl-4-(piperazin-1-yl)-pyrido[3',2':4,5]thieno[3,2-d]pyrimidine
2-Ethoxy-7-(4-methoxy-phenyl)-9-methyl-4-(piperazin-1-yl)-pyrido[3',2':4,5]thieno[3,2-d]pyrimidine
2-Ethoxy-9-ethyl-7-(4-methyl-phenyl)-4-(piperazin-1-yl)-pyrido[3',2':4,5]thieno[3,2-d]pyrimidine
2-Ethoxy-7-(4-ethoxy-phenyl)-9-ethyl-4-(piperazin-1-yl)-pyrido[3',2':4,5]thieno[3,2-d]pyrimidine
2-Ethoxy-9-ethyl-7-(2,3-dihydrobenzo[b][1,4]dioxin-6-yl)-4-(piperazin-1-yl)-pyrido[3',2':4,5] thieno[3,2-d]pyrimidine
4-Ethoxy-9-trifluoromethyl-7-(pyrazin-2-yl)-2-piperazino-pyrido[3',2':4,5]thieno[3,2-d]pyrimidine
4-Ethoxy-9-ethyl-7-(4-(pyrimidyl-2-yl)piperazin-1-yl)-2-piperazin-1-yl-pyrido[3',2' :4,5]thieno[3,2-d]pyrimidine
4-Ethoxy-7-morpholino-9-phenyl-2-(piperazin-1-yl)-pyrido[3',2':4,5]thieno[3,2-d]pyrimidine
4-Ethoxy-7-phenyl-9-methyl-2-(piperazin-1-yl)-pyrido[3',2':4,5]furo[3,2-d]pyrimidine
4-Ethoxy-7,9-dimethyl-8-(4-nitro-benzyl)-2-(piperazin-1-yl)-pyrido[3',2':4,5]thieno[3,2-d]pyrimidine
4-Ethoxy-7-(3,4-dimethoxy-phenyl)-9-methyl-2-(piperazin-1-yl)-pyrido[3',2':4,5]thieno[3,2-d]pyrimidine
4-Ethoxy-9-ethyl-7-(4-methyl-phenyl)-2-(piperazin-1-yl)-pyrido[3',2':4,5]thieno[3,2-d]pyrimidine
4-Ethoxy-7-(4-fluorophenyl)-9-methyl-2-(piperazin-1-yl)-pyrido[3',2':4,5]furo[3,2-d]pyrimidine
7-(4-Ethoxy-phenyl)-9-ethyl-2-(piperazin-1-yl)-4-n-propoxy-pyrido[3',2':4,5]thieno[3,2-d]pyrimidine
4-Ethoxy-7-(4-ethoxy-phenyl)-9-ethyl-2-(piperazin-1-yl)-pyrido[3',2':4,5]thieno[3,2-d]pyrimidine
4-Ethoxy-7-(4-(pyrimidin-2-yl)piperazin-1-yl)-9-ethyl-2-(piperazin-1-yl)-pyrido[3',2':4,5]thieno[3,2-d]pyrimidine
4-Ethoxy-9-ethyl-7-(4-methylthio-phenyl)-2-(piperazin-1-yl)-pyrido[3',2':4,5]thieno[3,2-d]pyrimidine
4-Ethoxy-7-(4-methyl-phenyl)-9-*iso*-propyl-2-(piperazin-1-yl)-pyrido[3',2':4,5]thieno-[3,2-d]pyrimidine
4-Ethoxy-9-ethyl-7-[4-(4-methoxyphenoxy)phenyl]-2-(piperazin-1-yl)-pyrido[3',2':4,5] thieno[3,2-d]pyrimidine
4-Ethoxy-9-ethyl-7-(2,3-dihydrobenzo[b][1,4]dioxin-6-yl)-2-(piperazin-1-yl)-pyrido[3',2':4,5] thieno[3,2-d]pyrimidine
4-Ethoxy-9-ethyl-7-(3,4-methylenedioxyphenyl)-2-piperazin-1-yl-pyrido[3',2':4,5]thieno-[3,2-d]pyrimidine
4-Ethoxy-7-(4-methoxy-phenyl)-2-(piperazin-1-yl)-9-trifluoromethyl-pyrido[3',2':4,5]thieno[3,2-d]pyrimidine
4-Ethoxy-7-(4-methyl-phenyl)-2-(piperazin-1-yl)-9-trifluoromethyl-pyrido[3',2':4,5]thieno[3,2-d]pyrimidine
7-(2,3-Dihydrobenzo[b][1,4]dioxin-6-yl)-4-ethoxy-2-(piperazin-1-yl)-9-trifluoromethyl-pyrido[3',2':4,5] thieno[3,2-d]pyrimidine
4-Ethoxy-7-(4-(1*H*-imidazol-1-yl)phenyl)-2-(piperazin-1-yl)-9-trifluoromethyl-pyrido[3',2':4,5]thieno[3,2-d]pyrimidine
4-[4-Ethoxy-2-(piperazin-1-yl)-9-trifluoromethyl-pyrido[3',2':4,5]thieno[3,2-d]pyrimidin-7-yl-benzoic acid ethyl ester
4-Ethoxy-2-(piperazin-1-yl)-7-(4-(piperidin-1-yl)phenyl)-9-trifluoromethyl-pyrido[3',2':4,5]thieno[3,2-d]pyrimidine
4-Ethoxy-9-ethyl-7-(4-methoxyphenyl)-2-(piperazin-1-yl)-pyrido[3',2':4,5]thieno[3,2-d]pyrimidine
4-Ethoxy-7-(3,4-methylenedioxyphenyl)-2-piperazin-1-yl-9-trifluoromethyl-pyrido[3',2':4,5]thieno-[3,2-d]pyrimidine
4-Ethoxy-7-(4-methylthiophenyl)-2-(piperazin-1-yl)-9-trifluoromethyl-pyrido[3',2':4,5]thieno[3,2-d]pyrimidine
4-Ethoxy-7-(4-(1H-pyrazol-1-yl)-phenyl)-9-ethyl-2-piperazin-1-yl-pyrido[3',2' :4,5]thieno[3,2-d]pyrimidine
4-Ethoxy-7-(4-iodo-phenyl)-2-(piperazin-1-yl)-9-trifluoromethyl-pyrido[3',2':4,5]thieno[3,2-d]pyrimidine
4-Ethoxy-7-(4-(1H-pyrazol-1-yl)-phenyl)-2-piperazin-1-yl-9-trifluoromethyl-pyrido[3',2':4,5]thieno[3,2-d]pyrimidine
7-(4-Bromo-phenyl)-4-ethoxy-9-ethyl-2-(piperazin-1-yl)-pyrido[3',2':4,5]thieno[3,2-d]pyrimidine
4-Ethoxy-9-ethyl-7-(4-iodo-phenyl)-2-(piperazin-1-yl)-pyrido[3',2':4,5]thieno[3,2-d]pyrimidine
4-Ethoxy-7-(3-nitro-phenyl)-2-piperazin-1-yl-9-trifluoromethyl-pyrido[3',2' :4,5]thieno[3,2-d]pyrimidine
7-(3-Cyano-phenyl)-4-ethoxy-2-piperazin-1-yl-9-trifluoromethyl-pyrido[3',2':4,5]thieno[3,2-d]pyrimidine
7-(4-Cyano-phenyl)-4-ethoxy-2-piperazin-1-yl-9-trifluoromethyl-pyrido[3',2':4,5]thieno[3,2-d]pyrimidine
7-(3-Amino-phenyl)-4-ethoxy-2-piperazin-1-yl-9-trifluoromethyl-pyrido[3',2':4,5]thieno[3,2-d]pyrimidine
7-(4-Amino-phenyl)-4-ethoxy-2-piperazin-1-yl-9-trifluoromethyl-pyrido[3',2':4,5]thieno[3,2-d]pyrimidine
4-Ethoxy-9-methyl-7-(4-methylsulfinyl-phenyl)-2-(piperazin-1-yl)-pyrido[3',2':4,5]thieno[3,2-d]pyrimidine
N-(4-(4-Ethoxy-9-trifluoromethyl-2-piperazin-1-yl-pyrido[3',2':4,5]thieno[3,2-d]pyrimidin-7-yl)-phenyl)-formamide
N-(4-(4-Ethoxy-9-trifluoromethyl-2-piperazin-1-yl-pyrido[3',2':4,5]thieno[3,2-d]pyrimidin-7-yl)-phenyl)-isonicotinamide
4-Ethoxy-9-trifluoromethyl-2-piperazin-l-yl-7-(4-(1H-pyrrol-1-yl)-phenyl)-pyrido[3',2':4,5]thieno[3,2-d]pyrimidine
N-(4-(4-Ethoxy-9-trifluoromethyl-2-piperazin-1-yl-pyrido[3',2':4,5]thieno[3,2-d]pyrimidin-7-yl)-phenyl)-nicotinamide-N-oxide
4-(4-Ethoxy-9-trifluoromethyl-2-piperazin-1-yl-pyrido[3',2':4,5]thieno[3,2-d]pyrimidin-7-yl)-phenyl-carbamic acid ethyl ester
N-(4-(4-Ethoxy-9-trifluoromethyl-2-piperazin-1-yl-pyrido[3',2':4,5]thieno[3,2-d]pyrimidin-7-yl)-phenyl)-nicotinamide
N-(4-Ethoxy-9-trifluoromethyl-2-piperazin-1-yl-pyrido[3',2':4,5]thieno[3,2-d]pyrimidin-7-yl)-benzamide trifluoroacetate
N'-Hydroxy-4-(4-ethoxy-9-trifluoromethyl-2-piperazin-1-yl-pyrido[3',2':4,5]thieno[3,2-d]pyrimidin-7-yl)-benzamidine bistrifluoroacetate
7-(3'-Aminobiphen-4-yl)-4-ethoxy-9-methyl-2-piperazin-1-yl-thieno[2,3-d:4,5-d']dipyrimidine
4-Ethoxy-7-(4-[3-furyl]-phenyl)-9-methyl-2-piperazin-1-yl-thieno[2,3-d:4,5-d']dipyrimidine
4-Ethoxy-7-(4-[2-furyl]-phenyl)-9-methyl-2-piperazin-1-yl-thieno[2,3-d:4,5-d']dipyrimidine
4-Ethoxy-9-methyl-2-piperazin-1-yl-7-(4-[3-pyridyl]-phenyl)-thieno[2,3-d:4,5-d']dipyrimidine
4-Ethoxy-9-methyl-2-piperazin-1-yl-7-(4-[4-pyridyl]-phenyl)-thieno[2,3-d:4,5-d']dipyrimidine
7-(4'-Aminobiphen-4-yl)-4-ethoxy-9-methyl-2-piperazin-1-yl-thieno[2,3-d:4,5-d']dipyrimidine
4-Ethoxy-9-methyl-2-piperazin-1-yl-7-(3',4',5'-trimethoxybiphen-4-yl)-thieno[2,3-d:4,5-d']dipyrimidine
4-Ethoxy-9-methyl-7-(4-phenoxy-phenyl)-2-piperazin-1-yl-thieno[2,3-d:4,5-d']dipyrimidine
2'-Amino-4-(4-ethoxy-9-methyl-2-piperazin-1-yl -thieno[2,3-d:4,5-d']dipyrimidin-7-yl)-biphenyl-4'-carboxylic acid methyl ester
7-(3',4'-Dimethoxybiphen-4-yl)-4-ethoxy-9-methyl-2-piperazin-1-yl-thieno[2,3-d:4,5-d']dipyrimidine

7. The compound according to claim 1, comprising formula 1(c) (I) or 1(c) (II): 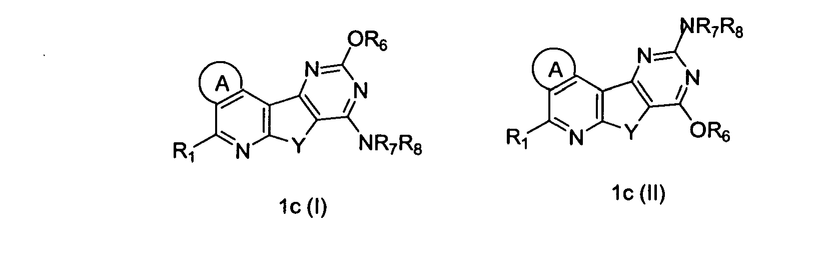 wherein R¹, R⁶, R⁷, R⁸ as well as A and Y have the meanings mentioned above with respect to formula 1(c).

8. The compound according to claim 1, comprising formula 1(c) or 1(d), which is selected from the group:
10-Ethoxy-5-morpholino-8-piperazin-1-yl-pyrimido[4',5':4,5]thieno[2,3-c]-1,2,3,4-tetrahydro-isoquinoline
7-Ethoxy-2,3-dihydro-4-morpholino-9-piperazin-lyl-1H-cyclopenta[4',5']pyrido[3',2':4,5]thieno[3,2-d]pyrimidine
8-Ethoxy-5-piperidino-10-piperazin-1-yl-pyrimido[4',5':4,5]thieno[2,3-c]-1,2,3,4-tetrahydro-isoquinoline-hydrochloride
8-Ethoxy-5-morpholino-10-piperazin-1-yl-pyrimido[4',5':4,5]thieno[2,3-c]-1,2,3,4-tetrahydro-isoquinoline-hydrochloride
8-Ethoxy-5-phenyl-10-piperazin-1-yl-pyrimido[4',5':4,5]thieno[2,3-c]-1,2,3,4-tetrahydro-isoquinoline-hydrochloride
10-Ethoxy-5-phenyl-8-piperazin-1-yl-pyrimido[4',5':4,5]thieno[2,3-c]-1,2,3,4-tetrahydro-isoquinoline
8-Ethoxy-5-morpholino-10-piperazin-1-yl-pyrimido[4',5':4,5]thieno[2,3-c]-1,2,3,4-tetrahydro-isoquinoline
7-Ethoxy-2,3,4,5-tetrahydro-4-morpholino-9-piperazin-1yl-1H-cyclohepta[4',5']pyrido[3',2':4,5]thieno[3,2-d]pyrimidine

9. A pharmaceutical composition containing at least one of the compounds according to any of claims 1 to 8 and, where appropriate,
pharmaceutically compatible excipients and/or carriers.

10. The pharmaceutical composition according to claim 9 additionally containing one or more of the following active substances:
- β₂-adrenoceptor agonists
- disodium cromoglycate
- corticosteroids
- leukotriene antagonists (either enzyme inhibitors [such as 5-lipoxygenase inhibitors or arachidonic acid enzyme inhibitors] or receptor antagonists),
- antihistaminics (preferably those having mast cell-stabilizing properties or leukotriene-antagonizing aspects)
- theophylline
- muscarine receptor antagonists
- (monoclonal) antibodies against TNF-alpha or other active substances inhibiting the formation and/or release of TNF-alpha or the activity of TNF-alpha.

11. Use of at least one of the compounds according to any of claims 1 to 8 for the production of a medicament for inhibiting the phosphodiesterase 4 (PDE4) enzyme and/or TNF alpha release.

12. Use according to claim 11 for treating bronchial asthma, COPD, rheumatoid arthritis (RA), osteoarthritis, cystic fibrosis, Guillain-Barré syndrome, Crohn's disease, ulcerative colitis, psoriasis, atopic dermatitis, allergic eczemas, allergic rhinitis, allergic conjunctivitis, systemic scleroderma, graft versus host disease (GvHD), systemic lupus erythematosus (SLE), diabetes mellitus type I, neurodegenerative diseases, in particular Alzheimer's disease and Parkinson's disease, post-traumatic multiorgan failure, toxic shock syndrome, acute glomerulonephritis, acute and chronic pains, arteriosclerosis, cardiac infarction, apoplexy, tumoral diseases, axonal degeneration, viral diseases.

13. Use according to claim 12, wherein the tumoral disease is leukemia.

14. Use according to claim 13, wherein the tumoral disease is CLL.

## Revendications

1. Liaisons des formules générales 1a, 1b, 1c ou 1d 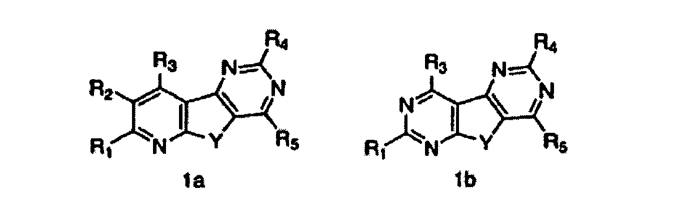 où ce qui suit signifie :
Y S, O ou N-H
R¹
- hydrogène,
- alcoyle C₁₋₁₀, alkényle -C₂₋₁₂, alkynyle -C₂₋₁₂, chacun à chaîne droite, ramifié ou cyclique ainsi qu'éventuellement substitué une fois, deux fois ou trois fois par des restes R^{§} sélectionnés indépendamment l'un de l'autre,
- aralkyl- avec aryle C₆₋₁₂ et alcoyle C₁₋₅, éventuellement substitué une fois, deux fois ou trois fois par des restes R^{§} sélectionnés indépendamment l'un de l'autre,
- phényle, éventuellement substitué une fois, deux fois ou trois fois par des restes R^{§} sélectionnés indépendamment l'un de l'autre,
- 1-naphtyle, 2-naphtyle
- pyridyl-N-oxyde, (éventuellement substitué par R^{§}),
- hétérocycles saturés monocycliques ou bicycliques ou non saturés une ou plusieurs fois avec de 5 à 14 atomes annulaires parmi lesquels de 1 à 4 atomes hétéros qui sont, de préférence, N, O et S, éventuellement substitués une fois, deux fois ou trois fois par des restes R^{§} sélectionnés indépendamment l'un de l'autre,
- alcoxy C₁₋₁₀, à chaîne droite, ramifié ou cyclique ainsi qu'éventuellement substitué une fois, deux fois ou trois fois par des restes R^{§} sélectionnés indépendamment l'un de l'autre,
- aralkyloxy- avec aryle C₆₋₁₂ et alcoyle C₁₋₅, éventuellement substitué une fois, deux fois ou trois fois par des restes R^{§} sélectionnés indépendamment l'un de l'autre,
- alkylacyle C₂₋₁₂ (éventuellement substitué par R^{§}),
- benzoyle, 1- et 2-naphtoyle (chacun éventuellement substitué par R^{§}),
- hydroxy, sulfhydryle, formyle, carboxyle, CONH₂, cyano, rhodano, nitro, SO₃H,
- alkylthio, alkylsulfinyle, SO₂0Alk et alkylsulfonyle, chacun à chaîne droite, ramifié ou cycliquement C₁₋₆ ainsi qu'éventuellement substitué une fois, deux fois ou trois fois par des restes R^{§} sélectionnés indépendamment l'un de l'autre,
- arylthio, arylsulfinyle, arylsulfonyle, hétéro-arylthio, hétéro-arylsulfinyle et hétéro-arylsulfonyle, chacun éventuellement substitué une fois, deux fois ou trois fois par des restes R^{§} sélectionnés indépendamment l'un de l'autre,
- alcoxycarbonyle, CONHAlk et CONAlk₂, chacun à chaîne droite, ramifié ou cycliquement C₁₋₆ ainsi qu'éventuellement substitué une fois, deux fois ou trois fois par des restes R^{§} sélectionnés indépendamment l'un de l'autre,
- aryloxycarbonyle, arylcarbamoyle, arylamido, N-aryle,N-alkylamido, N-aryle,N-alkylcarbamoyle, aralkyloxycarbonyle et aralkylcarbamoyle, avec alcoyle C₁₋₅, aryle C₆₋₁₀ et éventuellement substitué une fois, deux fois ou trois fois par des restes R^{§} sélectionnés indépendamment l'un de l'autre,
- hétérocyclylcarbonyle, hétérocyclylcarbamoyle, hétérocyclylamido, N-hétérocyclyle,N-alkylcarbamoyle, N-hétérocyclyle,N-alkylamido, hétérocyclylalkyloxycarbonyle et hétérocyclylkylcarbamoyle, avec un hétérocycle saturé monocyclique ou bicyclique ou un hétérocycle non saturé une ou plusieurs fois avec de 5 à 14 atomes annulaires parmi lesquels de 1 à 4 atomes hétéro qui sont, de préférence, N, O et S, éventuellement substitué une fois, deux fois ou trois fois par des restes R^{§} sélectionnés indépendamment l'un de l'autre et alcoyle C₁₋₅,
- chlore, brome, iode, fluor,
- amino, alkylamino C₁₋₆, di(C₁₋₅)alkylamino, chacun à chaîne droite, ramifié ou cycliquement C₁₋₆ ainsi qu'éventuellement substitué une fois, deux fois ou trois fois par des restes R^{§} sélectionnés indépendamment l'un de l'autre,
- arylamino C₆₋₁₀, éventuellement substitué une fois, deux fois ou trois fois par des restes R^{§} sélectionnés indépendamment l'un de l'autre,
- hétérocyclylamino, saturé monocyclique ou bicyclique, ou non saturé une ou plusieurs fois, avec de 5 à 14 atomes annulaires parmi lesquels de 1 à 4 atomes hétéros qui sont, de préférence, N, O et S, éventuellement substitué une fois, deux fois ou trois fois par des restes R^{§} sélectionnés indépendamment l'un de l'autre,
- arylhydrazino, éventuellement substitué une fois, deux fois ou trois fois par des restes R^{§} sélectionnés indépendamment l'un de l'autre,
- L_{A}-A-L_{B}-B
où ce qui suit signifie :
L_{A}:
- liaison simple,
- NR^{#}, O, S, SO, S0₂
A:
- phényle, éventuellement substitué une fois, deux fois ou trois fois par des restes R^{§} sélectionnés indépendamment l'un de l'autre,
- naphtyle, naphtyle substitué une ou plusieurs fois par R^{§}
- hétérocycles saturés monocycliques ou bicycliques ou non saturés une ou plusieurs fois avec de 4 à 14 atomes annulaires parmi lesquels de 1 à 5 atomes hétéros qui sont, de préférence, N, O et S, pouvant porter un ou plusieurs atomes d'oxygène à C, N et/ou S et sont éventuellement substitués une fois, deux fois ou trois fois par des restes R^{§} sélectionnés indépendamment l'un de l'autre,
- L_{A}-A : peut par ailleurs aussi signifier une liaison directe tricycle- L_{B}, L_{B} n'étant, dans ce cas, pas une liaison simple,
L_{B}:
- liaison simple
- NR^{#}, O, S, SO, SO₂, -CHR⁵-, -CH₂-O-, -O-CH₂,
- les groupes fonctionnels suivants: 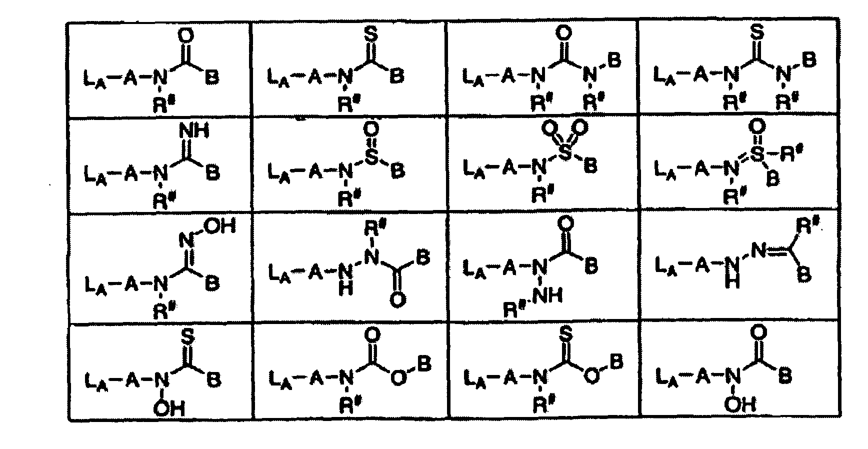
B:
- hydrogène
- alcoyle, éventuellement substitué une fois, deux fois ou trois fois par des restes R^{§} sélectionnés indépendamment l'un de l'autre,
- -CONH₂, -CONHAlk (Alk éventuellement substitué par R^{§}), -CONHAryle (aryle éventuellement substitué par R^{§}), -CONHHétaryle (hétaryle éventuellement substitué par R^{§}), -COOH, -COOAlk (Alk éventuellement substitué par R^{§}),
- -COAlk (Alk éventuellement substitué par R^{§}), -COArylr (aryle éventuellement substitué par R^{§}), -COHétaryle (hétaryle éventuellement substitué par R^{§})
- -CH₂-CONH₂, -CH₂-CONHAlk (Alk éventuellement substitué par R^{§}), -CH₂-CONHAryle (aryle éventuellement substitué par R^{§}), -CH₂-CONHHétaryle (hétaryle éventuellement substitué par R^{§}), -CH₂-COOH, -CH₂-COOAlk (Alk éventuellement substitué par R^{§})
- -CH₂COAlk (Alk éventuellement substitué par R^{§}), -CH₂COAryle (aryle éventuellement substitué par R^{§}), -CH₂-COHétaryl (hétaryle éventuellement substitué par R^{§})
- phényle, éventuellement substitué une fois, deux fois ou trois fois par des restes R^{§} sélectionnés indépendamment l'un de l'autre,
- naphtyle, éventuellement substitué une fois, deux fois ou trois fois par des restes R^{§} sélectionnés indépendamment l'un de l'autre,
- hétérocycles saturés monocycliques ou bicycliques ou non saturés une ou plusieurs fois par de 4 à 14 atomes annulaires parmi lesquels de 1 à 5 atomes hétéros qui sont, de préférence, N, O et S pouvant porter un ou plusieurs atomes d'oxygène à C, N et/ou S et pouvant éventuellement être substitués une fois, deux fois ou trois fois par des restes R^{§} sélectionnés indépendamment l'un de l'autre,
R^{§}
- hydrogène, alcoyle, éventuellement substitué par R^{§}
R²
- hydrogène,
- alcoyle C₁₋₁₂,
- alkényle C₁₋₁₂ et alkynyle C₁₋₁₂,
- benzyle, phényl(C₂₋₆)alkyle (éventuellement substitué par R^{§} une ou plusieurs fois, de manière identique ou différente à la partie de molécule aromatique et/ou aliphatique);
- phénacyle (éventuellement substitué par R^{§} une ou plusieurs fois, de manière identique ou différente à la partie de molécule aromatique);
- carboxyle, alcoxycarbonyle C₁₋₄, -CONH₂, -CONHAlk et CONAlk₂ (avec "Alk" chaque fois C₁₋₆),
- R^{#}C(O)- (où R^{#} a la même signification que ci-dessus),
- cyano, nitro, amino, alkylamino C₁₋₆, di(C₁₋₆)alkylamino, -N=N-C₆H₅, -N=N-C₆H₄-R^{§},
- 1,3-diphényl-pyrazol-4-yl, thiazolin-2-yl, imidazolin-2-yl et 3,4,5,6-tétrahydro-pyrimidinyle;
R³
- hydrogène,
- alcoyle C₁₋₁₀, alkényle -C₂₋₁₂, alkynyle -C₂₋₁₂, chacun à chaîne droite, ramifié ou cyclique ainsi qu'éventuellement substitué une fois, deux fois ou trois fois par des restes R^{§} sélectionnés indépendamment l'un de l'autre,
- trifluorméthyle
- aralkyl- avec aryle C₆₋₁₂ et alcoyle C₁₋₅, éventuellement substitué une fois, deux fois ou trois fois par des restes R^{§} sélectionnés indépendamment l'un de l'autre,
- phényle, éventuellement substitué une fois, deux fois ou trois fois par des restes R^{§} sélectionnés indépendamment l'un de l'autre,
- 1-naphtyle, 2-naphtyle
- pyridyl-N-oxyde, (éventuellement substitué par R^{§}),
- alcoxy C₁₋₁₀, à chaîne droite, ramifié ou cyclique ainsi qu'éventuellement substitué une fois, deux fois ou trois fois par des restes R^{§} sélectionnés indépendamment l'un de l'autre,
- aralkyloxy- avec aryle C₆₋₁₂ et alcoyle C₁₋₅, éventuellement substitué une fois, deux fois ou trois fois par des restes R^{§} sélectionnés indépendamment l'un de l'autre,
- alkylacyle (éventuellement substitué par R^{§}),
- benzoyle, 1- et 2-naphtoyle (chacun éventuellement substitué par R^{§}),
- hydroxy, sulfhydryle, formyle, carboxyle, CONH₂, cyano, rhodano, nitro, SO₃H
- alkylthio, alkylsulfinyle, SO₂OAlk et alkylsulfonyle, à chaîne droite, ramifié ou cyclique C₁₋₆ ainsi qu'éventuellement substitué une fois, deux fois ou trois fois par des restes R^{§} sélectionnés indépendamment l'un de l'autre,
- arylthio. arylsulfinyle, arylsulfonyle, hétéro-arylthio, hétéro-arylsulfinyle et hétéro-arylsulfonyle, chacun éventuellement substitué une fois, deux fois ou trois fois par des restes R^{§} sélectionnés indépendamment l'un de l'autre,
- alcoxycarbonyle, CONHAlk et CONAlk₂, chacun à chaîne droite, ramifié ou cyclique C₁₋₆ ainsi qu'éventuellement substitué une fois, deux fois ou trois fois par des restes R^{§} sélectionnés indépendamment l'un de l'autre,
- aryloxycarbonyle, arylcarbamoyle, arytamido, N-aryle,N-alkylamido, N-aryle,N-alkylcarbamoyle, aralkyloxycarbonyle et aralkylcarbamoyle, avec alcoyle C₁₋₅, aryle C₆₋₁₀ et éventuellement substitués une fois, deux fois ou trois fois par des restes R^{§} sélectionnés indépendamment l'un de l'autre,
- chlore, brome, iode, fluor,
- amino, alkylamino C₁₋₆, di(C₁₋₅)alkylamino, chacun à chaîne droite, ramifié ou cyclique ainsi qu'éventuellement substitués une fois, deux fois ou trois fois par des restes R^{§} sélectionnés indépendamment l'un de l'autre,
- arylamino C₆₋₁₀, éventuellement substitué une fois, deux fois ou trois fois par des restes R^{§} sélectionnés indépendamment l'un de l'autre,
- arylhydrazino, éventuellement substitué une fois, deux fois ou trois fois par des restes R^{§} sélectionnés indépendamment l'un de l'autre,
R⁴
- alcoyle C₂₋₁₄, cycloalcoyle C₃₋₁₄, alkényle C₂₋₁₄, cycloalkényle C₃₋₁₄, alkynyle C₂₋₁₄ (chacun éventuellement substitué au squelette C des restes aliphatiques précités par R^{§});
- phényle, 4-R^{§}-phényle, 3-R^{§}-phényle, 2-R^{§}-phényle (toutefois à l'exclusion de: 2-NO₂-phényle), 3-R^{§},4-R^{§}-phényle, 3-R^{§},4-R^{§}-, 5-R^{§}-phényle,
- 1-naphtyle, 2-naphtyle (chacun éventuellement substitué par R^{§});
- S-alcoyle, SO-alcoyle, SO₂-alcoyle, (chacun C₂-C₈),
S-alkényle, SO-alkényle, SO₂-alkényle (chacun C₂-C₈)
S-alkynyle, SO-alkynyle, SO₂-alkynyle (chacun C₂-C₈)
(éventuellement chacun substitué au squelette C des restes aliphatiques précités par -OH, -CN, -SCN, -NO₂, phényle ou cycloalcoyle C₃-C₇);
- reste saturé monocyclique, bicyclique ou tricyclique ou non saturé une ou plusieurs fois hétérocyclique avec en tout de 4 à 14 atomes annulaires dont de 1 à 5 atomes hétéros qui sont, de préférence, N, O, S et Se (chacun éventuellement substitué par R^{§});
- OR⁶, où R⁶ signifie
- CH₃, C₂H₅, (CH₂)₂CH₃, (CH₂)₃CH₃, C(CH₃)₃, CH₂CH₂OH, CH₂CH₂SH, CH₂CH₂SCH₃, CH₂CF₃, CH₂CCl₃, CH₂CHF₂₎, CH₂CHCl₂, CH₂CH₂F, CH₂CH₂Cl, CH₂CH₂BR,
- cycloalcoyle C₃-₇ [par exemple cylopropyle, cylopropylméthyle, cyclobutyle, cyclobutylméthyle, cyclopentyle, cyclopentylméthyle, cylohexyle, cyclohexylméthyl] (éventuellement substitué au squelette C par R^{§}),
- alkényle C₂-₅ et alkynyle C₂-₅,
- cycloalkényle C₃-₇,
- aryle et hétéro-aryle comme restes aromates ou hétéro-aromates monocycliques, bicycliques ou tricycliques avec éventuellement de 6 à 14 atomes annulaires [par exemple phényle, 4-R^{§}-phényle, 3-R^{§}-phényle, 2-R^{§}-phényle, 3-R^{§},4-R^{§}-phényle, 3-R^{§},4-R^{§}-,5-R^{§}-phényle],
- 1-naphtyle, 2-naphthyle (chacun éventuellement substitué par R^{§}),
- pyridyle, isochinolinyle, chinolinyle, acridinyle;
- NR⁷R⁸, où ce substituant signifie dans l'ensemble:
- morpholino, thiomorpholino, thiomorpholino-S,S-dioxyde, pyrrolidino, piperidino, 1-piperazino, 1-homopiperazino, 4-C₁-₆alkyl-1-piperazino, 4-(2-hydroxyéthyl)-1-piperazino, 4-benzyl-1-piperazino, 4-aryl-1-piperazino (chacun éventuellement substitué par R^{§} à l'anneau hétérocyclo-aliphatique),
- autres restes amino d'amines cycloaliphatiques secondaires, monocycliques ou polycycliques avec en tout de 5 à 14 atomes annulaires, y compris le représentant substitué par R^{§} au squelette C;
- restes amino d'amines secondaires aliphatiques et aromatiques, R⁷R⁸NH,
où R⁷ et R⁸ peuvent être identiques ou différents indépendamment l'un de l'autre et signifient:
- alcoyle C₁-₆, benzyle, phényle, 1- et 2-naphtyle, 2-, 3- et 4-pyridyle, chinolinyle, isochinolinyle, 2-thiényle, 2-furyle (chacun éventuellement substitué par R^{§});
- restes amino d'amines primaires, R⁷NH2, (où R⁷ signifie la même chose que plus haut),
- amino, NH₂, avec la restriction que R⁵ n'a alors que la signification de OR⁶;
R⁵
- OR⁶, où R⁶ signifie la même chose que plus haut,
- NR⁷R⁸, avec la même signification que plus haut, toutefois avec la restriction que R⁴ n'a pas non plus la signification de NR⁷R⁸, ainsi qu'à l'exception de morpholino, lorsque R⁴ = aryle,
- azido, hydroxylamino, O-(C₁-₃)alkylhydroxylamino,
- N-(C₁-₃)alkylhydroxylamino, N,N-di(C₁-₃)alkylhydroxylamino,
- hydrazino, (C₁-₄)alkyl- et di(C₁-₄)alkylhydrazino,
- benzylhydrazino, acylhydrazino, N,N-diacylhydrazino,
- carbamoylamino,
- 1-imidazolyle, 1,2,4-triazol-lyle, 1-pyridinium,
- 1-pyrazinium, 1-pyridazinium, y compris les représentants substitués alcoyle de ces azahétérocycles;
R^{§}
- OH, -SH, -O-C₁-₈alcoyle, -O-C₆-₁₄aryle, -S-C₁-₄alcoyle, -S-C₆-₁₄aryle,
- SO-C₁-₁₄alcoyle, -SO-C₆-₁₄aryle, -SO₂- C₁-₄alcoyle, -SO₂-C₆-₁₄aryle, -SO₃H,
- OSO₂C₁-₈alcoyle, -OSO₂C₆-₁₄aryle 1, -COOH, -COOC₁-₈alcoyle,
- (CO)C₁-₈alcoyle,
- COOH, -CONH₂, -CONHC₁-₈alcoyle, -CON(C₁-₈alcoyle)₂,
- NH₂, -NHC₁-₆alcoyle, -N(C₁-₆alcoyle)₂. -NHC₆-₁₄aryle, -NH-hétaryle,
- N(C₆-₁₄aryle)₂, -N(C₁-₆alcoyle)(C₆·₁₄aryle).
- alcoyle C₁-₆, alkényle -C₂-₁₂, alkynyle -C₂-₁₂, chacun à chaîne droite, ramifié ou cyclique ainsi qu'éventuellement substitué une fois, deux fois ou trois fois indépendamment l'un de l'autre par un halogène,
- halogène (F, -Cl, -Br, -I),
- CH₂CH₂SH, -CH₂CH₂OH, -CH₂CH₂SCH₃,
- sulfamoyle, alkylsulfamoyle, dialkylsulfamoyle avec alcoyle C₁-₅, éventuellement substitué par méthoxy,
- amidino, hydroxyamidino
- sulfo, phosphono,
- -CN, -NO₂, et -SCN
Les liaisons suivantes de la formule 1(a) sont exceptées de la protection:
I) Y = S, R¹ = OC₂H₅, R² = CN, R³ = C₆H₅, R⁴ = N(CH₃)₂, R⁵ = OC₂H₅
II) Y = S, R¹ = CH₃, R² = H, R³ = CH₃, R⁴ = phényle, R⁵ = hydrazino
III) Y = S, R¹ = CH₃, R² = H, R³ = CH₃, R⁴ = phényle, R⁵ = NH-(CH₂)₂-OH
IV) Y = S, R¹ = phényle, R² = H, R³ = phényle, R⁴ = thiopheno, R⁵ = NH-(CH₂)₂-OH
V) Y = S, R¹ = phényle, R² = H, R³ = phényle, R⁴ = phényle substitué trois fois avec methoxy, R⁵ = NH-(CH₂)₂-OH 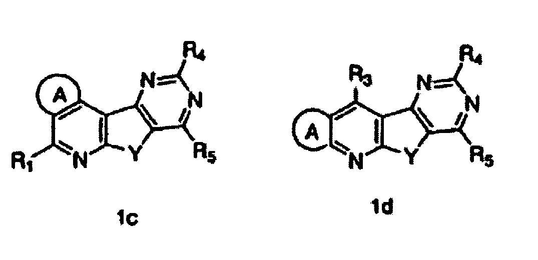 S, N-H ou O
- carbocycle fermé en anneau avec de 5 à 8 atomes annulaires, non saturé une fois ou plusieurs fois ainsi qu'aromatique (éventuellement substitué par R* et/ou éventuellement substitué par R^{§});
- bicycle fermé en anneau avec de 6 à 11 atomes annulaires, non saturé une fois ou plusieurs fois, pouvant également contenir de l'azote, de l'oxygène et/ou du soufre (éventuellement substitué par R* et/ou éventuellement substitué par R^{§});
- hétérocycle fermé en anneau avec de 5 à 8 atomes annulaires, non saturé une fois ou plusieurs fois ainsi qu'aromatique, pouvant également contenir de l'azote, de l'oxygène et du soufre une ou plusieurs fois, de manière identique ou différente indépendamment l'un de l'autre dans le cycle (éventuellement substitué par R* et/ou éventuellement substitué par R^{§}),
R¹ et R³
- hydrogène (sauf lorsque A est un anneau de cyclopentène non substitué fermé en anneau combiné avec R⁴' R⁵ égal à -OH)
- alcoyle C₁-₁₀ (éventuellement substitué par R^{§}),
- alkényle C₂₋₁₂ et alkynyle C₂-₁₂ (chacun éventuellement substitué par R^{§}),
- difluorméthyle, trifluorméthyle,
- benzyle, phényle-(C₂-₆)alcoyle (chacun éventuellement substitué par R^{§}),
phényle (éventuellement substitué par R^{§}), en particulier: 4-R^{§}-phényle, 3-R^{§}-phényle, 2-R^{§}-phényle, 3-R^{§},4-R^{§}-phényle, 3-R^{§},4-R^{§}-,5-R^{§}-phényle, 2-R^{§}, 4-R^{§}-phényle,
- 1-naphtyle, 2-naphtyle (chacun éventuellement substitué par R^{§}),
- cycloalcoyle C₃-₁₄, cycloalkényle C₃-₁₄ (chacun éventuellement substitué par R^{§}),
- hétérocycles saturés monocycliques ou bicycliques ou non saturé une ou plusieurs fois avec de 4 à 14 atomes annulaires parmi lesquels de 1 à 5 atomes hétéros qui sont, de préférence, N, O et S pouvant porter un ou plusieurs atomes d'oxygène à C, N et/ou S et éventuellement substitués une fois, deux fois ou trois fois par des restes R^{§} sélectionnés indépendamment l'un de l'autre,
- alkylacyle C₂-₁₂ (éventuellement substitué par R^{§}),
- benzoyle, 1- et 2-naphtoyle (chacun éventuellement substitué par R^{§}),
- hétérocyclylacyle [par exemple nicotinoyle, isonicotinoyle, 2-picolinoyle, 2-thiénoyle, 2-furoyle] (éventuellement substitué par R^{§})
- hydroxy,
- sulfhydryle,
- alcoxy C₁-₁₀,
- alkylthio, alkylsulfinyle et alkylsulfonyle (chacun C₁-₆)
- formyle, carboxyle, alcoxycarbonyle C₁-₄;
- CONH2, CONHAlk et CONAlk₂ (avec "Alk" chaque fois C₁-₆),
- cyano, rhodano, nitro, SO₃H, SO₂OAlk (avec "Alk": C₁-₅),
- chlore, brome, iode, fluor,
- amino, alkylamino C₁-₆, di(C₁-₅)alkylamino (chacun éventuellement substitué par R^{§} au reste alcoyle),
- morpholino, thiomorpholino, thiomorpholino-S-oxyde, thiomorpholino-S,S-dioxyde, pyrrolidino,
- piperidino, 1-piperazino, 4-méthyl-1-piperazino, 4-hydroxyéthyl-1-piperazino, 4-phényl-1-piperazino,
- cycloalkylamino, arylamino C₃-₁₄ et hétéro-arylamino [par exemple phényl-, 1- et 2-naphthyl-, 2-, 3- ou 4-pyridyl-, chinolinyl-, isochinolinyl-, acridinyl-, phénothiazinyl-, 2-thiényl- et 2-furylamino] (éventuellement chacun substitué aux anneaux carbocycliques ou hétérocycliques par R^{§});
R⁴
- alcoyle C₂-₁₄, cycloalcoyle C₃-₁₄, alkényle C₂-₁₄, cycloalkényle, alkynyle C₂-₁₄ (chacun éventuellement substitué au squelette C des restes précités par R^{§});
- benzyle, phényl-(C₂-₆)alkyl (chacun éventuellement substitué par R^{§}), phényle (éventuellement substitué par R^{§}), en particulier: 4-R^{§}-phényle, 3-R^{§}-phényle, 2-R^{§}-phényle, 3-R^{§},4-R^{§}-phényle, 3-R^{§},4-R^{§}-,5-R^{§}-phényle, 2-R^{§}, 4-R^{§}-phényle,
- 1-naphtyle, 2-naphtyle (chacun éventuellement substitué par R^{§});
- S-alcoyle (C₃-C₈), SO-alcoyle, SO₂-alcoyle (chacun C₁-C₈),
S-alkényle, SO-alkényle, SO₂-alkényle (chacun C₂-C₈)
S-alkynyle, SO-alkynyle, SO₂-alkynyle (chacun C₂-C₆)
(éventuellement chacun substitué au squelette C des restes précités par -OH, -CN, -SCN, -NO₂, phényle ou cycloalcoyle C₃ - C₇);
- reste saturé monocyclique, bicyclique ou tricyclique ou non saturé hétérocyclique une ou plusieurs fois avec en tout de 4 à 14 atomes annulaires parmi lesquels de 1 à 5 atomes hétéros qui sont, de préférence, N, O, S et Se (chacun éventuellement substitué par R^{§});
- OR⁶, où R⁶ signifie
- hydrogène
- CH₃, C₂H₅, (CH₂)₂CH₃, (CH₂)₃CH₃, CH(CH₃)₂, CH₂CH₂(CH₃)₂, C(CH₃)₃, CH₂CH₂OH, CH₂CH₂SH, CH₂CH₂SCH₃, CH₂CF₃, CH₂CCl₃, CH₂CHF₂₎, CH₂CHCl₂, CH₂CH₂F, CH₂CH₂Cl, CH₂CH₂Br,
- cycloalcoyle C₃-₇ [par exemple cylopropyle, cylopropylméthyle, cyclobutyle, cyclobutylméthyle, cyclopentyle, cyclopentylméthyle, cylohexyle, cyclohexylméthyl] (éventuellement substitué au squelette C par R^{§}),
- alkényle C₂-₅ et alkynyle C₂-₅,
- cycloalkényle C₃-₇,
- aryle et hétéro-aryle comme restes aromates ou hétéro-aromates monocycliques, bicycliques ou tricycliques avec éventuellement de 6 à 14 atomes annulaires [par exemple phényle, 4-R^{§}-phényle, 3-R^{§}-phényle, 2-R^{§}-phényle, 3-R^{§},4-R^{§}-phényle, 3-R^{§},4-R^{§}-,5-R^{§}-phényle],
- 1-naphtyle, 2-naphthyle (chacun éventuellement substitué par R^{§}),
- pyridyle, isochinolinyle, chinolinyle, acridinyle;
- NR⁷R⁸, où ce substituant signifie dans l'ensemble:
- morpholino, thiomorpholino, thiomorpholino-S-oxyde, thiomorpholino-S,S-dioxyde, pyrrolidino, piperidino, 1-piperazino, 1-homopiperazino, 4-C₁-₆-alkyl-1-piperazino, 4-(2-hydroxyéthyl)-1-piperazino, 4-benzyl-1-piperazino, 4-aryl-1-piperazino (chacun éventuellement substitué par R^{§} à l'anneau hétérocyclo-aliphatique),
- autres restes amino d'amines cycloaliphatiques secondaires, monocycliques ou polycycliques avec en tout de 5 à 14 atomes annulaires, y compris le représentant substitué par R^{§} au squelette C;
- restes amino d'amines secondaires aliphatiques et aromatiques, R⁷R⁸NH,
où R⁷ et R⁸ peuvent être identiques ou différents indépendamment l'un de l'autre et signifient:
- alcoyle C₁-₆, benzyle, phényle, 1- et 2-naphtyle, 2-, 3- et 4-pyridyle, chinolinyle, isochinolinyle, 2-thiényle, 2-furyle (chacun éventuellement substitué par R^{§});
- restes amino d'amines primaires, R⁷NH2, (où R⁷ signifie la même chose que plus haut),
- amino, NH₂, avec la restriction que R⁵ n'a alors que la signification de OR⁶;
- hydrogène,
- hétérocyclyle, à de cinq à sept éléments, éventuellement substitué par R³,
- phényle, éventuellement substitué par R³,
- OR⁶, où R⁶ signifie la même chose que plus haut,
- NR⁷ R⁸, avec la même signification que plus haut
- azido, hydroxylamino, O-(C₁₋₃)alkylhydroxylamino,
- N-( C₁₋₃)alkylhydroxylamino, N,N-di(C₁₋₃)alkylhydroxylamino,
- hydrazino, (C₁₋₄)alkyl- et di(C₁₋₃)alkylhydrazino,
- benzylhydrazino, acylhydrazino, N,N-diacylhydrazino,
- carbamoylamino,
- 1-imidazolyl, 1,2,4-triazol-lyl, 1-pyridinium,
- 1-pyrazinium, 1-pyridazinium, y compris les représentants substitués alcoyle de ces azahétérocycles;
R* =O, =S, =N-H, = =N-Aryl, =N-OH, =N-O-C₁₋₈alcoyle,
=N-O- C₆₋₁₄aryle
R^{§} tel que défini plus haut (pour les formules 1a ou 1b)
les liaisons suivantes de la formule 1 (c) étant exclues de la protection:
1,4-dihydro-2,2-diméthyl-5-(4-morpholinyl)-10-propylthio-2H-pyrano[4",3":4',5']pyrido[3',2':4',5']thiéno[3,2-d]pyrimidin-8(9H)-one.
10-buthylthio-1,4-dihydro-2,2-diméthyl-5-(4-morpholinyl)-2H-pyrano[4",3":4',5']pyrido[3',2':4',5']thiéno[3,2-d]pyrimidin-8(9H)-one
et 2-méthyl-5-thiophène-2-yl-1,2,3,4-tétrahydro-11H-7-thia-2,6,9,11-tétra-aza-benzo[c]fluorène-8, 10-dione
ainsi que les sels, solvates et tautomères pharmaceutiquement acceptables de ces liaisons.

2. Liaison selon la revendication 1, à la formule générale 1(a)(I) ou 1(a)(II): 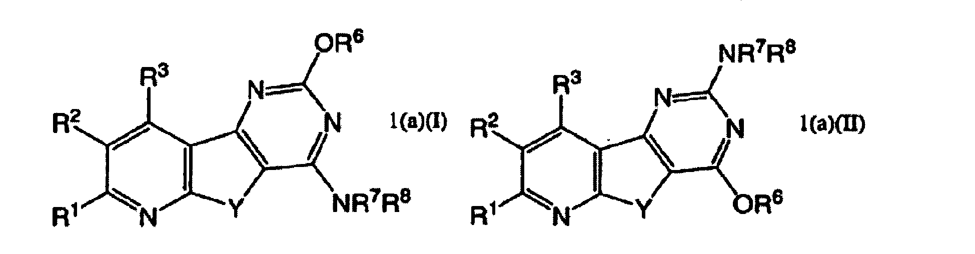 où R¹, R², R³, R⁶, R⁷, R⁸ et Y possèdent les significations précitées en ce qui concerne la formule 1(a).

3. Liaison selon la revendication 1, à la formule 1(a), Y = S et les substituants R¹, R², R³, R⁴, R⁵ ayant les significations suivantes:
| R₁ | R₂ | R₃ | R₄ | R₅ |
|---|---|---|---|---|
| 3,4-(MeO)₂-Ph | H | Me | piperazin-1-yl | OEt |
| 4-MeO-Ph | H | Me | piperazin-1-yl | OEt |
| 4-F-Ph | H | Me | piperazin-1-yl | OEt |
| Ph | H | Me | piperazin-1-yl | O/Pr |
| Ph | H | Me | piperazin-1-yl | OCH₂CHF₂ |
| Ph | H | Ph | piperazin-1-yl | OEt |
| Me | 4'-NO₂-Ph-CH₂ | Me | piperazin-1-yl | OEt |
| Me | 4'-NH2-Ph-CH₂ | Me | piperazin-1-yl | OEt |
| 3,4-méthylène-dioxyphényle | H | Me | piperazin-1-yl | OEt |
| Ph | H | Me | piperazin-1-yl | O*n*Pr |
| Ph | H | Me | piperazin-1-yl | OEt |
| Ph | H | Me | piperazin-1-yl | OCH₂CH₂OH |
| 3,4-(MeO)₂-Ph | H | Me | OEt | piperazin-1-yl |
| 4-MeO-Ph | H | Me | OEt | piperazin-1-yl |
| Ph | H | Me | OCH₂CHF₂ | piperazin-1-yl |
| Me | 4'-NO₂-Ph-CH₂ | Me | OEt | piperazin-1-yl |
| Ph | 4'-NO₂-Ph-CH₂ | Me | OEt | piperazin-1-yl |
| 3,4-(MeO)₂-Ph | H | Me | *n*Pr | piperazin-1-yl |
| Ph | H | Me | OCH₂CH₂OH | piperazin-1-yl |
| Ph | H | Me | OEt | piperazin-1-yl |
| Ph | H | Me | O/Pr | piperazin-1-yl |
| 3,4-méthylène-dioxyphényle | H | Me | OEt | piperazin-1-yl |
| 4-(pyrimidyl-2-yl)piperazin-1-yl)- | H | Et | piperazin-1-yl | OEt |
| | | | | |
|---|---|---|---|---|
| 4-Me-Ph | H | Et | piperazin-1-yl | OEt |
| 4-EtO-Ph | H | Et | piperazin-1-yl | OEt |
| 4-EtO-Ph | H | Et | piperazin-1-yl | OProp |
| 4-MeS-Ph | H | Et | piperazin-1-yl | OEt |
| 4-Me-Ph | H | Et | piperazin-1-yl | O/Pr |
| 4-(4-MeOPhO)Ph | H | Et | piperazin-1-yl | OEt |
| 2,3-dihydrobenzo [b][1,4]-dioxin-6-yl | H | Et | piperazin-1-yl | OEt |
| 3,4-méthylène-dioxyphényle | H | Et | piperazin-1-yl | OEt |
| 4-MeO-Ph | H | Et | piperazin-1-yl | OEt |
| 4-(1H-pyrazol-1-yl)-Ph | H | Et | piperazin-1-yl | OEt |
| 4-Br-Ph | H | Et | piperazin-1-yl | OEt |
| 4-Cl-Ph | H | Et | piperazin-1-yl | OEt |
| 4-1-Ph | H | Et | piperazin-1-yl | OEt |
| morpholine | H | Ph | piperazin-1-yl | OEt |
| 4-MeO-Ph | H | CF₃ | piperazin-1-yl | OEt |
| 4-Me-Ph | H | CF₃ | piperazin-1-yl | OEt |
| 2,3-dihydrobenzo [b][1,4]-dioxin-6-yl | H | CF₃ | piperazin-1-yl | OEt |
| 4-imidazol-1-yl-Ph | H | CF₃ | piperazin-1-yl | OEt |
| 4-Imidazol-1-yl-Ph | H | CF₃ | piperazin-1-yl | OCH₂CH₂OH |
| 4-(piperidin-1-yl)-Ph | H | CF₃ | iiperazin-1-yl | OEt |
| 4-morpholino-Ph | H | CF₃ | piperazin-1-yl | OEt |
| 3,4-méthylène-dioxyphényle | H | CF₃ | piperazin-1-yl | OEt |
| 4-MeS-Ph | H | CF₃ | piperazin-1-yl | OEt |
| 4-EtOOC-Ph | H | CF₃ | piperazin-1-yl | ÓÉt |
| | | | | |
|---|---|---|---|---|
| 4-(1H-pyrazol-1-yl)-Ph | H | CF₃ | piperazin-1-yl | OEt |
| 4-F-Ph | H | CF₃ | piperazin-1-yl | OEt |
| 4-I-Ph | H | CF₃ | piperazin-1-yl | OEt |
| 3-NO₂-Ph | H | CF₃ | piperazin-1-yl | OEt |
| 4-NO₂-Ph | H | CF₃ | píperazin-1-yl | OEt |
| 3-CN-Ph | H | CF₃ | piperazin-1-yl | OEt |
| 4-CN-Ph | H | CF₃ | piperazin-1-yl | OEt |
| 3-NH₂-Ph | H | CF₃ | piperazin-1-yl | OEt |
| 4-NHa-Ph | H | CF₃ | piperazin-1-yi | OEt |
| 4-MeS-Ph | H | CF₃ | piperazin-1-yl | OEt |
| 4-MeS(O)-Ph | H | CF₃ | piperazin-1-yl | OEt |
| pyridin-4-yl | H | CF₃ | piperazin-1-yl | OEt |
| pyridin-4-yl-N-oxyde | H | CF₃ | piperazin-1-yl | OEt |
| pyridin-3-yl | H | CF₃ | Piperazin-1-yl | OEt |
| 4-(pyrazin-2-yl) | H | CF₃ | piperazin-1-yl | OEt |
| 4(HCONH)-Ph | H | CF₃ | piperazin-1-yl | OEt |
| 4-(pyridin-4-yl-CONH).Ph | H | CF₃ | piperazin-1-yl | OEt |
| 4-(pyridin-3-yl-CONH)-Ph | H | CF₃ | piperazin-1-yl | OEt |
| 4-(pyridin-3-yl-N-Oxyde-CONH)-Ph | H | CF₃ | piperazin-1-yl | OEt |
| 4-(1H-1,2,4-triazol-4-yl)-Ph | H | CF₃ | piperazin-1-yl | OEt |
| 4-(1H-pyrrol-1-yl)-Ph | H | CF₃ | piperazin-1-yl | OEt |
| 4(CH₃OOCNH)-Ph | H | CF₃ | piperazin-1-yl | OEt |
| *iso*Pr | H | *iso*Pr | piperazin-1-yl | OEt |
| 2,3-dihydro-benzo [b][1,4]-dioxin-6-yl | H | Et | OEt | piperazin-1-yl |
| 4-EtO-Ph | H | Et | OEt | piperazin-1-yl |
| 4-Me-Ph | H | Et | OEt | piperazin-1-yl |
| 4-Me-Ph | H | Et | OCH₂CH₂OH | piperazin-1-yl |

4. Liaison selon la revendication 1, à la formule 1(a), Y = O et les substituants R¹, R², R³, R⁴, R⁵ ayant les significations suivantes:
| R₁ | R₂ | R₃ | R₄ | R₅ |
|---|---|---|---|---|
| Ph | H | Me | piperazin-1-yl | OEt |
| Ph | H | Me | OEt | piperazin-1-yl |

5. Liaison selon la revendication 1, à la formule 1(b), Y = S et les substituants R¹, R³, R⁴, R⁵ ayant les significations suivantes:
| R₁ | R₃ | R₄ | R₅ |
|---|---|---|---|
| 3-NH₂-Ph-4-Ph | Me | piperazin-1-yl | OEt |
| 4-(3-furyl)-Ph | Me | piperazin-1-yl | OEt |
| 4-(2-furyl)-Ph | Me | piperazin-1-yl | OEt |
| 4-(3-pyridyl)-Ph | Me | piperazin-1-yl | OEt |
| 4-(4-pyridyl)-Ph | Me | piperazin-1-yl | OEt |
| 4-NH₂-Ph-Ph | Me | piperazin-1-yl | OEt |
| 3,4,5-(CH₃0)₃-Ph-Ph | Me | piperazin-1-yl | OEt |
| 4-PhO-Ph | Me | piperazin-1-yl | OEt |
| 2-NH₂-4-CH₃OOC-Ph-Ph | Me | piperazin-1 -yl | OEt |
| 3-CH₃OOC-Ph-Ph | Me | piperazin-1-yl | OEt |
| 3,4-(CH₃O)₂-Ph-Ph | Me | piperazin-1-yl | OEt |
| 3,5-(CH₃)₂-isoxazol-4-yl-Ph | Me | piperazin-1-yl | OEt |
| Phényle | Me | piperazin-1-yl | OEt |
| 4-Br-Ph | Me | piperazin-1-yl | OEt |
| 3-NH₂-Ph | Me | piperazin-1-yl | OEt |
| 4-(pyrrol-3-yl)-Ph | Me | piperazin-1-yl | OEt |
| 4-(pyrimidin-5-yl)-Ph | Me | piperazin-1-yl | OEt |
| 4-(pyrid-3-yl-hydroxyméthyle)-Ph | Me | piperazin-1-yl | OEt |
| Ph-Ph | Me | piperazin-1-yl | OEt |
| 4-(*iso*PropO)-Ph | Me | piperazin-1-yl | OEt |
| 4-vinyle-Ph | Me | piperazin-1-yl | OEt |

6. Liaison selon la revendication 1, à la formule 1(a) ou 1(b), celle-ci étant
2-(2-hydroxyéthoxy)-9-méthyl-7-phényl-4-(piperazin-1-yl)-pyrido[3',2':4,5]thiéno [3,2-d]pyrimidine;
2-éthoxy-7-(3,4-diméthoxy-phényl)-9-méthyl-4-(piperazin-1-yl)-pyrido[3',2':4,5] thiéno [3,2-d]pyrimidine;
2-éthoxy-7-(4-méthoxy-phényl)-9-méthyl-4-(piperazin-1-yl)-pyrido[3',2':4,5]thiéno [3,2-d]pyrimidine;
2-éthoxy-9-éthyl-7-(4-méthyl-phényl)-4-(piperazin-1-yl)-pyrido[3',2':4,5]thiéno[3,2-d] pyrimidine;
2-éthoxy-7-(4-éthoxy-phényl)-9-éthyl-4-(piperazin-1-yl)-pyrido[3',2':4,5]thiéno[3,2-d] pyrimidine;
2-éthoxy-9-éthyl-7-(2,3-dihydrobenzo[b][1,4]díoxin-6-yl)-4-(piperazin-1-yl)-pyrido [3',2':4,5]thiéno[3,2-d] pyrimidine;
4-éthoxy-9-trifluorméthyl-7-(pyrazin-2-yl)-2-piperazino-pyrido[3',2':4,5]thiéno[3,2-d] pyrimidine;
4-éthoxy-9-éthyI-7-(4-(pyrimidyl-2-yl)piperazin-1-yl)-2-piperazin-1-yl-pyrido [3',2':4,5]thiénol[3,2-d]pyrimidine:
4-éthoxy-7-morpholino-9-phényl-2-(piperazin-1-yl)-pyrido[3',2':4,5]thiéno[3,2-d] pyrimidine;
4-éthoxy-7-phényl-9-méthyl-2-(piperazin-1-yl)-[3',2':4,5]furo[3,2-d]pyrimidine;
4-éthoxy-7,9-diméthyl-8-(4-nitro-benzyl)-2-(piperazin-1-yl)-pyrido[3',2':4,5]thiéno [3,2-d]pyrimidine;
4-éthoxy-7-(3,4-diméthoxy-phényl)-9-méthyl-2-(piperazin-1-yl)-pyrido[3',2':4,5] thiéno[3,2-d]pyrimidine;
4-éthoxy-9-éthyl-7-(4-méthyl-phényl)-2-(piperazin-1-yl)-pyrido[3',2':4,5]thiéno[3,2-d] pyrimidine;
4-éthoxy-7-(4-fluorphényl)-9-méthyl-2-(piperazin-1-yl)-pyrido[3',2':4,5]furo[3,2-d] pyrimidine;
7-(4-éthoxy-phényl)-9-éthyl-2-(piperazin-1-yl)-4-*n*-propoxy-pyrido[3',2':4,5]thiéno [3,2-d]pyrimidine;
4-éthoxy-7-(4-éthoxy-phényl)-9-éthyl-2-(piperazin-1-yl)-pyrido[3',2':4,5]furo[3,2-d] pyrimidine;
4-éthoxy-7-(4-(pyrimidin-2-yl)piperazin-1-yl)-9-éthyl-2-(piperazin-1-yl)-pyrido [3',2':4,5]thiéno[3,2-d]pyrimidine;
4-éthoxy-9-éthyl-7-(4-méthylthio-phényl)-2-(piperazin-1-yl)-pyrido[3',2':4,5]thiéno [3,2-d]pyrimidine;
4-éthoxy-7-(4-méthyl-phényl)-9-iso-propyl-2-(piperazin-1-yl)-pyrido[3',2':4,5]thiéno [3,2-d]pyrimidine;
4-éthoxy-9-éthyl-7'[4;(4-méthoxyphénoxy)phényl]-2-(piperazin-1-yl)-pyrido[3',2':4,5] thiéno[3,2-d]pyrimidine;
4-éthoxy-9-éthyl-7-(2,3-dihydrobenzo[b][1,4]dioxin-6-yl)-2-(piperazin-1-yl)-pyrido [3',2':4,5]thiéno[3,2-d]pyrimidine;
4-éthoxy-9-éthyl-7-(3,4-méthylènedioxyphényl)-2-piperazin-1-yl-pyrido[3',2':4,5] thiéno[3,2-d]pyrimidine;
4-éthoxy-7-(4-méthoxy-phényl)-2-(piperazin-1-yl)-9-trifluormétihyl-pyrido[3',2':4,5] thiéno[3,2-d]pyrimidine;
4-éthoxy-7-(4-méthyl-phényl)-2-(piperazin-1-yl)-9-trifluorméthyl-pyrido[3',2':4,5] thiéno[3,2-d]pyrimidine;
7-(2,3-dihydrobenzo[b][1,4]díoxin-6-yl)-4-éthoxy-2-(piperazin-1-yl)-9-trifluorméthyl-pyrido[3',2':4,5]thiéno[3,2-d]pyrimidine;
4-éthoxy-7-(4-(1H-imidazol-1-yl)phényl)-2-(piperazin-1-yl)-9-trifluorméthyl-pyrido [3',2':4,5]thiéno[3,2-d]pyrimidine;
benzoate d'éthyle de 4-[4-éthoxy-2-(piperazin-1-yl)-9-trifluorméthyl-pyrido[3',2':4,5] thiéno[3,2-d]pyrimidin-7-yl;
4-éthoxy-2-(piperazin-1-yl)-7-(4-(piperidin-1-yl)phényl)-9-trifluorméthyl-pyrido [3',2':4,5]thiéno[3,2-d]pyrimidine;
4-éthoxy-9-éthyl-7-(4-méthoxyphényl)-2-(piperazin-1-yl)-pyrido[3',2':4,5]thiéno[3,2-d] pyrimidine;
4-éthoxy-7-(3,4-méthylènedioxyphényl)-2-piperazin-1-yl-9-trifluorméthyl-pyrido [3',2':4,5]thiéno[3,2-d]pyrimidine;
4-éthoxy-7-(4-méthylthiophényl)-2-(piperazin-1-yl)-9-trifluorméthyl-pyrido[3',2':4,5] thiéno[3,2-d]pyrimidine;
4-éthoxy-7-(4-(1H-pyrazol-1-yl)-phényl)-9-éthyl-2-piperazin-1-yl-pyrido[3',2':4,5] thiéno[3,2-d]pyrimidine;
4-éthoxy-7-(4-iode-phényl)-2-(piperazin-1-yl)-9-trifluorméthyl-pyrido[3',2':4,5]thiéno [3,2-d]pyrimidine;
4-éthoxy-7-(4-(1H-pyrazol-1-yl)-phényl)-2-piperazin-1-yl-9-trifluorméthyl-pyrido [3',2:4,5]thiéno[3,2-d]pyrimidine;
7-(4-brome-phényl)-4-éthoxy-9-éthyl-2-(piperazin-1-yl)-pyrido[3',2:4,5]thiéno[3,2-d] pyrimidine;
4-éthoxy-9-éthyl-7-(4-iode-phényl)-2-(piperazin-1-yl)-pyrido[3',2':4,5]thiéno[3,2-d] pyrimidine;
4-éthoxy-7-(3-nitro-phényl)-2-piperazin-1-yl-9-trifluorméthyl-pyrido[3',2':4,5]thiéno [3,2-d]pyrimidine;
7-(3-cyano-phényl)-4-éthoxy-2-piperazin-1-yl-9-trifluorméthyl-pyrido[3',2':4,5]thiéno [3,2-d]pyrimidine;
7-(4-cyano-phényl)-4-éthoxy-2-piperazin-1-yl-9-trifluorméthyl-pyrido[3',2':4,5]thiéno [3,2-d]pyrimidine;
7-(3-amino-phényl)-4-éthoxy-2-piperazin-1-yl-9-trifluorméthyl-pyrido[3',2':4,5]thiéno [3,2-d]pyrimidine;
7-{4-amino-phényl)-4-éthoxy-2-piperazin-1-yl-9-trifluorméthyl-pyrido[3',2':4,5]thiéno [3,2-d]pyrimidine;
4-éthoxy-9-méthyl-7-(4-méthylsulfinyl-phényl)-2-(piperazin-1-yl)-pyrido[3',2':4,5] thiéno[3,2-d]pyrimidine;
formamide de N-(4-(4-éthoxy-9-trifluorméthyl-2-piperazin-1-yl-pyrido[3',2':4,5]thiéno [3,2-d]pyrimidin-7-yl)-phényle);
isonicotinamide de N-(4-(4-éthoxy-9-trifluorméthyl-2-piperazin-1-yl-pyrido[3',2':4,5] thiéno[3,2-d]pyrimidin-7-yl)-phényle);
4-éthoxy-9-trifluorméthyl-2-piperazin-1-yl-7-(4-(1H-pyrrol-1-yl)-phényl)-pyrido [3',2':4.5]thiéno[3,2-d]pyrimidine;
nicotinamid-N-oxyde de N-(4-(4-éthoxy-9-trifluorméthyl-2-piperazin-1-yl-pyrido [3',2':4,5]thiéno[3,2-d]pyrimidin-7-yl)-phényle);
carbamate de méthyle de 4-(4-éthoxy-9-trifluorméthyl-2-piperazin-1-yl-pyrido [3',2':4,5]thiéno[3,2-d]pyrimidin-7-yl)-phényle;
nicotinamide de N-(4-(4-éthoxy-9-trifluorméthyl-2-piperazin-1-yl-pyrido[3',2':4,5] thiéno[3,2-d] pyrimidin-7-yl)-phényle);
trifluoracétate de N-(4-éthoxy-9-trifluorméthyl-2-piperazin-1-yl-pyrido[3',2':4,5]thiéno [3,2-d]pyrimidin-7-yl)-benzamide;
bistrifluoracétate de N'-hydroxy-4-(4-éthoxy-9-trifluorméthyl-2-piperazin-1-yl-pyrido [3',2':4,5]thiéno[3,2-d]pyrimidin-7-yl)-benzamidine;
7-(3'-aminobiphène-4-yl)-4-éthoxy-9-méthyl-2-piperazin-1-yl-thiéno[2,3-d:4,5-d'] dipyrimidine;
4-éthoxy-7-(4-[3-furyl]-phényl)-9-méthyl-2-piperazin-1-yl-thiéno[2,3-d:4,5-d'] dipyrimidine:
4-éthoxy-7-(4-[2-furyl]-phényl)-9-méthyl-2-piperazin-1-yl-thiéno(2,3-d:4.5-d'] dipyrimidine;
4-éthoxy-9-méthyl-2-piperazin-1-yl-7-(4-[3-pyridyl-phényl)-thiéno[2,3-d:4,5-d'] dipyrimidine;
4-éthoxy-9-méthyl-2-piperazin-1-yl-7-(4-[4-pyridyl]-phényl)-thiéno[2,3-d:4.5-d'] dipyrimidine;
7-(4'-aminobiphène-4-yl)-4-éthoxy-9-méthyl-2-piperazin-1-yl-thiéno[2,3-d:4,5-d'] dipyrimidine;
4-éthoxy-9-méthyl-2-piperazin-1-yl-7-(3',4',5'-triméthoxybiphène-4-yl)-thiéno [2,3-d:4,5-d']dipyrimidine;
4-éthoxy-9-méthyl-7-(4-phénoxy-phényl)-2-piperazin-1-yl-thiéno[2,3-d:4,5-d'] dipyrimidine;
4'-carbonate de méthyle de 2'-amino-4-(4-éthoxy-9-méthyl-2-piperazin-1-yl-thiéno [2,3-d:4,5-d']dipyrimidin-7-yl)-biphényle;
7-(3',4'-diméthoxybiphène-4-yl)-4-éthoxy-9-méthyl-2-piperazin-1-yl-thiéno [2,3-d:4,5-d']dipyrimidine.

7. Liaison selon la revendication 1 à la formule 1(c) (I) ou 1(c) (II): 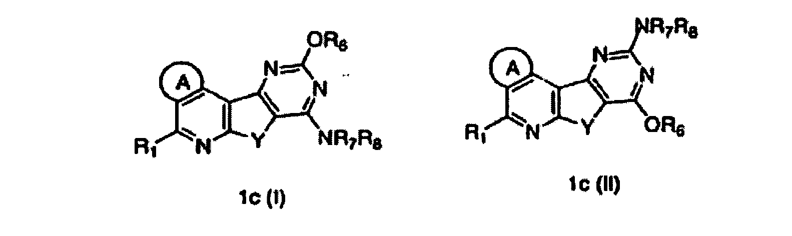 où R¹, R⁶, R⁷, R⁸ ainsi que A et Y possèdent les significations précitées en ce qui concerne la formule 1(c).

8. Liaison selon la revendication 1 à la formule 1(c) ou 1(d), celle-ci étant choisie parmi le groupe composé de:
10-éthoxy-5-morpholino-8-piperazin-1-yl-pyrimido[4',5':4,5]thiéno[2,3-c]-1,2,3,4-tétrahydro-isochinoline
7-éthoxy-2,3-dihydro-4-morpholino-9-piperazin-1yl-1H-cyclopenta[4',5']pyrido [3',2':4,5]thiéno[3,2-d]pyrimidine
hydrochlorure de 8-éthoxy-piperidino-10-piperazin-1yl-pyrimido[4',5':4,5]thiéno [2,3-c]-1,2,3,4-tétrahydro-isochinoline.
hydrochlorure de 8-éthoxy-5-morpholino-10-piperazin-1-yl-pyrimido[4',5':4,5]thiéno [2,3-c]-1,2,3,4-tétrahydro-isochinoline.
hydrochlorure de 8-éthoxy-5-phényl-10-piperazin-1-yl-pyrimido[4',5':4,5]thiéno [2,3-c]-1 ,2,3,4-tétrahydro-isochinoline
10-éthoxy-5-phényl-8-piperazin-1-yl-pyrimido[4',5':4,5]thiéno[2,3-c]-1,2,3,4-tétrahydro-isochinoline
8-éthoxy-5-morpholino-10-piperazin-1-yl-pyrimido[4',5':4,5]thiéno[2,3-c]-1,2,3,4-tétrahydro-isochinoline
7-éthoxy-2,3,4,5-tétrahydro-4-morpholino-9-piperazin-1yl-1H-cyclohepta[4',5']pyrido [3',2':4,5]thiéno[3,2-d]pyrimidine

9. Composition pharmaceutique contenant au moins l'une des liaisons selon l'une des revendications 1 à 8 ainsi qu'éventuellement des substances auxiliaires et/ou porteurs acceptables pharmaceutiquement.

10. Composition pharmaceutique selon la revendication 9, contenant en outre une ou plusieurs des susbtances actives suivantes:
- agonistes de l'adrénocepteur β₂
- cromoglycate de disodium
- corticostéroïdes
- antagonistes de leucotriènes (soit inhibiteurs d'enzymes [tels qu'inhibiteurs de 5-lipoxygénase ou inhibiteurs d'enzymes de l'acide arachidonique] ou antagonistes de récepteurs),
- antihistaminiques (de préférence ceux à propriétés de stabilisation de mastocytes ou aspects antagonisants des leucotriènes)
- théophylline
- antagonistes de récepteurs muscariniques
- anticorps (monoclonaux) contre TNF alpha ou d'autres substances actives inhibant la formation ou la libération de TNF alpha ou l'activité de TNF alpha.

11. Utilisation d'au moins l'une des liaisons selon l'une des revendications 1 à 8 pour la préparation d'un médicament pour inhiber l'enzyme phosphodiestérase 4 (PDE4) et/ou la libération de TNF alpha.

12. Utilisation selon la revendication 11 pour le traitement de l'asthme bronchial, BPCO, arthrite rheumatoïde (RA), ostéoarthrite, sclérose multiple, syndrome de Guillain-Barré, maladie de Crohn, colite ulcéreuse, psoriasis, dermatite atopique, eczémas allergiques, rhinite allergique, conjonctivite allergique, sclérodermie systémique, « graft versus host disease » (GvHO) (= greffe contre hôte), lupus érythémateux systémique (SLE), diabète sucré type I, maladies neurodégénératives, en particulier la maladie d'Alzheimer et la maladie de Parkinson, déficiences multi-organiques post-traumatiques, syndrome de choc toxique, glomérulonéphrite aigue, douleurs aigues et chroniques, artériosclérose, infarctus du myocarde, apoplexie, maladies tumorales, génération axonale, maladies virales.

13. Utilisation selon la revendication 12, la maladie tumorale étant la leucémie.

14. Utilisation selon la revendication 12, la maladie tumorale étant la LLC.
